# EUROPEAN PATENT APPLICATION

(11) **EP 4 039 094 A1**
(43) Date of publication of application: **10.08.2022**
(21) Application number: 21154940.7
(22) Date of filing: 03.02.2021
(51) Int. Cl.: A01N 43/40, A01N 43/48, A01N 43/54, C07D 239/72, C07D 471/04

(54) **NOVEL ANTIFUNGAL COMPOUNDS**

(71) Applicant: Max-Planck-Gesellschaft zur Förderung der Wissenschaften e.V., 80539 München (DE)
(72) Inventor: BISCHOFF, Matthias, 44227 Dortmund (DE); LAMPE, Philipp, 58454 Witten-Stockum (DE); SIEVERS, Sonja, 44143 Dortmund (DE); POLYCHRONIDOU, Vasiliki, 44221 Dortmund (DE); ANTONCHICK, Andrey, 44225 Dortmund (DE); THOMA, Annika, 10247 Berlin (DE); LUDWIG, Nicole, 53123 Bonn (DE); KAHMANN, Regine, 35037 Marburg (DE); MÜNCH, Karin, 35083 Wetter (DE); REIßMANN, Stefanie, 35037 Marburg (DE)
(74) Representative: Arth, Hans-Lothar

(57) **Abstract**

The present invention relates to a compound or a combination of compounds which can be used as anti-fungal agents directed against plant pathogenic basidiomycete fungi , and especially for inhibiting the virulence of corn smut fungus *Ustilago maydis* and related smut fungi, and of rust fungus *Uromyces fabae* and related rust fungi , as well as compositions comprising at least one of those compounds and/or agriculturally acceptable salts thereof as agriculturally active ingredients.

SEQUENCE LISTING

<110>  Max-Planck-Gesellschaft zur Foerderung der Wissenschaften

<120>  Novel antifungal compounds

<130>  GAR-P04311EP

<160>  77

<170>  PatentIn version 3.5

<210>  1
<211>  541
<212>  PRT
<213>  Melanopsichium pennsylvanicum

<400>  1

Met Arg Ala Leu Leu Ser Leu Asn Met Thr Lys Leu Leu Ala Leu Leu
1               5                   10                  15

Leu Val Leu Met Pro Phe Leu Leu Val Ala Asp Gly His Gln Tyr Ile
            20                  25                  30

Asp Asn Met Pro His Gln Ile Arg Met Val Arg His Arg Arg Ile Glu
        35                  40                  45

Ser Arg Ser Phe Met Ser Trp Met Thr His Ile Ser Asp Lys Ala Ser
    50                  55                  60

Asp Trp Met Phe Gly Ser Val His Asn Pro Ala Asn Asp Glu Ser His
65                  70                  75                  80

Ile Pro Lys Pro Ala Val Gly Gly Val Asp Val Leu Pro Lys Met Pro
                85                  90                  95

Tyr Pro Tyr Ala Thr Gly Met Ile Pro Arg Leu Ala Ser Glu Thr Leu
            100                 105                 110

Ser Gly Asn Gly Leu Val Gly Gly Trp Met Cys Ile His Ile Gln Pro
        115                 120                 125

Pro Gly Asn Ala Gln Ala Ser Leu Thr Arg Glu Gly Gly Ala Ser Gly
    130                 135                 140

Gly Gly Ser Gly Ala Gly Gly Gly Ala Gly Leu Gly Ala Gly Gly Gly
145                 150                 155                 160

Ala Gly Leu Gly Ala Gly Ala Gly Ala Gly Leu Gly Ala Gly Gly Gly
                165                 170                 175

Ala Gly Leu Gly Ala Gly Gly Gly Ala Gly Leu Gly Ala Gly Gly Gly
        180                 185                 190

Ala Gly Leu Gly Ala Gly Gly Gly Ala Gly Leu Gly Ala Gly Ala Gly
    195                 200                 205

Ala Gly Leu Gly Ala Gly Gly Ala Gly Gly Gly Ala Gly Leu Gly Ala
    210                 215                 220

Gly Gly Gly Ala Gly Leu Gly Ala Gly Gly Gly Ala Gly Leu Gly Ala
225                 230                 235                 240

Gly Gly Gly Ala Gly Leu Gly Ala Gly Gly Gly Ala Gly Leu Gly Ala
                245                 250                 255

Gly Gly Gly Ala Gly Leu Gly Ala Gly Gly Gly Ala Gly Leu Gly Ala
            260                 265                 270

Gly Gly Gly Ala Gly Leu Gly Gly Gly Thr Gly Ser Pro Tyr Leu Gly
        275                 280                 285

Tyr Gly Gly Tyr Tyr Gly Ser Gly Gly Gly Ala Gly Gly Met Asn Ser
    290                 295                 300

Gly Thr Gly Met Gly Arg Gly Gly Gly Ser Ser Gly Tyr Ser Gly Gly
305                 310                 315                 320

Asn Gly Leu Leu Ser Gly Ser Gly Ser Met Gly Gly Gly Ser Gly Gly
                325                 330                 335

Asn Tyr Met Ser Gly Ser Gly Ile Gly Gly Gly Asn Gly Tyr Ser Arg
            340                 345                 350

Ser Gly Phe Gly Thr Gly Ser Val Gly Asn Gly Val Gly Thr Gly Asp
        355                 360                 365

Phe Gly Ala Gly Gly Ser Gln Gly Gly Ser Ile Pro Pro Gly Tyr Ile
    370                 375                 380

Arg Tyr Gln Gly Gln Val Met Thr Ile Ala Gln Phe Gln Gln Ala Leu
385                 390                 395                 400

Ala Ser Gly Ala Pro Ser Gly Gly Ser Ser Ser Pro Tyr Val Arg Arg
                405                 410                 415

Arg Ser Leu Phe Ser Ser Asp Ser Ser Ser Leu Ser Thr Asn Thr Phe
            420                 425                 430

Leu Asp Lys Arg Gln Ala Pro Asp Ser Ala Pro Pro Ala Leu Asp Ser
        435                 440                 445

Ala Pro Pro Ala Asp Lys Lys Pro Ala Lys Ile Phe Met Cys Ile Asn
    450                 455                 460

Gly Asp Pro Asp Val Pro Pro Ile Lys Phe Asn Thr Met Thr Gly Leu
465                 470                 475                 480

Lys Ile Arg Asp Pro Glu Ala Gln Gln Asp Gly Val Val Glu Ile Thr
                485                 490                 495

Asn Leu Pro Asn Tyr Gln Met Pro Trp Met Pro Phe Leu Pro Thr Ala
            500                 505                 510

Glu Met Trp Glu Glu Gln Gln Thr Leu Met Leu Ala Gln Glu Glu Met
        515                 520                 525

Ile Glu Thr Asn Met Thr Thr Thr Pro Pro Arg Ser Arg
    530                 535                 540

<210>  2
<211>  462
<212>  PRT
<213>  Sporisorium scitamineum

<400>  2

Met Arg Ala Lys Leu Ser Leu Asn Met Thr Lys Leu Leu Ala Leu Leu
1               5                   10                  15

Leu Val Leu Met Pro Ile Leu Leu Ala Val Glu Ala Gln Ser Gly Lys
            20                  25                  30

Leu His His Gln Arg Pro Arg Met Met Arg Arg Arg Ile Glu Pro Arg
        35                  40                  45

Ser Val Ala Ser Trp Leu Thr Lys Ile Ser Ser Lys Ala Ser Asp Trp
    50                  55                  60

Val Phe Gly Ser Val Pro Asn Pro Val Thr Asp Pro Lys Asn Ile Pro
65                  70                  75                  80

Lys Pro Gln Val Gly Gly Val Ala Val Leu Pro Gln Met Leu Tyr Pro
                85                  90                  95

Tyr Ala Thr Gly Met Val Pro Arg Leu Ala Ser Glu Thr Leu Ser Gly
            100                 105                 110

Asn Gly Leu Pro Gly Gly Trp Met Cys Met His Ile Thr Pro Pro Ala
        115                 120                 125

Glu Glu Asp Asp Asp Ala Gly Gly Leu Gly Gly Gly Leu Gly Gly Gly
    130                 135                 140

Ala Gly Leu Gly Ala Gly Gly Met Gly Gly Glu Glu Trp Val Leu Ala
145                 150                 155                 160

Glu Trp Ala Gly Glu Glu Trp Val Leu Ala Glu Trp Ala Gly Glu Glu
                165                 170                 175

Trp Val Leu Ala Glu Trp Ala Gly Glu Glu Trp Val Leu Ala Glu Trp
            180                 185                 190

Ala Gly Arg Gly Gly Met Gly Gly Gly Gly Met Gly Thr Gly Gly Met
        195                 200                 205

Gly Gly Gly Gly Met Gly Thr Gly Gly Met Gly Val Gly Gly Gly Leu
    210                 215                 220

Ala Gly Gly Gln Gln Asn Gly Ala Ala Gly Gly Phe Ala Gly Gly Ala
225                 230                 235                 240

Gly Thr Gly Phe Gly Val Gly Ala Gly Ala Gly Tyr Ala Gly Ala Gly
                245                 250                 255

Gly Gln Gln Gly Phe Gly Ala Gly Gly Gln Gln Gly Phe Gly Ala Gly
            260                 265                 270

Gly Gln Gln Gly Phe Gly Ala Gly Val Gly Gly Gly Met Gly Thr Gly
        275                 280                 285

Gly Leu Gly Val Gly Ala Gly Ala Gly Thr Gly Ser Ala Ala Val Pro
    290                 295                 300

Ala Gly Tyr Ile Arg Tyr Asn Gly Gln Leu Met Thr Ile Gly Gln Tyr
305                 310                 315                 320

Gln Gln Ala Leu Ala Ala Ser Ser Gly Thr Pro Ser Ser Leu Ser Arg
                325                 330                 335

Arg Asp Val Tyr Gln Ser Asp Thr Ser Ala Phe Leu Asn Thr Phe Met
            340                 345                 350

His Lys Arg Gln Ser Ser Asp Ala Pro Ala Pro Gln Pro Ala Asp Asp
        355                 360                 365

Gly Asp Ala Ala Pro Lys Lys Lys Pro Ala Lys Val Leu Met Cys Ile
    370                 375                 380

Asn Gly Asp Pro Asp Val Pro Pro Ile Lys Phe Asp Thr Met Thr Gly
385                 390                 395                 400

Leu Arg Ile Arg Asp Pro Gln Ala Gln Lys Asp Gly Val Val Glu Ile
                405                 410                 415

Thr Asn Leu Pro Asn Tyr Gln Met Pro Trp Met Ala Phe Leu Pro Thr
            420                 425                 430

Val Asp Met Trp Asn Glu Gln Gln Ala Leu Met Ile Ala Gln Glu Lys
        435                 440                 445

Val Ile Glu Ile Asn Met Thr Thr Ala Pro Pro Pro Ala Arg
    450                 455                 460

<210>  3
<211>  340
<212>  PRT
<213>  Ustilago hordei (host: barley)

<400>  3

Met Arg Ala Asp Phe Leu Ser Leu Asn Met Ala Lys Leu Leu Ala Leu
1               5                   10                  15

Leu Leu Val Ile Met Pro Phe Leu Leu Ala Ile Glu Ala His Gly Ser
            20                  25                  30

His Pro Asp Thr Leu Ser Met His Leu Arg Met Thr Lys Gln Arg Arg
        35                  40                  45

Ile Glu Ser Arg Ser Phe Met Ser Trp Leu Thr Arg Ile Ser Ser Lys
    50                  55                  60

Ala Ser Asp Trp Ile Phe Gly Asp Val Ser Ala Pro Lys Lys Asp Glu
65                  70                  75                  80

Ser Leu Ile Pro Lys Pro Val Val Gly Gly Val Pro Val Leu Pro Lys
                85                  90                  95

Met Pro Tyr Pro Tyr Ala Ser Gly Met Val Pro Arg Leu Ala Ser Glu
            100                 105                 110

Thr Leu Asn Gly Asn Gly Leu Val Gly Gly Trp Ile Cys Met His Val
        115                 120                 125

Thr Pro Pro Thr Asp Asp Ser Ala Gly Ala Gly Ala Gly Ala Gly Ala
    130                 135                 140

Gly Ala Gly Gly Ala Asp Gly Ser Ala Gly Ala Ser Gly Thr Ser Val
145                 150                 155                 160

Gly Gly Phe Asn Asp Arg Gly Ala Gly Gly Ser Gly Ala Gly Gly Ser
                165                 170                 175

Tyr Asp Gly Ala Ala Arg Gly Phe Thr Gly Gly Ala Gly Ala Asn Pro
            180                 185                 190

Ala Phe Gly Gly Gln Gln Gly Leu Met Thr Ile Glu Gln Tyr Gln Gln
        195                 200                 205

Ala Ile Ala Ser Thr Gly Ser Ser Ser Pro Ser Leu Ser Arg Arg Glu
    210                 215                 220

Leu Leu Gln Ala Glu Thr Ala Asn Ile Met Val Asp Ser Leu Leu His
225                 230                 235                 240

Lys Arg Gln Ser Phe Asp Thr Ala Pro Ala Ala Pro Lys Lys Gln Ala
                245                 250                 255

Lys Val Leu Met Cys Ile Asn Gly Asp Pro Ala Ile Pro Pro Ile Lys
            260                 265                 270

Phe Asn Thr Met Thr Gly Leu Arg Ile Arg Asp Ala Ser Glu Lys Lys
        275                 280                 285

Asp Gly Val Val Ala Ile Thr Asn Leu Pro Asn Tyr Gln Met Pro Trp
    290                 295                 300

Met Pro Phe Leu Pro Thr Glu Glu Met Trp Ala Glu Gln Gln Thr Leu
305                 310                 315                 320

Met Leu Glu Gln Glu Lys Met Ile Glu Gln Asn Met Thr Thr Ala Pro
                325                 330                 335

Pro Pro Lys Gln
            340

<210>  4
<211>  509
<212>  PRT
<213>  Ustilago bromivira

<400>  4

Met Arg Ala Asp Phe Leu Ser Leu Asn Met Ala Lys Leu Leu Ala Leu
1               5                   10                  15

Leu Leu Val Ile Met Pro Phe Leu Leu Ala Ile Glu Ala His Gly Ser
            20                  25                  30

His Pro Asp Thr Pro Ser Ile His Phe Arg Met Thr Lys Gln Arg Arg
        35                  40                  45

Ile Glu Ser Arg Ser Phe Met Ser Trp Leu Thr Arg Ile Ser Ser Lys
    50                  55                  60

Ala Ser Asp Trp Ile Phe Gly Asp Val Ser Ala Pro Lys Lys Asp Glu
65                  70                  75                  80

Ser Leu Ile Pro Lys Pro Ala Val Gly Gly Val Pro Val Leu Pro Lys
                85                  90                  95

Met Pro Tyr Pro Tyr Ala Ser Gly Met Val Pro Arg Leu Ala Ser Glu
            100                 105                 110

Thr Leu Asn Gly Asn Gly Leu Val Gly Gly Trp Met Cys Met His Val
        115                 120                 125

Thr Pro Pro Thr Asp Asp Ser Ala Gly Ala Gly Ala Ser Ala Gly Ala
    130                 135                 140

Gly Gly Ala Asp Gly Ser Ala Gly Ala Ser Gly Thr Ser Val Gly Gly
145                 150                 155                 160

Phe Asn Asn Leu Gly Ala Gly Gly Ser Gly Ala Ser Val Gly Ser Gly
                165                 170                 175

Thr Gly Phe Lys Gly Ala Gly Gly Ser Tyr Asp Gly Ala Ala Gly Gly
            180                 185                 190

Phe Thr Gly Gly Ala Gly Ala Asn Pro Ala Phe Gly Gly Gln Gln Gly
        195                 200                 205

Val Ile Pro Ala Thr Gly Gly Tyr Tyr Gly Gly Ala Ala Gly Gly Ala
    210                 215                 220

Gly Ala Gly Gly Gln Gln Ala Thr Gly Tyr Tyr Asn Gly Gly Gly Ala
225                 230                 235                 240

Gly Gly Ala Gly Val Gly Gly Gln Gln Ala Thr Gly Gly Tyr Tyr Asn

245                 250                 255

Gly Gly Gly Ala Gly Gly Ala Gly Val Gly Gly Gln Gln Ala Thr Gly
            260                 265                 270

Tyr Tyr Asn Gly Gly Gly Ala Gly Gly Ala Gly Val Gly Gly Gln Gln
        275                 280                 285

Ala Thr Gly Tyr Tyr Asn Gly Gly Gly Ala Gly Gly Ala Gly Val Gly
    290                 295                 300

Gly Gln Gln Ala Thr Gly Gly Tyr Tyr Asn Gly Gly Ala Gly Val Gly
305                 310                 315                 320

Gly Gly Asp Gly Gly Gly Met Met Gly Gly Gly Gly Ala Leu Asn Gly
                325                 330                 335

Ala Gly Gly Ala Pro Val Tyr Asn Gly Ala Gly Val Ser Gln Gln Gly
            340                 345                 350

Phe Gly Gly Gly Thr Val Pro Ala Gly Lys Ile Arg Tyr Asn Gly Gln
        355                 360                 365

Leu Met Thr Ile Glu Gln Tyr Gln Gln Ala Ile Ala Ser Ser Gly Ser
    370                 375                 380

Ser Ser Pro Ser Leu Ser Arg Arg Glu Leu Leu Gln Ala Lys Thr Ala
385                 390                 395                 400

Asn Ile Met Val Asp Ser Leu Leu His Lys Arg Gln Ser Phe Asp Thr
                405                 410                 415

Ala Pro Ala Ala Pro Lys Lys Gln Ala Lys Val Leu Met Cys Ile Asn
            420                 425                 430

Gly Asp Ser Ala Val Pro Pro Ile Lys Phe Asn Thr Ile Thr Gly Leu
        435                 440                 445

Arg Ile Arg Asp Ala Ser Glu Lys Lys Asp Gly Val Val Ala Ile Thr
    450                 455                 460

Asn Leu Pro Asn Tyr Gln Met Pro Trp Met Pro Phe Leu Pro Thr Glu
465                 470                 475                 480

Glu Met Trp Ala Glu Gln Gln Thr Leu Met Leu Ala Gln Glu Lys Met
                485                 490                 495

Ile Glu Gln Asn Met Thr Thr Ala Pro Pro Pro Lys Gln
            500                 505

<210> 5
<211> 544
<212> PRT
<213> Sporisorium reilianum f. sp. zeae

<400> 5

Met Arg Ala Thr Leu Ser Leu Asn Met Thr Lys Leu Leu Thr Val Leu
1               5                   10                  15

Leu Val Leu Leu Pro Ile Leu Leu Ala Val Glu Ala Ser Gln Ser Gly
            20                  25                  30

Ala Leu His His Arg Tyr Pro Arg Met Met Arg Gln Arg Arg Ile Glu
        35                  40                  45

Gln Arg Ser Val Ala Ser Trp Leu Thr Lys Ile Ser Ser Lys Val Ser
    50                  55                  60

Asp Trp Val Phe Gly Ser Val Pro Asn Pro Val Thr Asp Pro Lys Asn
65                  70                  75                  80

Ile Pro Lys Ser Gln Val Gly Gly Val Ala Val Leu Pro Gln Met Pro
                85                  90                  95

Tyr Pro Tyr Ala Thr Gly Met Ile Pro Arg Leu Ala Thr Glu Thr Leu
            100                 105                 110

Ser Gly Asn Gly Leu Pro Gly Ala Trp Met Cys Ile His Val Thr Pro
        115                 120                 125

Pro Ala Glu Lys Asp Asp Asp Asp Asp Gly Gly Ala Gly Ala Gly Ala
    130                 135                 140

Gly Ala Gly Ala Gly Ala Gly Ala Gly Met Gly Ala Gly Ala Gly Ala
145                 150                 155                 160

Gly Ala Gly Ala Gly Gly Ala Gly Gly Leu Gly Gly Gly Leu Gly Gly
                165                 170                 175

Gly Leu Gly Gly Gly Leu Gly Gly Gly Leu Gly Gly Gly Val Gly Gly
            180                 185                 190

Gly Tyr Ala Gly Gly Gly Tyr Ala Gly Ser Ala Gly Ser Gly Tyr Ala
        195                 200                 205

Gly Ser Ala Gly Ser Gly Tyr Ala Gly Ser Ala Gly Gly Gly Tyr Ala
    210                 215                 220

Gly Ser Thr Gly Gly Gly Tyr Ala Gly Ser Thr Gly Gly Gly Tyr Ala
225                 230                 235                 240

Gly Ser Thr Gly Gly Gly Tyr Ala Gly Ser Thr Gly Gly Gly Tyr Ala
                245                 250                 255

Gly Ser Thr Gly Gly Gly Tyr Ala Gly Ser Thr Gly Gly Gly Tyr Ala
            260                 265                 270

Gly Asn Gly Leu Gly Tyr Gly Gly Ala Ala Gly Gly Gly Tyr Ala Gly
        275                 280                 285

Ala Gly Asn Gly Leu Gly Tyr Ala Gly Gly Ala Gly Ala Gly Tyr Ala
    290                 295                 300

Gly Gly Ala Gly Gly Leu Gly Ala Gly Gly Gly Tyr Ala Gly Ala Gly
305                 310                 315                 320

Ala Gly Thr Gly Tyr Ala Gly Gly Met Gln Gly Ala Gly Ala Gly Ala
                325                 330                 335

Gly Leu Gly Gly Gly Met Ala Gly Gly Gly Gly Ala Gly Gly Tyr Val
            340                 345                 350

Gly Gly Gly Ala Gly Ala Gly Val Gly Gly Gly Asp Val Pro Ala Ser
        355                 360                 365

Pro Pro Ala Gly Tyr Ile Arg Tyr Arg Gly Gln Leu Met Thr Ile Asp
    370                 375                 380

Gln Tyr Gln Gln Ala Leu Ala Ser Gly Gly Ser Ser Ala Ser Leu Ser
385                 390                 395                 400

Arg Arg Asp Met Leu Gln Thr Asp Ala Ser Thr Val Phe Leu Asn Thr
                405                 410                 415

Phe Met His Lys Arg Gln Ala Pro Asp Ser Asn Asn Gly Asp Gly Asp
            420                 425                 430

Gly Asn Gly Asn Gly Asn Gly Asn Gly Asn Gly Asp Gly Asp Gly Asn
        435                 440                 445

Gly Asn Gly Asn Gly Asp Gly Asp Gly Asn Gly Asn Gly Asp Gly Glu
    450                 455                 460

Cys Ile Asn Gly Asp Pro Ala Val Pro Ala Ile Lys Phe Asp Thr Met
465                 470                 475                 480

Thr Gly Leu Arg Ile Arg Asp Pro Ala Ala Gln Gln Asp Gly Val Val
                485                 490                 495

Glu Ile Thr Asn Leu Pro Asn Tyr Gln Met Pro Trp Met Ala Phe Leu
            500                 505                 510

Pro Thr Ala Asp Met Trp Gly Glu Gln Gln Thr Leu Met Ile Ala Gln
        515                 520                 525

Glu Gln Val Ile Glu Ser Asn Met Thr Thr Ala Pro Pro Pro Thr Arg
    530                 535                 540

<210>  6
<211>  284
<212>  PRT
<213>  Sporisorium reilianum f. sp. sorghi

<400>  6

Met Arg Ala Thr Leu Ser Leu Asn Met Thr Lys Leu Leu Thr Val Leu
1               5                   10                  15

Leu Val Leu Leu Pro Ile Leu Leu Ala Val Glu Ala Ser Gln Ser Gly
            20                  25                  30

Ala Leu His His Arg Tyr Pro Arg Met Met Arg Gln Arg Arg Ile Glu
        35                  40                  45

Gln Arg Ser Val Ala Ser Trp Leu Thr Lys Ile Ser Ser Lys Val Ser
    50                  55                  60

Asp Trp Val Phe Gly Ser Val Pro Asn Pro Val Thr Asp Pro Lys Asn
65                  70                  75                  80

Ile Pro Lys Ser Gln Val Gly Gly Val Ala Val Leu Pro Gln Met Pro
                85                  90                  95

Tyr Pro Tyr Ala Thr Gly Met Ile Pro Arg Leu Ala Thr Glu Thr Leu
            100                 105                 110

Ser Gly Asn Gly Leu Pro Gly Ala Trp Met Cys Ile His Val Thr Pro
        115                 120                 125

Pro Ala Glu Lys Asp Asp Asp Asp Asp Gly Gly Ala Gly Ala Gly Ala
    130                 135                 140

Gly Ala Gly Ala Gly Ala Gly Ala Gly Ala Gly Ala Gly Met Gly Ala
145                 150                 155                 160

Gly Ala Gly Ala Gly Ala Gly Ala Gly Ala Gly Ala Gly Met Gly Ala
                165                 170                 175

Gly Ala Gly Ala Gly Ala Gly Ala Gly Ala Gly Ala Ala Thr Asp Gly
            180                 185                 190

Ala Pro Lys Lys Lys Gln Arg Ala Lys Val Leu Met Cys Ile Asn Gly
        195                 200                 205

Asp Pro Ala Val Pro Ala Ile Lys Phe Asp Thr Met Thr Gly Leu Arg
    210                 215                 220

Ile Arg Asp Pro Ala Ala Gln Gln Asp Gly Val Val Glu Ile Thr Asn
225                 230                 235                 240

Leu Pro Asn Tyr Gln Met Pro Trp Met Ala Phe Leu Pro Thr Ala Asp
                245                 250                 255

Met Trp Gly Glu Gln Gln Thr Leu Met Ile Ala Gln Glu Gln Val Ile
            260                 265                 270

Glu Ser Asn Met Thr Thr Gly Pro Pro Pro Thr Arg
        275                 280

<210>  7
<211>  515
<212>  PRT
<213>  Ustilago maydis

<400>  7

Met Arg Ala Val Leu Ser Leu Asn Met Thr Lys Leu Leu Ala Leu Leu
1               5                   10                  15

Leu Val Ile Leu Pro Ile Leu Val Ala Val Gln Ala Asn Gly Ser Ile
            20                  25                  30

Ser Asn Ala Ser His His His Gln Arg Arg Met Val Arg Gln Arg His
        35                  40                  45

Ile Glu Ala Arg Ser Ala Met Ser Trp Leu Thr Lys Ile Ser Ser Lys
    50                  55                  60

Ala Ser Asp Trp Met Phe Gly Ser Val His Ala Pro Asn Leu Asp Lys
65                  70                  75                  80

Lys Asp Leu Pro Lys Pro Leu Val Gly Gly Val Ala Val Met Pro Lys
                85                  90                  95

Met Pro Tyr Pro Tyr Ala Thr Gly Met Ile Pro Arg Leu Ala Ser Glu
            100                 105                 110

Thr Leu Ser Gly Asn Gly Leu Pro Gly Gly Trp Met Cys Ile His Val
        115                 120                 125

Thr Pro Pro Ala Asp Val Pro Lys Gln Ala Asp Gly Gly Ala Gly Thr
    130                 135                 140

Arg Ser Gly Ala Gln Gln Phe Gly Gly Gln Arg Val Gly Ala Asp Gly
145                 150                 155                 160

Thr Ala Ala Gly Thr Val Gly Gly Thr Ala Ala Gly Ala Ala Gly Ala
                165                 170                 175

Ala Gly Gly Leu Pro Gly Gly Val Pro Gly Gly Met Ser Gly Gly Tyr
            180                 185                 190

Val Gly Gly Ala Gly Pro Gly Thr Ala Gly Gly Ala Gly Gly Ala Ala
        195                 200                 205

Gly Gly Val Ala Gly Gly Ser Gly Gly Gly Phe Ala Gly Gly Ala Gly
    210                 215                 220

Ser Ser Phe Gly Tyr Pro Gly Gly Phe Gly Phe Gly Ala Asn Thr Gly
225                 230                 235                 240

Tyr Pro Gly Gly Gln Gln Gly Ala Ser Thr Gly Ser Gly Pro Phe Val
                245                 250                 255

Gly Gly Ala Gly Ala Gly Gly Met Gly Gly Gly Met Gly Ala Gly Thr
            260                 265                 270

Gly Ser Ser Gly Met Gly Ala Gly Met Gly Ala Gly Ile Gly Asn Gly
        275                 280                 285

Gly Met Gly Ala Ala Met Gly Ala Ala Met Gly Asn Gly Met Gly Ser
    290                 295                 300

Asp Thr Ala Ala Gly Met Gly Gly Ala Ser Tyr Gly Val Ala Gly Thr
305                 310                 315                 320

Gly Gly Asn Ser Ala Met Ala Gly Gly Gln Gly Ser Thr Val Pro Ala
                325                 330                 335

Gly Tyr Ile Arg Tyr Asn Gly Gln Leu Met Thr Val Asp Gln Tyr Glu
            340                 345                 350

Gln Ala Ile Ala Gly Gly Gly Ser Pro Gly Thr Ala Ala Ser Gln Gln
        355                 360                 365

Thr Leu Ser Arg Arg Asp Met Leu Gln Thr Asp Thr Ser Thr Leu Phe
    370                 375                 380

Leu Asn Thr Phe Ile His Lys Arg Gln Ala Ser Thr Ser Ala Thr Asp
385                 390                 395                 400

Gly Asp Ser Asp Gly Asp Asp Ser Gln Ala Ala Pro Asp Gly Ala Asp
                405                 410                 415

Ala Ser Thr Gln His Lys Lys Pro Ala Ser Asn Ala Asn Ala Pro Lys
            420                 425                 430

Val Leu Met Cys Ile Asn Gly Asp Pro Ala Val Pro Ala Ile Lys Phe
        435                 440                 445

Asn Thr Met Thr Gly Val Arg Ile Arg Asp Ala Ser Thr Gln Lys Asp
    450                 455                 460

Gly Val Val Asp Ile Thr Asn Leu Pro Asn Tyr Lys Met Pro Trp Met
465                 470                 475                 480

Pro Phe Leu Pro Thr Ala Asp Met Trp Gly Glu Gln Gln Asn Leu Met
                485                 490                 495

Leu Asp Gln Glu Lys Met Ile Glu Thr Asn Met Thr Met Ala Pro Pro
            500                 505                 510

Pro Ser Arg
        515

<210>  8
<211>  396
<212>  PRT
<213>  Ustilago esculenta

<400>  8

Met Arg Ala Thr Val Ala Leu Asn Leu Thr Arg Leu Leu Thr Leu Leu
1               5                   10                  15

Leu Val Ile Met Pro Phe Ile Leu Ala Ile Glu Ala His Gly Ser Arg
            20                  25                  30

Pro Phe Gly Gln His His Ser Pro Arg Met Leu Arg Gln Arg Arg Ile
        35                  40                  45

Glu Ala Arg Ser Phe Met Ser Trp Leu Thr His Ile Ser Thr Lys Ala
    50                  55                  60

Ser Asp Trp Val Phe Gly Lys Val Glu Met Pro Thr Arg Asp Glu Ser
65                  70                  75                  80

Gln Ile Pro Lys Pro Gln Val Gly Gly Val Asp Val Leu Pro Lys Met
                85                  90                  95

Pro Tyr Pro Tyr Ala Thr Gly Met Ile Pro Arg Leu Ala Ser Asp Thr
            100                 105                 110

Leu Asn Gly Asn Gly Leu Pro Gly Ala Phe Met Cys Leu His Val Pro
        115                 120                 125

Leu Pro Asp Asp Pro Gln Pro Ala Gly Ser Gly Ser Gly Ser Gly Ser
    130                 135                 140

Gly Gly Gly Ser Gly Gly Ile Gly Gly Gly Gly Gly Ile Gly Gly Gly
145                 150                 155                 160

Ser Gly Gly Tyr Gly Gly Gly Ser Gly Gly Ile Gly Gly Gly Tyr Gly
                165                 170                 175

Gly Gly Ile Gly Gly Gly Ile Gly Gly Gly Ser Ser Gly Gly Gly Tyr
            180                 185                 190

Gly Gly Gly Ser Gly Tyr Gly Gly Gly Ser Gly Thr Tyr Gly Gly Ser
        195                 200                 205

Gly Ser Ser Ser Gly Ile Gly Gly Gly Gly Asp Ala Tyr Gly Gly Gly
    210                 215                 220

Tyr Gly Gly Gly Pro Gly Ile Gly Gly Gly Ser Gly Tyr Gly Gly Ser
225                 230                 235                 240

Gly Ser Gly Tyr Gly Gly Ser Gly Ser Gly Tyr Ala Gly Ser Gly Ala
                245                 250                 255

Gly Ser Gly Ile Gly Gly Gly Tyr Gly Gly Gly Tyr Gly Gly Ala Ile
            260                 265                 270

His Arg Arg Ser Met Phe Gln Ser Asp Val Ser Ser Ile Phe Leu Asn

        275                 280                 285

Thr Val Ile His Lys Arg Gln Ser Pro Asp Pro Ser Gly Asp Ser Thr
    290                 295                 300

Glu Asp Gly Arg Thr Ser Lys Lys Pro Thr Tyr Val Leu Met Cys Ile
305                 310                 315                 320

Asn Gly Asp Pro Asp Val Gln Pro Ile Lys Phe Asn Thr Thr Thr Gly
                325                 330                 335

Ile Arg Met Arg Asp Ala Ser Ala Gln Arg Asp Gly Val Val Glu Ile
            340                 345                 350

Thr Asn Leu Pro Asn Tyr Glu Met Pro Trp Met Pro Phe Leu Pro Thr
        355                 360                 365

Glu Glu Met Trp Ala Glu Gln Gln Thr Leu Met Leu Ala Gln Glu Asp
    370                 375                 380

Thr Ile Glu Asn Lys Met Thr Thr Ser Pro Lys Pro
385                 390                 395

<210> 9
<211> 491
<212> PRT
<213> Ustilago trichophora

<400> 9

Met Arg Ala Met Leu Ser Ile Asn Met Ala Lys Leu Leu Thr Leu Leu
1               5                   10                  15

Leu Val Ile Met Pro Val Val Leu Ala Ile Asp Val Gly Gly Ser Gln
            20                  25                  30

Ala Met Met Pro His His Pro Arg Met Glu Arg Arg Arg Ile Glu Ala
        35                  40                  45

Arg Ser Phe Met Ser Trp Leu Thr His Ile Ser Ser Lys Ala Ser Asp
    50                  55                  60

Trp Met Phe Gly Pro Val Pro Val Pro Ala Arg Asp Glu Ser His Ile
65                  70                  75                  80

Pro Lys Pro Val Thr Gly Pro Val Asp Val Met Pro Lys Met Pro Tyr
                85                  90                  95

Pro Tyr Ala Thr Gly Met Ile Pro Arg Leu Val Ser Glu Thr Leu Glu

            100                 105                 110

Gly Asn Gly Leu Val Gly Gly Tyr Met Cys Leu His Val Thr Pro Pro
        115                 120                 125

Asp Asp Ala Arg Pro Ala Ser Asn Gly Val Gly Gly Gly Gly Ser Gly
    130                 135                 140

Met Ser Ala Asp Gly Met Asn Gly Gly Gly Gly Met Asn Gly Gly Gly
145                 150                 155                 160

Gly Met Asn Gly Gly Gly Gly Met Asn Gly Gly Gly Gly Met Gly Asn
                165                 170                 175

Gly Gly Met Gly Asn Gly Gly Met Gly Gly Gly Gly Met Gly Gly Gly
            180                 185                 190

Ala Gly Ser Tyr Gly Gly Gly Gly Gly Met Ser Gly Gly Gly Met Gly
        195                 200                 205

Gly Gly Gly Met Asn Gly Gly Gly Gly Met Gly Asn Gly Gly Met Gly
    210                 215                 220

Asn Gly Gly Met Gly Asn Gly Ala Gly Ser Tyr Gly Gly Gly Gly Gly
225                 230                 235                 240

Met Gly Gly Gly Gly Met Gly Gly Gly Gly Met Gly Gly Gly Gly Met
                245                 250                 255

Gly Gly Gly Gly Met Gly Gly Gly Gly Met Gly Gly Gly Gly Met Asn
            260                 265                 270

Gly Gly Gly Asp Ser Tyr Gly Gly Gly Gly Gly Gly Gly Met Gly Asn
        275                 280                 285

Gly Gly Met Gly Asn Gly Gly Met Gly Asn Gly Gly Gly Met Gly Gly
    290                 295                 300

Gly Gly Gly Gly Val Met Tyr Gly Gly Gly Gly Gly Gly Gly Gly Gly
305                 310                 315                 320

Met Gly Gly Asp Gly Ser Ser Tyr Gly Gly Ser Asp Ser Gln Asp Gly
                325                 330                 335

Ala Asn Tyr Gly Thr Gly Gly Gly Asn Ser Val Pro Ser Gly Tyr Ile
            340                 345                 350

Arg Tyr Gln Gly Gln Leu Met Thr Ile Asp Gln Tyr Thr Arg Ala Ile
        355                 360                 365

Ser Gly Gly Gly Asp Ser Ser Gly Asn Gly Leu Ser Arg Arg Asp Val
    370                 375                 380

Leu Glu Ser Asp Ser Gln Ile His Lys Arg Gln Thr Gly Asp Gly Met
385                 390                 395                 400

Asp Thr Thr Pro Lys Lys Asn Arg Pro Lys Val Leu Met Cys Ile Asn
                405                 410                 415

Gly Asp Pro Asp Val Pro Pro Ile Lys Phe Asn Thr Met Thr Gly Leu
            420                 425                 430

Arg Ile Arg Asp Ala Ser Asp Arg Val Asn Gly Val Val Glu Ile Ser
        435                 440                 445

Asn Leu Pro Asn Tyr Glu Leu Ser Trp Met Pro Phe Leu Pro Thr Asp
    450                 455                 460

Gln Met Trp Ala Glu Gln Gln Thr Leu Met Leu Ala Gln Glu Ala Met
465                 470                 475                 480

Ile Glu Thr Asn Met Thr Thr Ala Pro Pro Pro
                485                 490


<210>  10
<211>  448
<212>  PRT
<213>  Ustilago hordei (host: oats)

<400>  10

Met Arg Ala Asp Phe Leu Ser Leu Asn Met Ala Lys Leu Leu Ala Leu
1               5                   10                  15

Leu Leu Val Ile Met Pro Phe Leu Leu Ala Ile Glu Ala His Gly Ser
            20                  25                  30

His Pro Asp Thr Leu Ser Met His Leu Arg Met Thr Lys Gln Arg Arg
        35                  40                  45

Ile Glu Ser Arg Ser Met Ser Trp Leu Thr Arg Ile Ser Ser Lys Ala
    50                  55                  60

Ser Asp Trp Ile Phe Gly Asp Val Ser Ala Pro Lys Lys Asp Glu Ser
65                  70                  75                  80

Leu Ile Pro Lys Pro Val Val Gly Gly Val Pro Val Leu Pro Lys Met
                85                  90                  95

Pro Tyr Pro Tyr Ala Ser Gly Met Val Pro Arg Leu Ala Ser Glu Thr
            100                 105                 110

Leu Asn Gly Asn Gly Leu Val Gly Gly Trp Ile Cys Met His Val Thr
        115                 120                 125

Pro Pro Thr Asp Asp Ser Ala Gly Ala Gly Ala Gly Ala Gly Gly Ala
    130                 135                 140

Asp Gly Ser Ala Gly Ala Ser Gly Thr Ser Val Gly Gly Phe Asn Asp
145                 150                 155                 160

Arg Gly Ala Gly Gly Ser Gly Ala Gly Gly Ser Tyr Asp Gly Ala Ala
                165                 170                 175

Arg Gly Phe Thr Gly Gly Ala Gly Ala Asn Pro Ala Phe Gly Gly Gln
            180                 185                 190

Gln Gly Val Asn Pro Ala Thr Gly Gly Tyr Tyr Val Gly Ala Gly Gly
        195                 200                 205

Gly Ala Gly Val Gly Gly Gln Gln Thr Thr Gly Gly Tyr Tyr Gly Ser
    210                 215                 220

Gly Gly Tyr Tyr Gly Gly Gly Gly Ala Gly Val Gly Gly Gln Gln Thr
225                 230                 235                 240

Thr Gly Gly Tyr Tyr Gly Ser Gly Gly Tyr Tyr Gly Gly Gly Gly Ala
                245                 250                 255

Gly Val Gly Gly Gln Gln Thr Thr Gly Glu Tyr Tyr Gly Gly Gly Ala
            260                 265                 270

Gly Val Gly Gly Gly Ala Gly Gly Gly Met Met Gly Gly Gly Gly Ala
        275                 280                 285

Leu Asn Glu Ala Gly Gly Gly Thr Val Pro Ala Gly Lys Ile Arg Tyr
    290                 295                 300

Asn Gly Gln Leu Met Thr Ile Glu Gln Tyr Gln Gln Ala Ile Ala Ser
305                 310                 315                 320

Ser Gly Ser Ser Ser Pro Ser Leu Ser Arg Arg Glu Leu Leu Gln Ala
                325                 330                 335

Glu Thr Ala Asn Ile Met Val Asp Ser Leu Leu His Lys Arg Gln Ser
                340                 345                 350

Phe Asp Thr Ala Pro Ala Ala Ser Lys Lys Gln Ala Lys Val Leu Met
            355                 360                 365

Cys Ile Asn Gly Asp Pro Ala Ile Pro Pro Ile Lys Phe Asn Thr Met
    370                 375                 380

Thr Gly Leu Arg Ile Arg Asp Ala Ser Glu Lys Lys Asp Gly Val Val
385                 390                 395                 400

Ala Ile Thr Asn Leu Pro Asn Tyr Gln Met Pro Trp Met Pro Phe Leu
                405                 410                 415

Pro Thr Glu Glu Met Trp Ala Glu Gln Gln Thr Leu Met Leu Glu Gln
                420                 425                 430

Glu Lys Ile Ile Glu Gln Asn Met Thr Thr Ala Pro Pro Pro Lys Gln
            435                 440                 445

<210>  11
<211>  506
<212>  PRT
<213>  Ustilago tritici

<400>  11

Met Lys Ala Ile Leu Ser Ile Asn Met Thr Lys Leu Leu Ala Leu Leu
1               5                   10                  15

Leu Val Leu Leu Pro Leu Leu Leu Ser Ala Glu Val Tyr Gly Ser Ser
                20                  25                  30

His Ala Met Leu Gln Gln Gly His Ser Arg Met Met Arg Arg Arg Leu
            35                  40                  45

Glu Ala Arg Ser Leu Ala Ser Phe Leu Thr Lys Ile Ser Ser Lys Ala
    50                  55                  60

Ser Asp Trp Val Phe Gly Ser Val Pro Asn Pro Asn Thr Asp Pro Lys
65                  70                  75                  80

Arg Val Pro Lys Pro Gln Val Gly Gly Val Pro Val Leu Pro Lys Met
                85                  90                  95

Pro Tyr Pro Tyr Ala Thr Gly Met Ile Pro Arg Leu Ala Ser Glu Thr
                100                 105                 110

Leu Ser Gly Gly Gly Leu Pro Gly Gly Trp Met Cys Leu His Val Thr
        115                 120                 125

Pro Pro Leu Glu His Asp Lys Ala Ala Gln Thr Gly Ala Gly Ala Gly
    130                 135                 140

Ala Gly Ala Gly Ala Ala Gly Asp Ala Gly Leu Gly Gly Gly Ala Ala
145                 150                 155                 160

Gly Val Gly Ala Gly Ala Gly Leu Gly Ala Gly Gly Gly Ala Gly Ala
                165                 170                 175

Gly Ala Val Gly Met Ala Gly Gly Ala Gly Ala Ala Val Gly Val Gly
            180                 185                 190

Ala Gly Ala Gly Leu Gly Gly Gly Lys Ser Ala Val Gly Leu Gly Gly
        195                 200                 205

Gly Phe Gly Gly Gly Gln Ser Gly Gly Ala Gly Ile Gly Ala Gly Thr
    210                 215                 220

Gly Thr Gly Ala Gly Val Gly Ala Gly Ala Gly Ser Ala Gly Gly Ala
225                 230                 235                 240

Gly Gly Ala Val Gly Val Gly Ala Gly Thr Gly Phe Ala Gly Gly Gln
                245                 250                 255

Gln Gly Leu Gly Gly Gly Ala Gln Gly Pro Gly Ala Gly Gly Tyr Val
            260                 265                 270

Gly Ala Gly Ala Gly Val Gly Gly Leu Gly Ile Gly Pro Gly Thr Gly
        275                 280                 285

Val Gly Leu Gly Asn Gly Phe Asp Gly Val Gly Ala Val Gly Thr Gly
    290                 295                 300

Gly Ala Asp Thr Ser Ile Gly Asn Gly Ala Gly Ala Gly Leu Gly Ala
305                 310                 315                 320

Ser Ala Gly Val Gly Thr Gly Ala Gly Ala Gly Ala Gly Ala Gly Pro
                325                 330                 335

Val Pro Ala Gly Tyr Ile Arg Tyr Asn Gly Gln Leu Leu Thr Ile Asp
            340                 345                 350

Gln Tyr Gln Gln Ala Leu Ala Ser Ser Gly Thr Gly Ser Ser Leu Ser
        355                 360                 365

Arg Arg Asp Leu Phe Gln Pro Glu Thr Ser Asn Ile Leu Leu Asn Thr
    370                 375                 380

Phe Ile His Lys Arg Gln Ala Gly Ala Asp Asp Ser Asp Ala Gly Gln
385                 390                 395                 400

Pro Gly Asp Asp Ser Asp Val Gly Asp Ala Thr Asp Pro Ala Pro Lys
                405                 410                 415

Lys Asn Thr Pro Ala Lys Val Leu Met Cys Ile Asn Gly Asp Pro Ala
            420                 425                 430

Val Pro Ala Ile Lys Phe Asp Thr Met Thr Gly Leu Arg Ile Arg Asp
        435                 440                 445

Ala Ser Asp Lys Gln Asp Gly Val Val Ala Ile Thr Asn Leu Pro Arg
    450                 455                 460

Tyr Asn Thr Pro Trp Met Pro Phe Leu Pro Thr Ala Glu Met Trp Ser
465                 470                 475                 480

Glu Gln Gln Thr Leu Met Leu Thr Gln Glu Glu Ile Ile Glu Lys Asn
                485                 490                 495

Met Thr Thr Gly Pro Pro Pro Ser Thr Lys
            500                 505

<210>  12
<211>  231
<212>  PRT
<213>  Melanopsichium pennsylvanicum

<400>  12

Met Leu Thr Ile Lys Ser Phe Ser Leu Tyr Leu Cys Ile Ala Val Val
1               5                   10                  15

Phe Leu Ile Thr Cys Ser Arg Ala Ser Gln Pro Val Ser Tyr Ala Leu
            20                  25                  30

Gln Gln Lys Arg Ala Arg Arg Pro Leu Ala Asn Ser Gly Met Ile Lys
        35                  40                  45

Arg Asn Leu Gly Gly Lys Ile Lys Asp Trp Tyr Leu Met Gln Val Thr
    50                  55                  60

His Asn Tyr Phe Cys Thr Ser Ser Ala Arg Val Val Pro Val Asp Pro
65                  70                  75                  80

Asp Thr Gly His Met Leu Leu Lys Ser Leu Arg Lys Arg Asp Phe Leu
                85                  90                  95

Asp Gly Trp Leu Gly His Ser Lys Thr Thr Glu Lys Gly Leu Cys Lys
            100                 105                 110

Asp Ala Ser Trp Val Leu Pro Asp Gly Trp Thr Gln Pro Ile Pro Lys
        115                 120                 125

Ser Gln Phe Leu Asn Gln Tyr Pro Trp Met Asp Val Asp Asp Arg Gly
    130                 135                 140

Gln Pro Arg Phe Lys Pro Glu Phe Phe Ala Trp Leu Gln Gln Arg Ala
145                 150                 155                 160

Ala Asn Gly Pro Ser Gly Val Ala Asp Ser Ala Ala Ala Ser Gly Gln
                165                 170                 175

Tyr Leu Pro Leu Thr Asn Gly Ala Asn Gly Ala Ala Val Pro Tyr Gly
            180                 185                 190

Gly Val Gly Tyr Val Pro Ala Ser Tyr Leu Ser Tyr Gln Gln Gly Thr
        195                 200                 205

Ser Gly Thr Ala Gly Leu Gly Gln Asn Thr Leu Gly Asn Gly Val Thr
    210                 215                 220

Ala Gly Ala Ala Ala His Ile
225                 230

<210>  13
<211>  218
<212>  PRT
<213>  Sporisorium scitamineum

<400>  13

Met Leu Val Ile Arg Ala Arg Leu Ile Leu Ser Leu Leu Ala Ile Val
1               5                   10                  15

Leu Leu Ile Val Asn Cys His Ala Leu Gln Asn Lys Lys Ala Val Pro
            20                  25                  30

Phe Gly Asn Asn Lys Val Glu Lys Arg Asn Trp Gly Gly Gln Ile Lys
        35                  40                  45

Asp Trp Trp Leu Ala Gln Asn Thr His Asn Tyr Phe Cys Thr Asp Thr
    50                  55                  60

Gly Arg Leu Val Pro Val Asp Pro Ala Thr Gly His Met Leu Leu Arg
65                  70                  75                  80

Gly Leu Arg Lys Arg Ser Trp Phe Asp Asp Leu Val Gly His Ser His
                85                  90                  95

Ala Thr Glu Lys Gly Leu Cys Lys Asn Pro Thr Trp Val Leu Pro Gln
            100                 105                 110

Gly Trp Thr Gln Pro Ile Pro Val Thr Gln Phe Leu Ala Gln Tyr Pro
        115                 120                 125

Trp Met Thr Val Asp Asp Arg Gly Gln Ile Arg Leu Arg Asp Asp Phe
    130                 135                 140

Arg Ala Trp Val Asp Gln Gln Ala Ala Ala Arg Ala Ala Asn Pro Ala
145                 150                 155                 160

Gln Ala Gly Gln Ala Met Ala Ala Ser Ser Gly Ala Pro Gly Ala Val
                165                 170                 175

Pro Asp Gly Gly Ala Gly Tyr Val Pro Pro Ser Ala Met Gln Val Ala
            180                 185                 190

Pro Gly Thr Gly Thr Thr Ser Gly Phe Gly Ser Asn Ala Phe Gly Asn
        195                 200                 205

Gly Val Thr Ala Gly Asn Ala Ala His Val
    210                 215

<210>  14
<211>  221
<212>  PRT
<213>  Ustilago hordei (host: barley)

<400>  14

Met Ala Thr Thr Pro Phe Gln Phe Leu Phe Ile Leu Leu Leu Ala Leu
1               5                   10                  15

Ala Leu Leu Ser Gly Asn Asn Ala Ala Glu Ser Lys Lys Ala Val Pro
            20                  25                  30

Phe Gly Lys Arg Asn Ile Gly Gly His Phe Lys Asp Trp Tyr Leu Ser
        35                  40                  45

Lys Met Thr Lys Asn Tyr Phe Cys Thr Val Asn Gly Arg Met Val Pro
    50                  55                  60

Val Asp Pro Arg Thr Gly His Met Leu Leu Lys Gly Leu Arg Lys Arg
65                  70                  75                  80

Asp Ile Thr Asp Ala Phe Asp Asp Gln Leu Gly His Ser Ala Lys Thr
                85                  90                  95

Glu Thr Gly Ser Cys Arg Thr Pro Thr Trp Val Leu Pro Gln Gly Trp
            100                 105                 110

Thr Gln Pro Ile Pro Val Thr Gln Phe Val Ala Gln Tyr Pro Trp Met
        115                 120                 125

Asp Val Asp Glu Arg Gly Gln Met His Leu Lys Pro Asp Tyr Arg Ala
    130                 135                 140

Trp Leu Glu Gln Gln Gly Glu Leu Arg Gln Gln Gln Ala Ala Gln Gly
145                 150                 155                 160

Gly Gly Gln Ala Leu Glu Ala Gly Gly Ala Thr Gly Ala Ala Ala Arg
                165                 170                 175

Gly Val Val Gln Asp Gly Gly Val Gly Tyr Val Pro Pro Ser Phe Leu
            180                 185                 190

Asn Val Gly Thr Gly Met Thr Thr Ser Ser Gly Leu Gly Gly Asn Asn
        195                 200                 205

Phe Gly Asn Gly Val Ala Ala Gly Ser Ala Ala His Phe
    210                 215                 220

<210>  15
<211>  221
<212>  PRT
<213>  Ustilago bromivira

<400>  15

Met Ala Thr Thr Pro Phe Gln Phe Leu Phe Ile Leu Leu Leu Ala Leu
1               5                   10                  15

Ala Leu Leu Ser Gly Asn Asn Ala Ala Glu Ser Lys Lys Ala Val Pro
            20                  25                  30

Phe Gly Lys Arg Asn Ile Gly Gly His Phe Lys Asp Trp Tyr Leu Ser
        35                  40                  45

Lys Met Thr Lys Asn Tyr Phe Cys Thr Val Asn Gly Arg Leu Val Pro
    50                  55                  60

Val Asp Pro Arg Thr Gly His Met Leu Leu Lys Ser Leu Arg Lys Arg
65                  70                  75                  80

Asp Ile Phe Asp Ala Phe Asp Glu Pro Leu Gly His Ser Ala Lys Thr
                85                  90                  95

Glu Thr Gly Ser Cys Arg Asn Pro Thr Trp Val Leu Pro Gln Gly Trp
            100                 105                 110

Thr Gln Pro Ile Pro Val Thr Gln Phe Leu Ala Gln Tyr Pro Trp Met
        115                 120                 125

Asp Val Asp Glu Arg Gly Gln Met His Leu Lys Pro Asp Tyr Arg Ala
    130                 135                 140

Trp Leu Glu Gln Gln Gly Glu Leu Arg Gln Gln Gln Ala Ala Gln Gly
145                 150                 155                 160

Gly Gly Gln Ala Val Glu Ala Gly Gly Ala Thr Asp Ala Thr Ala Gly
                165                 170                 175

Gly Val Val Gln Asp Gly Gly Val Gly Tyr Val Pro Pro Ser Phe Leu
            180                 185                 190

Asn Val Gly Thr Gly Met Ala Ala Ser Ser Gly Leu Gly Gly Asn Asn
        195                 200                 205

Phe Gly Asn Gly Val Ala Ala Gly Ser Ala Ala His Val
    210                 215                 220

<210>  16
<211>  221
<212>  PRT
<213>  Sporisorium reilianum f. sp. zeae

<400>  16

Met Leu Val Ile Lys Ser Thr Thr Ile Leu Ser Leu Leu Ala Val Val
1               5                   10                  15

Leu Leu Ile Val Asp Cys Gln Ala Val Gln Pro Lys Arg Ala Leu Pro
            20                  25                  30

Leu Gly Ser Asn Ala Val Lys Lys Arg Asn Leu Gly Gly Gln Ile Lys
        35                  40                  45

Asp Trp Tyr Leu Ala Gln Glu Thr His Asn Tyr Phe Cys Thr Asp Thr
    50                  55                  60

Gly Arg Leu Val Pro Val Asp Pro Asp Thr Gly His Met Leu Leu Lys
65                  70                  75                  80

Gly Leu Arg Lys Arg Gly Trp Ile Asp Asp Met Leu Gly His Ser His
                85                  90                  95

Gln Thr Glu His Gly Leu Cys Lys Asn Pro Ser Trp Val Leu Pro Pro
            100                 105                 110

Gln Trp Thr Gln Pro Ile Pro Val Thr Gln Phe Leu Ala Gln Tyr Pro
        115                 120                 125

Trp Met Thr Val Asp Glu Arg Gly Gln Ile Arg Leu Arg Asp Asp Phe
    130                 135                 140

Arg Ala Trp Ile Asp Gln Arg Thr Ala Ala Asn Ala Ala Asn Pro Ala
145                 150                 155                 160

Ala Ala Ala Gly Gly Ala Gly Gly Ala Gly Gly Ala Gly Gly Gly Gln
                165                 170                 175

Gly Ala Val Pro Asp Gly Gly Ala Gly Tyr Ile Pro Pro Ser Ala Met
            180                 185                 190

Gln Val Ala Pro Gly Thr Gly Thr Thr Ser Gly Phe Gly Thr Ala Ala
        195                 200                 205

Phe Gly Asn Gly Val Ala Ala Gly Ala Ala Ala His Val
    210                 215                 220

<210>  17
<211>  218
<212>  PRT
<213>  Sporisorium reilianum f. sp. sorghi

<400>  17

Met Leu Val Ile Lys Ser Thr Thr Ile Leu Ser Leu Leu Ala Val Val
1               5                   10                  15

Leu Leu Ile Val Asp Cys Gln Ala Val Gln Pro Lys Arg Ala Leu Pro
            20                  25                  30

Leu Gly Ser Asn Ala Val Lys Lys Arg Asn Leu Gly Gly Gln Ile Lys
        35                  40                  45

Asp Trp Tyr Leu Ala Gln Glu Thr His Asn Tyr Phe Cys Thr Asp Thr
    50                  55                  60

Gly Arg Leu Val Pro Val Asp Pro Asp Thr Gly His Met Leu Leu Lys
65                  70                  75                  80

Gly Leu Arg Lys Arg Gly Trp Ile Asp Asp Met Leu Gly His Ser His
                85                  90                  95

Gln Thr Glu His Gly Leu Cys Lys Asn Pro Ser Trp Val Leu Pro Pro
            100                 105                 110

Gln Trp Thr Gln Pro Ile Pro Val Thr Gln Phe Leu Ala Gln Tyr Pro
        115                 120                 125

Trp Met Thr Val Asp Glu Arg Gly Gln Ile Arg Leu Arg Asp Asp Phe
    130                 135                 140

Arg Ala Trp Ile Asp Gln Arg Ala Ala Ala Asn Ala Ala Asn Pro Ala
145                 150                 155                 160

Ala Ala Ala Gly Gly Ala Gly Gly Ala Gly Gly Gly Gln Gly Ala Val
                165                 170                 175

Pro Asp Gly Gly Ala Gly Tyr Ile Pro Pro Ser Ala Met Gln Val Ala
            180                 185                 190

Pro Gly Thr Gly Thr Thr Ser Gly Phe Gly Thr Ala Ala Phe Gly Asn
        195                 200                 205

Gly Val Ala Ala Gly Ala Ala Ala His Val
    210                 215

<210>  18
<211>  218
<212>  PRT
<213>  Ustilago maydis

<400>  18

Met Met Leu Pro Phe Gln Ser Tyr Ile Leu Phe Ser Leu Leu Ala Thr
1               5                   10                  15

Ile Phe Trp Thr Ile Gly Cys Asp Ala Leu Gln Gln Lys Arg Pro Ile
            20                  25                  30

Ala Phe Gly Ser Ser Val Glu Lys Arg Asn Leu Gly Gly Lys Leu Lys
        35                  40                  45

Asp Trp Tyr Leu Ala Gly Val Thr Lys Asn Tyr Met Cys Thr Asp Thr
    50                  55                  60

Gly Arg Leu Val Pro Val Asp Pro Gln Thr Gly His Met Leu Leu Lys
65                  70                  75                  80

Gly Leu Arg Lys Arg Asp Ser Trp Phe Asp Asp Leu Val Gly His Ser
                85                  90                  95

His Asp Thr Glu Gln Gly Leu Cys Arg Asn Pro Thr Trp Val Leu Pro
            100                 105                 110

Pro Gly Trp Thr Gln Pro Ile Pro Val Thr Gln Phe Asn Ala Gln Tyr
        115                 120                 125

Pro Trp Val Thr Val Asp Asp Arg Gly Gln Ile Arg Phe Arg Glu Glu
    130                 135                 140

Tyr Ser Asn Trp Leu Lys Gln Arg Ala Gly Ser Gly Val Gly Ser Gln
145                 150                 155                 160

Thr Met Gly Gln Pro Ala Ser Gly Thr Ser Gly Ala Pro Ala Ala Val
                165                 170                 175

Pro Asp Gly Gly Val Gly Tyr Val Pro Pro Ser Ala Leu Asn Val Ala
            180                 185                 190

Ala Gly Thr Gly Thr Ser Ser Gly Leu Gly Gln Asn Asp Phe Gly Asn
        195                 200                 205

Gly Ile Ser Ala Gly Asn Val Ala His Val
    210                 215


<210>  19
<211>  193
<212>  PRT
<213>  Ustilago esculenta

<400>  19

Met Leu Ala Ile Arg Ile Cys Phe Leu Leu Pro Leu Leu Val Phe Val
1               5                   10                  15

Leu Leu Ala Val Asp Cys His Ala Ile Gln Arg Lys Arg Ala Val Val
            20                  25                  30

Phe Gly Asp Lys Thr Leu Asp Lys Arg Asn Ile Gly Gly Gln Leu Lys
        35                  40                  45

Asp Met Tyr Leu Ser Lys Phe Thr Gln Asn Tyr Phe Cys Thr Asp Thr
    50                  55                  60

Phe Arg Leu Val Pro Val Asp Pro Glu Thr Gly His Met Leu Leu Lys
65                  70                  75                  80

Gly Leu Arg Lys Arg Ser Phe Ile Asp Asp Phe Phe Gly His Ser His
                85                  90                  95

Ser Thr Glu His Gly Glu Cys Arg Asn Arg Thr Trp Val Leu Pro Pro
            100                 105                 110

Gly Trp Thr Gln Pro Ile Pro Lys Thr Gln Phe Glu Ala Gln Tyr Pro
        115                 120                 125

Trp Ala Tyr Val Asp Asp Arg Gly Arg Ile Arg Leu Lys Glu Asp Tyr
    130                 135                 140

Arg Asp Trp Leu Glu Gln Gln Ser Gln Ala Arg Leu Ala Gly Ala Thr
145                 150                 155                 160

Asn Pro Ser Glu Gly Gly Ser Thr Met Pro Gly Gly Pro Asn Gly Ala
                165                 170                 175

Val Val Pro Asp Gly Gly Val Gly Tyr Val Pro Pro Ser Tyr Leu His
            180                 185                 190

Val

<210>  20
<211>  224
<212>  PRT
<213>  Ustilago trichophora

<400>  20

Met Leu Ser Ile Gly Ser Cys His Leu Phe Val Leu Leu Ile Leu Leu
1               5                   10                  15

Leu Ser Phe Ser Gly Ser His Ala Thr Gln His Lys Arg Ala Val Pro
            20                  25                  30

Phe Thr Ser Ala Ser Leu His Lys Arg Asn Leu Gly Gly Gln Phe Lys
        35                  40                  45

Asp Trp Tyr Leu Ser Lys Met Thr Lys Asn Tyr Phe Cys Thr Asp Thr
    50                  55                  60

Gly Arg Leu Val Pro Val Asp Pro Glu Thr Gly His Met Leu Leu Lys
65                  70                  75                  80

Gly Leu Arg Lys Arg Asp Phe Leu Asp Thr Leu Leu Gly His Thr His
                85                  90                  95

Glu Thr Glu Lys Gly Ser Cys Gly Thr Ala Ser Trp Ile Leu Leu Glu
            100                 105                 110

Asn Trp Ser Gln Pro Ile Pro Val Thr Gln Phe Leu Ala Gln Tyr Pro
        115                 120                 125

Trp Met Tyr Val Asp Glu Arg Gly Gln Ala Arg Leu Lys Pro Asp Tyr
    130                 135                 140

Leu Glu Trp Leu Glu Gln Gln Ala Ala Ala Arg Ala Ala Asn Pro Ala
145                 150                 155                 160

Ala Ala Ala Ala Pro Gly Gly Gln Met Arg Thr Ala Pro Asp Gly Thr
                165                 170                 175

Tyr Thr Gly Gly Gln Val Gln Asp Gly Gly Val Gly Tyr Val Pro Pro
            180                 185                 190

Ser Tyr Leu Asn Thr Gly Ala Gly Met Gly Thr Thr Ser Gly Leu Gly
        195                 200                 205

Gln Thr Gly Phe Gly Asn Gly Ile Thr Ala Gly Thr Ala Ala His Val
    210                 215                 220

<210>  21
<211>  221
<212>  PRT
<213>  Ustilago hordei (host: oats)

<400>  21

Met Ala Thr Thr Pro Phe Gln Phe Leu Phe Ile Leu Leu Leu Ala Leu
1               5                   10                  15

Ala Leu Leu Ser Gly Asn Asn Ala Ala Glu Ser Lys Lys Ala Val Pro
            20                  25                  30

Phe Gly Lys Arg Asn Ile Gly Gly His Phe Lys Asp Trp Tyr Leu Ser
        35                  40                  45

Lys Met Thr Lys Asn Tyr Phe Cys Thr Val Asn Asn Arg Leu Val Pro
    50                  55                  60

Val Asp Pro Arg Thr Gly His Met Leu Phe Lys Ser Leu Arg Lys Arg
65                  70                  75                  80

Asp Ile Thr Asp Ala Phe Asp Asp Gln Leu Gly His Ser Ala Lys Thr
                85                  90                  95

Glu Thr Gly Leu Cys Arg Asn Arg Thr Trp Val Leu Pro Gln Gly Trp
            100                 105                 110

Thr Gln Pro Ile Pro Val Thr Gln Phe Val Ala Gln Tyr Pro Trp Met
        115                 120                 125

Asp Val Asp Glu Arg Gly Gln Ile His Leu Lys Pro Asp Tyr Arg Ala
    130                 135                 140

Trp Leu Glu Gln Gln Gly Glu Leu Arg Gln Lys Gln Ala Ala Gln Gly
145                 150                 155                 160

Gly Gly Gln Ala Leu Glu Ala Gly Gly Ala Thr Gly Ala Ala Ala Arg
                165                 170                 175

Gly Val Val Gln Asp Gly Gly Val Gly Tyr Val Pro Pro Ser Phe Leu
            180                 185                 190

Asn Val Gly Thr Gly Met Thr Thr Ser Leu Gly Leu Gly Gly Asn Asn
        195                 200                 205

Phe Gly Asn Gly Val Ala Ala Gly Ser Ala Ala His Phe
    210                 215                 220


<210>  22
<211>  212
<212>  PRT
<213>  Ustilago tritici

<400>  22

Met Ser Ile Ile Arg Ala Glu Leu Ile Val Ala Ile Leu Ala Met Ser
1               5                   10                  15

Leu Leu Val Met Tyr Cys His Ala Gln Gln Asp Lys Lys Ala Phe Pro
            20                  25                  30

Phe Asp Ser Ser Ser Val Lys Lys Arg Asn Leu Gly Gly His Ile Lys
        35                  40                  45

Asp Trp Tyr Leu Ser Lys Met Thr His Asn Tyr Phe Cys Thr Asp Thr
    50                  55                  60

Gly Arg Leu Val Pro Val Asp Pro Glu Thr Gly His Met Leu Leu Lys
65                  70                  75                  80

Gly Leu Arg Lys Arg Asn Phe Val Asp Asp Met Val Gly Lys Ser His
                85                  90                  95

Glu Thr Glu Thr Gly Gly Cys His Asn Pro Thr Trp Val Leu Pro Glu
            100                 105                 110

Lys Trp Thr Gln Pro Ile Pro Val Thr Gln Phe Glu Ala Gln Tyr Pro
        115                 120                 125

Trp Met Thr Val Asp Asp Arg Gly Gln Ile Arg Leu Lys Asp Asp Tyr
    130                 135                 140

Arg Gln Trp Leu Glu Gln Arg Ala Ala Ala Asn Pro Ala Gln Ile Gly
145                 150                 155                 160

Gly Gly Ala Gly Ala Ala Pro Gly Val Val Pro Asp Gly Gly Val Gly
                165                 170                 175

Tyr Val Ala Pro Ser Tyr Leu Asn Val Ala Pro Gly Met Ala Ser Thr
            180                 185                 190

Ser Gly Leu Gly Thr Thr Asn Phe Gly Asn Gly Val Thr Ala Gly Thr
        195                 200                 205

Ala Ala His Ile
    210

<210>  23
<211>  325
<212>  PRT
<213>  Melanopsichium pennsylvanicum

<400>  23

Met Gly Leu Thr Arg Thr Trp Leu Val Leu Leu Gly Thr Ile Ser Leu
1               5                   10                  15

Ser Leu Leu Pro Leu Val Leu Ala Asp Thr Gln Leu Ala Asp Pro Asn
            20                  25                  30

Leu Ala Val Ile Ser Glu Phe Asn Val Val Leu Thr Asp Asn Thr Lys
        35                  40                  45

Trp Phe Asp Leu Gly Cys Cys Met Ser Thr Asn Ala Asp Leu Ala Ser
    50                  55                  60

Ile Gly Gln Tyr Ser Cys Val Pro His Lys Asp Leu Gln Leu Gly Gln
65                  70                  75                  80

Ala Pro Gly Ser Arg Arg Ile Gly Ile Gln Phe Cys His His Arg Ser
                85                  90                  95

Asp Gly Ser Ser Pro Asp Gln Val Lys Ser Ile Phe Lys Ser Val Cys
            100                 105                 110

Glu Lys Asn Thr Gly Ser Trp Leu Glu Pro Asp Gly Ser Tyr Cys Pro
        115                 120                 125

Gln Ile Trp Pro Asn Tyr Lys Asp Gly Tyr Lys Lys Pro Ala Pro Pro
    130                 135                 140

Ala Ala Pro Lys Ala Asp Pro Asp Pro Ala Pro Pro Pro Pro Pro Pro
145                 150                 155                 160

Ser Asn Pro Asp Leu Ser Gly Lys Thr Thr Asn Asp Glu Asn Gly Phe
                165                 170                 175

Lys Thr Glu Gly Thr Phe Asn Tyr Ala Leu Val Asp Tyr Ala Ile Thr
            180                 185                 190

Arg Ser Met Ala Cys Cys Lys Ala Ser Thr Lys Pro Ile Val Arg Pro
        195                 200                 205

Ala Lys Tyr Lys Cys Gly Gln Arg Lys Val Ser Ser Gln Val Phe Leu
    210                 215                 220

Lys Gln Cys Val Lys Ile Leu Pro Thr Ile Thr Lys Ala Lys Lys Leu
225                 230                 235                 240

Ala Gly Tyr Thr Thr Ile Gly Asp Gly Asn Asp Glu Ile Val Asp Asp
                245                 250                 255

Ala Thr Lys Gln Gln Cys Ser Asn Met Trp Thr Asn Ala Leu Thr Tyr
            260                 265                 270

Thr Tyr Gly Phe Cys Lys Leu Asp Tyr Asp Gln Ala Gln Asp Glu Ala
        275                 280                 285

Thr Lys Ala Phe Ser Asp Asp Cys Thr Ala Lys Gly Gly Glu Leu Lys
    290                 295                 300

Asp Pro His Asn Gly Met Cys Leu Trp Glu Val Asp Asp Ala Ala Gly
305                 310                 315                 320

Ala Ser Gly Phe Arg

                325

<210> 24
<211> 332
<212> PRT
<213> Sporisorium scitamineum

<400> 24

Met Gln Leu Lys Arg Thr Trp Leu Ala Leu Leu Gly Ala Val Ser Leu
1               5                   10                  15

Ala Leu Ile Ser Gln Val Ala Ala Asp Asp Pro Gln Ile Thr Asn Pro
            20                  25                  30

Tyr Leu Thr Val Ile Arg Gln Phe Asn Ile Val Leu Thr Asp Asn Thr
        35                  40                  45

Lys Val Tyr Asp Leu Ala Cys Cys Met Gly Val Pro Asn Glu Leu Ala
    50                  55                  60

Thr Thr Gln Thr Arg Arg Cys Phe Glu His Gln Asp Leu Ala Met Ser
65                  70                  75                  80

His Thr Pro Gly Ala Arg Arg Met Gly Leu Gln Tyr Cys His His Leu
                85                  90                  95

Pro Gly Ser Thr Pro Asp Glu Ala Thr Gln Arg Phe Lys Glu Arg Cys
            100                 105                 110

Thr Leu Asp Gly Gly Glu Ile Ile Thr Pro Asp Lys Ser Tyr Cys Pro
        115                 120                 125

Gln Ile Trp Pro Asn Tyr Asp Asp Lys Tyr Val Lys Pro Ala Pro Ala
    130                 135                 140

Pro Ala Pro Ala Pro Gly Gly Asp Gly Ala Pro Pro Pro Ala Pro Asn
145                 150                 155                 160

Pro Ala Pro Asp Ala Ala Gly Arg Ser Thr Asn Asp Lys Asp Gly Phe
                165                 170                 175

Lys Ser Glu Gly Thr Phe His Tyr Thr Leu Val Asp Tyr Ala Ile Thr
            180                 185                 190

Arg Ser Leu Ala Cys Cys Lys Ala Ser Ser Lys Pro Ile Val Lys Pro
        195                 200                 205

Val Ser Gln Pro Asp Lys Pro Lys Leu Cys Ser Cys Ala Gln Arg Lys

210                 215                 220

Val Ala Ser Asp Thr Phe Met Ala Gln Cys Ala Lys Ile Leu Pro Thr
225                 230                 235                 240

Ile Thr Lys Ser Lys Lys Leu Ser Gly Tyr Thr Thr Ile Lys Gly Asp
                245                 250                 255

Gly Asp Ala Ile Val Asp Asp Lys Thr Lys Glu Gln Cys Ser Ala Met
            260                 265                 270

Trp Thr Asn Ala Leu Ser Tyr Thr Tyr Gly Phe Cys Gln Leu Asp Asn
        275                 280                 285

Asp Ser Ala Arg Asp Asp Ala Ile Lys Ala Phe Gln Tyr Asp Cys Thr
    290                 295                 300

Ser Lys Gly Gly Glu Pro Lys Glu Pro His Asn Gly Met Cys Leu Trp
305                 310                 315                 320

Asn Val Asp Asp Ala Ala Ala Asp Ser Thr Thr Ala
                325                 330

<210>  25
<211>  329
<212>  PRT
<213>  Ustilago hordei (host: barley)

<400>  25

Met Gln Pro Asn Gly Ser Trp Leu Thr Leu Leu Gly Ala Ile Cys Val
1               5                   10                  15

Ser Leu Leu Pro Ser Ile Ser Ala Thr Thr Gln Ile Ser Asp Pro Asn
            20                  25                  30

Leu Ala Val Ile Leu Gln Tyr Asn Ile Val Leu Thr Asp Asn Thr Lys
        35                  40                  45

Val Asn Asp Leu Phe Cys Cys Met Gly Thr Ser Ala Glu Ile Pro Ser
    50                  55                  60

Ala Ala Glu Tyr Lys Cys Phe Glu His Gln Ala Leu Asp Met Gly Ser
65                  70                  75                  80

Ala Pro Gly Ser Arg Arg Met Gly Ile Gln Phe Cys His His Leu Pro
                85                  90                  95

Gly Ser Lys Pro Glu Glu Ala Val Ala Ala Phe Lys Asp Ser Cys Ser

            100                 105                 110

Lys Tyr Pro Gly Glu Tyr Ile Asn Pro Asp Lys Gly Gln Cys Pro Gln
        115                 120                 125

Ile Trp Pro Asn Tyr Lys Asp Asp Tyr Lys Lys Pro Ala Pro Ala Pro
    130                 135                 140

Ala Pro Asp Ala Pro Ala Pro Lys Pro Gly Thr His Asp Pro Ala Ser
145                 150                 155                 160

Gly Thr Pro Ala Ser Asn Pro Asn Ala Ser Gly Lys Gly Thr Asn Asp
                165                 170                 175

Lys Asp Gly Phe Lys Ala Gln Gly Thr Phe His Tyr Ser Leu Val Asp
            180                 185                 190

His Ser Ile Thr Gly Ala Leu Ala Cys Cys Lys Ala Ser Asn Ala Pro
        195                 200                 205

Ile Leu Thr Pro His Lys Ser Lys Cys Ala Gln Arg Lys Val Thr Asn
    210                 215                 220

Ser Ala Phe Met Arg Gln Cys Ala Asn Val Leu Pro Thr Ile Thr Asn
225                 230                 235                 240

Ser Lys Lys Leu Lys Gly Tyr Thr Thr Ile Asp Gly Gly Asp Asp Thr
                245                 250                 255

Ile Val Asp Glu Lys Thr Lys Gln Gln Cys Ser Ala Met Trp Thr Asn
            260                 265                 270

Ala Ile Ser Tyr Thr Tyr Gly Phe Cys Gln Leu Asp Tyr Asp Tyr Ala
        275                 280                 285

Ala Asp Glu Ala Ile Lys Ala Phe Gly Asp Asp Cys Ala Ala Lys Gly
    290                 295                 300

Gly Glu Ser Lys Asp Pro His Ile Gly Met Cys Leu Trp Asn Val Asp
305                 310                 315                 320

Asp Ala Ala Ala Asp Ser Ser Thr Gly
                325

<210> 26
<211> 326
<212> PRT
<213> Ustilago bromivira

<400>  26

Met Gln Arg Asn Gly Ser Trp Leu Thr Leu Leu Gly Ala Ile Cys Val
1               5                   10                  15

Ser Leu Leu Pro Ser Ile Ser Ala Asp Thr Gln Ile Ser Asp Pro Asn
            20                  25                  30

Leu Asp Val Ile Arg Gln Phe Asn Ile Val Leu Thr Asp Asn Thr Lys
        35                  40                  45

Val Ser Asp Leu Phe Cys Cys Met Gly Thr Pro Ala Glu Leu Pro Ser
    50                  55                  60

Ala Ala Gln Tyr Lys Cys Phe Glu His Gln Ala Leu Gln Met Gly Leu
65                  70                  75                  80

Ala Pro Gly Ser Arg Arg Met Gly Ile Gln Tyr Cys His His Leu Pro
                85                  90                  95

Gly Ser Thr Pro Asp Asp Ala Leu Ala Ala Phe Lys Asp Ser Cys Ser
            100                 105                 110

Lys Ala Thr Gly Glu Tyr Ile Asn Pro Asp Lys Gly Gln Cys Pro Gln
        115                 120                 125

Val Trp Pro Asn Tyr Lys Asp Asp Tyr Lys Lys Pro Ala Pro Ala Ala
    130                 135                 140

Ala Pro Ala Ala Lys Pro Gly Thr Pro Asp Pro Ala Ser Ala Thr Pro
145                 150                 155                 160

Ala Ser Asn Pro Asn Ala Ser Gly Lys Gly Thr Asn Asp Lys Asp Gly
                165                 170                 175

Phe Lys Thr Gln Gly Thr Phe His Tyr Ser Leu Val Asp His Ser Ile
            180                 185                 190

Thr Arg Ala Leu Thr Cys Cys Lys Ala Ser Asn Ala Pro Ile Val Thr
        195                 200                 205

Pro Gln Lys Tyr Lys Cys Ala Gln Arg Lys Val Thr Asn Ser Val Phe
    210                 215                 220

Met Arg Gln Cys Ala Lys Val Leu Pro Thr Ile Thr Asn Ser Lys Lys
225                 230                 235                 240

Leu Gln Gly Tyr Thr Thr Ile Asp Gly Ala Asp Asp Thr Ile Val Asp
                245                 250                 255

Glu Lys Thr Lys Gln Gln Cys Ser Ala Met Trp Thr Asn Ala Ile Ser
            260                 265                 270

Tyr Thr Tyr Gly Phe Cys Lys Leu Asp Tyr Asp His Ala Ala Asp Glu
        275                 280                 285

Ala Ile Lys Ala Phe Gly Asp Asp Cys Ala Ala Lys Gly Gly Glu Ser
    290                 295                 300

Lys Glu Pro His Ser Gly Met Cys Leu Trp Asn Val Asp Asp Ala Ala
305                 310                 315                 320

Ala Asp Ser Ser Thr Gly
                325

<210>  27
<211>  326
<212>  PRT
<213>  Sporisorium reilianum f. sp. zeae

<400>  27

Met Arg Thr Trp Leu Ala Leu Leu Gly Ala Val Ser Leu Ala Leu Val
1               5                   10                  15

Ser His Val Ala Ala Asp Asp Ala Gln Ile Thr Asn Pro Asn Leu Ala
            20                  25                  30

Val Ile Arg Thr Phe Asn Ile Ile Leu Thr Asp Asn Thr Lys Val Ser
        35                  40                  45

Asp Leu Thr Cys Cys Met Gly Val Pro Gly Glu Phe Ala Thr Ser Pro
    50                  55                  60

Gln Phe Gln Cys Phe Glu His Gln Asp Met Ala Met Gly Ser Ala Pro
65                  70                  75                  80

Gly Ser Arg Arg Leu Gly Leu Gln Tyr Cys His His Leu Pro Gly Ser
                85                  90                  95

Thr Pro Asp Glu Ala Ser Gln Ala Phe Lys Asp Met Cys Thr Lys Ala
            100                 105                 110

Thr Gly Glu Val Leu Thr Thr Asp Lys Gly Tyr Cys Pro Gln Arg Trp
        115                 120                 125

Pro Asn Tyr Asp Asp Lys Tyr Val Lys Pro Ala Pro Ala Pro Ala Pro
    130                 135                 140

Gly Ala Ala Ser Asp Pro Ala Pro Pro Pro Ala Pro Asn Pro Val Pro
145                 150                 155                 160

Asp Gly Ser Gly Lys Ser Thr Asn Asp Lys Asp Gly Phe Lys Thr Gln
                165                 170                 175

Gly Thr Phe His Tyr Ala Leu Val Asp Tyr Ala Ile Thr Arg Ser Leu
            180                 185                 190

Ala Cys Cys Lys Ala Ser Thr Lys Pro Ile Ile Lys Pro Val Ala Arg
        195                 200                 205

Ala Asp Ser Pro Lys Leu Cys Ser Cys Ala Gln Arg Lys Val Ala Ser
    210                 215                 220

Ser Val Phe Met Ser Gln Cys Ala Lys Met Leu Pro Thr Ile Thr Gly
225                 230                 235                 240

Ser Lys Lys Leu Thr Gly Tyr Ser Thr Leu Gly Gly Asp Ala Asp Ala
                245                 250                 255

Ile Val Asp Asp His Thr Lys Gln Gln Cys Ser Ala Met Trp Thr Asn
            260                 265                 270

Ala Ile Thr Tyr Thr Tyr Gly Phe Cys Gln Leu Asp Tyr Asp Gly Ala
        275                 280                 285

Arg Asp Asp Ala Ile Lys Ala Phe Gln Tyr Asp Cys Thr Ala Lys Gly
    290                 295                 300

Gly Glu Pro Lys Asp Pro His Asn Gly Met Cys Leu Trp Asn Val Asp
305                 310                 315                 320

Asp Ala Ala Ala Asp Ser
                325

<210>  28
<211>  326
<212>  PRT
<213>  Sporisorium reilianum f. sp. sorghi

<400>  28

Met Arg Thr Trp Leu Ala Leu Leu Gly Ala Val Ser Leu Ala Leu Val
1               5                   10                  15

Ser His Val Ala Ala Asp Asp Ala Gln Ile Thr Asn Pro Asn Leu Ala
            20                  25                  30

Val Ile Arg Thr Phe Asn Ile Ile Leu Thr Asp Asn Thr Lys Val Ser
        35                  40                  45

Asp Leu Thr Cys Cys Met Gly Val Pro Gly Glu Phe Ala Thr Ser Pro
    50                  55                  60

Gln Phe Gln Cys Phe Glu His Gln Asp Met Ala Met Gly Ser Ala Pro
65                  70                  75                  80

Gly Ser Arg Arg Leu Gly Leu Gln Tyr Cys His His Leu Pro Gly Ser
                85                  90                  95

Thr Pro Asp Glu Ala Ser Gln Ala Phe Lys Asp Met Cys Thr Lys Ala
            100                 105                 110

Thr Gly Glu Val Leu Thr Thr Asp Lys Gly Tyr Cys Pro Gln Arg Trp
        115                 120                 125

Pro Asn Tyr Asp Asp Lys Tyr Val Lys Pro Ala Pro Ala Pro Ala Pro
    130                 135                 140

Gly Ala Ala Ser Asp Pro Ala Pro Pro Pro Ala Pro Asn Pro Val Pro
145                 150                 155                 160

Asp Gly Ser Gly Lys Ser Thr Asn Asp Lys Asp Gly Phe Lys Thr Gln
                165                 170                 175

Gly Thr Phe His Tyr Ala Leu Val Asp Tyr Ala Ile Thr Arg Ser Leu
            180                 185                 190

Ala Cys Cys Lys Ala Ser Thr Lys Pro Ile Ile Lys Pro Val Ala Arg
        195                 200                 205

Ala Asp Ser Pro Lys Leu Cys Ser Cys Ala Gln Arg Lys Val Ala Ser
    210                 215                 220

Ser Val Phe Met Ser Gln Cys Ala Lys Met Leu Pro Thr Ile Thr Gly
225                 230                 235                 240

Ser Lys Lys Leu Thr Gly Tyr Ser Thr Leu Gly Gly Asp Ala Asp Ala
                245                 250                 255

Ile Val Asp Asp His Thr Lys Gln Gln Cys Ser Ala Met Trp Thr Asn
            260                 265                 270

Ala Ile Thr Tyr Thr Tyr Gly Phe Cys Gln Leu Asp Tyr Asp Gly Ala
        275                 280                 285

Arg Asp Asp Ala Ile Lys Ala Phe Gln Tyr Asp Cys Thr Ala Lys Gly
    290                 295                 300

Gly Glu Pro Lys Asp Pro His Asn Gly Met Cys Leu Trp Asn Val Asp
305                 310                 315                 320

Asp Ala Ala Ala Asp Ser
                325

<210>  29
<211>  327
<212>  PRT
<213>  Ustilago maydis

<400>  29

Met Gln Leu Asn Arg Thr Trp Ile Ala Leu Leu Gly Ala Ile Ser Leu
1               5                   10                  15

Thr Leu Val Ser Arg Val Ser Ala Asp Asn Pro Gln Ile Thr Asp Pro
            20                  25                  30

Asn Leu Lys Val Ile Arg Gln Phe Asn Ile Val Leu Thr Asp Asn Thr
        35                  40                  45

Lys Val Ser Asp Leu Leu Cys Cys Met Gly Thr Pro Asn Glu Leu Pro
    50                  55                  60

Ser Ile Lys Glu Tyr Gln Cys Phe Glu His Gln Asp Met Gln Met Gly
65                  70                  75                  80

Ser Ala Pro Gly Ser Arg Arg Met Gly Ile Gln Phe Cys Ser His Leu
                85                  90                  95

Pro Gly Ser Thr Pro Asp Asp Ala Leu Asn Ala Phe Lys Glu Thr Cys
            100                 105                 110

Thr Lys Ala Thr Gly Glu Val Leu Thr Pro Asp Lys Gly Tyr Cys Pro
        115                 120                 125

Gln Ile Trp Pro Asn Tyr Asp Asp Lys Tyr Val Lys Pro Ala Pro Val
    130                 135                 140

Thr Val Ala Gly Asn Gly Gly Asp Val Ala Pro Thr Pro Ala Ser Pro
145                 150                 155                 160

Pro Pro Ser Ala Pro Asp Ala Gly Gly Lys Thr Thr Asn Asp Ala Asp
                165                 170                 175

Gly Phe Lys Thr Lys Gly Thr Phe Leu Tyr Thr Leu Val Asp Tyr Ala
            180                 185                 190

Ile Thr Arg Ser Leu Ala Cys Cys Lys Gly Ser Ser Gln Pro Ile Ile
        195                 200                 205

Lys Pro Gln Lys Tyr Lys Cys Ala Gln Arg Lys Val Glu Gly Lys Val
    210                 215                 220

Phe Met Ser Gln Cys Thr Gln Met Leu Ala Ser Ile Thr Lys Ala Lys
225                 230                 235                 240

Lys Leu Glu Gly Phe Thr Thr Leu Glu Gly Asp Ser Ser Val Ile Val
                245                 250                 255

Asp Asp Lys Thr Lys Gln Gln Cys Ser Ala Thr Trp Thr Asn Ala Met
            260                 265                 270

Ser Phe Val Tyr Gly Phe Cys Gln Leu Asp Tyr Asp Gly Ala Arg Asp
        275                 280                 285

Asp Ala Ile Lys Ala Phe Gln Asp Asp Cys Ser Ala Lys Gly Gly Glu
    290                 295                 300

Ala Lys Glu Pro His Asp Gly Met Cys Leu Trp Asn Val Asp Asp Ala
305                 310                 315                 320

Ala Ile Asp Ser Thr Ser Ala
                325

<210> 30
<211> 322
<212> PRT
<213> Ustilago esculenta

<400> 30

Met Gln Ala Asn Arg Ser Trp Leu Ala Leu Leu Gly Ala Val Ser Leu
1               5                   10                  15

Arg Leu Leu Ala Gln Val Ser Ala Glu Ser Gln Val Ala Asp Pro Asn
            20                  25                  30

Leu Ala Val Ile Arg Glu Phe Asn Thr Val Leu Thr Asp Asn Thr Lys
        35                  40                  45

Leu Tyr Asp Ile Gly Cys Cys Met Asp Thr Asp Ser Thr Ile Gly Asp
    50                  55                  60

Asp Lys Ala Phe Lys Cys Phe Leu Pro Glu Asp Met Ala Leu Gly Glu
65                  70                  75                  80

Thr Pro Gly Ala Lys Arg Val Gly Leu Gln Phe Cys His His Leu Pro
                85                  90                  95

Asp Thr Thr Pro Asp Gln Ala Thr Asp Ala Phe Lys Val Gln Cys Ser
            100                 105                 110

Gln Arg Asn Gly Glu Trp Ile Glu Pro Asp Gln Arg Tyr Cys Pro Gln
        115                 120                 125

Lys Trp Glu Asn Tyr Asp Pro Asn Tyr Lys Lys Lys Thr Pro Pro Pro
    130                 135                 140

Ala Val Gln Pro Thr Pro Glu Glu Pro Lys Pro Gln Pro Lys Pro Asp
145                 150                 155                 160

Lys Asp Gly Lys Gly Thr Asn Asp Pro Asp Gly Phe Lys Thr Glu Gly
                165                 170                 175

Thr Phe His Tyr Thr Leu Val Asp Tyr Ala Ile Thr Arg Ser Leu Ala
            180                 185                 190

Cys Cys Lys Ala Ser Asn Lys Pro Ile Val Lys Pro Asn Lys Tyr Gln
        195                 200                 205

Cys Ala Gln Arg Lys Ala Thr Lys Ser Val Phe Met Lys Gln Cys Ala
    210                 215                 220

Lys Ile Leu Val Thr Ile Thr Lys Ala Gln Lys Ala Ser Gly Phe Thr
225                 230                 235                 240

Thr Ile Asn Gly Asp Ser Asp Val Val Asp Glu Ala Thr Lys Lys Gln
                245                 250                 255

Cys Ser Thr Met Trp Thr Asn Ala Ile Thr Tyr Thr Tyr Gly Phe Cys
            260                 265                 270

Gln Leu Asp Tyr Asp Phe Ala Val Asp Asp Ala Thr Lys Ala Phe Ser
        275                 280                 285

Asp Asp Cys Thr Ala Leu Gly Gly Glu Leu Lys Asp Pro His Phe Gly
    290                 295                 300

Met Cys Leu Trp Asn Val Asp Asp Ala Ala Gly Asp Ser Ser Thr Thr
305                 310                 315                 320

Ser Ser

<210>  31
<211>  323
<212>  PRT
<213>  Ustilago trichophora

<400>  31

Met Gln Leu Asn His Ser Trp Leu Ala Leu Leu Gly Ala Val Ser Leu
1               5                   10                  15

Ser Leu Leu Pro Val Val Ser Gly Asp Thr Gln Ile Ala Asp Pro Asn
            20                  25                  30

Leu Ala Val Ile Arg Asp Phe Asn Ile Val Leu Thr Asp Asn Thr Lys
        35                  40                  45

Leu Tyr Asp Leu Ala Cys Cys Gln Gly Leu Pro Gly Glu Leu Pro Ser
    50                  55                  60

Leu Asp Val Asn Tyr Lys Cys Phe Pro His Gln Ala Met Ala Met Gly
65                  70                  75                  80

Asn Ala Pro Gly Ser Arg Arg Val Gly Ile Gln Tyr Cys His His Leu
                85                  90                  95

Pro Ala Gly Lys Thr Pro Asp Glu Val Lys Arg Ala Phe Glu Asp Ala
            100                 105                 110

Cys Lys Gly Ala Thr Gly Glu Val Leu Thr Pro Asp Lys Gly Phe Cys
        115                 120                 125

Pro Gln Ile Trp Pro Asn Tyr Asp Asp Lys Tyr Lys Lys Pro Val Pro
    130                 135                 140

Pro Thr Pro Asn Pro Asp Pro Ala Pro Val Lys Pro Asp Pro Ala Pro
145                 150                 155                 160

Lys Glu Asp Gly Lys Asn Thr Asn Asp Lys Asp Gly Phe Lys Met Glu
                165                 170                 175

Gly Val Phe Asn Tyr Ala Leu Val Asp Tyr Ala Ile Thr Arg Ser Ile
            180                 185                 190

Ala Cys Cys Lys Ala Ser Asn Lys Pro Ile Met Ala Pro Ala Lys Ser
        195                 200                 205

Lys Cys Ala Gln Arg Lys Val Ser Asn Ser Val Phe Met Lys Gln Cys
    210                 215                 220

Cys Lys Met Leu Pro Thr Ile Thr Lys Ala Lys Gln Ile Thr Gly Tyr
225                 230                 235                 240

Ser Ala Ile Gly Asp Ala Ser Glu Ile Val Asp Glu Ala Thr Lys Gln
                245                 250                 255

Gln Cys Ser Ala Met Trp Thr Asn Ala Val Ser Tyr Thr Tyr Gly Phe
            260                 265                 270

Cys Gln Leu Asp Tyr Asp Phe Ala Ala Glu Glu Ala Ser Lys Ser Phe
        275                 280                 285

Glu Asp Ala Cys Thr Ser Lys Gly Gly Thr Met Lys Asp Pro His Asn
    290                 295                 300

Gly Met Cys Leu Trp Asn Val Asp Asp Ala Ala Ser Asp Ser Gly Ser
305                 310                 315                 320

Ser Asp Ser

<210>  32
<211>  329
<212>  PRT
<213>  Ustilago hordei (host: oats)

<400>  32

Met Gln Arg Asn Gly Ser Trp Leu Thr Leu Leu Gly Ala Ile Cys Val
1               5                   10                  15

Ser Leu Leu Pro Ser Ile Ser Ala Thr Thr Gln Ile Ser Asp Pro Asn
            20                  25                  30

Leu Ala Val Ile Leu Gln Tyr Asn Ile Val Leu Thr Asp Asn Thr Lys
        35                  40                  45

Val Asn Asp Leu Phe Cys Cys Met Gly Thr Ser Ala Glu Ile Pro Ser
    50                  55                  60

Ala Ala Glu Tyr Lys Cys Phe Glu His Gln Ala Leu Asp Met Gly Ser
65                  70                  75                  80

Ala Pro Gly Ser Arg Arg Met Gly Ile Gln Phe Cys His His Leu Pro
                85                  90                  95

Gly Ser Lys Pro Glu Glu Ala Val Ala Ala Phe Lys Asp Ser Cys Ser
            100                 105                 110

Lys Tyr Pro Gly Glu Tyr Ile Asn Pro Asp Lys Gly Gln Cys Pro Gln
        115                 120                 125

Ile Trp Pro Asn Tyr Lys Asp Asp Tyr Lys Lys Pro Ala Pro Ala Pro
    130                 135                 140

Ala Pro Val Thr Pro Ala Pro Lys Pro Gly Thr His Asp Pro Ala Ser
145                 150                 155                 160

Gly Thr Pro Ala Ser Asn Pro Asn Ala Ser Gly Lys Gly Thr Asn Asp
                165                 170                 175

Lys Asp Gly Phe Lys Ala Gln Gly Thr Phe His Tyr Ser Leu Val Asp
            180                 185                 190

His Ser Ile Thr Gly Ala Leu Ala Cys Cys Lys Ala Ser Asn Ala Pro
        195                 200                 205

Ile Leu Thr Pro His Lys Ser Lys Cys Ala Gln Arg Lys Val Thr Asn
    210                 215                 220

Ser Ala Phe Met Arg Gln Cys Ala Asn Val Leu Pro Thr Ile Thr Asn
225                 230                 235                 240

Ser Lys Lys Leu Gln Gly Tyr Thr Thr Ile Asp Gly Gly Asp Asp Ile
                245                 250                 255

Ile Val Asp Glu Lys Thr Lys Gln Gln Cys Ser Ala Met Trp Thr Asn
            260                 265                 270

Ala Ile Ser Tyr Thr Tyr Gly Phe Cys Gln Leu Asp Tyr Asp Tyr Ala
        275                 280                 285

Ala Asp Glu Ala Ile Lys Ala Phe Gly Asp Asp Cys Ala Ala Lys Gly
    290                 295                 300

Gly Glu Ser Lys Asp Pro His Ile Gly Met Cys Leu Trp Asn Val Asp
305                 310                 315                 320

Asp Ala Ala Ala Asp Ser Ser Thr Gly

                325

<210>  33
<211>  334
<212>  PRT
<213>  Ustilago tritici

<400>  33

Met Gln Leu Lys His Ser Trp Leu Gly Leu Leu Gly Ala Val Ser Leu
1               5                   10                  15

Gly Leu Ile Pro Ser Ile Ala Ala Asp Val Gln Ile Ser Asp Pro Asn
            20                  25                  30

Leu Ala Val Ile Arg Glu Tyr Asn Val Val Leu Thr Asp Asn Thr Lys
        35                  40                  45

Val Ser Asp Leu Leu Cys Cys Met Gly Ala Asp Asn Glu Leu Lys Ser
    50                  55                  60

Leu Ala Gln Phe Gln Cys Phe Asp His Gln Asp Met Leu Met Gly Arg
65                  70                  75                  80

Ala Pro Gly Ser Arg Arg Val Gly Ile Gln Tyr Cys Ser His Leu Pro
                85                  90                  95

Gly Ser Thr Pro Asp Glu Ala Ser Ala Ser Phe Lys Thr Ala Cys Thr
            100                 105                 110

Gly Gly Asn Gly Glu Val Leu Thr Pro Asp Lys Gly Tyr Cys Pro Gln
        115                 120                 125

Val Trp Pro Asn Tyr Lys Asp Gly Tyr Lys Lys Pro Ala Pro Ala Pro
    130                 135                 140

Ala Pro Ala Pro Asp Ala Gly Ala Gly Gly Ala Asp Ala Asp Ala Pro
145                 150                 155                 160

Pro Pro Ser Asp Ala Pro Gly Pro Asp Gly Lys Gly Thr Asn Asp Lys
                165                 170                 175

Asp Gly Phe Lys Thr Thr Gly Thr Phe His Tyr Thr Leu Val Asp Tyr
            180                 185                 190

Ala Ile Thr Arg Ser Leu Ala Cys Cys Lys Ala Ser Asn Lys Pro Leu
        195                 200                 205

Val Lys Pro Thr Thr Ala Ala Gln Thr Lys Thr Tyr Met Cys Ala Gln

210                 215                 220

Arg Lys Val Ala Ser Ser Thr Phe Met Ser Gln Cys Ala Lys Thr Leu
225                 230                 235                 240

Ala Thr Ile Thr Lys Ala Lys Pro Leu Ala Gly Tyr Ser Thr Leu Asn
                245                 250                 255

Gly Gly Asp Ser Thr Ile Val Asp Asp Lys Thr Lys Ser Gln Cys Ser
            260                 265                 270

Ala Met Trp Thr Asn Ala Met Ser Phe Thr Tyr Gly Phe Cys Gln Leu
        275                 280                 285

Asp His Asp Gly Ala Arg Asp Asp Ala Ile Lys Ala Phe Gln Asp Asp
    290                 295                 300

Cys Thr Ala Lys Gly Gly Glu Ser Lys Asp Pro His Met Gly Met Cys
305                 310                 315                 320

Leu Trp Asn Val Asp Asp Ala Ala Gln Asp Ser Thr Gly Ala
                325                 330

<210>  34
<211>  143
<212>  PRT
<213>  Melanopsichium pennsylvanicum

<400>  34

Met Arg Ile Lys Arg Thr Ser Thr Tyr Ser Phe Gly Met Arg Leu Gln
1               5                   10                  15

Ile Leu Phe Met Leu Ile Ser Thr Ile Thr Ile Ser Ser Met Val Val
            20                  25                  30

Val Gln Val Glu Ala Lys Thr Gln Arg Val Gly Asp Glu Cys Asn Trp
        35                  40                  45

Ser Lys Asn Cys Gln Gln Asn Ala Asp Gly Val Gly Pro Ala Trp Ala
    50                  55                  60

Asn Gly Glu Ile Thr Cys Ala Ile Pro Gln Asn Gln Ser Pro Glu Thr
65                  70                  75                  80

Cys Ser Ser His Arg Lys Thr Arg Asn Ala Phe Trp Gly Thr Cys Lys
                85                  90                  95

Ala Val Gly Thr Leu Thr Asp Thr Glu Tyr Gly Cys Pro Cys Gly Val

100                 105                 110

His Lys Gly Asn Cys Pro Ile Ser Pro Met Phe Glu Arg Ile Phe Asp
        115                 120                 125

Pro Ala Gly Phe Met Gln Lys Ala Tyr Asp Tyr Ile Asn Asn Asn
    130                 135                 140

<210>  35
<211>  132
<212>  PRT
<213>  Sporisorium scitamineum

<400>  35

Met Arg Arg Pro Thr Thr Phe Leu Ser Trp Ala Phe Gly Leu Leu Val
1               5                   10                  15

Ile Leu Leu Ala Val Thr Met Val Glu Ala Ala Thr Gln Arg Val Gly
            20                  25                  30

Asp Glu Cys Asn Tyr Lys Lys Asn Cys Gln Ser His Ala Asp Gly Val
        35                  40                  45

Gly Pro Asp Trp Ala Lys Gly Glu Ile Thr Cys Ala Val Pro Gln Ala
    50                  55                  60

Asn Ala Pro Glu Thr Cys Gly Ser Gly Asn Lys Lys Asp Arg Asn Arg
65                  70                  75                  80

Phe Tyr Gly Thr Cys Lys Ala Val Gly Thr Leu Glu Ala Thr Glu Tyr
                85                  90                  95

Gly Cys Ala Cys Gly Val His Gly Gly Asp Cys Pro Glu Thr Ser Pro
            100                 105                 110

Phe Ser Arg Ile Phe Trp Pro Ala Asp Trp Met Asp Lys Tyr Trp Gln
        115                 120                 125

Ala Val Gly His
    130

<210>  36
<211>  131
<212>  PRT
<213>  Ustilago hordei (host: barley)

<400>  36

Met Phe Asn Arg Ile Pro Ile Leu Ala Leu Ala Leu Thr Leu Ala Val
1               5                   10                  15

Leu Phe Ser Asn Leu Met Met Val Val Glu Ala Lys Thr Gln Arg Val
            20                  25                  30

Gly Asp Glu Cys Asn Trp Lys Arg Asn Cys Gln Gln Tyr Ala Asp Gly
        35                  40                  45

Val Gly Pro Asp Trp Ala Lys Gly Glu Ile Thr Cys Ala Val Pro Gln
    50                  55                  60

Gly Asn Ser Pro Glu Thr Cys Gly Ser Gly Asp Asp Arg Arg Thr Asp
65                  70                  75                  80

Phe Tyr Gly Thr Cys Lys Ala Val Gly Thr Leu Glu Ala Thr Glu Tyr
                85                  90                  95

Gly Cys Pro Cys Gly Val His Gly Ala Asp Cys Pro Ala Thr Ser Pro
            100                 105                 110

Phe Ser Arg Ile Phe Trp Pro Gln Asp Trp Met Lys Asn Ala Trp Ala
        115                 120                 125

Gln Ile Asn
    130

<210>  37
<211>  131
<212>  PRT
<213>  Ustilago bromivira

<400>  37

Met Phe Asn Arg Ile Gln Ile Leu Ala Leu Ala Leu Thr Leu Ala Val
1               5                   10                  15

Leu Phe Ser Asn Leu Met Met Val Val Glu Ala Gly Thr Gln Arg Val
            20                  25                  30

Gly Asp Glu Cys Asn Trp Lys Lys Asn Cys Gln Ser Tyr Ala Asp Gly
        35                  40                  45

Val Gly Pro Asp Trp Ala Lys Gly Glu Ile Thr Cys Ala Val Pro Gln
    50                  55                  60

Ser Asn Ser Pro Glu Thr Cys Gly Ser Gly Asp Asp Asn Arg Ser Ala
65                  70                  75                  80

Phe Tyr Gly Thr Cys Lys Ala Val Gly Thr Leu Glu Ala Thr Glu Tyr
                85                  90                  95

Gly Cys Gly Cys Gly Val His Lys Gly Pro Cys Pro Ala Thr Ser Pro
        100                 105                 110

Phe Asn Arg Ile Phe Trp Pro Gln Asp Trp Met Lys Asn Ala Trp Ala
        115                 120                 125

Gln Val His
    130

<210>  38
<211>  130
<212>  PRT
<213>  Sporisorium reilianum f. sp. zeae

<400>  38

Met Arg Arg Gly Thr Val Ser Leu Thr Ala Leu Val Ala Leu Leu Val
1               5                   10                  15

Val Ala Ser Gly Leu Val Glu Ala Lys Thr Gln Arg Val Gly Asp Glu
            20                  25                  30

Cys Asn Trp Lys Lys Asn Cys Gln Ala Gln Ala Asp Gly Val Gly Pro
        35                  40                  45

Ala Trp Ala Asn Gly Ala Ile Thr Cys Ala Val Pro Gln Ala Asn Ala
    50                  55                  60

Pro Glu Thr Cys Gly Tyr Gly Asp Lys Lys Ala Arg Asn Arg Phe Tyr
65                  70                  75                  80

Gly Thr Cys Lys Ala Val Gly Thr Leu Glu Ala Thr Glu Tyr Gly Cys
                85                  90                  95

Ala Cys Gly Val His Gly Val Asp Cys Pro Ala Asp Ser Pro Phe Ser
            100                 105                 110

Arg Ile Phe Trp Pro Gln Asp Trp Leu Asn Lys Phe Trp Ala Asp Val
        115                 120                 125

His Asn
    130

<210>  39
<211>  130
<212>  PRT
<213>  Sporisorium reilianum f. sp. sorghi

<400>  39

Met Arg Arg Gly Thr Val Ser Leu Thr Ala Leu Val Ala Leu Leu Val
1               5                   10                  15

Val Ala Ser Gly Leu Val Glu Ala Lys Thr Gln Arg Val Gly Asp Glu
            20                  25                  30

Cys Asn Trp Lys Lys Asn Cys Gln Ala Gln Ala Asp Gly Val Gly Pro
        35                  40                  45

Ala Trp Ala Asn Gly Ala Ile Thr Cys Ala Val Pro Gln Ala Asn Ala
    50                  55                  60

Pro Glu Thr Cys Gly Tyr Gly Asp Lys Lys Ala Arg Asn Arg Phe Tyr
65                  70                  75                  80

Gly Thr Cys Lys Ala Val Gly Thr Leu Glu Ala Thr Glu Tyr Gly Cys
                85                  90                  95

Ala Cys Gly Val His Gly Val Asp Cys Pro Ala Asp Ser Pro Phe Ser
            100                 105                 110

Arg Ile Phe Trp Pro Gln Asp Trp Leu Asn Lys Phe Trp Ala Asp Val
        115                 120                 125

His Asn
    130

<210>  40
<211>  129
<212>  PRT
<213>  Ustilago maydis

<400>  40

Met His Arg Pro Thr Ser Leu Tyr Val Thr Leu Ile Cys Leu Leu Gly
1               5                   10                  15

Thr Val Met Ser Val Arg Ala Ala Thr Gln Arg Val Gly Asp Ser Cys
            20                  25                  30

Ser Tyr Lys Gln Asn Cys Gln Asp Trp Ala Glu Gly Val Gly Pro Asp
        35                  40                  45

Trp Ala Lys Gly Ala Ile Thr Cys Ala Val Pro Gln Asp Gly Lys Pro
    50                  55                  60

Glu Lys Cys Gly Ser Gly Asp Lys Lys Gly Arg Asp Glu Phe Ser Gly
65                  70                  75                  80

Val Cys Lys Ala Val Gly Thr Leu Ser Ala Thr Glu Tyr Gly Cys Ala
                85                  90                  95

Cys Gly Val His Lys Ala Asp Cys Pro Glu Thr Ser Pro Phe Ser Arg
            100                 105                 110

Ile Phe Trp Pro Gln Asp Trp Leu Glu Thr Ala Trp Ser Gln Val His
        115                 120                 125

His

<210>  41
<211>  126
<212>  PRT
<213>  Ustilago esculenta

<400>  41

Met Ala Arg Thr Phe Arg Leu Val Ala Leu Leu Val Leu Val Thr Leu
1               5                   10                  15

Phe Val Gly Gly Phe Val Glu Ala Ala Thr Gln Arg Val Gly Asp Asp
            20                  25                  30

Cys Asn Trp Lys Gln Asn Cys Gln Ser Asn Ile Pro Gly Ile Gly Pro
        35                  40                  45

Ala Trp Ala Asn Gly Glu Ile Thr Cys Ala Val Pro Ser Asp Arg Lys
    50                  55                  60

Pro Glu Thr Cys Thr Asn Lys Arg Glu Gln Arg Asn Glu Phe Ser Gly
65                  70                  75                  80

Thr Cys Lys Pro Val Gly Gln Leu Met Glu His Glu Tyr Gly Cys Pro
                85                  90                  95

Cys Gly Val His Gly Gly Asp Cys Gly Ser Ile Ala Met Val Ser Arg
            100                 105                 110

Ile Phe Trp Pro Lys Asp Trp Leu Gln Gly Leu Trp Lys Asn
        115                 120                 125

<210>  42
<211>  138
<212>  PRT
<213>  Ustilago trichophora

<400>  42

Met Ala Ile Phe Gly Lys Arg Asn Val Ser Pro Leu Val Leu Ile Val
1               5                   10                  15

Ile Leu Thr Leu Phe Asn Gly Leu Phe Trp Gln Val Val Glu Ala His
            20                  25                  30

Gly Glu Val Gln Ile Gly Gly Glu Cys Asn Trp Arg Lys Asn Cys Gln
        35                  40                  45

Gln Phe Val Asp Gly Ala Gly Phe Asp Trp Ala Asn His Glu Val Thr
    50                  55                  60

Cys Ala Val Pro Ala Thr Gln Ser Pro Glu His Cys Asn Ser Asn Lys
65                  70                  75                  80

His Glu Arg Asn Asp Phe Trp Gly Thr Cys Lys Ala Ile Gly Thr Leu
                85                  90                  95

Asp Pro Gln Glu Tyr Gly Cys Pro Cys Gly Val His Gly Pro Asp Cys
            100                 105                 110

Pro Phe Val Ser Thr Leu Asn Arg Ile Phe Trp Pro Gln Asn Trp Ile
        115                 120                 125

Lys Asp Ala Trp Ala Asn Leu Gln His His
    130                 135

<210>  43
<211>  131
<212>  PRT
<213>  Ustilago hordei (host: oats)

<400>  43

Met Phe Asn Arg Ile Ser Ile Leu Ser Leu Ala Leu Thr Leu Ala Val
1               5                   10                  15

Leu Phe Ser Asn Leu Met Met Val Val Glu Ala Lys Thr Gln Arg Val
            20                  25                  30

Gly Asp Glu Cys Asn Trp Lys Arg Asn Cys Gln Gln Tyr Ala Asp Gly
        35                  40                  45

Val Gly Pro Asp Trp Ala Lys Gly Glu Ile Thr Cys Ala Val Pro Gln
    50                  55                  60

Gly His His Pro Glu Thr Cys Gly Ser Gly Asp Asp Arg Arg Thr Asp
65                  70                  75                  80

Phe Tyr Gly Thr Cys Lys Ala Val Gly Thr Leu Glu Ala Thr Glu Tyr
                85                  90                  95

Gly Cys Pro Cys Gly Val His Gly Ala Asp Cys Pro Ala Thr Ser Pro
            100                 105                 110

Phe Ser Arg Ile Phe Trp Pro Gln Asp Trp Met Lys Asn Ala Trp Ala
        115                 120                 125

Gln Ile His
    130

<210>  44
<211>  127
<212>  PRT
<213>  Ustilago tritici

<400>  44

Met Arg Leu Ala Thr Val Ala Phe Ala Asn Val Val Phe Gly Met Phe
1               5                   10                  15

Ala Leu Val Asp Ala Ala Thr Gln Arg Val Gly Asp Glu Cys Asn Trp
            20                  25                  30

Ser Lys Asn Cys Ala Ser Pro Val Ala Gly Val Gly Pro Ala Trp Ala
        35                  40                  45

Asn Gly Glu Val Thr Cys Ala Val Pro Gln Pro Asn Lys Pro Glu Ser
    50                  55                  60

Cys Gly Ser Gly Asp Lys Lys His Arg Asn Asp Phe Tyr Gly Ile Cys
65                  70                  75                  80

Lys Ala Val Gly Thr Leu Asp Ala Thr Glu Tyr Gly Cys Ala Cys Gly
                85                  90                  95

Val His Asn Gly Asn Cys Pro Thr Thr Ser Pro Phe Ser Arg Ile Phe
            100                 105                 110

Trp Pro Gln Asp Trp Leu Pro Asn Ala Trp Lys Asp Met His Ser
        115                 120                 125

<210>  45
<211>  1143
<212>  PRT
<213>  Melanopsichium pennsylvanicum

<400>  45

Met Arg Val Glu Leu Leu Leu Leu Leu Ser Leu Ala Cys Leu Ala Phe

1               5                   10                  15

Thr Ala Gln Ala Ala Pro Gly Pro Ala Gln Asp Ala Leu Arg His His
            20                  25                  30

Lys Phe Lys Lys Arg Ser Phe Glu Ser Lys Arg Glu Lys Ala Lys Leu
        35                  40                  45

Lys Asn Ala Ser His Leu Gly Gln Ser Leu His Lys His His Glu Ser
    50                  55                  60

Ser Pro Lys Lys Thr Arg Gly Val Pro Pro Leu Thr Glu Ile Gln Val
65                  70                  75                  80

Ala Gln Glu Ala Thr Ser Asn Leu Lys Leu Gln Glu His Val Leu Ala
                85                  90                  95

Arg Leu Glu Asp Glu Ile Ala Asp Ile Glu Ala Lys Gly Arg Gln Ala
            100                 105                 110

Tyr Lys Asp Lys Lys Gln Ser Met His Val Glu Asp Ala Leu His Ile
        115                 120                 125

Ala Glu Thr Gly Arg Pro Pro Pro Lys Phe Pro Thr Ile Thr Arg Ile
    130                 135                 140

Gly Ser Ala Ile Gly Ile Thr Ser Gly Ile Leu Asn Met Val Asn Phe
145                 150                 155                 160

Leu Phe Ala Ala Lys Thr Thr Ala Leu Ser Thr Ser Gln Phe Ser Ala
                165                 170                 175

Pro Pro Thr Pro Pro Thr Leu Ile Asp Thr Ala Ala Arg Trp Asp Gly
            180                 185                 190

Ser His Tyr Asp Ile Met Pro Val Pro Glu Val Ser His Asp Ser Ser
        195                 200                 205

Gly Ser Gly Glu Lys Gln Arg Arg Ser Ile Pro Val Ile Arg Ala Gln
    210                 215                 220

Lys Tyr Leu Lys Lys Arg Ser Leu Ser Asp Lys Val Asp Ala Ser Ser
225                 230                 235                 240

His Val Gln Asn Arg Met Val Lys Arg Lys Val Ser Arg Asp Arg Glu
                245                 250                 255

His His Lys Gly Gln Ala Glu Asn Val Lys Lys Arg Met Leu Ala Ala
            260                 265                 270

Thr Gly Ile Gly Leu Ile Leu Gly Ala Ala Ile Val Pro Gly Val Pro
        275                 280                 285

Ser Met Val Met Gly Lys Lys Pro Val Ile Asp Lys Pro Pro Phe Asp
    290                 295                 300

Pro Tyr Asp Pro Asn Met Leu Lys Gln Lys Asp Glu Phe Ala Ala Ala
305                 310                 315                 320

Ala Ala Asp Pro Asn Lys Ala Leu Pro Val Leu Ala Pro Pro Gln Ser
                325                 330                 335

Gln Ala Phe Gly Asn Ala Leu Gly Asn Pro Ala Ser Gly Pro Pro Asp
            340                 345                 350

Met Ala Asp Ile Arg Phe Pro Asp Ser Ala Tyr Ser Pro Glu Leu Gln
        355                 360                 365

Ile Gln Pro Gln Pro His Tyr Val Pro Tyr Tyr Gln Gln Ala Phe Ser
    370                 375                 380

Thr Ser Thr Gly Ser Ala His Asp Tyr Ser Gly Asp Ser Thr Ala Ser
385                 390                 395                 400

Ser Thr Ser Ala Ser Pro Gln Pro Val Leu Arg Lys Arg Ala Leu Val
                405                 410                 415

Ser Glu His Lys Thr Asp Val Lys Asn Asp Lys Ala Lys Ala Arg Ser
            420                 425                 430

Arg Ile Asp Lys Arg Met Phe Ala Gly Gly Pro Trp Gly Arg Val Ala
        435                 440                 445

Thr Ala Val Thr Ile Gly Ser Val Ile Pro Leu Met Ile Thr Thr Leu
    450                 455                 460

Lys His Thr Arg Trp Arg Gln Tyr Asn Thr Arg Asp Arg Asp Ile Leu
465                 470                 475                 480

Lys Asp Leu Asp Asp Thr Pro Thr Tyr Gly Ala Val Gly Val Pro Gly
                485                 490                 495

Arg Ile Pro Gly Gln Leu Ala Thr Asn Ala Met Pro Gly Tyr Thr Asn
            500                 505                 510

Tyr Val Thr Gln Leu Gln Thr Gln Gln Leu Arg Gln Gln Ala Asn Ala
        515                 520                 525

Gln Thr Ala Ser Gln Gln Gln Tyr Leu Ala Asp Gln Ala Ala Ala Leu
    530                 535                 540

Arg Glu Tyr Gln Arg Asn Gln Met Ala Leu Ser Ser Ser Thr Thr Ile
545                 550                 555                 560

Asn Gly Glu Pro Ser Thr Thr Asp Ala Gln Pro Val Leu Arg Lys Arg
                565                 570                 575

Asp Ser His Arg Ala Gln Thr Arg Leu His Lys Arg Gly Thr Leu Phe
            580                 585                 590

Asn Met Gly Leu Gly Ala Ala Gly Leu Leu Leu Gly Tyr Ala Gln Ser
        595                 600                 605

Val Thr Met Phe Pro Gly Asp Lys Asp Glu Glu Arg Asp Gln Lys Lys
    610                 615                 620

Arg Glu Arg Lys Gln Gln Glu Ala Glu Ala Met Ala Ala Gln Gln Ala
625                 630                 635                 640

Ala Ser Tyr Gly Gly Ser Pro Pro Gly Ser Gly Gly Gly Gly Ala Gly
                645                 650                 655

Ser Phe Ala Gly Leu Pro Val Ala Ala Gly Asp Pro Val Gly Gly Val
            660                 665                 670

Gly Ile Ala Tyr Pro Ala Ala Gly Asp Gly Tyr Ala Ser Thr Gly Val
        675                 680                 685

Tyr Ser Ala Ser Gly Ala Gly Gly Ala Gly Ala Gly Gly Tyr Gly Ala
    690                 695                 700

Ala Pro Thr Tyr Arg Ser Gly Ile Ser Met Val Gly Ser Ser Tyr Tyr
705                 710                 715                 720

Gly Gly Gly Gly Gly Gly Gly Gly Gly Phe Thr Gly Gly Gly Gly Ser
                725                 730                 735

Pro Ser Leu Ala Ser Ser Leu Asn Ser Ala Ala Gly Met Gln Ala Gly
            740                 745                 750

Ser Tyr Ser Ala Gly Gly Asn Thr Val Ser Leu Arg Lys Arg Asp Leu
        755                 760                 765

Pro Lys Thr Thr Gly Asn Glu His Glu Ala Gln Ser Leu Ser Lys Arg
    770                 775                 780

Ala Pro Gly Ile Gly Thr Phe Leu Ser Val Gly Gly Thr Ala Met Phe
785                 790                 795                 800

Val Gly Thr Leu Gly Ala Asn Leu Val Val Ala Glu Lys Gln Lys His
                805                 810                 815

Lys Pro Val Pro Leu Val Thr Pro Asn Ala Asn Thr Leu Gly Pro Met
            820                 825                 830

Val Pro Gly Gly Asp Leu Tyr Thr Gln Tyr Leu Met Ser Lys Arg Pro
        835                 840                 845

Ala Thr Thr Gly Ala Gly Ala Gly Phe Gly Val Gly Ala Gly Gln Met
    850                 855                 860

Gly Gly Tyr Ser Met Ala Ala Gly Thr Pro Ala Ser Ser Asn Ser Leu
865                 870                 875                 880

Ala Thr Thr Ser Pro Gly Phe Thr Pro Ala Tyr Ser Gly Arg Tyr Ala
                885                 890                 895

Asn Gly Ala Pro Pro Ile Leu Asn Gly Val Phe Asn Asn Gly Gly Thr
            900                 905                 910

Thr Ile Ser Pro Gln Ser Tyr Asp Glu Ala Asp Gly Pro Ala Pro Val
        915                 920                 925

Leu Lys Lys Arg Ser Thr Gln Thr Phe Asp Lys Arg Ala Pro Leu Gly
    930                 935                 940

Phe Ala Gly Ala Val Glu Ala Glu Ser Ala Ala Ala Ser Leu Phe Thr
945                 950                 955                 960

Lys Gly Pro Val Leu Ala Glu Ser Ala Gly Gly Ala Ala Ser Leu Leu
                965                 970                 975

Ser Lys Ala Pro Val Val Thr Glu Thr Gly Val Gly Ala Thr Ser Arg
            980                 985                 990

Leu Gly Val Gly Ala Ala Asn Ser  Arg Phe Ser Ala Leu  Pro Gly Tyr
        995                 1000                1005

Ser Arg  Phe Pro Arg Glu Gly  Ala Phe Ala Ser Glu  Ala Gly Leu

1010                    1015                    1020

Gly Ala  Phe Gly Arg Asn Ser  Met Ala Gly Glu Ser  Ala Tyr Val
    1025                    1030                    1035

Asn Thr  Phe Arg Thr Asn Pro  Ala Glu Trp Gly Thr  Ser Arg Pro
    1040                    1045                    1050

Ala Thr  Ile Pro Asn Phe Gly  Arg Thr Gly Glu Ala  Gly Lys Pro
    1055                    1060                    1065

Ala Thr  Leu Glu Gly Leu Ser  Asp Gly Val Gly Arg  Pro Ala Pro
    1070                    1075                    1080

Asn Gly  Leu Thr Gln Gln Gln  Gly Met Gly Gly Asp  Pro Lys Ala
    1085                    1090                    1095

Ala Ala  Phe Ala Gly Glu Met  Gly Ser Leu Asp Arg  Ala Val Glu
    1100                    1105                    1110

Glu Pro  Val Lys Lys Gly Met  Ser Phe Lys Lys Ala  Val Ala Ile
    1115                    1120                    1125

Leu Gly  Gly Gly Ala Gly Ala  Ser Met Thr Phe Asn  Gly Leu Gly
    1130                    1135                    1140

<210>  46
<211>  1093
<212>  PRT
<213>  Sporisorium scitamineum

<400>  46

Met Arg Val Gly Val Val Leu Leu Phe Cys Leu Val Cys Leu Ala Phe
1               5                   10                  15

Thr Ala Gln Ala Ala Pro Ala Pro Ala Thr Asp Thr Leu His Ala Pro
            20                  25                  30

Arg Glu Lys Asp Arg Asn Asp Asp Lys His Lys Asn Gly Lys His Gly
        35                  40                  45

Arg His His His Arg His Glu Ser His Lys Lys Asp Thr Asp His Ser
    50                  55                  60

Ser Asn Ala Ala Ser Arg Leu Lys Ser Arg Gly Ala Pro Pro Gly Ala
65                  70                  75                  80

Ala Ala Asp Leu Glu Lys Val Leu Leu Pro His Pro Gln Asp Asp Leu

                85                  90                  95

Phe Thr Lys Ile Ser Glu Glu Val Thr Ala Arg Glu Gln Ala Ile Arg
            100                 105                 110

Asn Arg Lys Lys Ala Glu Leu His His Ile Gln Ile Glu Asp Ala Ile
        115                 120                 125

His Val Ala Lys His Gly Lys Glu Pro Pro Lys Phe Pro Glu Ile Thr
    130                 135                 140

Arg Ala Ala Ala Gly Ile Gly Leu Thr Ala Ala Ile Leu Asn Val Val
145                 150                 155                 160

Asn Phe Ile Tyr Ser Ala Lys Thr Thr Ala Leu Ala Thr Thr Gln Phe
                165                 170                 175

Ser Thr Pro Pro Thr Pro Pro Thr Leu Ile Asp Thr Ala Ala Leu Trp
            180                 185                 190

Asp Gly Asn Gln Tyr Thr Leu Met Pro Leu Pro Glu Glu Lys Pro Asp
        195                 200                 205

Ala Ala Ala Ile Gly Lys Val Lys Arg Gly Leu Ala Glu Asp Ser Ile
    210                 215                 220

Asn Arg Trp Leu Arg Lys Arg His Leu Ala Ala Glu Ser Thr Ala Glu
225                 230                 235                 240

Gly Glu Thr Gln Glu Pro Ile Leu Ser Lys Arg Trp Ile Val Ser Ser
                245                 250                 255

Val Val Gly Ala Gly Ile Gly Ala Phe Leu Ala Pro Gly Phe Phe Ser
            260                 265                 270

Thr Ile Arg Gly Lys Lys Pro Glu Val Asp Leu Pro Pro Phe Asp Pro
        275                 280                 285

Tyr Asp Pro Asn Ile Leu Lys Pro Lys Asp Ser Phe Ser Ala Ala Ala
    290                 295                 300

Ala Thr Leu Asp Gln Pro Leu Pro Ala Val Gln Ala Ser Ala Leu Asn
305                 310                 315                 320

Gly Gly Ala Ala Pro Gly Ala Pro Pro Asn Val Asp Asn Thr Arg Phe
                325                 330                 335

Pro Asn Asp Ala Ser Tyr Leu Pro Val Thr Ala Asp Ser Arg Thr Gln
            340                 345                 350

Pro Leu Val Asn Pro Ser Thr Arg Gln Ser Leu Ala Val Asn Pro Gly
        355                 360                 365

Gly Ala Tyr Val Gly Ser Gly Ser Glu Ser Ser Ser Ser Ser Glu Ala
    370                 375                 380

Gln Ala Val Leu Arg Lys Arg Thr Val Val Pro Ala Ser His Ser Ser
385                 390                 395                 400

Asp Gly Glu Ile Asp Ala Asn Ser Ser Ser Glu Leu Asp Lys Arg Phe
                405                 410                 415

Leu Phe Ser Gly Gly Thr Ala Ser Lys Ile Ala Gly Ala Ile Thr Val
            420                 425                 430

Gly Ser Ile Leu Pro Leu Val Tyr Gly Gly Val Thr His Ala Arg Trp
        435                 440                 445

Arg Thr Glu Asn Pro Arg Asp Arg Asp Ile Leu Lys Glu Leu Asp Asp
    450                 455                 460

Thr Pro Thr Tyr Gly Ala Val Gly Val Pro Gly Arg Gln Pro Gly Gln
465                 470                 475                 480

Leu Ala Thr Asn Ala Met Pro Gly Tyr Ser Asp Tyr Val Leu Lys Leu
                485                 490                 495

Glu Gln Gln Gln Ala Gln Gln Gln Ala Ser Leu Gln Ala Ala Gln Gln
            500                 505                 510

Pro Gln Met Pro Gly Thr Gln Leu Glu Gln Ala Ser Pro Thr Thr Thr
        515                 520                 525

Asp Ala Thr Gly Gly Gly Gly Asp Pro Val Leu Arg Arg Arg Asp Asn
    530                 535                 540

Gln Ser His Leu Glu Lys Arg Gly Leu Leu Ala Asn Gly Ala Leu Ala
545                 550                 555                 560

Leu Phe Gly Ala Gly Leu Gly Tyr Ala Gln Ser Ala Ser Gln Phe Pro
                565                 570                 575

Thr Asp Arg Asp Glu Glu Lys Asp Lys Lys Lys Arg Glu Ala Arg Lys
            580                 585                 590

Lys Gln Gln Glu Met Met Ala Ala Gln Gln Gln Gln Ala Gly Tyr Pro
        595                 600                 605

Gly Ala Glu Gly Ala Ala Val Gly Gly Asp Pro Leu Ala Gly Ile Pro
    610                 615                 620

Glu Lys Pro Leu Pro Gly Thr Ala Asp Ala Asn Gly Ser Ala Ala Gly
625                 630                 635                 640

Ala Asp Pro Leu Ala Gly Ile Pro Glu Lys Pro Leu Pro Gly Thr Val
                645                 650                 655

Ser Gly Thr Gly Ile Ser Asp Tyr Ser Asp Gly Gly Ala Ser Ala Gly
            660                 665                 670

Ala Val Tyr Thr Pro Thr Val Gly Ser Gly Ser Gly Ser Tyr Leu Pro
        675                 680                 685

Ala Thr Tyr Gly Gly Ala Gly Phe Gly Ser Ser Thr Tyr Val Ser Gly
    690                 695                 700

Gly Gly Phe Thr Gly Gly Ala Gly Gly Ala Ser Ser Leu Gly Ala Ser
705                 710                 715                 720

Leu Asn Ala Ala Ala Gly Tyr Arg Thr Ser Ser Ser Gly Gly Gly Gly
                725                 730                 735

Gly Gly Glu Ser Val Leu Lys Lys Arg Asp Thr Ser Ser Ala Ala Asp
            740                 745                 750

His Lys Leu Glu Lys Arg Thr Pro Gly Phe Gly Ser Ala Leu Thr Ile
        755                 760                 765

Gly Gly Thr Thr Met Phe Ala Gly Thr Leu Leu Ala Asn Val Phe Val
    770                 775                 780

Ser Gln Lys Gln Lys Ser Lys Pro Val Pro Leu Val Thr Pro Asn Ala
785                 790                 795                 800

Asp Thr Arg Gly Pro Met Ile Pro Gly Gly Asp Leu Tyr Thr Gln Tyr
                805                 810                 815

Leu Glu Ser Lys Arg Ile Gln Pro Ser Gly Ala Gly Gly Ser Ala Leu
            820                 825                 830

Ala Gly Gly Ala Pro Ala Ala Ile Asn Tyr Gly Ala Ser Met Pro Ala
        835                 840                 845

Thr Val Asn Thr Ala Pro Val Ser Arg Thr Asp Thr Pro Ser Ser Gly
    850                 855                 860

Ser Gly Gly Ala Ala Ile Pro Asp Ser Arg Ser Ser Thr Asp Ala Ser
865                 870                 875                 880

Asp Ser Ala Thr Thr Gly Ala Leu Asp Pro Val Leu Lys Lys Arg Asp
                885                 890                 895

Met Gln Leu Glu Leu Gln Lys Arg Ser Pro Lys Ala Phe Ala Val Glu
            900                 905                 910

Leu Glu Ala Ala Ala Pro Gln Leu Leu Arg Ala Gly Ala Gly Ala Ala
        915                 920                 925

Ser Val Leu Gly Lys Gly Ala Ala Ala Thr Glu Ala Gly Val Gly Ala
    930                 935                 940

Ala Ser Ala Ile Gly Arg Gly Gly Ala Ala Ala Ser Glu Ala Gly Ala
945                 950                 955                 960

Gly Ala Phe Ser Arg Val Gly Met Ala Gly Arg Gly Gly Gly Met Tyr
                965                 970                 975

Arg Phe Pro Glu Ser Ala Arg Pro Glu Leu Ser Arg Phe Gly Thr Thr
            980                 985                 990

Ala Ala Glu Gly Gly Phe Ser Ser Trp Glu Arg Val Gln Leu Arg Asn
        995                 1000                1005

Asn Pro Phe Gly Asn Gly Phe Ala Ala Ser Val Tyr Arg Pro Gly
    1010                1015                1020

Gly Ala Ala Leu Gly Ala Ala Arg Pro Ala Met Val Ser Asp Val
    1025                1030                1035

Lys Val Ala Thr Gly Thr Ala Gly Thr Pro Ala Thr Val Lys Gly
    1040                1045                1050

Ala Ala Ala Ala Glu Ala Glu Gly Glu Ala Ala Glu Thr Asn Lys
    1055                1060                1065

Gly Met Thr Pro Leu Lys Ala Met Gly Thr Ile Ser Ala Gly Ala
    1070                1075                1080

Gly Ala Thr Met Met Met Asn Gly Leu Gly

1085                1090

<210>  47
<211>  1106
<212>  PRT
<213>  Ustilago hordei (host: barley)

<400>  47
   Met Arg Phe Glu Leu Leu Leu Phe Leu Ser Leu Ala Cys Leu Ala Phe
   1               5                   10                  15
   Thr Ala Gln Thr Ala Pro Ala Ser Ala Lys Asp Ala Leu Gln Glu Gln
               20                  25                  30
   Lys His Lys Gln Arg Asp Ser Glu Pro His Lys His Lys Pro Lys Ser
           35                  40                  45
   Gly Pro Arg Leu His Arg His Pro Lys Gln His His Tyr Lys Ser Ser
       50                  55                  60
   His Leu Gly His Gly Asn Asn Leu Lys Tyr Thr Arg Gly Leu Thr Ser
   65                  70                  75                  80
   Tyr Ser Val Glu Val Val Gln Asp Thr Lys Thr Leu Phe Val Pro Glu
                   85                  90                  95
   His Glu Ser Lys Leu Arg Ala Lys Ile Asp Ala Ile Glu Lys Thr Ser
               100                 105                 110
   Leu Glu Lys Glu Lys Leu Lys Gln His Glu Ile Glu Arg Ala Asp Ile
           115                 120                 125
   Ile Glu Thr Ala Lys His Gly Ala Val Pro Pro Lys Phe Pro Leu Val
       130                 135                 140
   Thr Arg Ile Ser Ser Phe Ile Gly Ile Val Ala Ala Ile Ala Asn Val
   145                 150                 155                 160
   Ala Asn Phe Ile Phe Ala Val Lys Thr Thr Ala Leu Ala Thr Lys Gln
                   165                 170                 175
   Phe Ser Ala Pro Pro Thr Pro Pro Thr Phe Ile Asp Thr Ala Gly Arg
               180                 185                 190
   Trp Asp Gly Gln Gln Tyr Gln Leu Met Pro Leu Pro Gln Glu Pro Asp
           195                 200                 205
   Asp Thr Lys Ser Ala Arg Lys Lys Arg Ser Leu Pro Asp Glu Cys Ala
   210 215 220
   Thr Val Pro Pro Ala His Lys Arg Met Val Lys Arg Lys Arg Pro His
   225 230 235 240
   His Thr Glu Gln Ser Gln Lys Leu Asp Glu Ala Gln His Asp Pro Lys
   245 250 255
   Leu Ser Lys Arg Phe Val Thr Ala Ala Gly Val Gly Leu Leu Leu Gly
   260 265 270
   Gly Ala Ile Ser Pro Gly Met Pro Ser Leu Val Met Gly Trp Lys Pro
   275 280 285
   Glu Val Asp Lys Thr Pro Phe Asp Pro Tyr Asp Pro Asn Val Leu Lys
   290 295 300
   Pro Lys Asp Pro Phe Ala Val Ala Ala Ala Asp Pro Asp His Ala Leu
   305 310 315 320
   Pro Ala Leu Pro Met Arg Lys Pro Ala Pro Ser Gly Ser Ser Asn Gly
   325 330 335
   Leu Pro Ser Leu Arg Asn Ile Val Phe Pro Ala Ser Ala Tyr Ser Ser
   340 345 350
   Ser Ala Thr Ser Ile Leu Gln Gln Gln Gln Gln Gln Leu Thr Ala Ala
   355 360 365
   Thr Ala Gln Gln Gly Ser Ala Ile Ser Ser Ala Asn Val Pro Ala Arg
   370 375 380
   Gly Ser Ser Thr Ala Ser Ala Ser Asp Glu Val Gln Thr Val Leu Arg
   385 390 395 400
   Lys Arg Ser Val Glu Ala Lys His Ser Thr Glu Ile Glu His Gln Val
   405 410 415
   Arg Glu Ser Ser Lys Pro Leu Glu Lys Arg Phe Leu Phe Gly Lys Gly
   420 425 430
   Pro Val Gly Lys Val Thr Gly Ala Val Ala Leu Gly Ser Ile Leu Tyr
   435 440 445
   Val Leu Tyr Lys Gly Val Ser His Ser Arg Trp Arg Gln Glu Asn Pro
   450 455 460
   Arg Asp Arg Asp Ile Leu Lys Asp Leu Asp Asp Thr Pro Met Tyr Gly
   465                 470                 475                 480
   Ala Val Gly Val Pro Lys Arg Ile Pro Gly Gln Leu Ala Thr Lys Ala
                   485                 490                 495
   Met Pro Gly Tyr Ser Asp Tyr Val Ala Lys Leu Ala Ala Gln Gln Gln
               500                 505                 510
   Thr Gln Gln Gln Ala Ala Met Gln Gln Gln Gln Trp Gln Gln Leu Ala
           515                 520                 525
   Thr Thr Gln Ala Gln Ala Arg Asp Ala Leu Thr Glu Gln Ala Ile Ala
       530                 535                 540
   Ala Gln Ser Ser Ser Gly Gly Val Gly Val Ala Ala Ala Asp Ala Gly
   545                 550                 555                 560
   Ser Asn Asp Pro Val Leu Arg Arg Arg Ser Ser Asp Ala Ser Ser Ala
                   565                 570                 575
   Val Ser Ala Lys Pro Val Phe Gly Asp Ala Gly Gln Thr Arg Leu Asp
               580                 585                 590
   Lys Arg Gly Gly Arg Ala Val Leu Ile Gly Gly Gly Leu Thr Val Ile
           595                 600                 605
   Gly Ile Ala Leu Gly Tyr Leu Gln Ser Ala Thr Met Phe Pro Thr Asp
       610                 615                 620
   Arg Asp Glu Glu Lys Asp Lys Lys Lys Arg Glu Arg Lys Lys Gln Gln
   625                 630                 635                 640
   Glu Gln Gln Leu Leu Met Gln Gln Gln Gln Ala Leu Val Gln Gly Gln
                   645                 650                 655
   Ala Ala Gln Ser Ala Gly Met Gly Tyr Ala Pro Ser Gly Ala Ser Ala
               660                 665                 670
   Pro Leu Val Ser Ser Ser Asp Gly Gly Gly Ile Ala Phe Pro Ser Ser
           675                 680                 685
   Ala Tyr Ser Ser Ser Gly Ser Thr Tyr Pro Pro Ser Met Gly Ala Gly
       690                 695                 700
   Ser Phe Ser Ser Ser Ala Gly Thr Glu Ser Ser Thr Gly Gly Ala Ser
   705                 710                 715                 720
   Ser Ala Ser Ala Ala Leu Asn Ala Ala Ala Gly Tyr Ser Ser Ala Gly
                   725                 730                 735
   Gly Ser Ala Ser Gly Ser Val Leu Lys Lys Arg Asp Ile Ser Gly His
               740                 745                 750
   Pro Asp Gln Glu Ser Glu Thr Arg Ser Leu Ser Lys Arg Ser Pro Gly
           755                 760                 765
   Val Gly Ser Phe Leu Ser Ile Ala Gly Thr Thr Met Phe Phe Gly Thr
       770                 775                 780
   Leu Ala Gly Asn Leu Ile Val Gly Glu Lys Glu Lys His Lys Pro Val
   785                 790                 795                 800
   Pro Tyr Val Thr Pro Asn Ala Asp Thr Arg Gly Pro Met Val Pro Gly
                   805                 810                 815
   Gly Glu Leu Tyr Thr Gln Tyr Leu Gln Ser Lys Arg Pro Pro Ser Thr
               820                 825                 830
   Ser Gly Asp Ala Phe Gly Asp Val Gly Ala Ser Leu Ala Thr Gly Thr
           835                 840                 845
   Pro Ala Ser Ile Thr Gly Gln Asn Met Ala Pro Ala Ser Ala Pro Ala
       850                 855                 860
   Ser Ile Tyr Gly Gln Thr Pro Val Val Gly Ser Gln Leu Gly Ala Asn
   865                 870                 875                 880
   Thr Gly Thr Ala Ala Ser Pro Asn Ala Pro Asp Val Gly Arg Gly Gly
                   885                 890                 895
   Glu Ala Asp Pro Val Leu Lys Lys Arg His Ser Pro Arg Leu Glu Lys
               900                 905                 910
   Arg Val Pro Leu Ser Phe Ala Gly Ala Arg Ala Glu Ala Ala Ala Gly
           915                 920                 925
   Leu Leu Thr Lys Gly Val Pro Met Val Glu Ala Glu Val Gly Ala Ala
       930                 935                 940
   Ser Ala Leu Ser Arg Ser Ala Ala Leu Ser Glu Ala Gly Ala Ala Ser
   945                 950                 955                 960
   Arg Phe Ala Pro Thr Thr Arg Phe Ser Ser Leu Ser Gly Val Arg Gly
                   965                 970                 975
   Gly Ala Ala Ala Ser Glu Ala Gly Phe Ala Gly Leu Gly Arg Ala Ser
               980                 985                 990
   Met Ala Gly Glu Gly Ser Thr Ala  Ala Arg Ile Tyr Gln  Pro Asn Ala
           995                 1000                 1005
   Ala Ala  Val Gly Ser Ala Arg  Pro Ala Met Leu Ser  Asp Leu Gly
       1010                 1015                 1020
   Gly Ala  Ser Gly Arg Ala Gly  Thr Pro Ala Thr Phe  Ala Arg Ser
       1025                 1030                 1035
   Ala Ser  Thr Arg Gln Leu Ser  Pro Ala Glu Met Gln  Gly Gln Gln
       1040                 1045                 1050
   Gln Arg  Val Ala Ala Gly Gln  Ala Ala Asp Met Asn  Pro Gly Val
       1055                 1060                 1065
   Lys Gly  Met Ser Gly Gln Ala  Pro Glu Glu Ala Lys  Lys Gly Trp
       1070                 1075                 1080
   Thr Ser  Gln Lys Val Leu Gly  Ile Val Gly Gly Ala  Ala Gly Ala
       1085                 1090                 1095
   Thr Met  Leu Phe Pro Gly Ala  Gly
       1100                 1105

<210>  48
<211>  1112
<212>  PRT
<213>  Ustilago bromivira

<400>  48

Met Arg Phe Glu Leu Leu Leu Phe Leu Ser Leu Ala Cys Leu Ala Phe
1               5                   10                  15

Thr Ala Gln Thr Ala Pro Ala Ser Ala Lys Asp Ala Leu Gln Glu Gln
            20                  25                  30

Lys His Lys Gln Arg Asp Ser Glu Pro His Lys His Lys Pro Lys Ser
        35                  40                  45

Gly Pro His Leu His Arg His Pro Lys Gln His His Asp Asn His His
    50                  55                  60

Lys Ser Ser His Pro Asp His Gly Asn Asn Pro Lys His Thr Arg Ser
65                  70                  75                  80

Val Leu Ser Glu Ser Ala Gln Val Val Gln Glu Thr Lys Thr Leu Leu
                85                  90                  95

Val Pro Glu Tyr Glu Ser Lys Ile Gln Ala Lys Ile Gly Arg Ile Glu
            100                 105                 110

Lys Thr His Leu Glu Lys Glu Lys Glu Lys Gln His Glu Ile Glu Arg
        115                 120                 125

Ala Asp Ile Ile Glu Thr Ala Lys His Gly Ala Val Pro Pro Lys Phe
    130                 135                 140

Pro Leu Val Thr Arg Ile Ser Ser Phe Ile Gly Ile Thr Ala Ala Val
145                 150                 155                 160

Ala Asn Ile Ala Asn Phe Ile Phe Ser Ala Lys Thr Thr Ala Leu Ala
                165                 170                 175

Thr Glu Gln Phe Ser Ala Pro Pro Thr Pro Pro Thr Phe Ile Asp Thr
            180                 185                 190

Ala Gly Arg Trp Asp Gly Gln Gln Tyr Gln Leu Met Pro Leu Pro Gln
        195                 200                 205

Glu Pro Asp Asp Thr Lys Ser Ala Arg Lys Lys Arg Ser Leu Pro Asp
    210                 215                 220

Glu Ser Ala Thr Val Pro Pro Ala His Lys Arg Met Val Lys Arg Lys
225                 230                 235                 240

Arg Pro His His Thr Gly Gln Ser Gln Lys Leu Asp Glu Ala Gln His
                245                 250                 255

Asp Pro Lys Leu Ser Lys Arg Phe Ala Thr Ala Ala Gly Val Gly Leu
            260                 265                 270

Leu Leu Gly Gly Ala Leu Ser Pro Gly Ile Pro Ser Leu Val Met Gly
        275                 280                 285

Trp Glu Pro Glu Val Asp Lys Thr Pro Phe Asn Pro Tyr Asp Pro Asn
    290                 295                 300

Val Leu Lys Pro Lys Asp Pro Phe Ala Val Ala Ala Ala Asp Pro Asp
305                 310                 315                 320

His Ala Leu Pro Ala Leu Pro Met Gln Asn Pro Ala Pro Ser Gly Ser

                325                 330                 335

Ser Asn Ala Leu Pro Ser Leu Arg Asn Ile Val Phe Pro Ala Ser Ala
            340                 345                 350

Tyr Ser Ser Ser Ala Thr Ser Ile Leu Gln Gln Gln Gln Gln Gln Leu
        355                 360                 365

Thr Ala Ala Thr Ala Gln Gln Gly Ser Ala Ile Ser Ser Ala Asn Val
    370                 375                 380

Pro Ala Arg Gly Ser Ser Pro Ala Ser Asp Lys Val Gln Thr Val Leu
385                 390                 395                 400

Arg Lys Arg Ser Val Glu Ala Lys His Ser Thr Lys Val Glu His Gln
                405                 410                 415

Glu Ser Glu Ser Ser Thr Pro Leu Glu Lys Arg Phe Leu Phe Gly Lys
            420                 425                 430

Gly Pro Trp Gly Lys Val Ser Gly Gly Val Ala Leu Gly Ser Ile Ala
        435                 440                 445

Tyr Val Leu Tyr Lys Gly Val Ser His Ala Arg Trp Arg Gln Glu Asn
    450                 455                 460

Pro Arg Asp Arg Glu Ile Leu Lys Asp Leu Asp Asp Thr Pro Met Tyr
465                 470                 475                 480

Gly Ala Val Gly Val Pro Lys Arg Ile Pro Gly Gln Leu Ala Ile Lys
                485                 490                 495

Ala Met Pro Gly Tyr Ser Asp Tyr Val Ala Lys Leu Ala Ala Gln Gln
            500                 505                 510

Gln Thr Gln Gln Gln Ala Ala Met Gln Arg Gln Gln Arg Gln His Leu
        515                 520                 525

Ala Thr Thr Gln Ala Gln Ala Arg Asp Ala Leu Thr Glu Gln Ala Ile
    530                 535                 540

Ala Ala Gln Ser Ser Ser Gly Gly Val Gly Val Ala Ala Ala Asp Ala
545                 550                 555                 560

Gly Gly Asn Asp Pro Val Leu Arg Arg Arg Ser Ser Asp Ala Ser Ser
                565                 570                 575

Ala Val Ser Ala Lys Arg Val Phe Cys Asp Ala Gly Gln Thr Arg Leu
            580                 585                 590

Asp Lys Arg Gly Asn Lys Leu Leu Ile Glu Gly Gly Leu Ile Ala Val
        595                 600                 605

Gly Leu Ala Leu Gly Tyr Leu Gln Ser Ala Thr Met Phe Pro Ser Asp
    610                 615                 620

Arg Asp Glu Glu Lys Asp Lys Gln Lys Arg Glu Arg Lys Lys Gln Glu
625                 630                 635                 640

Glu Gln Gln Leu Leu Met Gln Gln Gln Gln Ala Thr Ala Leu Ala Pro
                645                 650                 655

Ala Ala Gln Ser Ala Gly Met Gly Tyr Ala Pro Ser Gly Ala Ala Pro
            660                 665                 670

Asp Ala Ser Ala Pro Pro Val Ser Ser Ser Asn Gly Gly Gly Phe Ala
        675                 680                 685

Phe Ser Ser Ser Ala Tyr Gly Ser Ser Gly Ser Thr Ser Pro Pro Ser
    690                 695                 700

Met Gly Ala Gly Ser Phe Ser Pro Ser Ala Gly Thr Gly Ser Ser Thr
705                 710                 715                 720

Gly Gly Ala Ser Ser Ala Ser Ala Ala Leu Asn Ala Ala Ala Gly Tyr
                725                 730                 735

Ser Ser Thr Gly Gly Ser Ala Ser Gly Ser Val Leu Lys Lys Arg Asp
            740                 745                 750

Ile Ser Gly His Pro Asp Gln Lys Ser Asp Thr Arg Ser Leu Ser Lys
        755                 760                 765

Arg Ser Pro Gly Ile Gly Ser Phe Ile Pro Ile Val Gly Thr Thr Met
    770                 775                 780

Phe Leu Gly Thr Leu Ala Gly Asn Ile Val Val Gly Glu Asn Gln Lys
785                 790                 795                 800

His Lys Pro Val Pro Tyr Val Thr Pro Asn Ala Asp Thr Arg Gly Pro
                805                 810                 815

Met Val Pro Gly Gly Glu Leu Tyr Thr Gln Tyr Leu Gln Ser Lys Arg
            820                 825                 830

Pro Pro Ser Thr Ser Gly Asp Ala Leu Gly Asp Val Gly Ala Gly Leu
        835                 840                 845

Ala Met Gly Thr Pro Ala Ser Ile Asn Gly Gln Asn Met Gly Pro Ala
    850                 855                 860

Ser Ala Pro Ala Ser Ile Tyr Gly Gln Thr Pro Val Val Gly Ser Gln
865                 870                 875                 880

Leu Gly Ala Asn Thr Gly Thr Ala Ala Ser Pro Asp Ala Pro Asp Val
                885                 890                 895

Gly Arg Gly Gly Gly Ala Asp Pro Ala Leu Lys Lys Arg His Ser Pro
            900                 905                 910

Arg Leu Gly Lys Arg Val Pro Leu Ser Phe Ala Gly Ala Ala Met Glu
        915                 920                 925

Ala Glu Thr Ala Ala Gly Leu Leu Thr Lys Gly Ile Pro Met Val Glu
    930                 935                 940

Ala Glu Val Gly Ala Ala Ser Ala Leu Ser Arg Ser Ala Ala Leu Ser
945                 950                 955                 960

Glu Ala Gly Ala Ala Ser Arg Phe Ala Pro Val Thr Arg Phe Ser Ser
                965                 970                 975

Leu Ser Gly Val Arg Gly Gly Ala Ala Ala Ser Glu Ala Gly Phe Ala
            980                 985                 990

Gly Leu Gly Arg Ala Ser Met Ala Gly Glu Gly Ser Thr Ala Ala Arg
        995                 1000                1005

Ile Tyr Gln Pro Asn Ala Ala Ala Val Gly Ser Ala Arg Pro Ala
    1010                1015                1020

Met Leu Ser Asp Leu Gly Gly Ala Ser Gly Arg Ala Gly Thr Pro
    1025                1030                1035

Ala Thr Phe Ala Arg Ser Ala Ser Thr Gly Gln Leu Ser Pro Ala
    1040                1045                1050

Glu Met Gln Gly Leu Gln Gln Lys Val Ala Pro Gly Arg Ala Ala
    1055                1060                1065

Asp Met Asn Pro Gly Val Lys Glu Met Pro Gly Gln Ala Pro Glu
    1070                1075                1080

Glu Ala Lys Lys Gly Trp Thr Leu Gln Lys Ala Met Gly Thr Val
    1085                1090                1095

Gly Thr Ala Ser Gly Ala Thr Met Leu Phe Asn Gly Ala Gly
    1100                1105                1110

<210> 49
<211> 1072
<212> PRT
<213> Sporisorium reilianum f. sp. zeae

<400> 49

Met Arg Val Glu Leu Leu Phe Leu Phe Cys Leu Ala Cys Leu Ala Phe
1               5                   10                  15

Thr Ala Gln Ala Ala Pro Thr Ser Ala Thr Glu Ala Leu Arg Val Pro
            20                  25                  30

Arg His Lys Glu Arg Ser Asn His Asp Lys His Asp Lys His Glu Asp
        35                  40                  45

Ser Lys His Gly His His His His Arg His Glu Ser His Arg Glu Lys
    50                  55                  60

Glu Thr Ser His Ser Ser His Ala Ala Ser Arg Leu Lys Asn Arg Ala
65                  70                  75                  80

Glu Ile Pro Gly Glu Val Ala Ala Ala Lys Gly Ala Ala Glu Arg Leu
                85                  90                  95

Leu Pro Gln Pro Gln Pro Glu Leu Tyr Ser Ser Leu Ser Asp Gln Val
            100                 105                 110

Ala Glu Gln Glu Arg Leu Ile Arg Gln Arg Lys Thr Leu Lys Glu Gln
        115                 120                 125

His Val Gln Val Glu Asp Ala Ile His Val Ala Arg Tyr Gly Lys Glu
    130                 135                 140

Pro Pro Lys Phe Pro Glu Ile Thr Arg Ala Ala Ala Ala Ile Gly Leu
145                 150                 155                 160

Thr Ala Ala Leu Leu Asn Val Ala Asn Phe Leu Phe Ser Ala Lys Thr
                165                 170                 175

Thr Ala Leu Ala Thr Thr Gln Phe Ser Thr Pro Pro Thr Pro Pro Thr
            180                 185                 190

Leu Ile Asp Thr Ala Ala Leu Trp Asp Gly Lys Gln Tyr Arg Leu Met
        195                 200                 205

Pro Leu Pro Glu Gly Lys Ser Ala Ala Ile Ala Ala Ala Gly Lys Val
    210                 215                 220

Lys Arg Ser Leu Val Gln Glu Ser Ile Asn Arg Gln Leu Arg Lys Arg
225                 230                 235                 240

His Leu Val Ala Lys Arg Trp Ile Val Ser Thr Leu Val Gly Thr Ala
                245                 250                 255

Leu Gly Ala Ala Leu Ala Pro Gly Phe Thr Ser Glu Ile Arg Gly Lys
            260                 265                 270

Lys Ala Glu Val Asp Lys Pro Pro Phe Asp Pro Tyr Asp Pro Asn Met
        275                 280                 285

Leu Lys Pro His Asp Ser Phe Ser Ala Ala Ala Ala Ser Val Asp Gln
    290                 295                 300

Pro Leu Pro Ala Val Pro Ala Ser Ala Leu Thr Gly Gly Ser Ala Pro
305                 310                 315                 320

Gly Thr Pro Pro Asn Val Asp Ser Ile Arg Phe Pro Val Gln Ala Ser
                325                 330                 335

Tyr Leu Pro Val Ser Ala Asp Pro Arg Thr Gln Pro Leu Val Asn Pro
            340                 345                 350

Gly Ser Ser Tyr Val Ala Ser Ser Ser Arg Thr Ser Ala Ser Ser Glu
        355                 360                 365

Ala Gln Pro Val Leu Arg Lys Arg Ser Val Val Ala Ala Ser His Ser
    370                 375                 380

Ser Asp Gly Asp Ser Asp Gly Gly Ser Ser Ser Glu Leu Asp Lys Arg
385                 390                 395                 400

Phe Leu Phe Gly Gly Gly Gly Val Ser Gly Lys Leu Met Gly Ala Ala
                405                 410                 415

Thr Leu Gly Ser Val Leu Pro Leu Leu Tyr Lys Gly Val Thr His Ala
            420                 425                 430

Arg Trp Arg Thr Ser Asn Pro Arg Asp Arg Asp Ile Leu Lys Glu Leu

435                 440                 445

Asp Asp Thr Pro Thr Tyr Gly Ala Val Gly Val Pro Gly His Gln Pro
    450                 455                 460

Gly Gln Leu Ala Thr Asn Ala Met Pro Gly Tyr Thr Asp Tyr Val Ser
465                 470                 475                 480

Gln Leu Glu Gln Gln Gln Ala Arg Ala Gln Arg Ala Ala Asp Thr Gln
                485                 490                 495

Ala Ala Ser Leu Ala Gly Thr Pro Glu Ala Ala Thr Ser Ala Ala Ala
            500                 505                 510

Thr Gly Thr Ala Gly Gly Gly Gly Gly Asp Pro Val Leu Arg Arg Arg
        515                 520                 525

Ala Asp His Ser His Leu Glu Lys Arg Gly Trp Leu Leu Gly Gly Ser
    530                 535                 540

Leu Thr Val Leu Gly Gly Leu Leu Gly Tyr Ala Gln Ser Ala Thr Gln
545                 550                 555                 560

Phe Pro Thr Asp Arg Asp Glu Glu Lys Asp Lys Lys Lys Arg Glu Ala
                565                 570                 575

Arg Lys Lys Gln Glu Glu Met Met Ala Ala Gln Gln Gln Gln Ala Gly
            580                 585                 590

Tyr Pro Gly Ala Ala Gly Ala Gly Ala Gly Ala Gly Ala Gly Ala Asp
        595                 600                 605

Pro Leu Ala Gly Ile Pro Glu Arg Pro Leu Pro Gly Thr Met Gly Gly
    610                 615                 620

Gly Gly Leu Ser Asp Gly Ser Ala Tyr Gly Ala Thr Thr Gly Gly Gly
625                 630                 635                 640

Gly Gly Gly Gly Tyr Ala Pro Ala Ala Gly Ser Gly Ser Gly Met Tyr
                645                 650                 655

Ser Pro Thr Thr Tyr Gly Gly Ser Gly Tyr Gly Ser Ser Ser Ser Ser
            660                 665                 670

Ser Leu Tyr Ser Thr Asp Gly Ala Ser Thr Gly Gly Thr Gly Gly Gly
        675                 680                 685

Ser Ser Leu Gly Ala Ser Leu Asn Ala Ala Ala Gly Tyr Gly Ala Ser
    690                 695                 700

Ser Ser Ala Gly Ser Gly Gly Gly Gly Gly Ser Val Leu Lys Lys Arg
705                 710                 715                 720

Ser Pro Ser Ser Ala Ala Ala Glu His Glu Leu Glu Lys Arg Thr Pro
                725                 730                 735

Gly Phe Gly Ser Ala Leu Thr Ile Gly Gly Thr Ala Met Phe Gly Gly
            740                 745                 750

Thr Leu Leu Ala Asn Leu Trp Val Ser Gly Lys Gln Arg Ser Lys Pro
        755                 760                 765

Val Pro Val Val Thr Pro Asn Ala Asp Thr Arg Gly Pro Met Ile Pro
    770                 775                 780

Gly Gly Asp Leu Tyr Ala Gln Tyr Leu Ala Ser Lys Arg Ile Gln Pro
785                 790                 795                 800

Gly Ser Ala Gly Gly Thr Gly Leu Ala Gly Gly Gly Ala Pro Ala Ala
                805                 810                 815

Val Asn Tyr Gly Ala Ser Met Pro Ala Ala Val Asn Thr Ala Ser Ala
            820                 825                 830

Pro Asp Glu Ser Ser Ala Ala Val Val Pro Gly Thr Gly Pro Ser Ser
        835                 840                 845

Asp Ala Ser Asp Ser Ala Ala Thr Gly Gly Leu Asp Pro Val Leu Lys
    850                 855                 860

Lys Arg Asp Thr Gln Pro Gly Leu Gln Lys Arg Ser Pro Glu Ala Pro
865                 870                 875                 880

Leu Ile Glu Leu Glu Ser Gln Val Pro Asn Leu Leu Lys Ala Ala Gly
                885                 890                 895

Ala Ala Ser Gly Leu Ala Arg Gly Ala Ala Ala Ser Glu Ala Gly Val
            900                 905                 910

Gly Ala Ala Ser Met Leu Gly Arg Gly Gly Ala Ala Ala Ser Glu Ala
        915                 920                 925

Gly Ala Gly Ala Phe Ser Arg Val Ser Ala Val Gly Arg Glu Gly Gly
    930                 935                 940

Gly Phe Ala Arg Leu Ser Glu Ser Ala Arg Pro Gly Leu Ser Arg Tyr
945                 950                 955                 960

Gly Gly Thr Thr Ala Ala Glu Gly Gly Leu Ser Ser Phe Glu Arg Thr
                965                 970                 975

Pro Leu Ser Ser Asn Pro Phe Glu Thr Ser Phe Ala Ser Ser Phe Tyr
            980                 985                 990

Arg Pro Gly Gly Thr Ala Leu Ser  Gly Ala Ala Arg Pro  Ala Met Val
        995                 1000                 1005

Ser Asp  Phe Lys Val Ala Thr  Gly Thr Ala Gly Ala  Pro Ala Thr
    1010                 1015                 1020

Ile Glu  Gly Ala Ala Ala Ala  Glu Ala Glu Ala Gly  Ala Asp Ala
    1025                 1030                 1035

Ala Ala  Ala Ala Thr Gly Lys  Arg Lys Gly Met Thr  Pro Leu Lys
    1040                 1045                 1050

Met Val  Ser Ala Val Gly Ser  Gly Ala Gly Val Thr  Met Met Met
    1055                 1060                 1065

Asn Gly  Leu Gly
    1070

<210>  50
<211>  1078
<212>  PRT
<213>  Sporisorium reilianum f. sp. sorghi

<400>  50
   Met Arg Val Glu Leu Leu Phe Leu Phe Cys Leu Ala Cys Leu Ala Phe
   1               5                   10                  15
   Thr Ala Gln Ala Ala Pro Thr Ser Ala Thr Glu Ala Leu Arg Val Pro
               20                  25                  30
   Arg His Lys Glu Arg Ser Asn His Asp Lys His Asp Lys His Glu Asp
           35                  40                  45
   Ser Lys His Gly His His His His Arg His Glu Ser His Arg Glu Lys
       50                  55                  60
   Glu Thr Ser His Ser Ser His Ala Ala Ser Ser Leu Lys Asn Arg Ala
   65                  70                  75                  80
   Glu Ile Pro Gly Glu Val Ala Ala Ala Lys Lys Ala Ala Glu Arg Leu
   85 90 95
   Leu Pro Gln Pro Gln Pro Glu Leu Tyr Ser Ser Leu Ser Asp Lys Val
   100 105 110
   Ala Glu Gln Glu Arg Leu Ile Arg Gln Arg Lys Lys Leu Lys Glu Gln
   115 120 125
   His Val Gln Val Glu Asp Ala Ile His Val Ala Arg Tyr Gly Lys Glu
   130 135 140
   Pro Pro Lys Phe Pro Glu Ile Thr Arg Ala Ala Ala Ala Ile Gly Leu
   145 150 155 160
   Thr Ala Ala Leu Leu Asn Val Ile Asn Phe Leu Phe Ser Ala Lys Thr
   165 170 175
   Thr Ala Leu Ala Thr Thr Gln Phe Ser Ala Pro Pro Thr Pro Pro Thr
   180 185 190
   Leu Ile Asp Thr Ala Ala Leu Trp Asp Gly Lys Gln Tyr Arg Leu Met
   195 200 205
   Pro Leu Pro Glu Glu Lys Ser Ala Ala Ile Ala Ala Ala Gly Lys Ala
   210 215 220
   Lys Arg Ser Leu Val Gln Glu Ser Ile Asn Arg Gln Leu Arg Lys Arg
   225 230 235 240
   His Leu Val Ala Lys Arg Trp Ile Val Ser Thr Leu Val Gly Thr Ala
   245 250 255
   Leu Gly Ala Ala Leu Ala Pro Gly Phe Thr Ser Gln Ile Arg Gly Lys
   260 265 270
   Lys Ala Glu Ala Asp Lys Pro Pro Phe Asp Pro Tyr Asp Pro Asn Met
   275 280 285
   Leu Lys Pro His Asp Pro Phe Ser Ala Ala Ala Ala Ser Val Asp Gln
   290 295 300
   Pro Leu Pro Thr Val Pro Ala Ser Ala Leu Thr Gly Gly Ser Ala Pro
   305 310 315 320
   Arg Thr Pro Pro Asn Val Asp Asn Ile Arg Phe Pro Val Gln Ala Ser
   325 330 335
   Asp Leu Pro Val Ser Ala Glu Pro Arg Thr Gln Pro Leu Val Asn Pro
   340 345 350
   Gly Ser Ser Tyr Val Ala Ser Gly Ser Arg Thr Ser Ala Ser Ser Glu
   355 360 365
   Ala Gln Pro Val Leu Arg Lys Arg Ser Val Val Ala Ala Ser His Ser
   370 375 380
   Ser Asp Gly Asp Ser Asp Gly Gly Ser Ser Ser Glu Leu Asp Lys Arg
   385 390 395 400
   Phe Leu Phe Gly Gly Gly Gly Val Ser Gly Lys Leu Met Gly Ala Ala
   405 410 415
   Thr Leu Gly Ser Val Leu Pro Leu Leu Tyr Glu Gly Val Thr His Ala
   420 425 430
   Arg Trp Arg Thr Ser Asn Pro Arg Asp Arg Asp Ile Leu Lys Glu Leu
   435 440 445
   Asp Asp Thr Pro Thr Tyr Gly Ala Val Gly Val Pro Ser His Gln Pro
   450 455 460
   Gly Gln Leu Ala Thr Asn Ala Met Pro Gly Tyr Thr Asp Tyr Val Ser
   465 470 475 480
   Gln Leu Glu Gln Gln Gln Ala Arg Ala Gln Arg Ala Ala Asn Thr Gln
   485 490 495
   Ala Ala Ser Gln Ala Gly Thr Gln Glu Ala Gly Thr Pro Ala Ala Ala
   500 505 510
   Thr Glu Thr Ala Gly Gly Gly Gly Gly Asp Pro Val Leu Arg Arg Arg
   515 520 525
   Asp Glu Gln Ser His Leu Glu Lys Arg Gly Trp Leu Leu Gly Gly Ser
   530 535 540
   Leu Thr Val Leu Gly Gly Leu Leu Gly Tyr Ala Gln Ser Ala Thr Gln
   545 550 555 560
   Phe Pro Thr Asp Arg Asp Glu Glu Lys Asp Lys Lys Lys Arg Glu Ala
   565 570 575
   Arg Lys Lys Gln Glu Glu Met Leu Ala Ala Gln Gln Gln Gln Ala Gly
   580 585 590
   Tyr Pro Gly Ala Ala Glu Ala Gly Ala Gly Ala Gly Ala Gly Ala Gly
   595 600 605
   Ala Gly Ala Gly Ala Asp Pro Leu Ala Gly Ile Pro Glu Arg Pro Leu
   610 615 620
   Pro Gly Thr Thr Gly Gly Gly Gly Leu Ser Asp Gly Ser Ala Tyr Gly
   625 630 635 640
   Ala Thr Thr Gly Gly Gly Gly Gly Gly Gly Tyr Ala Pro Ala Ala Gly
   645 650 655
   Ser Gly Ser Gly Met Tyr Ser Pro Thr Thr Tyr Gly Gly Ser Gly Tyr
   660 665 670
   Gly Ser Ser Ser Ser Ser Ser Leu Tyr Ser Thr Asp Gly Ala Ser Thr
   675 680 685
   Gly Gly Thr Gly Gly Gly Ser Ser Leu Gly Ala Ser Leu Asn Ala Ala
   690 695 700
   Ala Gly Tyr Gly Ala Ser Ser Ser Ala Gly Ser Gly Gly Gly Gly Gly
   705 710 715 720
   Ser Val Leu Lys Lys Arg Ser Pro Ser Ser Ala Ala Ala Glu His Glu
   725 730 735
   Leu Glu Lys Arg Thr Pro Gly Phe Gly Ser Ala Leu Thr Ile Gly Gly
   740 745 750
   Thr Ala Met Phe Gly Gly Thr Leu Leu Ala Asn Leu Trp Val Ser Gly
   755 760 765
   Lys Gln Arg Ser Lys Pro Val Pro Val Val Thr Pro Asn Ala Asp Thr
   770 775 780
   Arg Gly Pro Met Ile Pro Gly Gly Asp Leu Tyr Ala Gln Tyr Leu Ala
   785 790 795 800
   Ser Lys Arg Ile Gln Pro Gly Ser Ala Gly Gly Thr Gly Leu Ala Gly
   805 810 815
   Gly Gly Ala Pro Ala Ala Val Asn Tyr Gly Thr Ser Met Pro Ala Ala
   820 825 830
   Phe Asn Thr Ala Ser Ala Pro Asp Glu Ser Ser Ala Ala Val Val Pro
           835                 840                 845
   Gly Thr Gly Pro Ser Ser Asp Ala Ser Asp Ser Thr Ala Thr Gly Gly
       850                 855                 860
   Leu Asp Pro Val Leu Lys Lys Arg Asp Thr Gln Pro Gly Leu Gln Lys
   865                 870                 875                 880
   Arg Ser Pro Glu Ala Pro Leu Ile Glu Leu Glu Ser Gln Val Pro Asn
                   885                 890                 895
   Leu Leu Lys Ala Ala Gly Ala Ala Ser Gly Leu Ala Arg Gly Ala Ala
               900                 905                 910
   Ala Ser Glu Ala Gly Val Gly Ala Ala Ser Met Leu Gly Arg Gly Gly
           915                 920                 925
   Ala Ala Ala Ser Glu Ala Gly Ala Gly Ala Phe Ser Arg Val Ser Ala
       930                 935                 940
   Val Gly Arg Glu Gly Gly Gly Phe Ala Arg Phe Ser Glu Ser Ala Arg
   945                 950                 955                 960
   Pro Gly Leu Ser Arg Tyr Gly Gly Thr Thr Ala Ala Glu Gly Gly Leu
                   965                 970                 975
   Ser Ser Phe Glu Arg Thr Pro Leu Ser Ser Asn Pro Phe Glu Thr Ser
               980                 985                 990
   Phe Ala Ser Ser Phe Tyr Arg Pro  Gly Gly Thr Ala Leu  Ser Gly Ala
           995                 1000                1005
   Ala Arg  Pro Ala Met Val Ser  Asp Phe Lys Val Ala  Thr Gly Thr
       1010                1015                1020
   Ala Gly  Ala Pro Ala Thr Ile  Glu Gly Ala Ala Ala  Ala Glu Ala
       1025                1030                1035
   Glu Ala  Gly Ala Asp Ala Ala  Ala Ala Ala Thr Gly  Lys Arg Lys
       1040                1045                1050
   Gly Met  Thr Pro Leu Lys Met  Val Ser Ala Val Gly  Ser Gly Ala
       1055                1060                1065
   Gly Val  Thr Met Met Met Asn  Gly Leu Gly
       1070                1075

<210>  51
<211>  1107
<212>  PRT
<213>  Ustilago maydis

<400>  51
   Met Arg Val Gly Ala Leu Leu Leu Phe Cys Leu Ala Cys Leu Ala Phe
   1               5                   10                  15
   Thr Ala Gln Ala Ala Pro Ala Ser Ala Lys Asp Glu Leu Leu Ala Gln
               20                  25                  30
   Lys Leu Arg Gln Arg Asp Gly Gly Asp Lys Lys Asn Ser Ser Asn Gly
           35                  40                  45
   Glu His Gly Arg His His Gln Glu Ala Arg Gln Pro Lys Lys His Arg
       50                  55                  60
   Arg Ser Glu Gly Val Val Asp Arg Leu His Ala Arg Glu Leu Thr Pro
   65                  70                  75                  80
   Gly Glu Ala Gln Lys Val Gly Asp Thr Val Ser Lys Leu Val Pro Gln
                   85                  90                  95
   Glu Asp Leu Leu Asn Asp Leu Gln Thr Lys Val Thr Gly Ile Glu Asn
               100                 105                 110
   Ala Arg Arg Glu Ala Glu Arg Gln Lys Gln His Thr Met Gln Val Glu
           115                 120                 125
   Asp Ser Ile His Met Ala Lys Tyr Gly Lys Ala Pro Pro Lys Phe Pro
       130                 135                 140
   Ser Ile Thr Arg Ala Ala Ser Ala Ile Gly Leu Ser Ala Ala Val Leu
   145                 150                 155                 160
   Asn Val Val Asn Phe Ile Phe Ser Thr Lys Thr Thr Leu Gln Ala Thr
                   165                 170                 175
   Tyr Gln Phe Ser Ala Pro Pro Thr Pro Pro Thr Leu Ile Asp Thr Ala
               180                 185                 190
   Ala Arg Trp Asp Gly Ser Gln Tyr Thr Leu Met Ser Leu Pro Thr Asp
           195                 200                 205
   Lys Pro Asp Glu Ser Gln Ser His Lys Ser Lys Arg Gln Val Glu Lys
       210                 215                 220
   Ala Gln Leu Thr Arg Leu Gln Lys Arg His Leu Ser Asn Gly Ala Val
   225                 230                 235                 240
   Ala Glu Ser Asp Val Gln Arg Arg Phe Val Arg Ser Arg Gln Leu Pro
                   245                 250                 255
   Pro Ala Asp Pro Ser Gly Gln Leu Lys Glu Ser Val Gln Lys Leu Lys
               260                 265                 270
   Leu Ser Lys Arg Gln Tyr Ile Ser Ser Val Val Gly Ala Gly Leu Gly
           275                 280                 285
   Ala Leu Leu Ser Pro Gly Phe Thr Ser Thr Val Met Gly Lys Lys Lys
       290                 295                 300
   Glu Asp Met Asp Thr Pro Pro Phe Asn Pro Tyr Asp Pro Asn Ile Leu
   305                 310                 315                 320
   Lys Pro Lys Asp Ala Phe Ser Ala Ala Ala Ala Thr Pro Gly Gln Thr
                   325                 330                 335
   Leu Pro Val Leu Ala Ser Ser Asn Pro Asp Tyr Ser Ser Ala Leu Ser
               340                 345                 350
   Thr Pro Pro Asn Val Asp Gly Ile Lys Phe Pro Val Asp Val Tyr Asp
           355                 360                 365
   Thr Glu Gly Ala Ser Gln Ser Gln Ala Gln Asn Leu Val Tyr Pro Leu
       370                 375                 380
   Ser Ser Gln Ser Gln Ser Val Leu Arg Lys Arg Glu Val Asn Gly Asp
   385                 390                 395                 400
   Arg Asp Arg Ile Asp Ala Arg Ala Ser Ser Gln Leu Asp Lys Arg Phe
                   405                 410                 415
   Leu Phe Thr Gly Gly Pro Trp Ser Lys Leu Ala Gly Ala Ile Thr Val
               420                 425                 430
   Gly Ser Val Val Pro Leu Ile Tyr Gly Gly Val Thr His Ala Arg Trp
           435                 440                 445
   Arg Gln Tyr Asn Pro Arg Asp Arg Asp Ile Leu Lys Asp Ile Asp Asp
       450                 455                 460
   Arg Pro Thr Tyr Gly Ala Val Gly Val Pro Asp Arg Gln Pro Gly Gln
   465                 470                 475                 480
   Leu Ala Thr Asn Ala Met Pro Gly Tyr Ser Asn Tyr Val Thr Gln Leu
                   485                 490                 495
   Gln Gln Glu Gln Ala Leu Gln Gln Ala Arg Ala Gln Thr Ala Ala Arg
               500                 505                 510
   Glu Gln Gly Gln Ala Ser Ser Pro Asp Glu Leu Ser Gln Ser Ser Thr
           515                 520                 525
   Ser Ala Ala Ser Gly Val Thr Ala Pro Asp Gly Ala Ala Ser Gly Gln
       530                 535                 540
   Ser Gly Val Pro Val Leu Lys Arg Arg Ala Ala Asp Ser Leu Glu Lys
   545                 550                 555                 560
   Arg Gly Gly Leu Leu Ala Ser Gly Ala Leu Val Val Val Gly Gly Leu
                   565                 570                 575
   Leu Gly Tyr Ala Gln Ser Ala Thr Gln Phe Pro Thr Asp Arg Asp Glu
               580                 585                 590
   Glu Lys Asp Glu Lys Lys Arg Glu Ala Lys Lys Lys Gln Gln Glu Leu
           595                 600                 605
   Met Ala Gln Gln Gln Gln Gln Gln Gln Gln Ala Thr Gln Met Gln Gly
       610                 615                 620
   Gly Ala Gly Ala Leu Ala Ala Asp Pro Thr Gly Gly Ala Ser Gly Gly
   625                 630                 635                 640
   Ser Gln Thr Asp Pro Leu Ala Gly Ile Pro Val Lys Pro Leu Pro Gly
                   645                 650                 655
   Ser Asp Val Ser Ala Ala Tyr Asp Ser Ser Gly Gly Gly Tyr Leu Ala
               660                 665                 670
   Ala Ser Gly Asp Gly Gly Ala Ser Gly Phe Ser Pro Pro Thr Tyr Gly
           675                 680                 685
   Ser Gly Gly Ala Ser Ser Gly Gly Ile Ser Ser Leu Gly Asp Ser Leu
       690                 695                 700
   Asn Ala Ala Ala Gly Tyr Gln Ala Pro Ala Ser Gly Gly Gly Gly Gly
   705                 710                 715                 720
   Gly Gly Ser Gly Gly Ser Val Leu Lys Lys Arg Asp Ala Ser Ala Asp
   725                 730                 735
   Gly Asn Thr Ala His Thr Ala Ser Asp His Gln Val Glu Ser Arg Ser
           740                 745                 750
   Leu Ser Lys Arg Thr Pro Gly Phe Gly Thr Ala Leu Thr Ile Gly Gly
       755                 760                 765
   Thr Ala Met Phe Val Gly Thr Leu Ala Gly Asn Ile Phe Val Gly Glu
       770                 775                 780
   Lys Gln Lys Asn Lys Pro Val Pro Leu Val Thr Pro Asn Ala Asn Thr
   785                 790                 795                 800
   Arg Gly Pro Met Ile Pro Gly Gly Glu Leu Tyr Thr Ser Tyr Leu Glu
                   805                 810                 815
   Ala Gln Arg Gly Gln Thr Gly Thr Gly Ala Gly Ser Tyr Ser Pro Met
           820                 825                 830
   Ser Gly Val Gly Thr Gly Leu Ala Thr Gly Ala Pro Ala Ser Ile Asn
       835                 840                 845
   Tyr Gly Ala Ala Ala Met Ser Ser Ala Gly Gly Gly Met Pro Ser Ser
       850                 855                 860
   Ala Gly Val Ser Thr Leu Ser Thr Ser Gly Asp Gly Thr Pro Gly Asp
   865                 870                 875                 880
   Thr Ser Ala Gly Thr Ala Asn Gly Asp Thr Ser Gly Leu Ala Gly Ala
                   885                 890                 895
   Ala Asp Pro Val Leu Lys Lys Arg Ser Met Leu Arg Lys Arg Val Pro
           900                 905                 910
   Leu Gly Phe Ala Gly Ala Ala Val Ala Ala Glu Thr Glu Ala Val Ala
       915                 920                 925
   Leu Ala Arg Ala Gly Ala Gly Ala Ala Ser Val Leu Gly Arg Gly Ala
       930                 935                 940
   Ala Val Ser Gly Ala Glu Ala Gly Thr Ala Ala Leu Ala Arg Gly Ala
   945                 950                 955                 960
   Thr Val Ser Gly Ala Asp Val Gly Thr Ala Ser Leu Ala Arg Gly Ala
                   965                 970                 975
   Gly Leu Ser Glu Ser Gly Thr Ala Arg Ala Ala Phe Ala Gly Arg Gly
               980                 985                 990
   Gly Gly Leu Gly Gly Glu Ala Ala  Ser Leu Ser Arg Gly  Glu Ala Gly
           995                 1000                1005
   Leu Ala  Ser Glu Gly Gly Ala  Gly Met Leu Gly Arg  Gly Thr Trp
       1010                 1015                1020
   Met Gly  Arg Gly Gly Ser Ala  Ala Ser Leu Tyr Arg  Thr Asp Ala
       1025                 1030                1035
   Ala Met  Gly Ser Ala Arg Pro  Ala Met Val Ser Asp  Met Pro Ala
       1040                 1045                1050
   Val Thr  Pro Gly Thr Ala Gly  Ser Pro Ala Thr Ile  Gln Gly Ala
       1055                 1060                1065
   Met Ser  Ala Glu Ala Gly Ala  Gly Lys Glu Ala Ala  Lys Gly Gly
       1070                 1075                1080
   Met Ser  Phe Lys Lys Val Ala  Ala Ile Val Gly Gly  Gly Ala Ala
       1085                 1090                1095
   Ala Thr  Met Thr Phe Asn Gly  Ile Gly
       1100                 1105

<210>  52
<211>  1116
<212>  PRT
<213>  Ustilago esculenta

<400>  52

Met Arg Val His Leu Leu Leu Leu Leu Cys Leu Ala Cys Leu Ala Phe
1               5                   10                  15

Thr Ala Gln Ala Ala Pro Ala Ser Ala Arg Glu Ala Leu Leu Ala Gln
            20                  25                  30

Lys Gln Lys Gln His Ser Asp Glu Gly Lys Lys Lys Lys Pro Asn Asn
        35                  40                  45

Gly Ala Arg His His Asp Ala Lys His His His His Lys Ser Gly His
    50                  55                  60

Ser Gly His Thr Ser Ser Pro Lys Glu Thr Arg Gly Val Ser Glu Ala
65                  70                  75                  80

Gly Ile His Ile Val Glu Gly Ser Ala Ser His Leu His Pro Gln Glu
                85                  90                  95

Asn Leu Leu Glu Glu Leu Asn Ala Glu Val Ala Lys Leu Glu Glu Thr
            100                 105                 110

Arg Arg Gln Gln Leu Lys Asn Lys Lys His Val Met Gln Val Ala Asp
        115                 120                 125

Ser Ile His Met Ala Asn Thr Gly Arg Ala Pro Pro Lys Phe Pro Ile
    130                 135                 140

Ile Thr Arg Val Gly Ser Ala Ile Gly Leu Ala Ser Ala Val Leu Asn
145                 150                 155                 160

Val Val Asn Phe Leu Phe Ala Ala Lys Thr Thr Ala Leu Ala Thr Thr
                165                 170                 175

Gln Phe Ser Ala Pro Pro Thr Pro Pro Thr Leu Ile Asp Thr Ala Ala
            180                 185                 190

Arg Trp Asp Gly Ser Gln Tyr Val Leu Met Pro Leu Pro Glu Asp Asn
        195                 200                 205

Asp Val Thr Pro Gly Arg Lys Glu Arg Arg Ser Ile Val Asp Lys Pro
    210                 215                 220

Asp Pro Ser Ser His Gly Asn Lys Arg Leu Ile Lys Arg Lys Lys Arg
225                 230                 235                 240

Gln Asn Gln His Glu Ser Val Pro Leu Ala His Lys Gly Asp Tyr Pro
                245                 250                 255

Lys Met Glu Lys Arg Trp Val Ile Ala Ala Ala Thr Gly Ala Leu Ile
            260                 265                 270

Gly Ala Ala Phe Ala Pro Gly Leu Pro Ser Met Val Thr Gly Lys Trp
        275                 280                 285

Lys Pro Glu Ile Glu Lys Pro Pro Tyr Asp Pro Tyr Asp Pro Asn Ile
    290                 295                 300

Leu Lys Pro Lys Asp Thr Phe Ser Ala Ala Ala Ala Ser Pro Asp Ala
305                 310                 315                 320

Pro Leu Pro Met Leu Pro Ala Pro Ser Pro Lys Tyr Pro Gly Lys Gly
                325                 330                 335

Pro Pro Tyr Leu Gly Asp Ile Lys Phe Pro Ser Phe Glu Tyr Gly Ser
            340                 345                 350

Pro Ala Pro Gly Asn Ile Tyr Arg Pro Pro Leu Val Asn Pro Tyr Ser
        355                 360                 365

Leu Gln Ser Ser Pro Asp Val Arg Gly Ala Ser Tyr Gly Gly Ala Arg
    370                 375                 380

Thr Ala Gly Thr Gln Thr Thr Thr Ser Asp Glu Ala Gln Asp Val Leu
385                 390                 395                 400

Arg Lys Arg Ser Val Leu Ser Gly Val Lys Val Asp Asp Val Gln Leu
                405                 410                 415

Asp Asp Ala Ala Ser Lys Ser Gln Leu Gly Lys Arg Ser Phe Phe Gly
            420                 425                 430

Gly Pro Phe Gly Thr Ala Val Ala Ala Gly Ser Ile Phe Pro Leu Leu
        435                 440                 445

Tyr Gly Gly Val Thr His Ala Arg Ala Arg Thr Tyr Asn Pro Arg Asp
    450                 455                 460

Arg Asp Ile Leu Lys Asp Leu Asp Thr Ser Asn Thr Tyr Gly Ala Val
465                 470                 475                 480

Gly Val Pro Gly Asn Leu Pro Gly Gln Leu Ala Thr Asn Ala Met Pro
                485                 490                 495

Gly Tyr Thr Asp Tyr Val Thr Arg Leu Arg Asn Ala Gln Ser Tyr Gln
            500                 505                 510

Gln Ala Gln Ala Leu Ala Ala Thr Arg Gln Arg Asp Ile Ala Ala Lys
        515                 520                 525

Val Ala Gln Met Arg Gln Phe Gln Gln Ala Gln Gln Ala Glu Ser Ser
    530                 535                 540

Thr Ala Val Ser Thr Ser Gly Gly Ser Ser Gly Glu Pro Val Leu Arg
545                 550                 555                 560

Arg Arg Ser Ile Ala Asp Ser Gln Thr Ile Pro His Asn Gly Gln Ser
                565                 570                 575

Arg Leu Glu Lys Arg Ala Ser Asn Trp Leu Val Gly Gly Gly Leu Leu
            580                 585                 590

Leu Val Gly Gly Leu Leu Gly Tyr Ala Gln Ser Ala Thr Gln Phe Pro
        595                 600                 605

Ser Asp Arg Gly Glu Glu Lys Asp Glu Lys Lys Arg Glu Ala Lys Lys
    610                 615                 620

Lys Gln Glu Ala Glu Met Ala Ala Leu Gln Gln Gln Gln Ala Ala Gln
625                 630                 635                 640

Ser Gly Gly Gly Tyr Pro Asp Pro Asn Ala Ala Val Gly Ala Pro Gly
                645                 650                 655

Ser Ser Pro Gly Pro Asp Pro Phe Ala Gly Ile Pro Ile Gln Pro Ala
            660                 665                 670

Pro Asp Ser Gly Gly Gly Val Ser Pro Ser Gly Ser Gly Gly Tyr Gly
        675                 680                 685

Ser Gly Gly Gly Tyr Gly Thr Gly Gly Gly Tyr Gly Ala Gly Gly Gly
    690                 695                 700

Tyr Gly Ala Gly Gly Gly Tyr Gly Ala Gly Gly Gly Tyr Gly Ser Gly
705                 710                 715                 720

Gly Asp Gly Thr Gly Gly Gly Tyr Gly Thr Gly Gly Ser Ser Ser Leu
                725                 730                 735

Gly Ala Ser Leu Asn Ala Ala Ala Gly Tyr His Thr Ser Gly Gly Gly
            740                 745                 750

Gly Gly Gln Ser Asn Val Leu Lys Lys Arg Asn Leu Pro Asp Ser Ser
        755                 760                 765

Glu Phe Val Glu Pro Asn Lys Ala His Ala Ser Ser His Gly Leu Glu
    770                 775                 780

Ala Arg Ser Leu Ser Lys Arg Thr Pro Gly Leu Gly Thr Ala Phe Ser
785                 790                 795                 800

Ile Gly Gly Thr Thr Met Phe Leu Gly Thr Leu Leu Gly Asn Leu Tyr
                805                 810                 815

Val Ser Ser Lys Gln Lys Asn Lys Pro Val Pro Leu Val Thr Pro Asn
            820                 825                 830

Ala Asp Thr Arg Gly Pro Met Ile Ile Gly Gly Asp Leu Tyr Thr Ala

        835                 840                 845

Tyr Leu Glu Ser Lys Arg Gly Gln Gly Thr Gly Ala Gly Gln Ser Thr
    850                 855                 860

Met Val Gly Gly Pro Pro Ala Ser Val Asn Phe Pro Gly Tyr Thr Pro
865                 870                 875                 880

Gly Asn Pro Pro Ala Gly Ser Val Gly Gly Ser Ser Leu Tyr Ala Ala
                885                 890                 895

Pro Glu Ala Thr Thr Pro Gly Thr Thr Leu Pro Tyr Asn Pro Gly Arg
            900                 905                 910

Gln Gln Asp Thr Ser Lys Ala Asp Asp Pro Glu Pro Val Leu Lys Lys
        915                 920                 925

Arg Asp Met Ser Ala Glu Ala Ser Ala Val Asn Lys Arg Ala Pro Leu
    930                 935                 940

Gly Trp Ala Ala Glu Ala Glu Thr Ala Ala Val Ser Leu Ala Arg Ala
945                 950                 955                 960

Pro Ala Ile Val Glu Ala Glu Ala Ser Ala Ala Ala Arg Leu Gly Ala
                965                 970                 975

Gly Ser Arg Met Ser Glu Leu Ser Gly Leu Gly Ser Arg Ala Arg Gly
            980                 985                 990

Met Ala Gln Gly Gly Ser Tyr Ala Ser Glu Ala Gly Val Arg Gly Phe
        995                 1000                1005

Gly Arg Thr Ser Leu Ala Ala Glu Gly Gly Gly Ser Ser Leu Phe
    1010                1015                1020

Gly Pro Gly Ala Ala Ala Trp Gly Arg Ala Lys Pro Ala Ile Val
    1025                1030                1035

Ser Asp Met Gly Arg Ala Gly Thr Ala Gly Ala Ala Gly Arg Pro
    1040                1045                1050

Ala Thr Leu Glu Gly Leu Tyr Gly Ser Ser Ser Ala Ser Tyr Arg
    1055                1060                1065

Lys Leu Ser Pro Gln Glu Ile Leu Glu Thr Glu Arg Arg Ala Glu
    1070                1075                1080

Ala Asp Ala Glu Ala Lys Gly Lys Gly Met Thr Gly Ala Lys Ala
    1085                1090                1095

Met Gly Ile Ile Gly Ser Gly Ala Gly Ala Thr Met Met Phe Asn
    1100                1105                1110

Gly Ile Gly
    1115

<210> 53
<211> 1220
<212> PRT
<213> Ustilago trichophora

<400> 53

Met Arg Ile Glu Leu Leu Leu Leu Phe Cys Leu Ala Cys Leu Val Phe
1               5                   10                  15

Thr Ala Gln Ala Ala Pro Ala Ser Glu Lys Asp Ala Leu Leu Glu Gln
            20                  25                  30

Lys Leu Lys Gln Arg Lys Gly Glu Gly Glu Gly Lys Gln His Lys Pro
        35                  40                  45

Asn Lys Pro His His Ser Gln Arg His His His Arg Lys His His Gly
    50                  55                  60

Leu Asn Gln Leu Asp His Ala Ser Ser Ala Lys His Thr Arg Ala Ala
65                  70                  75                  80

Pro Met Phe Asn Thr Gly Ala Ala Arg Ala Ala Glu Glu Ala Gly Lys
                85                  90                  95

Arg Leu Leu Pro Gln Glu His Leu Leu Ser Glu Leu Arg Gly Glu Ile
            100                 105                 110

Thr Ala Ala Glu Asn Asp His Arg Gln Leu Leu Lys Ser Lys Lys His
        115                 120                 125

Glu Met Gln Val Glu Asp Ser Leu His Ile Ala Thr Thr Gly Lys Leu
    130                 135                 140

Pro Pro Lys Phe Pro Thr Ile Thr Arg Ile Gly Ser Ala Leu Gly Leu
145                 150                 155                 160

Thr Ser Ala Val Leu Asn Val Ile Asn Ile Met Phe Ala Ala Lys Thr
                165                 170                 175

Thr Ala Leu Ala Thr Thr Gln Phe Ala Pro Pro Pro Ile Pro Pro Thr
        180                 185                 190

Leu Leu Asp Thr Ala Ala Arg Trp Asp Gly Asn Gln Tyr Thr Leu Leu
    195                 200                 205

Pro Pro Pro Ser Asp Gly Lys Gly Gly Phe Ile Asp Leu Arg Arg Arg
    210                 215                 220

Ser Leu Ile Asp Asn Thr Glu Pro Lys Asn Gly Ala His His Asn Leu
225                 230                 235                 240

Lys Lys Arg Ser Leu Ala Ser Leu Thr Ser Pro Ser Ser Arg Ala His
                245                 250                 255

Gly Arg Met Ile Lys Arg Arg Lys Pro Leu Thr Ser Glu Glu Pro His
            260                 265                 270

Ser Asn Ala Glu Glu Gly Asp Ser Arg Lys Leu Ala Lys Arg Trp Val
        275                 280                 285

Val Ser Ala Val Val Gly Ala Ala Leu Gly Ala Ala Leu Ala Pro Gly
    290                 295                 300

Phe Pro Ser Met Val Thr Gly Lys Trp Lys Ala Glu Val Asp Lys Pro
305                 310                 315                 320

Arg Phe Asp Pro Tyr Asn Pro Asp Ile Leu Lys Gln Asn Asp Pro Phe
                325                 330                 335

Ser Ala Ala Ala Ala Thr Pro Asp Gln Pro Leu Pro Met Pro Pro Leu
            340                 345                 350

Arg Ala Pro Pro Pro Arg Phe Pro Leu Asn Ala Pro Pro Asn Leu Gly
        355                 360                 365

His Ile Val Phe Pro Thr His Ala Asn Ser Met Gln Gln Gln Pro Pro
    370                 375                 380

Leu Asp Ala Tyr Ser Arg Gln Asn Lys Ser Thr Pro Ala Asp Thr Lys
385                 390                 395                 400

Ala Val Leu Arg Lys Arg Ala Val Glu Ser Asp Ala Gln Thr Asp Gly
                405                 410                 415

Lys His Glu Lys Ala Thr Ser Ser Ser His Leu Asp Lys Arg Phe Leu
            420                 425                 430

Phe Ser Gly Gly Pro Gly Ala Lys Leu Ala Gly Ala Val Thr Val Gly
        435                 440                 445

Ser Val Val Pro Leu Leu Tyr Gly Gly Val Thr His Ala Arg Trp Arg
    450                 455                 460

Thr Glu Asn Pro Arg Asp Arg Asp Ile Leu Lys Asp Leu Asp Asp Thr
465                 470                 475                 480

Pro Thr Tyr Gly Ala Val Gly Val Pro Gly His Val Pro Gly Gln Leu
                485                 490                 495

Ala Thr Asn Ala Met Pro Gly Tyr Ala Asp Tyr Ile Ala Arg Leu Gln
            500                 505                 510

Ala His Gln Ala Gln Gln Gln Ala Ala Ala Gln Ala Thr Ala Gln Arg
        515                 520                 525

Gln Tyr Leu Ala Thr Gln Ala Met Gln Leu Arg Glu Tyr Gln Gln Asn
    530                 535                 540

Arg Leu Ala Gln Thr Thr Thr Ala Tyr Thr Glu Ser Gly Gly Ser Ser
545                 550                 555                 560

Glu Pro Val Leu Arg Lys Arg Asp Thr Val Cys Ser Gln Thr Ser Gln
                565                 570                 575

Gly Thr Glu Cys Tyr Asn Asn Gly Gln Thr Arg Leu Asp Lys Arg Gly
            580                 585                 590

Asn Trp Leu Met Thr Gly Gly Leu Ala Leu Ala Gly Leu Ala Leu Gly
        595                 600                 605

Tyr Phe Gln Ser Ala Thr Gln Phe Pro Thr Asp Arg Asp Glu Glu Lys
    610                 615                 620

Asp Ala Lys Lys Arg Glu Met Lys Lys Gln Gln Lys Ala Glu Met Gln
625                 630                 635                 640

Ala Ala Gln Gln Gln Gln Glu Ala Gln Gly Gly Gly Gly Gly Gly Gly
                645                 650                 655

Gly Gly Ala Gly Ala Ala Thr Gly Ser Asp Pro Phe Ala Gly Met Gln
            660                 665                 670

Ile Ser Gly Thr Pro Leu Gly Ser Gly Gly Gly Asp Thr Ser Thr Tyr
        675                 680                 685

Ala Pro Ser Tyr Ser Ser Ser Gly Ser Gly Ser Ala Leu Ala Tyr Asp
    690                 695                 700

Gly Gly Ala Gly Leu Ser Gly Ala Gly Ala Gly Ala Gly Gly Tyr Ala
705                 710                 715                 720

Ser Thr Gln Lys Tyr Asp Ala Thr Gly Ala Ser Gly Tyr Asp Ser Ser
                725                 730                 735

Ser Ser Gly Tyr Gly Ser Ser Ala Thr Thr Tyr Ser Ser Gly Asn Thr
            740                 745                 750

Gly Ala Ser Ala Tyr Gly Ser Ser Ser Ala Tyr Gly Ser Asp Ser Ser
        755                 760                 765

Ser Ala Gly Gly Ser Ser Ser Leu Ala Ala Ser Leu Asn Ala Ala Ala
    770                 775                 780

Gly Tyr Ser Asn Ser Ala Gly Gly Gly Ala Ser Ser Gly Met Leu Lys
785                 790                 795                 800

Lys Arg Asp Thr Ser Asp Ala Ser Glu Pro Arg Ala Asn Asp Lys Ala
                805                 810                 815

Arg Pro Ser Ser His Glu Gln Asn Ala Pro Ser Leu Ser Lys Arg Thr
            820                 825                 830

Pro Gly Phe Ala Asn Ala Leu Thr Ile Gly Gly Thr Thr Leu Phe Val
        835                 840                 845

Gly Thr Leu Leu Gly Asn Leu Phe Val Ser Ser Lys Gln Lys Ser Lys
    850                 855                 860

Pro Val Pro Leu Val Thr Pro Asn Ala Asp Thr Arg Gly Pro Met Val
865                 870                 875                 880

Ile Gly Gly Asp Met Tyr Thr Ala Tyr Leu Glu Ser Lys Arg Ala Gly
                885                 890                 895

Gln Met Ala Gly Pro Gly Gln Gly Gly Val Gly Gly Ser Gly Leu Gly
            900                 905                 910

Pro Ala Ala Gly Ser Pro Ala Ser Ile Asn Asp Gly Ala Ala Ala Pro
        915                 920                 925

Ala Tyr Ser Pro Ser Tyr Gly Gly Ser Ser Tyr Gly Gly Ser Ser Tyr

930                 935                 940

Gly Gly Gly Ser Ser Ser Leu Ala Asp Thr Asn Thr Tyr Ser Thr Ala
945                 950                 955                 960

Ser Asn Ala Ala Thr Thr Gly Asn Ser Tyr Pro Gln Gln Asp Thr Ser
                965                 970                 975

Gly Ala Gly Gly Ser Ala Ala Val Leu Lys Lys Arg Ser Val Ile Lys
            980                 985                 990

Lys Pro Ser Leu Ser Ala Asp Ala  Asp Ala Leu Arg Lys  Arg Ser Pro
        995                 1000                 1005

Leu Gly  Met Ala Ala Ile Ala  Asp Ala Gly Ala Ala  Ala Ser Leu
    1010                 1015                 1020

Leu Gly  Lys Ala Pro Ala Met  Val Glu Ala Gly Ala  Gly Ala Ala
    1025                 1030                 1035

Ser Leu  Ile Ser Lys Ala Pro  Ala Met Thr Glu Leu  Gly Ala Gly
    1040                 1045                 1050

Ala Val  Ser Lys Leu Gly Ala  Ala Ser Arg Leu Ser  Gly Leu Ser
    1055                 1060                 1065

Glu Tyr  Gly Ser Ser Ala Ala  Gly Leu Ala Arg Gly  Gly Ala Phe
    1070                 1075                 1080

Ala Ser  Glu Ala Gly Leu Gly  Ser Leu Gly Arg Ser  Ser Met Ala
    1085                 1090                 1095

Ala Glu  Asn Thr Tyr Ala Arg  Met Phe Arg Pro Gly  Gln Ala Gly
    1100                 1105                 1110

Trp Gly  Ser Ala Asn Pro Ala  Thr Val Ser Ser Phe  Arg Gly Ala
    1115                 1120                 1125

Gly Gly  Thr Gly Gly Ala Gly  Val Pro Ala Thr Leu  Glu Asp Leu
    1130                 1135                 1140

Ser Gly  Ser Leu Gly Arg Ser  Ala Ser Ser Arg Thr  Gly Tyr Gln
    1145                 1150                 1155

Gly Phe  Ser Gln Gln Gln Ile  Met Glu Ala Glu Gln  Arg Ala Ala
    1160                 1165                 1170

Met Ala Ser Glu Lys Gly Leu Ala Gly Gln Gly Gly Ala Gly Ala
    1175                1180                1185

Ile Pro Glu Pro Lys Lys Lys Gly Met Thr Gly Met Lys Ala Ile
    1190                1195                1200

Gly Ile Val Gly Ala Gly Ser Gly Ala Ser Met Met Phe Asn Gly
    1205                1210                1215

Ile Gly
    1220

<210> 54
<211> 1099
<212> PRT
<213> Ustilago hordei (host: oats)

<400> 54

Met Arg Phe Glu Leu Leu Leu Phe Leu Ser Leu Ala Cys Leu Ala Phe
1               5                   10                  15

Thr Ala Gln Thr Ala Pro Ala Ser Ala Lys Asp Ala Leu Gln Glu Gln
            20                  25                  30

Lys His Lys Gln Arg Asp Ser Glu Pro His Lys His Lys Pro Lys Ser
        35                  40                  45

Gly Pro His Leu His Arg His Pro Lys Gln His His Tyr Lys Ser Ser
    50                  55                  60

His Pro Gly Tyr Gly Asn Asn Leu Ile Tyr Thr Arg Gly Leu Thr Gly
65                  70                  75                  80

Met Ser Val Glu Val Val Gln Asp Thr Lys Thr Leu Phe Val Pro Glu
                85                  90                  95

His Glu Ser Lys Leu Arg Ala Lys Ile Asp Gly Ile Glu Lys Thr Phe
            100                 105                 110

Leu Glu Lys Glu Lys Leu Lys Leu His Glu Ile Glu Arg Ala Asp Ile
        115                 120                 125

Ile Glu Thr Ala Lys His Gly Ala Val Pro Pro Lys Phe Pro Arg Val
    130                 135                 140

Thr Arg Ile Ser Ser Phe Ile Gly Ile Val Ala Ala Ile Ala Asn Val
145                 150                 155                 160

Ala Asn Phe Ile Phe Ala Ala Lys Thr Thr Asp Leu Ala Thr Lys Gln
                165                 170                 175

Phe Ser Ala Pro Pro Thr Pro Pro Thr Phe Ile Asp Thr Ala Gly Arg
            180                 185                 190

Trp Asp Gly Gln Gln Tyr Gln Leu Met Pro Leu Pro Gln Glu Pro Asp
        195                 200                 205

Asp Thr Met Ser Ala Arg Lys Lys Arg Ser Leu Pro Asp Glu Cys Ala
    210                 215                 220

Thr Val Pro Pro Ala His Lys Arg Met Val Lys Arg Lys Arg Pro His
225                 230                 235                 240

His Thr Glu Gln Ser Gln Lys Leu Asp Glu Ala Gln His Asp Pro Lys
                245                 250                 255

Leu Ser Lys Arg Ser Val Ile Ala Ala Gly Val Gly Ile Phe Leu Gly
            260                 265                 270

Gly Ala Phe Ser Pro Gly Leu Pro Ser Leu Val Met Gly Trp Glu Pro
        275                 280                 285

Glu Val Asp Lys Thr Pro Phe Asp Pro Tyr Asp Pro Asn Val Leu Lys
    290                 295                 300

Pro Lys Asp Pro Phe Ala Val Ala Ala Ala Asp Pro Asp His Ala Leu
305                 310                 315                 320

Pro Ala Leu Pro Met Arg Lys Pro Ala Pro Ser Gly Ser Ser Asn Gly
                325                 330                 335

Leu Pro Ser Leu Arg Asn Ile Val Phe Pro Val Ser Ala Tyr Ser Ser
            340                 345                 350

Ser Ala Thr Ser Ile Leu Gln Gln Gln Gln Gln Gln Leu Thr Ala Ala
        355                 360                 365

Thr Ala Gln Gln Gly Ser Ala Ile Ser Ser Ala Asn Val Pro Ala Arg
    370                 375                 380

Gly Ser Ser Thr Ala Ser Ala Ser Asp Glu Val Gln Thr Val Leu Arg
385                 390                 395                 400

Lys Arg Ser Val Glu Ala Lys His Ser Thr Glu Ile Glu His Gln Glu
                405                 410                 415

Arg Glu Ser Ser Lys Pro Leu Glu Lys Arg Phe Leu Phe Asp Lys Gly
            420                 425                 430

Leu Val Gly Lys Leu Thr Gly Ala Val Ala Leu Gly Ser Thr Leu Tyr
        435                 440                 445

Val Leu Tyr Lys Gly Ile Ser His Ser Arg Trp Arg Lys Glu Asn Pro
    450                 455                 460

Arg Asp Arg Asp Ile Leu Lys Asp Leu Asp Asp Thr Pro Met Tyr Gly
465                 470                 475                 480

Ala Val Gly Val Pro Lys Arg Ile Pro Gly Gln Leu Ala Thr Asn Ala
                485                 490                 495

Met Pro Gly Tyr Ser Asp Tyr Val Ala Lys Leu Ala Ala Gln Gln Gln
            500                 505                 510

Thr Gln Gln Gln Trp Gln Gln Leu Ala Thr Thr Gln Ala Gln Ala Arg
        515                 520                 525

Asp Ala Leu Thr Glu Gln Ala Ile Ala Ala Gln Ser Ser Ser Gly Gly
    530                 535                 540

Val Gly Val Ala Ala Ala Asp Ala Gly Ser Asn Asp Pro Val Leu Arg
545                 550                 555                 560

Arg Arg Ser Ser Asp Ala Ser Ser Ala Val Ser Ala Lys Pro Val Phe
                565                 570                 575

Gly Asp Ala Gly Gln Thr Arg Leu Asp Lys Arg Gly Gly Arg Ala Val
            580                 585                 590

Leu Ile Gly Gly Gly Leu Thr Val Ile Gly Ile Ala Leu Gly Tyr Leu
        595                 600                 605

Gln Ser Ala Thr Met Phe Pro Thr Asp Arg Asp Ala Glu Lys Asp Lys
    610                 615                 620

Arg Lys Arg Gln Arg Lys Lys Leu Glu Glu Gln Gln Leu Leu Met Gln
625                 630                 635                 640

Gln Gln Gln Ala Leu Val Gln Gly Glu Ala Ala Gln Ser Ala Gly Met
                645                 650                 655

Gly Tyr Ala Pro Ser Gly Ala Ser Ala Pro Leu Val Ser Ser Ser Asp
            660                 665                 670

Gly Gly Gly Ile Ala Phe Pro Ser Ser Ala Tyr Ser Ser Ser Gly Ser
        675                 680                 685

Thr Tyr Pro Pro Ser Met Gly Ala Gly Ser Phe Ser Ser Ser Ala Gly
    690                 695                 700

Thr Glu Pro Ser Thr Gly Gly Ala Ser Ser Ala Ser Ala Ala Leu Asn
705                 710                 715                 720

Ala Ala Ala Gly Tyr Ser Ser Ala Gly Gly Ser Ala Ser Gly Ser Val
                725                 730                 735

Leu Lys Lys Arg Asp Ile Ser Gly His Pro Asp Gln Glu Ser Glu Thr
            740                 745                 750

Arg Ser Leu Ser Lys Arg Ser Pro Gly Val Gly Ser Phe Leu Ser Ile
        755                 760                 765

Ala Gly Thr Thr Phe Phe Phe Gly Thr Leu Ala Gly Asn Leu Phe Val
    770                 775                 780

Gly Glu Lys Glu Lys His Lys Pro Val Pro Tyr Val Thr Pro Asn Ala
785                 790                 795                 800

Asp Thr Arg Gly Pro Met Val Pro Gly Gly Glu Leu Tyr Thr Gln Tyr
                805                 810                 815

Leu Gln Ser Lys Arg Pro Pro Ser Thr Ser Gly Asp Ala Phe Gly Asp
            820                 825                 830

Val Gly Ala Ser Leu Ala Thr Gly Thr Pro Ala Ser Ile Thr Gly Gln
        835                 840                 845

Asn Met Ala Pro Ala Ser Ala Pro Ala Ser Ile Tyr Gly Gln Thr Pro
    850                 855                 860

Val Val Gly Ser Gln Leu Gly Ala Asn Thr Gly Thr Ala Ala Ser Pro
865                 870                 875                 880

Asn Ala Pro Asp Val Gly Arg Gly Gly Glu Ala Asp Pro Val Leu Lys
                885                 890                 895

Lys Arg His Ser Pro Arg Leu Glu Lys Arg Val Pro Leu Ser Phe Ala
            900                 905                 910

Gly Ala Arg Ala Glu Ala Val Ala Gly Leu Leu Thr Lys Gly Val Pro

        915                 920                 925

Met Val Glu Ala Glu Val Gly Ala Ala Ser Ala Leu Ser Arg Ser Ala
    930                 935                 940

Ala Leu Ser Glu Ala Gly Ala Ala Ser Arg Phe Ala Pro Thr Thr Arg
945                 950                 955                 960

Phe Ser Ser Leu Ser Gly Val Arg Gly Gly Ala Ala Ala Ser Glu Ala
                965                 970                 975

Gly Phe Ala Gly Leu Gly Arg Ala Ser Met Ala Gly Glu Gly Ser Thr
            980                 985                 990

Ala Ala Arg Ile Tyr Gln Pro Asn Ala Ala Ala Val Gly Ser Ala Arg
        995                 1000                1005

Pro Ala Met Leu Ser Asp Leu Gly Gly Ala Ser Gly Arg Ala Gly
    1010                1015                1020

Thr Pro Ala Thr Phe Ala Arg Ser Ala Ser Thr Arg Gln Leu Ser
    1025                1030                1035

Pro Glu Glu Met Gln Gly Leu Gln Gln Arg Val Ala Ala Gly Arg
    1040                1045                1050

Ala Ala Asp Met Asn Pro Gly Val Lys Gly Met Ser Gly Gln Ala
    1055                1060                1065

Pro Glu Glu Ala Lys Lys Gly Trp Thr Leu Gln Lys Ala Leu Gly
    1070                1075                1080

Ile Val Gly Gly Ser Ala Gly Ala Ser Leu Leu Phe Ser Gly Ala
    1085                1090                1095

Gly

<210> 55
<211> 1157
<212> PRT
<213> Ustilago tritici

<400> 55

Met Arg Val Glu Leu Leu Leu Phe Phe Cys Leu Thr Cys Leu Ala Phe
1               5                   10                  15

Thr Ala Gln Ala Ala Pro Ala Ser Ala Lys Asp Ala Leu Leu Ala Gln

20                          25                          30

Thr Leu Lys Gln Arg Ser Asn Asp Asp Arg Pro Asn Asp Ser Lys His
            35                          40                          45

Asp Pro Pro His His Gln Gln Arg His His His Arg Glu Asp Arg Glu
    50                          55                          60

His His Glu His His Glu His His Gly Arg His His Arg Lys Gln Arg
65                          70                          75                          80

Val Asp His Ser Asn Asn Val Thr Gly His Leu Lys Asn Arg Asp Val
                        85                          90                          95

Ile Gln Gly Ala Arg Gly Ala Glu Glu Ala Ala Thr His Leu Leu Gln
                100                         105                         110

Pro His Ala Gln Glu Asp Leu Leu Ala Asn Ala Arg Ser Arg Phe Ala
            115                         120                         125

Ala Leu Gln Lys Ala Gly Arg Glu Arg Glu Leu Ser Lys Gln His Glu
    130                         135                         140

Ile Glu Ile Glu Asp Ala Ile His Val Ala Lys Tyr Gly Lys Thr Pro
145                         150                         155                         160

Pro Lys Phe Pro Ala Ile Thr Arg Val Ala Ser Ala Ile Gly Leu Ile
                        165                         170                         175

Ala Ala Val Met Asn Thr Val Asn Phe Leu Phe Ser Ala Lys Thr Thr
                180                         185                         190

Ala Leu Ala Thr Thr Gln Phe Ser Ala Pro Pro Asn Pro Pro Thr Phe
            195                         200                         205

Ile Asp Thr Ala Ala Met Trp Asp Gly Asn Gln Tyr Arg Leu Met Thr
    210                         215                         220

Leu Pro Glu Glu Lys Ala Val Ala Ser Gly Gly Ser Ala Lys Gln Lys
225                         230                         235                         240

Arg Ser Leu Pro Lys Gln Pro Ser Thr Gln Arg Leu Gln Lys Arg Gln
                        245                         250                         255

Phe Gly Val Lys Pro Val Ala Val Ala Glu Pro Gly Gln Leu Ser His
                260                         265                         270

Phe Thr Lys Arg Glu Thr Ser Asn Asp Ala Arg Thr Ala Gly Glu Arg
        275                 280                 285

Gly Ala Gly Ala Val Asp Pro Lys Leu Val Lys Arg Trp Ala Ile Ala
    290                 295                 300

Ala Gly Thr Gly Phe Val Leu Gly Gly Leu Val Ala Pro Glu Phe Ile
305                 310                 315                 320

Ser Thr Val Arg Gly Lys Lys Pro Glu Val Glu Lys Pro Pro Phe Asp
                325                 330                 335

Pro Tyr Asp Pro Asn Val Leu Lys Pro Lys Asp Pro Phe Thr Ala Ala
            340                 345                 350

Ala Ala Thr Ser Asp Gln Pro Leu Pro Ala Val Pro Leu Ser Gln Leu
        355                 360                 365

Ser Ala Ala Arg Ala Ala Gly Ala Pro Pro Asp Val Ser Asn Ile Lys
    370                 375                 380

Phe Pro Gly Ile Val Tyr Asn Asp Pro Ala Thr Ser Tyr Val Pro Ser
385                 390                 395                 400

His Ser Val Thr Asn Pro Ala Ser Arg Gln Asp Leu Ala Ala Ser Pro
                405                 410                 415

Ala Ser Ala Ala Gly Ser Asp Ala Gly Ala Ser Gln Ser Ser Gln Val
            420                 425                 430

Ser Pro Val Leu Arg Lys Arg Ala Asp Val Pro Asn Ala Arg Ser Ala
        435                 440                 445

Ser Asp Asn Ser Asp Ala Thr Ser Ser Ser Lys Leu Asp Lys Arg Phe
    450                 455                 460

Leu Phe Ser Gly Gly Pro Trp Ser Lys Val Ala Gly Phe Val Thr Val
465                 470                 475                 480

Gly Ser Val Val Pro Leu Leu Tyr Glu Gly Val Thr His Ala Arg Phe
                485                 490                 495

Arg Thr Gln Asn Pro Arg Asp Arg Asp Ile Leu Lys Glu Leu Asp Asp
            500                 505                 510

Lys Pro Thr Tyr Gly Ala Val Gly Val Pro Gly Arg Thr Pro Gly Gln
        515                 520                 525

Leu Ala Thr Asn Ala Met Pro Gly Tyr Ser Asp Tyr Val Thr Gln Leu
    530                 535                 540

Glu Gln Gln Gln Ala Arg Arg Gln Ala Ala Ala Pro Pro Asn Glu Met
545                 550                 555                 560

Gln Ala Gly Gln Ser Ala Gln Pro Ala Val Ala Ala Asp Ala Ser Thr
                565                 570                 575

Gly Ala Gly Ala Ser Glu Gly Gly Asn Gly Gly Gly Gly Gly Asp Pro
            580                 585                 590

Val Leu Arg Arg Arg Asp Leu Glu Ser Asn Gly Gln Thr Arg Val Glu
        595                 600                 605

Arg Arg Gly Trp Leu Leu Gly Gly Ala Leu Val Ala Gly Gly Ala Leu
    610                 615                 620

Leu Gly Tyr Ala Gln Ser Ala Thr Gln Phe Pro Thr Asp Arg Asp Ala
625                 630                 635                 640

Glu Lys Glu Glu Lys Lys Arg Glu Ala Lys Lys Lys Gln Gln Glu Met
                645                 650                 655

Met Ala Ala Gln Gln Ala Gln Gln Gly Ala Ala Pro Pro Ala Gly Pro
            660                 665                 670

Gly Tyr Ala Asp Ala Ser Gln Val Ala Ala Thr Asn Ile Ile Pro Gly
        675                 680                 685

Pro Ala Asp Thr Ser Ala Leu Gly Ala Ala Tyr Pro Val Ala Ala Gly
    690                 695                 700

Gly Gly Gly Ser Ser Ser Ser Tyr Gly Tyr Gly Gly Gly Leu Gly Ala
705                 710                 715                 720

Ala Ser Ser Tyr Thr Tyr Gly Gly Ser Ser Gly Ser Ser Pro Ser Tyr
                725                 730                 735

Gly Leu Gly Ser Gly Thr Ser Ser Ser Tyr Thr Thr Asn Pro Ala Ser
            740                 745                 750

Ser Tyr Gly Gly Ser Thr Ser Ser Leu Ala Ser Ser Leu Asn Ala Ala
        755                 760                 765

Ala Gly Leu Gln Gly Ser Ser Ala Ser Gly Gly Ser Val Leu Lys Lys
    770                 775                 780

Arg Asp Leu Ser Pro Leu Asp Arg Asn Leu Gln Ala Gly Ser Leu Ser
785                 790                 795                 800

Lys Arg Thr Pro Gly Trp Gly Ser Ala Leu Ser Ile Gly Gly Thr Thr
                805                 810                 815

Met Phe Phe Gly Thr Leu Ala Ala Asn Val Trp Val Gly Ser Lys Gln
            820                 825                 830

Lys Ser Lys Pro Val Pro Leu Val Thr Pro Asn Ala Asn Thr Arg Gly
        835                 840                 845

Pro Met Ile Pro Gly Gly Asp Leu Tyr Thr Gln Tyr Leu Glu Ser Lys
    850                 855                 860

Arg Pro Leu Ala Ala Gly Gly Met Ser Tyr Gly Ala Pro Gly Ser Asp
865                 870                 875                 880

Ala Thr Pro Ala Val Val Asn Tyr Gly Lys Pro Val Ser Val Ala Ala
                885                 890                 895

Lys Pro Gly Thr Gly Gly Ala Leu Val Gly Thr Pro Gly Gly Thr Pro
            900                 905                 910

Gly Ala Thr Pro Gly Gly Pro Pro Gly Gly Gly Val Pro Pro Ser Ala
        915                 920                 925

Gln Gly Gly Gly Val Asp Ala Gly Ser Asp Ala Ser Ser Asp Asp Pro
    930                 935                 940

Asn Ser Gly Met Val Gly Asp Lys Asp Pro Val Leu Lys Lys Arg Gly
945                 950                 955                 960

Val Ala Ser Val Leu Gln Lys Arg Thr Pro Gln Gly Ala Ala Ala Val
                965                 970                 975

Ala Ala Val Glu Thr Glu Ala Pro Phe Phe Ala Arg Leu Ala Ala Ser
            980                 985                 990

Leu Ala Ala Arg Leu Arg Gly Gly  Ala Ala Ala Ser Glu  Ala Ala Thr
        995                 1000                1005

Gly Ala  Thr Ala Ala Leu Gly  Arg Gly Ala Ala Ser  Glu Gly Ser
    1010                1015                1020

Ile Gly  Ala Ala Ala Arg Ala  Gly Glu Tyr Thr Pro  Tyr Ser Arg

1025                1030                1035

Leu Tyr  Ser Thr Ala Gly Gly  Gly Leu Thr Arg Glu  Thr Ala Ala
    1040                1045                1050

Ser Glu  Gly Ala Trp Gly Ala  Ser Arg Phe Ser Thr  Leu Ala Ser
    1055                1060                1065

Ser Pro  Met Thr Arg Ala Glu  Ala Gly Leu Ala Asn  Ser Val Tyr
    1070                1075                1080

Arg Pro  Tyr Gly Ala Ala Met  Gly Glu Ala Arg Pro  Ala Met Ala
    1085                1090                1095

Ala Asp  Val Lys Ala Ala Thr  Gly Thr Ala Gly Thr  Pro Ala Thr
    1100                1105                1110

Val Glu  Gly Ala Ala Ala Asp  Ala Gly Gln Glu Ala  Ala Ala Ala
    1115                1120                1125

Gly Lys  Ser Lys Gly Met Thr  Gly Thr Arg Ala Met  Glu Ile Val
    1130                1135                1140

Gly Ala  Gly Ala Gly Ala Thr  Met Leu Met Pro Gly  Thr Gly
    1145                1150                1155

<210>  56
<211>  1017
<212>  PRT
<213>  Melanopsichium pennsylvanicum

<400>  56

Met Lys Val Ala Phe Leu Ser Ala Ile Val Phe Gly Ala Ile Ser Val
1               5                   10                  15

Ala Ala Ser Ser Ser Gln Val Ile Ala Asp Glu His Phe Ile Gln His
            20                  25                  30

Ser Ala Arg Pro Ser Ser Tyr Ala Ser Thr Lys Glu Asn Val Arg Ala
        35                  40                  45

Asn Ser Lys Ser Ser Ala Ser Lys His Gln Ala Val His His His Ser
    50                  55                  60

Lys His Pro His Ser Lys His His Arg Gln Arg Leu Ser Gln Arg Glu
65                  70                  75                  80

Thr Leu Val Lys Arg Asp Glu Lys Gln Ile Leu Glu Leu Gly Asp Asn

                85                  90                  95

Leu Ala Gly Leu Leu Lys His Val Thr Gly Asp Gly Glu Lys Gly Gly
            100                 105                 110

Ser Thr Val Ile Asn His Tyr His Pro Gly Ser Gly Ala Gly Glu Ala
        115                 120                 125

Thr Ser His Ala Leu Gly Gly Ser Ala His Pro Asn Ala Pro Pro Gln
    130                 135                 140

Tyr Ala Thr Pro Tyr His Ser Ser Asp Pro Ala Val Met Ala Arg Leu
145                 150                 155                 160

Asp Ser Ile Gln Arg Gly Met Gly Asp Leu Lys His Ala His Arg Ala
                165                 170                 175

Ser Ala Glu Gln Ile Gln Leu Met Glu Glu Leu Ala Ala Asn Ser Met
            180                 185                 190

Lys Glu Arg Ala Glu Leu Glu Lys Val Thr Pro Leu Ala Pro Ser Pro
        195                 200                 205

Thr Lys Arg Gln Met Ser Lys Lys Ala Ile Ala Ala Leu Val Leu Gly
    210                 215                 220

Gly Gly Ala Leu Gly Trp Gly Ala Thr Lys Met Phe Asp Ser Lys Pro
225                 230                 235                 240

Ala Asp Thr Thr Ser Gly Tyr Asn Pro Gln Glu Met Ala Ala Leu Gln
                245                 250                 255

Ala Ala Gly Gly Ala Ala Pro Gly Thr Ala Ser Val Asp Gly Tyr Ser
            260                 265                 270

Ser Ser Gly Gly Ile Ser Ala Pro Ser Tyr Thr Ser Thr Gly Ala Val
        275                 280                 285

Ser Gly Gly Ala Asp Pro Gly Ala Asn Ala Ser Gly Gly Val Gln Ile
    290                 295                 300

Thr Thr Glu Pro Gln Gln Ile Pro Gly Thr Asn Ile Tyr Trp Val Leu
305                 310                 315                 320

Asp Ser Asn Asn Gln Ala His Thr Phe Ser Leu Thr Ala Ser Gly Asp
                325                 330                 335

Val Gln Glu Val Pro Trp Pro Gln Gly Trp Thr Pro Pro Thr Gly Ser
            340                 345                 350

Gly Ala Gly Ala Ala Ser Pro Asp Thr Val Ser Gly Ala Gln Gly Ala
        355                 360                 365

Gly Gln Ser Ala Gly Thr Pro Tyr Tyr Gln Gln Thr Gly Gly Gly Gln
    370                 375                 380

Ser Gly Gly Ala Pro Thr Ala Gln Thr Gly Gly Ser Gly Gln Gly Gly
385                 390                 395                 400

Thr Gly Ser Asp Asn Pro Gln Thr Phe Trp Asp Pro Gln Thr Gln Met
                405                 410                 415

Asn Tyr Val Ile Gly Pro Asn Asn Arg Lys Tyr Tyr Ile Ile Ser Asn
            420                 425                 430

Asn Tyr Leu Met Asp Glu Leu Thr Gly Asn Ile Ser Asp Ala Arg Thr
        435                 440                 445

Gly Glu Ile Val Ser Tyr Gly Glu Gly Gln Thr Asp Gly Gly Gly Asp
    450                 455                 460

Val Gly Thr Gly Glu Asp Gln Thr Ile Gly Gly Gly Gln Gly Ala Gly
465                 470                 475                 480

Gly Val Gln Gly Gly Ser Asn Ala Gly Asn Gly Asp Asp Ile Ser Gly
                485                 490                 495

Gly Gly Ser Gly Phe Gly Ser Gly Ser Gly Ser Gly Ser Gly Ser Gly
            500                 505                 510

Ser Gly Ser Gly Ser Gly Ser Gly Ser Gly Ser Gly Ser Gly Ser Gly
        515                 520                 525

Ser Gly Ser Gly Ser Gly Ser Gly Ser Gly Ser Gly Ser Gly Ala Gly
    530                 535                 540

Gly Ile Ala Ser Gly Ser Gly Arg Met Arg Lys Arg Ser Leu Arg His
545                 550                 555                 560

Arg Lys Leu Glu Ser Arg Gln Leu Gly Met Gly Gly Ile Gln Pro Val
                565                 570                 575

Ser Pro Ala Leu Arg Glu Gln Met Arg Leu Ala Met Leu Arg Gln Gln
            580                 585                 590

Ala Gly Val Ser Pro Ser Asp Ala Ala Ala Met Ala Gln Lys Ala Gly
        595                 600                 605

Lys Gly Asn Met Leu Val Lys Val Gly Leu Gly Leu Gly Ala Leu Ile
    610                 615                 620

Gly Leu Ala Val Gly Leu Asn Ala Tyr Ala His Pro Arg Arg Pro Asn
625                 630                 635                 640

Thr Thr Pro Asp Asn Glu Tyr Gly Leu His Ala Thr Thr Ala Cys Asp
                645                 650                 655

Pro Tyr Gly Lys Pro Ala Ile Leu Asp Phe Lys Gly Asp Tyr Phe Ser
            660                 665                 670

Lys Ser Thr Tyr Ser Arg Val Ala Pro Asn Asn Leu His Ala Cys Ser
        675                 680                 685

Val Gln Glu Leu Asn Asn Ala Gly Tyr Thr Thr Asp Ala Tyr Gln Gly
    690                 695                 700

Ala Glu Gly Gly Gly Gly Asp Gly Gly Gly Gly Ala Ala Arg Met Gln
705                 710                 715                 720

Lys Arg Gln Leu Pro Asp Leu Lys Leu Ser His Asp Gly Gln Val Glu
                725                 730                 735

Val Val Arg Lys Asp Ile Ala Asn Asn Gly Asn Ser Asn Gly Leu His
            740                 745                 750

Lys Pro Phe Gln Ala Lys Arg Ala Glu Arg Ser His Gly His Ile Leu
        755                 760                 765

Glu Lys Arg Gln Gly Gly Lys Leu Met Ala Thr Ala Gly Met Leu Leu
    770                 775                 780

Pro Ser Met Ser Gly Ser Gly Ala Leu Ala Ile Gly Ala Ala Ala Leu
785                 790                 795                 800

Ile Thr Val Ile Tyr Leu His Asn Ile His Glu Lys Lys Lys Gln Ala
                805                 810                 815

Leu Val Asp Val Trp Asn Ser Lys Tyr His Ser Ala Gly Glu Ala Ala
            820                 825                 830

Ser Ser Val Gly Asn Ala Ile Ala Thr Gly Ala Asn Gly Gln Leu Phe
        835                 840                 845

Asn Pro Leu Thr Gly Asn Leu Ile Leu Ile Asp Pro Asn Thr Asn Gln
    850                 855                 860

Tyr Tyr Asp Ser Gly Thr Gly Gln Pro Val Asp Ser Lys Thr Gly Met
865                 870                 875                 880

Pro Ser Ser Tyr Ser Gly Gly Asp Asp Asp Pro Ser Lys Gly Gly Ser
                885                 890                 895

Gly Ala Ser Ser Gln Leu Thr Gln Ile Pro Asp Pro Gln Leu Pro Ile
            900                 905                 910

Asp Ala Ser Gly Asn Ile Ser Gly Asp Asp Pro Leu Val Gln Gln Ala
        915                 920                 925

Leu Gln Gln Trp Asn Met Met Thr Pro Asp Gln Gln Gln Gln Leu Met
    930                 935                 940

Ala Gln Tyr Gly Gly Gln Ala Gln Gln Ile Leu Gln Ser Ser Ser Pro
945                 950                 955                 960

Gln Ala Ala Phe Asp Ala Trp Thr Lys Gln Gln Ser Leu Met Val Thr
                965                 970                 975

Gln Leu Ser Ser Pro Gly Ser Gln Gln Gln Ser Ser Leu Gln Pro Gln
            980                 985                 990

Tyr Thr Gln Leu Gln Gln Tyr Val  Pro Gln Gln Gln Gln  Gln Phe Ala
        995                 1000                1005

Pro Gln  Pro Gln Ser Ala Thr  Phe Lys
    1010                1015

<210>  57
<211>  1035
<212>  PRT
<213>  Sporisorium scitamineum

<400>  57

Met Lys Val Ala Leu Leu Cys Ala Ile Val Phe Gly Ala Leu Ser Val
1               5                   10                  15

Ala Ala Ser Ser Ser Gln Tyr Val Ala Asp Glu His Phe Val Gln Arg
            20                  25                  30

Ser His Gly Ser Ala Lys Ala Asn Ser Gly Thr Lys Asn Glu Lys Pro
        35                  40                  45

Ala Ser Lys His Gln Pro Lys His Gln Arg Asp Leu Thr Ser Gln Ala
    50                  55                  60

Gly His Ala Glu His Leu Phe Lys Arg Val Asp Gly Pro Ala Phe Leu
65                  70                  75                  80

Glu Ile Gly Asp Asn Leu Ala Ser Val Leu Lys Lys Ser Ser Gly Gly
                85                  90                  95

Glu Thr Val Ile Asn His Tyr Ser Gly Ala Asp Ala Ala Ala Ser His
            100                 105                 110

Phe Arg Gly Gly Pro Pro Pro Pro Tyr Tyr His Ser His Tyr Pro Pro
        115                 120                 125

Pro Tyr His Gly Gly Asp Pro Ala Thr Ala Ser Lys Leu Glu Gly Ile
    130                 135                 140

Asn Ser Lys Leu Asp His Leu His Arg Gly Gln Gln Met Asn Ala Gln
145                 150                 155                 160

Gln Ile Glu Leu Met Gln Glu Leu Gln Ala Lys Glu Met Glu Lys Ala
                165                 170                 175

Gly Ala Ala Ser Ala Ala Ala Ala Ala Lys Pro Lys Arg Gly Tyr Gly
            180                 185                 190

Lys Gly Ala Met Ala Leu Met Leu Ala Gly Gly Ala Leu Gly Gly Trp
        195                 200                 205

Gly Leu Thr Lys Val Phe Asp Thr Lys Pro Ala Asp Ser Thr Thr Gly
    210                 215                 220

Tyr Asn Pro Gly Asp Met Ala Ala Met Gln Ala Ala Gly Gly Ala Ala
225                 230                 235                 240

Pro Gly Thr Ala Pro Val Asp Gly Ala Pro Gln Pro Ser Ala Gly Gly
                245                 250                 255

Gln Ala Asp Gln Ala Asn Pro Phe Lys Glu Gly Pro Tyr Lys Ile Pro
            260                 265                 270

Gly Thr Gly Leu Val Trp Val Val Asp Gln Gln Asn Gln Val His Val
        275                 280                 285

Val Asp Pro Val Arg Gln Val Glu Val Ser Pro Pro Pro Gly Trp Asn

    290                 295                 300

Pro Tyr Gly Ser Ser Gly Gly Ala Ala Asp Gly Ser Thr Ala Gly Gly
305                 310                 315                 320

Asp Gly Ala Asp Ala Ala Gln Gln Gly Ala Arg Ala Thr Gln Gln Val
                325                 330                 335

Pro Gly Ala Ser Gly Gly Ala Pro Thr Thr Gln Ala Gly Asp Ser Ser
            340                 345                 350

Thr Ser Gly Val Asp Ala Ala Gln Gln Gly Ala Arg Ala Thr Gln Gln
        355                 360                 365

Val Pro Gly Ala Ser Gly Gly Ala Pro Thr Thr Gln Ala Gly Asp Ser
    370                 375                 380

Ser Thr Ser Gly Ala Gly Gln Gln Gly Asp Gly Gly Gln Val Ala Gln
385                 390                 395                 400

Gly Ser Gly Ala Ser Thr Ala Gln Asn Ala Ala Gln Gln Ala Pro Gln
                405                 410                 415

Asp Ala Ser Ala Asn Ala Asp Leu Ser Gly Ser Gly Asp Thr Lys Pro
            420                 425                 430

Val Trp Asp Pro Asn Val Lys Arg Tyr Trp Val Val Gly Pro Lys Asn
        435                 440                 445

Val Arg Tyr Tyr Ile Asp Asp Ser Thr Gly Phe Leu Leu Asn Ser Glu
    450                 455                 460

Thr Asn Asp Val Ser Asp Pro Arg Thr Gly Lys Ile Val Ile Ser Gly
465                 470                 475                 480

Lys Glu Leu Met Ala Glu Ala Glu Gly Asp Gly Gln Gly Gln Gly Gln
                485                 490                 495

Gly Ser Gly Gly Asp Thr Pro Gly Ser Gln Gly Gly Arg Asp Gly Gly
            500                 505                 510

Ala Ser Gln Gly Pro Gly Gln Asp Gly Ala Gly Ala Gly Gly Arg Met
        515                 520                 525

Gln Lys Arg Gly Glu Ser Gln Gly Gln Thr Ser Gly Ser Ser Asn Gly
    530                 535                 540

Phe Thr Thr Gly Ser Gln Gly Gln Asp Asp Gly Asn Gly Gly Gly Asn
545                 550                 555                 560

Arg Gln Gly Gly Phe Gly Arg Met Gln Lys Arg Ser Leu Arg His Arg
                565                 570                 575

Lys His Asp Lys Arg Ser Tyr Thr Ala Ala Ala Phe Gln Ala Ser Asn
            580                 585                 590

Pro Ala Leu Arg Glu Gln Ala Met Leu Ala Met Ala Gln Gly Ser Gln
        595                 600                 605

Gly Gly Ala Pro Val Ala Ala Ala Ala Lys Lys Ala Gly Val Gly Lys
    610                 615                 620

Thr Ile Ala Lys Ala Gly Val Gly Ile Ala Ala Leu Val Ala Val Val
625                 630                 635                 640

Val Gly Leu Ser Asp Tyr Ala His Pro Gln Pro Lys Gly Thr Thr Gln
                645                 650                 655

Asp Asn Pro Tyr Gly Leu Thr Pro Thr Asn Ile Cys Asp Val Tyr Gly
            660                 665                 670

Glu Pro Ile Ile Arg Asp Phe Arg Gly Val Leu Trp Asn Lys Ser Thr
        675                 680                 685

Tyr Ser Arg Val Asp Leu Asn Asn Pro Gly Gln Leu Tyr Gln Cys Pro
    690                 695                 700

Gln Ser Asp Gln Ala Gln Gly Asp Gly Val Tyr Ala Gly Ala Gly Tyr
705                 710                 715                 720

Gly Thr Gln Pro Gln Lys Arg Asp Leu His Glu Arg Gly Leu Arg Asp
                725                 730                 735

Lys Leu Ile Glu Trp Val Arg Pro Ser Val Ile Glu Thr Asp Val Ser
            740                 745                 750

Ser Asn Ala Asn Lys Lys Leu Leu Asp Glu Val Lys Lys Asp Val Asp
        755                 760                 765

Lys Gln Gly Asp Ser Thr Glu Asn His Ser Leu Glu Lys Arg Gln Met
    770                 775                 780

Lys Tyr Leu Lys Ala Leu Met Pro Ser Met Thr Ala Ser Gly Thr Met
785                 790                 795                 800

Ala Val Ala Gly Thr Val Ile Ile Thr Ala Ile Trp Leu His His Val
                805                 810                 815

His Glu Gln Lys Ile Lys Ala Leu Gln Glu Gln Trp Asn Ser Gln Tyr
            820                 825                 830

His Ser Ala Gly Glu Ala Ala Ser Ser Val Gly Asn Ala Ile Ser Thr
        835                 840                 845

Gly Ala Asn Gly Gln Leu Phe Asn Pro Ile Thr Gly Ser Leu Ile Leu
    850                 855                 860

Val Asp Pro Asn Thr Gln Val Tyr Tyr Asp Ser Ser Thr Gly Glu Gln
865                 870                 875                 880

Val Asn Pro Lys Thr Gly Met Pro Leu Arg Tyr Gly Asp Gly Asp Asp
                885                 890                 895

Gly Ser Lys Lys Gln Lys Glu Gln Gln Val Gln Leu Pro Ile Asp Arg
            900                 905                 910

Asn Gly Asn Leu Ser Gly Asp Ser Pro Glu Val Gln Gln Ala Met Ala
        915                 920                 925

Gln Trp Asn Ala Met Ser Pro Gln Gln Gln Gln Gln Val Leu Ala Gln
    930                 935                 940

Tyr Gly Asp Ala Ala Asp Gln Ile Leu Asn Ser Pro Asp Pro Asn Ala
945                 950                 955                 960

Ala Leu Gln Ala Trp Asn Gln Gln His Ser Gln Ala Leu Gln Gln Gln
                965                 970                 975

Phe Ala Ser Gln Gln Gln Leu Val Pro Gln Gln Gln Gln Gln Phe Ala
            980                 985                 990

Pro Gln Gln Leu Ala Pro Gln Gln Leu Ala Pro Gln Gln Leu Ala Pro
        995                 1000                1005

Gln Gln Leu Ala Pro Gln Gln Leu Ala Pro Gln Gln Gln Gln Met
    1010                1015                1020

Gln Pro Ala Ala Gly Gly Ala Ser Asn Leu Gly Met
    1025                1030                1035

<210> 58
<211> 990

<212>  PRT
<213>  Ustilago hordei (host: barley)

<400>  58

Met Lys Val Ala Leu Leu Ser Ala Ile Val Phe Gly Ala Ile Ser Ala
1               5                   10                  15

Ala Ala Ser Ser Ser His Val Leu Ala Asp Glu Tyr Tyr Leu Gln Asn
            20                  25                  30

Phe Ala His Ala His Tyr Pro Ser Tyr Ser Ser Ala Lys Lys Ser Thr
        35                  40                  45

Lys Lys Ser Lys Asp Phe Lys Ser Glu Tyr Gln His Ser Glu Tyr Gln
    50                  55                  60

Ser Glu His His Ser Lys His His Ile Ser Lys Thr Ala Pro Leu Leu
65                  70                  75                  80

Leu Lys Arg Val Asn Ala Gly Asn Thr Leu Glu Ile Gly Glu Ser Ala
                85                  90                  95

Ala Asn Val Ile Lys Ser Ala Leu Glu Gly Gly Lys Lys Ser Gly Asp
            100                 105                 110

Thr Ile Ile Asn His Tyr Gly Thr Ser Thr Ser Glu Ser Leu Gly Gly
        115                 120                 125

His Phe His Pro Gly Ser Gly Gly Asn Pro Gln Ala Tyr Ser Pro Tyr
    130                 135                 140

Ala His Pro Pro Tyr Ala His Pro Pro Tyr Ala His Pro Tyr Pro Pro
145                 150                 155                 160

Pro Gln Ala Ala Gly Gly Thr Asp Ala Leu Leu His Ala Lys Ile Asp
                165                 170                 175

Lys Ile Gly Arg Lys Phe Thr Lys Leu Lys Gly Ala Gln Ile Glu Asn
            180                 185                 190

Thr Lys Gln Ile Gln Ile Ile Gln Glu Met Gln Ala Arg Thr Met Met
        195                 200                 205

Glu Glu Gln Ala Gly Gly Ala Arg Gly Ala Thr Ala Ala Lys Gly Ala
    210                 215                 220

Thr Ala Ala Lys Gly Asp Pro Glu Ala Gln Gln Lys Lys Arg Ser Phe
225                 230                 235                 240

Gly Lys Gly Ser Met Phe Leu Thr Ala Ala Gly Gly Leu Ala Ala Gly
                245                 250                 255

Val Ile Gly Thr Lys Met Leu Asp Ser Lys Pro Ala Val Pro Asp Thr
            260                 265                 270

Ala Tyr Ser Pro Gln Asp Met Ala Ala Ile Gln Gln Ala Ser Ala Gly
        275                 280                 285

Thr Pro Gly Gly Ala Thr Ile Asp Gly Pro Thr Ala Gln Gly Gly Gln
    290                 295                 300

Gly Gln Gly Glu Ser Gln Gly Gly Gly Gly Gly Gly Ser Gly Gly Gly
305                 310                 315                 320

Gln Pro Glu Phe Leu Gly Pro Tyr Phe Asp Gln Ala Thr Gln Thr Arg
                325                 330                 335

Tyr Ile Ile Asp Gly Glu Gly Tyr Tyr His Phe Leu Gly Ser Gly Asn
            340                 345                 350

His Glu Ile Arg Pro Pro Pro Gly Trp Ser Lys Asn Gly Pro Val Arg
        355                 360                 365

Asp Asp Gly Ser Gly Gly Ala Glu Thr Asp Asp Gly Ser Asn Gly Gln
    370                 375                 380

Gln Glu Ala Ala Gly Gly Ala Ala Gly Gly Gly Ala Ala Gly Gly Gly
385                 390                 395                 400

Ala Ala Gly Gly Gly Ala Ala Gly Gly Gly Val Ala Arg Gly Ala Asp
                405                 410                 415

Ser Gly Pro Ala Ala Gly Phe Pro Gln Thr Ser Ala Asn Gly Ala Asp
            420                 425                 430

Val Ala Pro Gln Arg Asp Arg Gln Phe Pro Ser Ser Ala Glu Gly Gly
        435                 440                 445

Gln Ser Asp Ser Ser Asn Lys Pro Ile Trp Asp Asn Thr Ile Lys Met
    450                 455                 460

Tyr Tyr Val Val Gly Pro Asn Gly Val Lys Phe Tyr Ile Asp Thr Glu
465                 470                 475                 480

Thr Gly Tyr Leu Val Asn Ser Val Thr Leu Asp Ile Ser Asp Pro Lys

                485                 490                 495

Thr Gly Gln Ile Ile Met Ser Gly Lys Asp Val Met Lys Gly Ser Ser
            500                 505                 510

Thr Gly Pro Gly Gly Ala Ala Gly Gly Asn Ala Gly Gly Asn Ala Gly
        515                 520                 525

Gly Asn Ala Gly Arg Met Lys Lys Arg Ser Ile Arg His His Gln Phe
    530                 535                 540

His Lys Arg Ser Tyr Glu Gln Ala Ala Leu Tyr Ala Gln Ser Pro Ala
545                 550                 555                 560

Leu Arg Ala Gln Ala Arg Ile Ala Met Leu Arg Asp Ser Gly Arg Leu
                565                 570                 575

Gln Pro Thr Val Ser Ala Ala Ala Arg Lys Ala Gly Ile Gly Lys Thr
            580                 585                 590

Leu Val Lys Val Gly Leu Gly Ala Gly Leu Leu Ile Gly Thr Val Leu
        595                 600                 605

Ala Val Gly Ala Tyr Ala His Pro Asn Arg Pro Gly Thr Ser Pro Asp
    610                 615                 620

Asn Glu Tyr Gly Leu Val Pro Thr Ser Ile Cys Asp Val Tyr Gly Glu
625                 630                 635                 640

Pro Ile Val Gln Asp Phe Arg Lys Val Leu Trp Asn Lys Ser Thr Leu
                645                 650                 655

Thr Arg Leu Asp Pro Asn Asn Leu Pro Pro Leu His Thr Cys Ser Lys
            660                 665                 670

Gln Glu Leu Glu Asp Ala Gly Val Ser Thr Glu Gln Gln Gly Ala Met
        675                 680                 685

Gln Lys Arg Asp Ser Val Glu Ser Lys Ser Gln Asp Gln Met Ala Lys
    690                 695                 700

Leu Val Gln Asp Ala Lys Ala Asp His Ser Glu His Asn Leu Arg Lys
705                 710                 715                 720

Arg Ser Glu Thr Asn Thr Lys Glu Arg Asp His Ser Leu Glu Lys Arg
                725                 730                 735

Gln Gly Glu Gly Ala Val Ala Ser Thr Val Ala Lys Gly Ala Gly Leu
        740                 745                 750

Ala Phe Leu Pro Ser Ile Gly Trp Ala Gly Ala Ala Ala Ile Gly Ala
        755                 760                 765

Thr Ala Leu Val Thr Ala Ile Tyr Leu His Asn Ile His Lys Glu Lys
    770                 775                 780

Glu Leu Ala Ala Met Gln Glu Trp Asn Ser Gln Tyr His Ser Ala Gly
785                 790                 795                 800

Glu Ala Ala Ser Ser Val Gly Asn Ala Ile Ser Thr Gly Ala Asn Gly
                805                 810                 815

Gln Leu Phe Asn Pro Leu Thr Gly Ser Leu Ile Met Val Asp Pro Asn
            820                 825                 830

Thr Gly Leu Tyr Tyr Asp Ala Ser Thr Gly Glu Gln Val Asn Pro Lys
        835                 840                 845

Thr Gly Met Pro Ser Arg Tyr Gly Gly Glu Asp Ser Ser Gln Gln Gln
    850                 855                 860

Gln Gln Gln Gln Ala Pro Gln Leu Pro Leu Asp Asn Ala Gly Asn Met
865                 870                 875                 880

Ser Gly Asn Ser Pro Glu Val Gln Lys Ala Met Glu Gln Trp Asn Leu
                885                 890                 895

Met Ser Pro Gln Gln Gln Gln Gln Leu Leu Asn Gln Tyr Gly Ser Ala
            900                 905                 910

Ala Gln Gln Ile Leu Asp Ser Pro Asp Pro Asn Ala Ala Leu Gln Ala
        915                 920                 925

Trp Ser Gln Gln Gln Ala Gln Gln Ala Gln Ala Val Gln Pro Val Val
    930                 935                 940

Thr Gln Arg Gly Pro Val Ile Pro Gln Ser Gln Gln Gln Gln Gln Gln
945                 950                 955                 960

Leu Pro Pro Ser Arg Tyr Asn Met Ala Val Ser Asn Thr Ala Ser Ser
                965                 970                 975

Tyr Asp Gly Ala Gly Gly Gly Ala Gly Ala Gly Pro Ala Met
            980                 985                 990

<210>  59
<211>  992
<212>  PRT
<213>  Ustilago bromivira

<400>  59

Met Lys Val Ala Leu Leu Ser Ala Ile Val Phe Gly Ala Ile Ser Ala
1               5                   10                  15

Ala Ala Ser Ser Ser His Val Leu Ala Asp Glu Tyr Tyr Leu Gln Asp
            20                  25                  30

Phe Ala His Ala His Tyr Arg Ser Tyr Ser Ser Ala Lys Lys Ser Gly
        35                  40                  45

Lys Lys Thr Lys Gly Ser Arg Ser Lys His Gln His Ser Glu His Gln
    50                  55                  60

Ser Glu His His Ser Lys His His Ser Glu His His Val Ser Glu Gln
65                  70                  75                  80

His His Val Ser Lys Thr Ala Pro Leu Leu Lys Arg Val Asn Gly Glu
                85                  90                  95

Ser Thr Phe Glu Ile Gly Lys Asn Ala Ala Asp Leu Leu Arg Ser Ala
            100                 105                 110

Val Glu Gly Gly Lys Lys Ser Gly Asp Thr Ile Ile Asn His Tyr Gly
        115                 120                 125

Ala Ser Thr Thr Glu Ser Leu Gly Gly His Phe Arg Pro Gly Ser Gly
    130                 135                 140

Gly Asn Pro His Ala Tyr Pro Pro Tyr Ala His Pro Tyr Ala His Pro
145                 150                 155                 160

Tyr Pro Ala Pro Gln Ala Ala Ala Gly Thr Asp Ala Leu Leu His Ala
                165                 170                 175

Lys Ile Asp Lys Leu Gly His Lys Val Thr Lys Leu Lys Gly Gly Gln
            180                 185                 190

Met Glu Asn Ala Gln Gln Ile His Met Met Gln Glu Leu Gln Ala Lys
        195                 200                 205

Ser Met Val Glu His Ala Gly Gly Ala Pro Gly Ala Thr Ala Ala Gln
    210                 215                 220

Gln Ala Lys Arg Gly Phe Gly Lys Gly Ala Leu Phe Leu Gly Ala Ala
225                 230                 235                 240

Gly Gly Ile Gly Val Gly Val Ile Gly Thr Lys Met Phe Asp Thr Lys
                245                 250                 255

Pro Ala Val Pro Asp Thr Ala Tyr Ser Pro Gln Asp Met Ala Ala Ile
            260                 265                 270

Gln Gln Ala Ser Ala Gly Thr Pro Gly Gly Ala Thr Ile Asp Gly Ser
        275                 280                 285

Ala Gly Pro Ala Ala Gln Gly Gly Gln Gly Glu Ser Gln Gly Gly Gly
    290                 295                 300

Gly Gly Gly Ser Gly Gly Gly Gln Ser Asp Tyr Leu Gly Pro Tyr Phe
305                 310                 315                 320

Asp Gln Ala Thr Gln Thr Arg Tyr Val Ile Asp Gly Glu Gly Ser Tyr
                325                 330                 335

His Phe Ile Gly Ser Gly Asn Gln Glu Ile Arg Pro Pro Pro Gly Trp
            340                 345                 350

Ser Lys Asn Gly Pro Val Arg Asp Gly Ser Ser Gly Gly Ala Gly Thr
        355                 360                 365

Asp Asp Gly Ser Asn Gly Gln Gln Asp Ala Gly Gly Gly Ala Ala Gly
    370                 375                 380

Gly Gly Ala Ala Gly Gly Ala Ala Gly Gly Ala Ala Gly Gly Ala Ala
385                 390                 395                 400

Gly Gly Ala Asp Ser Gly Pro Ala Ala Gly Phe Pro Gln Thr Ser Ala
                405                 410                 415

Asn Gly Ala Asp Gly Ala Pro Gln Arg Asp Arg Gln Phe Pro Ser Ser
            420                 425                 430

Ala Gly Gly Gly Gln Ser Asp Ser Ser Asn Lys Pro Ile Trp Asp Ala
        435                 440                 445

Thr Asn Gln Met Tyr Tyr Val Val Gly Pro Asn Gly Val Lys Phe Tyr
    450                 455                 460

Ile Asp Asn Glu Thr Gly Tyr Leu Val Asn Ser Val Thr Leu Asp Ile
465                 470                 475                 480

Ser Asp Pro Lys Thr Gly Gln Ile Ile Met Ser Gly Lys Asp Val Met
                485                 490                 495

Asn Gly Ser Ser Thr Gly Pro Gly Gly Ala Ala Gly Gly Asn Thr Gly
            500                 505                 510

Gly Asp Thr Gly Gly Asn Thr Gly Gly Asn Thr Gly Gly Asn Ala Gly
        515                 520                 525

Arg Met Lys Lys Arg Ser Ile Arg His Arg Gln Phe His Lys Arg Ser
    530                 535                 540

Tyr Glu Gln Ala Ala Leu His Ala Gln Ser Pro Ala Leu Arg Ala Gln
545                 550                 555                 560

Ala Arg Ile Thr Met Leu Arg Asn Ser Gly Arg Leu Gln Pro Thr Val
                565                 570                 575

Ser Ala Ala Ala Gly Lys Ala Gly Ile Gly Lys Asn Leu Leu Lys Ile
            580                 585                 590

Gly Val Gly Ala Gly Val Leu Val Gly Thr Val Leu Ala Leu Ser Ala
        595                 600                 605

Tyr Ala His Pro Asp Arg Pro Gly Thr Ser Pro Asp Asn Glu Tyr Gly
    610                 615                 620

Leu Val Pro Thr Ser Ile Cys Asp Val Tyr Gly Glu Pro Ile Ile Gln
625                 630                 635                 640

Asp Phe Arg Gly Val Leu Trp Asn Lys Ser Thr Leu Thr Arg Leu Asp
                645                 650                 655

Ala Lys Asn Leu Pro Pro Leu His Gln Cys Ser Lys Gln Glu Leu Glu
            660                 665                 670

Asp Ala Gly Val Tyr Thr Glu Gln Gln Gly Ala Met Gln Lys Arg Asp
        675                 680                 685

Ser Val Glu Ser Lys Ser Asp Gln Met Ala Lys Leu Val Gln Asp Ala
    690                 695                 700

Lys Ala Asp Arg Ser Glu His Asn Leu Arg Lys Arg Ser Glu Thr Asn
705                 710                 715                 720

Thr Lys Glu Arg Asp His Ser Leu Glu Lys Arg Gln Thr Glu Ala Ala

                725                 730                 735

Val Thr Ser Thr Val Ala Lys Gly Ala Gly Leu Ala Phe Leu Pro Ser
            740                 745                 750

Ile Gly Trp Ala Gly Gly Ala Ala Ile Gly Ala Ala Ala Phe Val Thr
        755                 760                 765

Ala Ile Tyr Leu His Asn Ile His Lys Glu Lys Glu Leu Ala Ala Met
    770                 775                 780

Gln Gln Trp Ser Ser Gln Tyr His Ser Ala Gly Glu Ala Ala Ser Ser
785                 790                 795                 800

Val Gly Asn Ala Ile Ser Thr Gly Ala Asn Gly Gln Leu Phe Asn Pro
                805                 810                 815

Leu Thr Gly Ser Leu Ile Met Val Asp Pro Asn Thr Gly Leu Tyr Tyr
            820                 825                 830

Asp Ala Ser Thr Gly Glu Gln Val Asn Pro Lys Thr Gly Met Pro Ser
        835                 840                 845

Arg Tyr Gly Gly Gly Glu Asp Ser Ser Gln Gln Gln Gln Gln Gln Gln
    850                 855                 860

Val Pro Gln Leu Pro Leu Asp Asn Ala Gly Asn Met Ser Gly Asn Ser
865                 870                 875                 880

Pro Glu Val Gln Lys Ala Met Glu Gln Trp Lys Ser Met Ser Pro Gln
                885                 890                 895

Gln Gln Gln Gln Leu Met Ser Gln Tyr Gly Ser Ala Ala Gln Gln Ile
            900                 905                 910

Leu Asn Ser Pro Asp Pro Asn Ala Ala Leu Gln Ala Trp Ser Gln Gln
        915                 920                 925

Gln Ala Gln Gln Ala Gln Gln Ala Gln Gln Ala Gln Ala Val Gln Pro
    930                 935                 940

Val Val Thr Gln Pro Gly Pro Val Phe Pro Gln Ser Gln Gln Gln Gln
945                 950                 955                 960

Gln Gln Leu Pro Pro Ser Gly Tyr Asn Met Ala Gly Ser Asn Ile Ala
                965                 970                 975

Ser Gly Tyr Asp Gly Ala Gly Gly Gly Ala Gly Ala Gly Pro Ala Met
            980                 985                 990

<210>  60
<211>  923
<212>  PRT
<213>  Sporisorium reilianum f. sp. zeae

<220>
<221>  misc_feature
<222>  (325)..(325)
<223>  Xaa can be any naturally occurring amino acid

<400>  60

Met Lys Val Ala Leu Leu Cys Ala Ile Val Phe Gly Ala Leu Ser Val
1               5                   10                  15

Ala Ala Ser Ser Ser Gln His Val Ala Asp Glu His Phe Val Gln His
            20                  25                  30

Ser Arg Gly Ser Thr Lys Pro Lys Ser Gly Thr Lys Asn Lys Lys Pro
        35                  40                  45

Ala Ser Lys His His Gly Lys His His His Gly Lys Glu Pro Ser Gln
    50                  55                  60

Pro Ala His Ala Asp His Leu Val Lys Arg Asp Glu Ile Arg Pro Ile
65                  70                  75                  80

Ala Glu Ile Gly Glu Asn Leu Ala Ser Val Leu Lys Gly Ala Gln Gly
                85                  90                  95

Asp Gly Lys Lys Ala Gly Asp Val Ile Ile Asn Ser Tyr His Pro Pro
            100                 105                 110

Ser Gly Ala Gly Ala Ala Ser His Met Pro Gly Gly Gly Tyr Gly Asn
        115                 120                 125

Tyr Pro Pro Pro Ala His Tyr Pro Pro Pro Tyr Gln Val Thr Asp Pro
    130                 135                 140

Ala Leu Thr Ser Lys Leu Glu Gly Ile Asn Ser Lys Leu Asp His Leu
145                 150                 155                 160

His Gln Gly Gln Arg Thr Asn Thr Glu Gln Ile Glu Ile Met Gln Glu
                165                 170                 175

Leu Lys Ala Lys Glu Met Glu Gly Ala Ala Ala Gln Ser Ala Ala Val
            180                 185                 190

Ala Ala Lys Pro Lys Arg Gly Phe Gly Lys Gly Ala Met Ala Met Met
        195                 200                 205

Leu Ala Gly Gly Ala Leu Ala Gly Trp Gly Ala Thr Lys Met Leu Asp
    210                 215                 220

Thr Lys Pro Ser Asp Ser Thr Thr Gly Tyr Asn Gln Gly Asp Met Ala
225                 230                 235                 240

Ser Met Gln Ala Ala Gly Gly Ala Ala Pro Gly Thr Ala Pro Val Asp
                245                 250                 255

Gly Tyr Pro Gln Asp Gln Pro Pro Pro Gly Ala Gly Gly Ala Ala Gly
            260                 265                 270

Ser Asp Gln Asn Pro Phe Lys Glu Gly Pro Tyr Lys Ile Pro Gly Thr
        275                 280                 285

Pro Tyr Val Trp Val Arg Asp Gln Gln Asp Gln Ile His Val Val Asp
    290                 295                 300

Pro Val Arg Gln Val Glu Val Gln Pro Pro Pro Gly Trp Ser Pro Tyr
305                 310                 315                 320

Gly Asp Ser Ser Xaa Gln Gln Gly Gly Ala Arg Gln Thr Val Gln Gly
                325                 330                 335

Gly Ser Ala Ser Thr Gly Gly Gln Asn Gly Val Gln Gln Val Pro Gln
            340                 345                 350

Asp Ala Gln Arg Ala Asp Gln Gly Asp Asp Gly Gly Ser Gly Lys Pro
        355                 360                 365

Lys Pro Ile Trp Asp Ser Asp Met Gln Met Tyr Tyr Val Ile Gly Pro
    370                 375                 380

Lys Asn Val Lys Tyr Tyr Ile Asp Asp Gln Thr Gly Tyr Leu Ile Asn
385                 390                 395                 400

Ser Val Thr His Asp Val Ser Asp Pro Ser Thr Gly Lys Val Val Met
                405                 410                 415

Thr Gly Gln Glu Leu Glu Ala Gly Gly Asp Gly Gly Ala Gly Gln Gly
            420                 425                 430

Gln Gly Gly Ala Gln Thr Gly Ala Gly Gly Ala Ala Ala Gly Gly Arg

435                 440                 445

Pro Gln Lys Arg Asp Glu Asp Ser Asn Gly Asn Ala Gly Gln Thr Ser
    450                 455                 460

Gly Ser Gly Gly Asp Asp Thr Ser Ser Gln Gly Gly Gly Gly His Glu
465                 470                 475                 480

Asn Gly Asn Ser Asp Ser Gly Gly Gly Pro Gly Arg Met Glu Lys Arg
                485                 490                 495

Ser Leu Arg His Arg Lys His Asp Lys Arg Gly Tyr Pro Ala Ala Ala
            500                 505                 510

Phe Gln Ala Ala Asn Pro Ala Leu Arg Glu Gln Ala Met Leu Ala Met
        515                 520                 525

Ser Arg Thr Ala Gln Gly Gly Asn Thr Ala Ala Ala Ala Ala Gly Lys
    530                 535                 540

Ser Gly Val Gly Gly Lys Val Leu Lys Ala Gly Val Gly Val Ala Ala
545                 550                 555                 560

Leu Val Ala Leu Val Met Gly Leu Asp Ser Tyr Ala His Pro Arg Pro
                565                 570                 575

Pro Gly Thr Ser Glu Ala Asn Pro Tyr Gly Leu Gln Ala Thr Asp Val
            580                 585                 590

Cys Asp Met Tyr Gly Glu Pro Ile Ile Arg Asp Phe Arg Gly Val Leu
        595                 600                 605

Trp Asn Lys Ser Thr Tyr Thr Arg Val Asp Pro Asn Asn Pro Pro Pro
    610                 615                 620

Leu Tyr Gln Cys Pro Pro Asp Gln Gly Gln Ala Gly Ala Ala Gly Gly
625                 630                 635                 640

Asp Ala Gly Ala Gly Tyr Gly Pro Gln Gln Gln Lys Arg Asp Leu His
                645                 650                 655

Lys Arg Gly Leu Val Ser Ala Ile Ser Ala Phe Asp Asp Val Ala Ala
            660                 665                 670

Val Asn Asn Ala Gly Pro Gln Asn Glu Gln Lys Arg Ser Leu Glu Lys
        675                 680                 685

Arg Gln Met Lys Ala Met Ala Ala Leu Leu Pro Ser Leu Ser Ser Ser
    690                 695                 700

Gly Thr Met Ala Val Val Gly Thr Val Ala Val Thr Ala Ile Trp Leu
705                 710                 715                 720

His Tyr Val His Glu Gln Lys Leu Lys Ala Leu Gln Asp Gln Trp Asn
                725                 730                 735

Ser Gln Tyr His Ser Ala Gly Glu Ala Ala Ala Ser Val Gly Asn Ala
            740                 745                 750

Ile Ser Thr Gly Ala Asn Gly Gln Leu Phe Asn Pro Ile Thr Gly Ser
        755                 760                 765

Leu Ile Leu Val Asp Pro Asn Thr Gln Gln Tyr Tyr Asp Ser Ser Thr
    770                 775                 780

Gly Glu Gln Val Asp Pro Lys Thr Gly Met Pro Ala Arg Tyr Gly Asp
785                 790                 795                 800

Gly Ser Asp Gly Arg Lys Lys Lys Gln Glu Gln Pro Leu Gln Leu Pro
                805                 810                 815

Leu Asp Ser Asn Gly Asn Leu Ser Gly Asp Ser Pro Gln Val Gln Gln
            820                 825                 830

Ala Met Ala Gln Trp Asn Ser Leu Ser Pro Gln Gln Gln Gln Gln Ile
        835                 840                 845

Leu Ala Gln Tyr Gly Ala Ala Ala Asp Gln Ile Leu Asn Ser Pro Asp
    850                 855                 860

Pro Asn Ala Ala Leu Gln Ala Trp Asn Arg Gln His Ser Leu Ala Leu
865                 870                 875                 880

Gln Gln Gln Phe Val Pro Gln Gln Gln Gln Gln Phe Ala Pro Gln Gln
                885                 890                 895

Leu Ala Pro Gln Gln Gln Met Met Gln Pro Ala Ala Gly Gly Ala Ser
            900                 905                 910

Gly Leu Gly Gly Asp Gly Ser Gly Ala Gly Met
        915                 920

<210>  61
<211>  996
<212>  PRT

<213>  Sporisorium reilianum f. sp. sorghi

<400>  61

Met Lys Val Ala Leu Leu Cys Ala Ile Val Phe Gly Ala Leu Ser Val
1               5                   10                  15

Ala Ala Ser Ser Ser Gln His Val Ala Asp Glu His Phe Val Gln His
            20                  25                  30

Ser Arg Gly Ser Thr Lys Pro Lys Ser Gly Thr Lys Asn Lys Lys Pro
        35                  40                  45

Ala Ser Lys His His Gly Lys His His His Asp Lys Glu Pro Ser Gln
    50                  55                  60

Pro Ala His Ala Asp His Leu Val Lys Arg Asp Glu Ile Arg Pro Ile
65                  70                  75                  80

Ala Glu Ile Gly Glu Asn Leu Ala Ser Val Leu Lys Gly Ala Gln Gly
                85                  90                  95

Glu Asp Lys Lys Ala Gly Asp Val Ile Ile Asn Ser Tyr His Pro Pro
            100                 105                 110

Ser Gly Ala Gly Ala Ala Ser His Met Pro Gly Gly Gly Tyr Gly Asn
        115                 120                 125

Tyr Pro Pro Ala His Tyr Pro Pro Pro Tyr Gln Val Thr Asp Pro Ala
    130                 135                 140

Leu Thr Ser Lys Leu Glu Gly Ile Asn Ser Lys Leu Asp His Leu His
145                 150                 155                 160

Gln Gly Gln Arg Thr Asn Thr Glu Gln Ile Glu Ile Met Gln Met Leu
                165                 170                 175

Lys Ala Lys Glu Met Glu Gly Ala Ala Ala Gln Ser Ala Ala Val Ala
            180                 185                 190

Ala Lys Pro Lys Arg Gly Phe Gly Lys Gly Ala Met Ala Met Met Leu
        195                 200                 205

Ala Gly Gly Ala Leu Ala Gly Trp Gly Ala Thr Lys Met Leu Asp Thr
    210                 215                 220

Lys Pro Ser Glu Ser Thr Thr Gly Tyr Asn Gln Gly Asp Met Ala Ser
225                 230                 235                 240

Met Gln Ala Ala Gly Gly Ala Ala Pro Gly Thr Ala Pro Val Asp Gly
                245                 250                 255

Tyr Pro Arg Gly Gln Ala Pro Ala Gly Ala Gly Gly Ala Ala Gly Ser
            260                 265                 270

Asp Gln Asn Pro Phe Lys Glu Gly Pro Tyr Lys Ile Pro Gly Thr Pro
        275                 280                 285

Tyr Val Trp Val Arg Asp Gln Gln Asp Gln Ile His Val Val Asp Pro
    290                 295                 300

Val Arg Gln Val Glu Ile Gln Pro Pro Pro Gly Trp Ser Pro Tyr Gly
305                 310                 315                 320

Asp Ser Ser Gly Ala Gly Ala Gly Ala Gly Ala Gly Ala Ala Arg Gly
                325                 330                 335

Ala Ser Thr Thr Gln Ala Gly Asp Gly Ser Ala Ser Gly Ala Gly Gln
            340                 345                 350

Gln Gly Gly Ala Gly Gln Thr Val Gln Gly Gly Ser Ala Ser Thr Thr
        355                 360                 365

Gln Ala Gly Asp Gly Ser Ala Ser Gly Ala Gly Gln Gln Gly Gly Ala
    370                 375                 380

Gly Gln Gln Gly Gly Ala Gly Gln Gln Gly Gly Ala Gly Gln Gln Gly
385                 390                 395                 400

Gly Ala Gly Gln Thr Val Gln Gly Gly Ser Ala Ser Thr Gly Gly Gln
                405                 410                 415

Asn Gly Val Gln Gln Ala Gln Asp Ala Gln Arg Ala Asp Gln Gly Asp
            420                 425                 430

Asp Gly Gly Ser Gly Lys Pro Lys Pro Ile Trp Asp Ser Asp Met Gln
        435                 440                 445

Met Tyr Tyr Val Ile Gly Pro Lys Asn Val Lys Tyr Tyr Ile Asp Asp
    450                 455                 460

Gln Thr Gly Tyr Leu Ile Asn Ser Val Thr His Asp Val Ser Asp Pro
465                 470                 475                 480

Ser Thr Gly Lys Val Val Met Thr Gly Gln Glu Leu Glu Ala Gly Gly
                485                 490                 495

Asp Gly Gly Ala Gly Gln Gly Gln Gly Gly Ala Gln Thr Gly Ala Gly
        500                 505                 510

Gly Ala Ala Ala Gly Gly Arg Pro Gln Lys Arg Asp Glu Gly Ser Asn
    515                 520                 525

Gly Asn Ala Gly Gln Thr Ser Gly Ser Gly Gly Asp Asp Thr Ser Ser
    530                 535                 540

Gln Gly Val Gly Gly His Glu Asn Gly Asn Ser Asn Ser Gly Gly Gly
545                 550                 555                 560

Pro Gly Arg Met Glu Lys Arg Ser Leu Arg His Arg Lys His Asp Lys
                565                 570                 575

Arg Gly Tyr Pro Ala Ala Ala Phe Gln Ala Ala Asn Pro Ala Leu Arg
            580                 585                 590

Glu Gln Ala Met Leu Ala Met Thr Arg Thr Ala Gln Gly Gly Asn Thr
        595                 600                 605

Ala Ala Ala Ala Ala Gly Lys Ser Gly Val Gly Gly Lys Val Leu Lys
    610                 615                 620

Ala Gly Val Gly Val Ala Ala Leu Val Ala Leu Val Met Gly Leu Asp
625                 630                 635                 640

Ser Tyr Ala His Pro Arg Pro Pro Gly Thr Ser Glu Ala Asn Pro Tyr
                645                 650                 655

Gly Leu Gln Ala Thr Asp Val Cys Asp Met Tyr Gly Glu Pro Ile Ile
            660                 665                 670

Arg Asp Phe Arg Gly Val Leu Trp Asn Lys Ser Thr Tyr Thr Arg Val
        675                 680                 685

Asp Pro Asn Asn Pro Pro Pro Leu Tyr Gln Cys Pro Pro Asp Gln Gly
    690                 695                 700

Gln Ala Gly Ala Ala Gly Gly Asp Ala Gly Ala Gly Tyr Gly Pro Gln
705                 710                 715                 720

Gln Gln Lys Arg Asp Leu His Lys Arg Gly Leu Val Ser Ala Ile Ser
                725                 730                 735

Ala Phe Asp Asp Val Ala Ala Val Asn Asn Ala Gly Pro Gln Asn Glu

740                 745                 750

Gln Lys Arg Ser Leu Glu Lys Arg Gln Met Lys Ala Met Ala Ala Leu
        755                 760                 765

Leu Pro Ser Leu Ser Ser Ser Gly Thr Met Ala Val Val Gly Thr Val
    770                 775                 780

Val Val Thr Ala Ile Trp Leu His Tyr Val His Glu Gln Lys Leu Lys
785                 790                 795                 800

Ala Leu Gln Asp Gln Trp Asn Ser Gln Tyr His Ser Ala Gly Glu Ala
                805                 810                 815

Ala Ala Ser Val Gly Asn Ala Ile Ser Thr Gly Ala Asn Gly Gln Leu
            820                 825                 830

Phe Asn Pro Ile Thr Gly Ser Leu Ile Leu Val Asp Pro Asn Thr Gln
        835                 840                 845

Gln Tyr Tyr Asp Ser Ser Thr Gly Glu Gln Val Asp Pro Lys Thr Gly
    850                 855                 860

Met Pro Ala Arg Tyr Gly Asp Gly Ser Asp Gly Arg Lys Lys Lys Lys
865                 870                 875                 880

Lys Gln Glu Gln Pro Leu Gln Leu Pro Leu Asp Ser Asn Gly Asn Leu
                885                 890                 895

Ser Gly Asp Ser Pro Gln Val Gln Gln Ala Met Ala Gln Trp Asn Ser
            900                 905                 910

Leu Ser Pro Gln Gln Gln Gln Gln Ile Leu Ala Gln Tyr Gly Ala Ala
        915                 920                 925

Ala Asp Gln Ile Leu Asn Ser Pro Asp Pro Asn Ala Ala Leu Gln Ala
    930                 935                 940

Trp Asn Arg Gln His Ser Leu Ala Leu Gln Gln Gln Phe Val Pro Gln
945                 950                 955                 960

Gln Gln Gln Gln Phe Ala Pro Gln Gln Leu Ala Pro Gln Gln Gln Met
                965                 970                 975

Met Gln Pro Ala Ala Gly Gly Ala Ser Gly Leu Gly Gly Asp Gly Ser
            980                 985                 990

Gly Ala Gly Met
        995

<210>  62
<211>  953
<212>  PRT
<213>  Ustilago maydis

<400>  62

Met Lys Val Ala Leu Leu Cys Ala Ile Val Phe Gly Ala Ile Ser Val
1               5                   10                  15

Ala Ala Ser Ser Ser His Tyr Val Ala Asp Glu Gln Phe Ile Gln Arg
            20                  25                  30

Gly Val Pro His Arg Ser Ser Ala Ala Ser Lys Ala Lys Arg Asp Ala
        35                  40                  45

Asn Gly Ser Lys Val Gly Lys Lys Glu His Leu Leu Lys Pro Arg Asp
    50                  55                  60

Glu Thr Ser Val Val Arg Leu Gly Val Asp Ala Leu Lys Gly Ala Leu
65                  70                  75                  80

Gly Asp Gly Ala Lys Gly Gly Glu Thr Ile Ile Asn His Tyr His Ala
                85                  90                  95

Ser Pro Asn Ala Ala Ala Ala His Met Pro Gly Gly Ala Pro Met Ser
            100                 105                 110

His Pro Pro Ala Pro Tyr Tyr Pro Pro Pro Ala Tyr Gly His Asp Pro
        115                 120                 125

Gly Met Ala Ala Arg Phe Asp Lys Leu Asp Gln Lys Leu Gly Lys Leu
    130                 135                 140

Lys Glu Gly His Lys Ala Asn Ala Gln Gln Ile Gln Ile Met Gln Glu
145                 150                 155                 160

Leu Gln Ala Lys Gln Met Gln Ile Ala His Ala Glu Ala Ser Gly Ala
                165                 170                 175

Gln Ala Ala Ala Ala Val Pro Ala Ala Ala Lys Lys Arg Gly Met Ser
            180                 185                 190

Lys Thr Lys Met Ala Leu Leu Gly Gly Gly Gly Leu Ala Ala Gly Tyr
        195                 200                 205

Gly Leu Thr Lys Val Phe Asp Thr Lys Pro Ser Asp Ser Thr Thr Ala
    210                 215                 220

Tyr Asn Gln Gln Asp Met Ala Ala Leu Gln Ala Ala Gly Gly Ala Ala
225                 230                 235                 240

Pro Gly Ser Thr Pro Val Asp Gly Tyr Pro Gln Gly Ala Asp Pro Ser
                245                 250                 255

Gln Gly Ala Met Pro Gln Gly Ala Ala Gly Gln Asp Pro Ser Ala Gly
            260                 265                 270

Gly Ala Gly Gly Gly Ser Ala Gln Ile Ala Asp Gly Pro Tyr Arg Ile
        275                 280                 285

Pro Gly Ser Asp Leu Val Trp Val Thr Asp Arg Gln Gly Gln Tyr His
    290                 295                 300

Val Leu Lys Tyr Thr Pro Gln Gly Met Val Glu Val Asn Pro Pro Ala
305                 310                 315                 320

Gly Trp Gln Pro Pro Ser Ala Ala Gly Ser Gly Ala Ala Pro Gly Thr
                325                 330                 335

Ser Gly Gly Ala Gly Ala Pro Val Ala Ser Asp Gly Ser Ala Gly Ala
            340                 345                 350

Ser Thr Gly Ala Asp Gly Ala Ala Gly Gly Ala Gly Ser Ala Ala Gly
        355                 360                 365

Ala Asn Pro Val Gln Ser Gly Asp Ser Ser Ser Ser Gly Ala Gly Lys
    370                 375                 380

Gly Ala Gly Gly Gln Asn Pro Asn Gly Ala Ser Gln Asp Ala Gln Thr
385                 390                 395                 400

Ala Gly Ala Ala Gly Asn Gln Ser Gly Gly Ser Pro Lys Pro Glu Tyr
                405                 410                 415

Asp Ser Ala Ala Gly Met Tyr Tyr Val Val Gly Pro Asn Ser Val Lys
            420                 425                 430

Tyr Tyr Ile Asp Ser Gln Thr Gly Tyr Leu Ile Asn Ala Asn Thr Gly
        435                 440                 445

Asp Val Ser Asp Pro Lys Thr Gly Lys Ile Val Tyr Ser Ala Glu Glu
    450                 455                 460

Leu Met Asn Gly Ala Thr Gly Asn Asp Asn Gly Ala Gly Gln Gly Gln
465                 470                 475                 480

Ser Gln Ala Asn Gly Gly Gly Gln Ser Ser Gly Ala Gly Ala Gly Ala
                485                 490                 495

Gly Asn Gly Arg Met Gln Lys Arg Ser Leu Arg His Arg Lys Leu Asp
            500                 505                 510

Lys Arg Asp Tyr Ala Asn Ala Ala Phe Gln Ala Ser Ser Pro Ala Leu
        515                 520                 525

Arg Glu Gln Ala Gln Leu Ala Leu Leu Arg Gly Pro Asn Gln Ala Asn
    530                 535                 540

Pro Gly Ala Leu Ser Gly Thr Ala Thr Lys Ala Gly Pro Gly Leu Gly
545                 550                 555                 560

Lys Met Ala Ala Lys Gly Ala Leu Gly Val Gly Ala Leu Val Ala Leu
                565                 570                 575

Val Leu Gly Leu Gly Ala Tyr Ala His Pro Thr Arg Pro Asn Thr Thr
            580                 585                 590

Pro Asp Asn Glu Tyr Gly Leu Thr Pro Thr Ser Val Cys Asp Met Tyr
        595                 600                 605

Gly Glu Pro Ile Ile Arg Asp Phe Arg Gly Val Leu Trp Asn Lys Ser
    610                 615                 620

Thr Tyr Thr Arg Val Asp Leu Asn His Pro Pro Pro Leu His Gln Cys
625                 630                 635                 640

Ser Glu Gln Glu Leu Ala Asp Ala Gly Val Ser Val Gly Gly Gly Ala
                645                 650                 655

Gly Gly Ala Ala Pro Met Gln Lys Arg Ala Ser Lys Asp Arg Asp Ala
            660                 665                 670

Pro Gln Asp Arg Gln Leu Leu Ala Ala Val Arg Glu Asn Ile Asn Gln
        675                 680                 685

Glu His Leu Ser Lys Thr Ser Ser Val Glu Leu Ser Lys Arg Ser Gly
    690                 695                 700

Ser Asn Asp Gln Ser Leu Glu Lys Arg Gly Met Ser Gly Val Thr Ser
705                 710                 715                 720

Leu Leu Pro Ser Met Thr Thr Ser Gly Thr Met Ala Val Gly Ala Thr
                725                 730                 735

Leu Leu Ile Thr Ala Ile Tyr Leu His Asn Ile His Lys Glu Lys Leu
            740                 745                 750

Gln Ala Leu Lys Asp Gln Trp Asn Gly Gln Tyr His Ser Ala Gly Asp
        755                 760                 765

Ala Ala Ser Ala Val Gly Asn Ala Ile Asp Thr Gly Ala Asn Gly Gln
    770                 775                 780

Leu Phe Asn Pro Ile Thr Gly Asn Leu Val Leu Val Asp Ala Thr Thr
785                 790                 795                 800

Asn Met Tyr Tyr Asp Ser Gly Thr Gly Glu Gln Val Asn Pro Lys Thr
                805                 810                 815

Gly Met Pro Phe Lys Tyr Gly Gly Gly Asp Asn Asn Ser Ser Glu Ser
            820                 825                 830

Ser Asp Lys Gln Ala Pro Val His Leu Pro Leu Asp Arg Asn Gly Asn
        835                 840                 845

Met Ser Gly Asp Ser Pro Glu Val Gln Gln Ala Leu Ala Lys Trp Asn
    850                 855                 860

Thr Met Ser Pro Gln Gln Gln Gln Gln Leu Leu Ala Gln Tyr Gly Asp
865                 870                 875                 880

Ala Ala Ser Gln Ile Leu Asn Ser Pro Asp Pro Asn Ala Ala Leu Gln
                885                 890                 895

Ala Trp Ser Ala Gln Gln Gly Ala Ala Gln Pro Ile Ala Pro Gln Leu
            900                 905                 910

Ile Pro Gln Gln Gln Gln Leu Ala Pro Gln Gln Tyr Gln Thr Leu Val
        915                 920                 925

Pro Gly Gln Gln Gln Ser Pro Pro Ala Ser Gly Gly Ala Phe Gly Ser
    930                 935                 940

Ser Asp Thr Ala Ala Thr Ser Asp Met
945                 950

<210>  63

<211>  978
<212>  PRT
<213>  Ustilago esculenta

<400>  63

Met Lys Val Ala Leu Phe Thr Ala Ile Val Phe Gly Ala Ile Ser Val
1               5                   10                  15

Ala Ala Thr Ser Ser Gln Ala Leu Ala Asp Lys Arg Asp Val Gln His
            20                  25                  30

Ser Ala His Arg Arg Ser His Ala Ser Val Lys Lys Arg Asp Ser Ser
        35                  40                  45

Ser Lys Gln Pro Arg Ala Glu His Asp His Ala Glu Gln His Ile Ala
    50                  55                  60

Ser Ser Pro Gln Pro Phe Val Lys Arg Thr Asp Ala Lys Pro Ile Tyr
65                  70                  75                  80

Glu Ile Gly Asp Asn Leu Ala Asn Val Leu Lys Gly Thr Leu Gly Asp
                85                  90                  95

Gly Lys Lys Gly Gly Asp Thr Ile Ile His His Tyr Gly Pro Gly Ala
            100                 105                 110

Gly Pro His Val Pro Gly Gly Ala Pro Tyr Thr His Tyr Pro Pro Ser
        115                 120                 125

His Tyr Pro Pro Pro Tyr Gln Gly Gly Asp Pro Ala Met Ala Ala Arg
    130                 135                 140

Met Asp Lys Ile Asp Lys Arg Leu Gly Lys Leu Ala Asp Gly His Lys
145                 150                 155                 160

Ala Asn Thr Gln Gln Ile Glu Leu Met Glu Gln Leu His Ala Gln Arg
                165                 170                 175

Met Ala Glu Ser Ala Asn Ala Glu Arg Ala Ala Ala Ala Ala Ala His
            180                 185                 190

Pro Pro Lys Lys Ser Gly Phe Gly Lys Gly Ala Ile Ala Leu Ser Val
        195                 200                 205

Ile Gly Gly Leu Gly Val Gly Tyr Gly Leu Thr Lys Met Leu Asp Thr
    210                 215                 220

Lys Pro Ser Asp Ser Thr Thr Gly Tyr Ser Pro Glu Asp Met Ala Ala

225                 230                 235                 240

Leu Gln Ala Ala Gly Gly Ala Gly Pro Gly Ser Ala Pro Val Asn Gly
                245                 250                 255

Tyr Pro Pro Ser Gln Ala Ser Gly Gly Gln Ala Gln Gly Gly Ala Asp
            260                 265                 270

Asp Ser Met Ala Ala Tyr Pro Ile Gln Gly Thr Ser Leu Val Trp Ala
        275                 280                 285

Ile Asp Arg Gln Asn Val Val His Val Tyr Asp Lys Asp Gln Asn Tyr
    290                 295                 300

Gln Glu Val Pro Trp Pro Pro Gly Trp Gln Pro Pro Ile Pro Ser Gly
305                 310                 315                 320

Ala Gly Gly Gly Gly Gly Val Asp Pro Gly Thr Gly Ala Gly Gly Ser
                325                 330                 335

Thr Ser Ala Arg Ser Gln Ala Gly Gly Ser Ser Asn Tyr Pro Ser Gly
            340                 345                 350

Ala Pro Gln Pro Asp Gly Arg Pro Gln Arg Thr Trp Asp Pro Gln Ala
        355                 360                 365

Gln Met Tyr Phe Val Ile Gly Pro Asp Asn Val Arg Tyr Tyr Ile Asp
    370                 375                 380

Ser Gln Ser Gly Tyr Leu Ile Asn Ser Val Thr Gly Asp Val Ile Asp
385                 390                 395                 400

Pro Gln Thr Gly Gln Ile Val Tyr Arg Gly Ala Asp Leu Met Lys Asn
                405                 410                 415

Gly Ser Gly Gly Gln Ala Gly Gly Gly Gly Gln Ala Gly Gly Gly Gly
            420                 425                 430

Gln Thr Gly Gly Ala Gly Ala Gly Gly Ala Gly Ala Gly Gly Ala Gly
        435                 440                 445

Ala Gly Gly Ala Gly Ala Gly Gly Ala Gly Ala Gly Gly Ala Gly Ala
    450                 455                 460

Gly Gly Ala Gly Ala Gly Gly Ala Gly Ala Gly Gly Ala Gly Ala Gly
465                 470                 475                 480

Gly Ala Gly Ala Gly Gly Ala Gly Ala Gly Gly Ala Gly Ala Gly Ala
                    485                 490                 495

Gly Gly Ala Gly Ala Gly Gly Ala Gly Ala Gly Gly Gly Ala Asp Gly
                500                 505                 510

Gly Asp Gly Gly Gly Gly Phe Pro Ser Arg Asp Thr Gly Ser Ile Arg
            515                 520                 525

Lys Arg Phe Val Arg Gln Arg Lys Leu Asp Lys Arg Gln Tyr Thr Ser
    530                 535                 540

Ala Ala Leu Tyr Ala Thr Asn Pro Ala Leu Arg Glu Gln Ala Arg Leu
545                 550                 555                 560

Ala Met Leu Arg Gly Glu Ser Ala Asn Gly Leu Pro Ala Gly Met Ala
                565                 570                 575

Lys Pro Pro Gly Asn Gly His Thr Gly Leu Lys Val Ala Val Gly Leu
                580                 585                 590

Gly Ala Leu Val Ala Leu Val Leu Gly Leu Gly Ala Tyr Ala His Pro
            595                 600                 605

Thr Pro Pro Asn Thr Thr Pro Asp Asn Glu Tyr Gly Leu Thr Pro Thr
    610                 615                 620

Asn Val Cys Asp Ile Tyr Gly Gln Pro Ile Ile Arg Asp Phe Arg Gly
625                 630                 635                 640

Val Leu Trp Asn Lys Ser Thr Tyr Ala Arg Leu Asp Leu Lys Asn Pro
                645                 650                 655

Pro Pro Leu His Tyr Cys Ser Gln Gln Glu Leu Ser Asp Ala Gly Tyr
                660                 665                 670

Ala Pro Gln Thr Gly Gly Ala Thr Pro Val Gln Lys Arg Glu Leu Gln
            675                 680                 685

Asp Thr Arg Thr Phe Ala Glu Leu Ile Thr Val Val Lys Arg Asp Val
    690                 695                 700

Ala Arg Gly Thr Thr Ala Ser Arg Val Glu Asp Gly Thr Arg Lys Asp
705                 710                 715                 720

Ser Glu Gln Asp Pro Thr Gly Met Ser Gly Asp His Gly His Ser Leu
                725                 730                 735

Glu Lys Arg Gln Gly Lys Met Leu Ala Ala Gly Ala Ala Leu Leu Pro
            740                 745                 750

Ser Met Thr Gly Ser Gly Val Leu Ala Phe Gly Ala Ala Thr Leu Ile
        755                 760                 765

Thr Ala Val Tyr Leu Tyr Ser Ile His Ala Lys Lys Glu Lys Ala Ala
    770                 775                 780

Phe Asp Gln Trp Asn Ser Met Tyr His Ser Pro Gly Glu Ala Ala Ser
785                 790                 795                 800

Ser Val Gly Asn Ala Ile Gly Thr Gly Ala Asn Gly Gln Leu Phe Asn
                805                 810                 815

Pro Leu Thr Gly Asn Leu Ile Leu Val Asp Pro Ser Thr Asn Leu Tyr
            820                 825                 830

Tyr Asp Ser Gly Ser Gly Glu Gln Val Asn Pro Lys Thr Gly Met Pro
        835                 840                 845

Phe Arg Tyr Gly Gly Gly Asp Ser Gly Ser Gly Ser Asp Ser Ala Ala
    850                 855                 860

Asp Ala Gly Thr Asn Ala Pro Leu Pro Gln Leu Pro Leu Asp Ser Arg
865                 870                 875                 880

Gly Asn Met Ser Ser Glu Ser Pro Glu Val Gln Gln Ala Leu Ala Ser
                885                 890                 895

Trp Asn Ala Met Ser Pro Gln Gln Gln Gln Gln Leu Trp Ala Gln Tyr
            900                 905                 910

Gly Ser Thr Ala Asn Gln Ile Leu Ser Ser Pro Asp Pro Arg Ala Ala
        915                 920                 925

Phe Gln Ala Trp Ser Ser Gln Gln Val Gln Pro Gln Pro Tyr Pro Pro
    930                 935                 940

Gln Gln Pro Gln Pro Tyr Ser Pro Gln Gln Pro Gln Pro Tyr Ser Pro
945                 950                 955                 960

Gln Gln Pro Gln Pro Tyr Pro Pro Gln Gln Pro Gln Pro Tyr Pro Tyr
                965                 970                 975

Gly Ala

<210>  64
<211>  1073
<212>  PRT
<213>  Ustilago trichophora

<400>  64

Met Lys Val Ala Leu Leu Cys Gly Ile Val Phe Gly Ala Ile Ser Ala
1               5                   10                  15

Ala Ala Ser Ser Ser Gln Ala Leu Ala Asp Glu His Phe Gly Leu His
            20                  25                  30

Gly Gly His His His Arg Ser Arg Gly Ser Thr Lys Asp Asn Gly Gln
        35                  40                  45

Ala Gly Asn Lys Gly Ala Ala Pro Lys Asp Gln His His Ala Lys His
    50                  55                  60

His Gly His Lys His Arg Pro His Thr Glu Arg Glu Arg Ala Ser Thr
65                  70                  75                  80

His Glu His Leu Ser Lys Arg Ala Ala Phe Asn Ala Ala Glu Ala Leu
                85                  90                  95

Lys Ala Ala Asp Gly Leu Glu Asn Ile Gly Lys Ala Leu Arg Ser Ser
            100                 105                 110

Ala Gly Glu Val Ser Gly Arg Ser Lys Val Val Asn Asn Phe Tyr Gly
        115                 120                 125

Val Pro Pro Pro Ala Ser His Val Pro Gly Gly Ala Pro Tyr Ser His
    130                 135                 140

Met Leu Gly Gly Gly Leu Pro His Gln Tyr Pro Pro Pro Tyr Ala Pro
145                 150                 155                 160

His Tyr Pro Pro Met Pro Pro Ile Gly Thr Asp Pro Ala Leu Ala Gly
                165                 170                 175

Lys Ile Asp Lys Leu Glu Lys Ser Val Gly Lys Leu Lys Asp His Ser
            180                 185                 190

Lys Val Asn Ala Glu Gln Ile Lys Leu Met Gln Glu Leu Gln Ala Lys
        195                 200                 205

Gln Met Ala Ala Glu Ala Asn Ala Ala Glu Arg Thr Ala Leu Met Thr
    210                 215                 220

Thr Gln Gln Gly Lys Lys Gly Met Ser Lys Thr Lys Met Ala Met Leu
225                 230                 235                 240

Gly Ile Gly Gly Val Ala Ala Gly Trp Gly Leu Thr Lys Leu Leu Asp
                245                 250                 255

Thr Lys Pro Thr Asp Ser Thr Thr Gly Tyr Asn Pro Gln Asp Met Ala
            260                 265                 270

Ala Leu Arg Gly Ala Gly Gly Gly Pro Pro Ala Gly Gly Ala Pro Val
        275                 280                 285

Asp Gly Ser Gly Gln Gly Gln Asp Asp Gly Phe Ser Ile Leu Lys Gly
    290                 295                 300

Pro Asp Glu Ile Pro Gly Thr Asp Leu Val Leu Val Gln Leu Lys Asn
305                 310                 315                 320

Gly Glu Tyr His Ala Leu Thr Lys Asp Gly Lys Lys Glu Leu Pro Leu
                325                 330                 335

Pro Pro Gly Trp Thr Pro Gln Asp Pro Leu Pro Gly Met Gln Gly Pro
            340                 345                 350

Pro Ala Gly Ser Gly Arg Asp Gly Gly Gln Glu Gln Gly Gln Tyr Pro
        355                 360                 365

Ser Gly Ala Gly Gln Gly Gly Gly Gln Gly Gly Gly Gln Gly Gly Gly
    370                 375                 380

Gln Gly Gly Gly Gln Gly Gly Glu Asp Gly Ala Gln Ala Pro Gln Gly
385                 390                 395                 400

Val Asp Gly Arg Gln Asp Pro Arg Asn Glu Gly Leu Val Asp Asp Gly
                405                 410                 415

Gln Ile Arg Gly Thr Ala Thr Pro Gln Arg Gly Gly Gly Gln Gly Asn
            420                 425                 430

Asp Gly Pro Lys Pro Met Phe Asp Lys Gln Val Gln Met Phe Tyr Val
        435                 440                 445

Leu Gly Pro Asp Asn Thr Lys Tyr Phe Ile Glu Pro Gln Thr Gly Phe
    450                 455                 460

Leu Ile Asn Ser Gln Thr Phe Glu Val Ser Asp Pro Lys Thr Gly Lys

465                 470                 475                 480

Ile Val Lys Thr Lys Glu Gln Ile Met Gln Glu Ser Gly Asp Asp Leu
                485                 490                 495

Gly Ala Gly Gly Thr Asp Gly Ala Gly Gly Val Gly Glu Ala Gly Gly
            500                 505                 510

Ala Ala Gly Gly Gly Ala Gly Pro Ser Gly Pro Glu Ala Gly Gly Gly
        515                 520                 525

Arg Gly Ser Gly Gly Met Gln Lys Arg Ser Ile Arg His Arg Lys Val
    530                 535                 540

Asp Lys Arg Ser Tyr Glu Met Ala Ala Leu Gln Ala Ala Ser Pro Ala
545                 550                 555                 560

Leu Arg Glu Gln Ala Arg Leu Ala Ala Leu Arg Gly Asn Gln Tyr Ser
                565                 570                 575

Ala Ala Pro Met Met Asn Ala Ala Lys Lys Gly Gly Gly Gly Lys Thr
            580                 585                 590

Leu Leu Lys Ala Gly Ile Gly Val Gly Ala Leu Val Ala Val Val Ile
        595                 600                 605

Gly Leu Gly Asp Tyr Ala His Pro Met Arg Pro Asn Thr Thr Pro Asp
    610                 615                 620

Asn Glu Phe Gly Leu His Pro Thr Thr Val Cys Asp Ala Tyr Arg Gln
625                 630                 635                 640

Pro Ile Ile Ala Asp Phe Arg Gly Val Leu Trp Ser Lys Ser Thr Tyr
                645                 650                 655

Glu Arg Ile Asp Lys Gln Asn Pro Pro Pro Leu His Tyr Cys Thr Glu
            660                 665                 670

Gln Glu Leu Val Met Ala Gly Tyr Leu Gln Pro Asp Gly Gly Gly Ala
        675                 680                 685

Val Gly Gly Tyr Gly Gln Ala Gly Asp Gly Gly Ala Pro Met Gln Lys
    690                 695                 700

Arg Glu Met Pro Pro Lys Lys Pro Ala Asp Phe Glu Lys Leu Val Ser
705                 710                 715                 720

Ala Val Lys Asn Asp Val Glu Gln Gly Asn Glu Pro Asp Gly Ser Asn
                725                 730                 735

Gln Gly His Glu Lys Arg Ser Ile Gln Glu Ala Ser Ala Val Lys Glu
            740                 745                 750

Asp Ser Gln Ala Leu Glu Lys Arg Gly Met Leu Ala Lys Ala Gly Ala
        755                 760                 765

Leu Leu Val Pro Ser Met Thr Ala Ser Thr Gly Ile Leu Val Ala Gly
    770                 775                 780

Thr Ala Val Leu Thr Val Leu Tyr Leu His Ser Val His Gln Gln Lys
785                 790                 795                 800

Glu Lys Ala Arg Met Glu Glu Trp Asn Ser His Tyr His Ser Ala Gly
                805                 810                 815

Glu Ala Ala Ser Ala Val Gly Asn Ala Ile Ala Thr Gly Ala Asn Gly
            820                 825                 830

Gln Leu Phe Asn Pro Leu Thr Gly Asn Leu Val Leu Val Asp Pro Asn
        835                 840                 845

Ser His Asn Tyr Tyr Asp Ser Gly Thr Gly Glu Gln Val Asn Pro Lys
    850                 855                 860

Thr Gly Met Pro Phe Lys Tyr Gly Asp Asp Glu Ser Ser Glu Asn Ser
865                 870                 875                 880

Lys Gly Ala Gly Gly Ser Asn Gln Gln Val Pro Gln Leu Pro Ile Asp
                885                 890                 895

Asn Ser Gly Asn Val Ser Gly Asp Ser Pro Glu Val Gln Gln Ala Met
            900                 905                 910

Gln Gln Trp Asn Ser Met Ser Pro Gln Glu Gln Gln Gln Leu Met Asn
        915                 920                 925

Gln Tyr Gly Glu Ala Ala Asn Gln Ile Leu Asn Ser Pro Asn Pro Gln
    930                 935                 940

Ala Ala Phe Glu Asn Trp Ser Ser Gln Gln Ser Val Pro Pro Pro Gln
945                 950                 955                 960

Gln Tyr Pro Pro Gln Gln Gln Tyr Pro Pro Gln Gln Gln Tyr Pro Pro
                965                 970                 975

Gln Gln Gln Tyr Pro Leu Gln Gln Gln Tyr Pro Pro Gln Gln Gln Tyr
            980                 985                 990

Pro Pro Gln Gln Pro Gln Gln Gln  Phe Pro Ser Gln Gln  Pro Arg Tyr
        995                 1000                 1005

Met Pro  Gln Gln Gln Gln Gln  Tyr Ile Pro Gln Gln  Gln Pro Gln
    1010                 1015                 1020

Tyr Met  Leu Pro Gln Gln Gln  Pro Gln Tyr Met Leu  Gln Gln Gln
    1025                 1030                 1035

Pro Gln  Tyr Met Leu Gln Gln  Gln Pro Gln Gln Gln  Pro Gln Gln
    1040                 1045                 1050

Pro Ser  Ala Ser Ser Gly Gly  Ser Tyr Arg Gly Asp  Ala Gly Ala
    1055                 1060                 1065

Ala Ala  Pro Val Met
    1070

<210>  65
<211>  983
<212>  PRT
<213>  Ustilago hordei (host: oats)

<400>  65

Met Lys Val Ala Leu Leu Ser Ala Ile Val Phe Gly Ala Ile Ser Ala
1               5                   10                  15

Ala Ala Ser Ser Ser His Val Leu Ala Asp Glu Tyr Tyr Leu Gln Asn
            20                  25                  30

Phe Ala His Ala His Tyr Arg Ser Tyr Ser Ser Ala Lys Lys Ser Thr
        35                  40                  45

Lys Lys Ser Lys Asp Phe Glu Ser Lys Tyr Gln His Tyr Glu Tyr Gln
    50                  55                  60

Ser Lys His His Ser Lys His His Val Ser Lys Thr Ala Pro Leu Leu
65                  70                  75                  80

Lys Arg Val Asn Ala Gly Asn Thr Leu Glu Ile Gly Glu Gly Ala Ala
                85                  90                  95

Asn Val Ile Lys Ser Ala Leu Glu Gly Gly Lys Lys Ser Gly Asp Thr
            100                 105                 110

Ile Ile Asn His Tyr Gly Ala Ser Thr Ser Glu Ser Leu Gly Gly Arg
        115                 120                 125

Ile His Pro Gly Ser Gly Gly Asn Pro Gln Ala Tyr Ser Pro Tyr Ala
    130                 135                 140

His Pro Pro Tyr Ala His Pro Pro Tyr Ala His Pro Tyr Pro Pro Pro
145                 150                 155                 160

Gln Ala Ala Gly Gly Thr Asp Ala Leu Leu His Ala Lys Val Asp Arg
                165                 170                 175

Ile Gly Arg Lys Phe Thr Lys Leu Lys Gly Ala Gln Met Glu Asn Thr
            180                 185                 190

Lys Gln Ile Gln Val Leu Glu Glu Met Gln Ala Arg Thr Met Met Glu
        195                 200                 205

Glu Gln Ala Gly Gly Ala Arg Gly Ala Thr Ala Ala Lys Glu Pro Gly
    210                 215                 220

Ala Lys Glu Pro Glu Ala Glu Gln Lys Lys Arg Gly Phe Gly Lys Gly
225                 230                 235                 240

Ala Met Phe Leu Thr Ala Ala Gly Gly Ile Gly Ala Gly Val Leu Gly
                245                 250                 255

Thr Lys Leu Leu Asp Thr Val Pro Ala Val Ser Asp Thr Ala Tyr Arg
            260                 265                 270

Pro Glu Asp Met Ala Ala Met Gln Gln Ala Gly Ala Gly Thr Pro Gly
        275                 280                 285

Gly Ala Thr Ile Asp Gly Pro Thr Ala Gln Gly Gly Gln Gly Gln Gly
    290                 295                 300

Glu Ser Gln Gly Gly Gly Gly Gly Gly Ser Gly Gly Gly Gln Pro Glu
305                 310                 315                 320

Phe Leu Gly Pro Tyr Phe Asp Gln Ala Thr Gln Thr Arg Tyr Ile Ile
                325                 330                 335

Asp Gly Glu Gly Tyr Tyr His Phe Leu Gly Ser Gly Asn His Glu Ile
            340                 345                 350

Met Pro Pro Pro Gly Trp Ser Lys Asn Gly Pro Leu Arg Asp Asp Gly
        355                 360                 365

Ser Gly Gly Ala Glu Thr Asp Asp Gly Ser Asn Ser Gln Gln Glu Ala
    370                 375                 380

Ala Gly Gly Ala Ala Gly Gly Gly Ala Ala Arg Gly Ala Asp Ser Gly
385                 390                 395                 400

Pro Ala Ala Gly Phe Pro Gln Thr Ser Ala Asn Asp Ala Asp Val Ala
                405                 410                 415

Pro Gln Arg Asp Arg Gln Phe Pro Ser Ser Ala Glu Gly Gly Gln Arg
            420                 425                 430

His Ser Ser Asn Lys Pro Thr Leu Asp Asn Thr Ile Asn Met Tyr Tyr
        435                 440                 445

Val Asp Arg Gln Asp Gly Val Arg Phe Tyr Ile Asp Asn Lys Thr Gly
    450                 455                 460

Tyr Leu Val Asn Ser Val Thr Leu Asp Ile Ser Asp Pro Lys Thr Gly
465                 470                 475                 480

Gln Ile Ile Met Ser Gly Lys Asp Val Met Lys Gly Ser Ser Thr Gly
                485                 490                 495

Pro Gly Ser Ala Ala Gly Gly Asn Ala Gly Gly Asn Ala Gly Gly Asn
            500                 505                 510

Ala Gly Gly Asn Ala Gly Arg Met Lys Lys Arg Ser Ile Arg His His
        515                 520                 525

Lys Phe His Lys Arg Ser Tyr Glu Gln Ala Ala Leu Tyr Ala Gln Ser
    530                 535                 540

Ser Ala Leu Arg Ala Gln Ala Arg Ile Ala Met Leu Arg Asp Ser Gly
545                 550                 555                 560

Arg Leu Gln Pro Thr Val Ser Ala Ala Ala Arg Lys Ala Gly Ile Gly
                565                 570                 575

Lys Thr Leu Val Lys Val Gly Val Gly Ala Ser Val Leu Ile Ala Thr
            580                 585                 590

Ile Leu Thr Leu Glu Asp Phe Ala His Pro Lys Arg Pro Gly Thr Ser
        595                 600                 605

Pro Asp Asn Glu Tyr Gly Leu Val Pro Thr Ser Ile Cys Asp Val Tyr

610                 615                 620

Gly Glu Pro Ile Val Gln Asp Phe Arg Asn Val Leu Trp Asn Lys Ser
625                 630                 635                 640

Thr Leu Thr Arg Leu Asp Pro Lys Asn Leu Pro Pro Leu His Thr Cys
                645                 650                 655

Ser Lys Gln Glu Leu Glu Asp Ala Gly Val Ser Thr Glu Gln Gln Gly
            660                 665                 670

Ala Met Gln Lys Arg Asp Ser Val Glu Ser Lys Ser Asp Gln Met Ala
        675                 680                 685

Lys Leu Val Gln Asp Ala Lys Ala Asp His Ser Glu His Asn Leu Arg
    690                 695                 700

Lys Arg Ser Glu Thr Asn Thr Lys Glu Arg Asp His Ser Leu Glu Lys
705                 710                 715                 720

Arg Gln Gly Glu Gly Ala Val Ala Ser Thr Val Ala Lys Gly Glu Gly
                725                 730                 735

Ala Val Ala Ser Thr Val Ala Lys Gly Ala Gly Leu Ala Phe Leu Pro
            740                 745                 750

Ser Ile Gly Trp Ala Gly Ala Ala Ala Ile Gly Ala Thr Ala Leu Val
        755                 760                 765

Thr Ala Ile Tyr Leu His Asn Ile His Lys Glu Lys Glu Leu Ala Ala
    770                 775                 780

Met Gln Glu Trp Asn Ser Gln Tyr His Ser Ala Gly Glu Ala Ala Ser
785                 790                 795                 800

Ser Val Gly Asn Ala Ile Ser Thr Gly Ala Asn Gly Gln Leu Phe Asn
                805                 810                 815

Pro Leu Thr Gly Ser Leu Ile Met Val Asp Pro Asn Thr Gly Leu Tyr
            820                 825                 830

Tyr Asp Ala Ser Thr Gly Glu Gln Val Asn Pro Lys Thr Gly Met Pro
        835                 840                 845

Ser Arg Tyr Gly Gly Glu Asp Ser Ser Gln Gln Gln Gln Gln Gln Gln
    850                 855                 860

Ala Pro Gln Leu Pro Leu Asp Asn Ala Gly Asn Met Ser Gly Asn Ser
865                 870                 875                 880

Pro Glu Val Gln Lys Ala Met Glu Gln Trp Asn Leu Met Ser Pro Gln
                885                 890                 895

Gln Gln Gln Gln Leu Leu Asn Gln Tyr Gly Ser Ala Ala Gln Gln Ile
            900                 905                 910

Leu Asp Ser Pro Asp Pro Asn Ala Ala Leu Gln Ala Trp Ser Gln Gln
        915                 920                 925

Gln Ala Gln Ala Val Gln Pro Val Val Thr Gln Arg Gly Pro Val Ile
    930                 935                 940

Pro Gln Ser Gln Gln Gln Gln Gln Gln Leu Pro Pro Ser Arg Tyr Asn
945                 950                 955                 960

Met Ala Val Ser Asn Thr Ala Ser Ser Tyr Asp Gly Ala Gly Gly Gly
                965                 970                 975

Ala Gly Ala Gly Pro Ala Met
            980

<210>  66
<211>  945
<212>  PRT
<213>  Ustilago tritici

<400>  66

Met Lys Val Ala Leu Leu Cys Val Ile Val Phe Gly Ala Ile Ser Val
1               5                   10                  15

Ala Ala Ser Ser Ser Gln His Val Ala Asp Glu His Phe Val Gln His
            20                  25                  30

Ser Gly Gly His Arg Pro Lys Ala Ser Thr Lys Asn Arg Ser Ala Lys
        35                  40                  45

Lys Lys Ser Ala Ala Ser His His His Tyr Asp Lys His Glu His Lys
    50                  55                  60

Lys Asp Phe Thr Pro Val His Ala Lys His Leu Val Lys Arg Ala Glu
65                  70                  75                  80

Pro Pro Met Ala Gly Ile Ala Asp Ile Gly Glu Asn Met Ala Gly Val
                85                  90                  95

Leu Lys His Ala Met Gly Gly Gly Glu Arg Gly Ala Gly Val Ile Asn
        100                 105                 110

Arg Tyr Gly Ala Ser Ala Ala Glu His Val Pro Val Gly Thr Ala His
    115                 120                 125

Pro Tyr Tyr Ser Pro Pro Val Tyr Gly His Asp Ala Ala Ser Ala Ala
    130                 135                 140

Ser Leu Ala Lys Ile Asn Ser Lys Leu Asp Lys Leu Lys Asp Gly His
145                 150                 155                 160

Lys Ile Asn Ser Gln Gln Ile Glu Leu Met Gln Glu Leu Gln Ala Lys
                165                 170                 175

Gln Met Ala Asn Thr Glu Ala Ala Ala Ala Ala Ala Ala Lys Thr Lys
        180                 185                 190

Pro Gly Thr Asn Trp Gly Lys Met Ala Met Met Gly Ile Gly Gly Ile
    195                 200                 205

Gly Leu Gly Tyr Gly Ala Thr Lys Leu Phe Asp Thr Lys Pro Ser Asp
    210                 215                 220

Ser Thr Thr Gly Tyr Ser Gln Gln Asp Met Ala Ala Leu Gln Gly Gly
225                 230                 235                 240

Gly Ala Ala Pro Pro Gly Ser Val Pro Val Asp Gly Ala Pro Gly Ala
                245                 250                 255

Ala Gly Gly Gln Ser Val Gln Gly Gly Asp Pro Gly Tyr Asp Gly Pro
        260                 265                 270

Tyr Thr Ile Pro Asn Ser Lys Leu Val Trp Ile Val Asp Arg Ser Asn
    275                 280                 285

Gln Val His Val Leu Asp Pro Ala Gln Ser Tyr Ala Glu Val Pro Ala
    290                 295                 300

Pro Pro Gly Trp Gln Pro Pro Gly Ala Thr Leu Thr Gly Pro Ala Ser
305                 310                 315                 320

Ala Ser Gly Ala Gly Ala Gly Thr Gly Thr Gly Thr Gly Ala Gly Ala
                325                 330                 335

Ala Gly Ala Ala Gly Ala Gly Ala Ala Gly Ala Gly Ala Ala Asp Ser
        340                 345                 350

Ala Ser Thr Ala Gln Ala Asn Asp Gly Ser Ala Ser Ser Val Ala Gln
        355                 360                 365

Gly Ala Gly Gly Gln Thr Thr Gln Gly Gly Ser Gly Val Ser Ser Gln
    370                 375                 380

Lys Val Ala Gln Asp Thr Gly Ala Ala Ala Gly Gln Gly Asp Gly Ser
385                 390                 395                 400

Ser Lys Pro Ile Tyr Asp Lys Ala Ile Gly Met Tyr Tyr Val Val Gly
                405                 410                 415

Pro Gln Gly Val Lys Tyr Tyr Leu Asp Pro Gln Ser Gly Leu Leu Ile
            420                 425                 430

Asn Ser Val Thr Met Asp Ala Ser Asp Pro Lys Thr Gly Lys Ile Ile
        435                 440                 445

Tyr Ser Gly Lys Asp Leu Glu Asn Ile Ser Val Arg Gly Asp Gly Gly
    450                 455                 460

Ala Ala Ala Ala Gly Gly Ser Gly Ser Gly Gln Gly Gln Val Pro Asp
465                 470                 475                 480

Gly Ala Gly Gly Ser Pro Ala Gly Ala Gly Gly Asn Ser Ala Gly Ala
                485                 490                 495

Gly Ala Gly Lys Ser Ala Gly Gly Met Gln Lys Arg Ser Leu Arg Arg
            500                 505                 510

Arg Lys Leu Asp Lys Arg Ser Tyr Gly Thr Ile Ala Thr Gln Ala Thr
        515                 520                 525

Asn Pro Ala Leu Arg Ala Gln Ala Gln Ile Ala Met Leu Arg Glu Ser
    530                 535                 540

Gly Gly Ser Ala Ala Ala Ser Ala Val Lys Gly Gly Trp Gly Lys Thr
545                 550                 555                 560

Ala Ala Lys Ile Gly Leu Gly Val Ala Gly Leu Ala Ala Leu Val Ile
                565                 570                 575

Gly Leu Gly Glu Tyr Ala His Pro Gln Leu Pro Asn Thr Thr Glu Asp
            580                 585                 590

Asn Glu Tyr Gly Leu Thr Pro Thr Ser Val Cys Asp Ile Tyr Gly Glu
        595                 600                 605

Pro Ile Ile Met Asp Phe Lys Gly Arg Leu Trp Asn Lys Ser Thr Tyr
    610                 615                 620

Thr Gln Val Asp Met Asn Asn Pro Pro Pro Leu His Gln Cys Ser Gln
625                 630                 635                 640

Gln Glu Leu Ala Asp Ser Gly Val Ala Pro Thr Gly Pro Val Gly Pro
                645                 650                 655

Gly Met Ser Gln Gln Lys Arg Asn Ile His Ser Gly Glu Leu Pro Ala
            660                 665                 670

Gly Leu Ile Ile Ala Val Lys Arg Glu Val Asp Asn Asp Ser Ser Ala
        675                 680                 685

Glu Arg Gln Glu His Ala Glu Gly Glu Asn His Thr Leu Glu Lys Arg
    690                 695                 700

Gln Gly Lys Ala Ala Gly Ala Gly Ala Gly Ala Leu Gly Met Ala Ser
705                 710                 715                 720

Met Leu Ile Pro Ser Met Ser Thr Gly Thr Ala Trp Ala Leu Gly Ala
                725                 730                 735

Gly Val Leu Val Thr Ala Leu Tyr Leu His Gly Ile His Glu Gln Lys
            740                 745                 750

Leu Lys Ala Ala Gln Asp Glu Tyr Asn Ser Lys Phe His Ser Ala Gly
        755                 760                 765

Glu Ala Ala Ser Ser Val Gly Asn Ala Ile Ser Thr Gly Ala Asn Gly
    770                 775                 780

Gln Leu Phe Asn Pro Ile Thr Gly Asn Leu Ile Leu Val Asp Pro Asn
785                 790                 795                 800

Thr Asn Leu Tyr Tyr Asp Ser Gly Thr Gly Glu Gln Val Asn Pro Lys
                805                 810                 815

Thr Gly Met Pro Gly Gly Ser Asp Asp Asp Ala Ser Ser Ser Ser Lys
            820                 825                 830

Gln Gln Gln Val Gln Leu Pro Ile Asp Lys Asn Gly Asn Val Ser Gly
        835                 840                 845

Asp Ser Pro Gln Val Gln Gln Ala Met Ala Lys Trp Ser Ser Met Ser

850                 855                 860

Pro Gln Gln Gln Gln Gln Leu Leu Ala Gln Tyr Gly Asp Val Ala Asn
865                 870                 875                 880

Gln Ile Leu Asn Ser Pro Asp Pro Asn Ser Ala Leu Gln Ala Trp Ser
                885                 890                 895

Asn Lys Gln Ala Gln Ala Ser Ser Leu Ala Ile Gln Gln Ala Gln Gln
            900                 905                 910

Gln Gln Val Ala Pro Gln Gln Gln Phe Ser Pro Gln Gln Gln Gln Phe
        915                 920                 925

Ala Pro Gln Gln Ala Ala Gly Ala Gly Leu Ser Ala Ala Gly Ser Gly
    930                 935                 940

Met
945

<210>  67
<211>  177
<212>  PRT
<213>  Melanopsichium pennsylvanicum

<400>  67

Met Arg Ile Thr Leu Thr Thr Val Leu Ala Thr Phe Ala Ile Leu Leu
1               5                   10                  15

Phe Ala Ser Val Asp Gln Ala Val Ala Val Asp Thr Ser Thr Gly Val
            20                  25                  30

Gly Ile Pro Asp Tyr Thr Ala Asn Ala Pro Val Ile Ala Ser Ser Phe
        35                  40                  45

Ile Trp Phe Ser Ser Val Glu Val Gly Val Cys Tyr Ser Pro Ala Ala
    50                  55                  60

Arg Leu Gly Ser Ile Lys Gly Thr Ile His Cys Thr His Gln Glu Lys
65                  70                  75                  80

Tyr Asp Ile Asp Asn Asn Thr Trp Thr Leu Pro Gln Thr Cys Val Ala
                85                  90                  95

Leu Gly Pro Leu Gly Gly Pro Leu Ser Ser Gly Val Arg Asp Ala Cys
            100                 105                 110

Thr Asn Ala Lys Gly Thr Phe Asn Val Ile Thr Pro Ala Gly Ala Asn

115                 120                 125

Thr Asp Gly Ser Gln Ala Tyr Asn Ala Ile Gln Gln Gln Ser Gly Gly
    130                 135                 140

Ala Gly Ala Gly Ala Gly Ala Gly Ala Asp Asp Ser Gly Asp Thr Ser
145                 150                 155                 160

Thr Gln Ser Ser Gly Gly Pro Leu Ser Ser Leu Gly Lys Met Val Gly
                165                 170                 175

Phe

<210>  68
<211>  176
<212>  PRT
<213>  Sporisorium scitamineum

<400>  68

Met Arg Thr Thr Leu Thr Gln Ala Ile Leu Leu Ala Leu Ala Phe Val
1               5                   10                  15

Leu Thr Phe Thr Thr Pro Ser Ala Lys Ala Ser Asp Ala Ile Pro Leu
            20                  25                  30

Pro Asp Phe Lys Leu Val Gln Phe Pro Met Ala Ser Thr Phe Tyr Trp
        35                  40                  45

Tyr Ser Ser Val Glu Leu Gly Val Cys Tyr Ser Pro Gln Ala Arg Val
    50                  55                  60

Ala Ser Val Lys Gly Ala Leu His Cys Thr His Gln Glu Lys Tyr Asp
65                  70                  75                  80

Leu Asp Asn Asn Ser Trp Thr Leu Pro Gln Ser Cys Val Ala Leu Lys
                85                  90                  95

Pro Leu Gly Val Pro Leu Ser Asn Ala Val Arg Gln Ser Cys Val Asn
            100                 105                 110

Ala Lys Gly Thr Phe Asn Thr Ile Gln Pro Ala Ala Ser Asn Thr Asn
        115                 120                 125

Gly Gly Gln Ala Tyr Asp Thr Ile Gln Ser Lys Gly Ala Gly Gly Ala
    130                 135                 140

Gly Ala Ser Gly Ser Gln Pro Asp Ser Asn Asp Gln Glu Asp Gly Gly

145                 150                 155                 160

Lys Gln Asp Gly Gly Met Leu Ser Gly Leu Gly Asn Leu Phe Gly Lys
                165                 170                 175

<210>  69
<211>  175
<212>  PRT
<213>  Ustilago hordei (host: barley)

<400>  69

Met Lys Leu Thr Leu Asn Thr Ala Phe Leu Leu Thr Leu Ala Ser Leu
1               5                   10                  15

Leu Val Ile Ser Ile Asp Ser Val Asp Ala Asp Val Pro Leu Pro Asp
            20                  25                  30

Tyr Thr Ala His Asp Tyr Pro Leu Ala Ala Thr Phe Tyr Trp Phe Ser
        35                  40                  45

Ser Val Glu Val Gly Val Cys Tyr Ser Pro Lys Ala Arg Val Gly Ser
    50                  55                  60

Ile Lys Gly Ala Leu His Cys Thr His Gln Glu Asn Tyr Asp Ile Glu
65                  70                  75                  80

Asn Asn Thr Trp Thr Leu Pro Gln Thr Cys Val Ala Leu Lys Pro Leu
                85                  90                  95

Gly Glu Pro Leu Ser Asn Ala Val Arg Asp Ser Cys Thr Asn Ala Lys
            100                 105                 110

Gly Ser Phe Asn Leu Ile Lys Pro Ala Ser Ser Asn Ala Asp Gly Ser
        115                 120                 125

Gln Ala Tyr Asn Ala Ile Ser Asn Lys Gly Ala Asp Gly Ala Ala Ser
    130                 135                 140

Asp Pro Gly Ser Gln Thr Gly Ser Asp Asp Asp Glu Gly Asp Lys Lys
145                 150                 155                 160

Lys Asp Gly Gly Met Leu Gly Gly Leu Gly Lys Met Val Gly Leu
                165                 170                 175

<210>  70
<211>  174
<212>  PRT
<213>  Ustilago bromivira

<400>  70

Met Lys Leu Thr Leu Asn Thr Ala Phe Leu Leu Thr Leu Ala Ser Leu
1               5                   10                  15

Leu Val Ile Ser Ile Asp Ser Val Asp Ala Gln Val Pro Leu Pro Asp
            20                  25                  30

Tyr Thr Val His Asp Tyr Pro Leu Ala Ala Thr Phe Tyr Trp Phe Ser
        35                  40                  45

Ser Val Glu Val Gly Val Cys Tyr Ser Pro Lys Ala Arg Val Gly Ser
    50                  55                  60

Ile Lys Gly Ala Leu His Cys Thr His Gln Glu Asn Tyr Asp Leu Glu
65                  70                  75                  80

Asn Asn Thr Trp Thr Leu Pro Gln Thr Cys Val Ala Leu Lys Pro Leu
                85                  90                  95

Gly Glu Pro Leu Ser Asn Ala Val Arg Asp Ser Cys Ala Asn Ala Lys
            100                 105                 110

Gly Ser Phe Asn Ile Ile Lys Pro Ala Ser Asn Asn Ala Asp Gly Ser
        115                 120                 125

Gln Ala Tyr Asp Ala Ile Ser Asn Lys Gly Ala Gly Gly Ala Ala Ser
    130                 135                 140

Asp Pro Gly Ser Gln Thr Ser Ser Asp Asp Asp Glu Gly Asp Lys Lys
145                 150                 155                 160

Asp Gly Gly Met Leu Gly Gly Leu Gly Lys Met Phe Gly Leu
                165                 170


<210>  71
<211>  179
<212>  PRT
<213>  Sporisorium reilianum f. sp. zeae

<400>  71

Met Arg Thr Thr Leu Val Gln Thr Leu Ile Leu Thr Leu Thr Leu Leu
1               5                   10                  15

Leu Thr Thr Pro Cys Ile Arg Ala Asp Asp Ala Asn Gly Ala Met Gln
            20                  25                  30

Leu Pro Asp Phe Thr Pro Ala Lys Phe Pro Ile Ala Ser Thr Phe Tyr
        35                  40                  45

Trp Tyr Ser Ser Val Glu Val Gly Val Cys Tyr Ala Pro Gln Ala Arg
    50                  55                  60

Val Ala Ser Ile Lys Gly Ala Ile His Cys Thr His Gln Glu Asn Tyr
65                  70                  75                  80

Asp Leu Asp Asn Asn Ser Trp Thr Leu Pro Gln Ser Cys Val Ala Leu
                85                  90                  95

Lys Pro Leu Gly Thr Pro Leu Ser Asn Ala Val His Gln Ser Cys Val
            100                 105                 110

Asn Ala Lys Gly Thr Trp Asn Val Ile Lys Pro Ala Ala Thr Asn Ala
        115                 120                 125

Gly Gly Gly Gln Ala Tyr Asp Thr Ile Gln Ser Lys Gly Ala Gly Gly
    130                 135                 140

Ala Gly Gly Ala Asp Ala Asp Ser Ala Pro Asp Ser Asn Asp Gln Glu
145                 150                 155                 160

Asp Ala Gly Lys Lys Asp Gly Gly Leu Leu Gly Gly Ile Gly Ser Ala
                165                 170                 175

Leu Gly Met

<210>  72
<211>  179
<212>  PRT
<213>  Sporisorium reilianum f. sp. sorghi

<400>  72

Met Arg Thr Thr Leu Val Gln Thr Leu Ile Leu Thr Leu Thr Leu Leu
1               5                   10                  15

Leu Thr Thr Pro Cys Ile Arg Ala Asp Asp Ala Asn Gly Ala Met Gln
            20                  25                  30

Leu Pro Asp Phe Thr Pro Thr Lys Phe Pro Ile Ala Ser Thr Phe Tyr
        35                  40                  45

Trp Tyr Ser Ser Val Glu Val Gly Val Cys Tyr Ala Pro Gln Ala Arg
    50                  55                  60

Val Ala Ser Ile Lys Gly Ala Ile His Cys Thr His Gln Glu Asn Tyr
65                  70                  75                  80

Asp Leu Asp Asn Asn Ser Trp Thr Leu Pro Gln Ser Cys Val Ala Leu
                85                  90                  95

Lys Pro Leu Gly Thr Pro Leu Ser Asn Ala Val His Gln Ser Cys Val
            100                 105                 110

Asn Ala Lys Gly Thr Trp Asn Val Ile Lys Pro Ala Ala Ser Asn Ala
        115                 120                 125

Gly Gly Gly Gln Ala Tyr Asn Thr Ile Gln Ser Lys Gly Ala Gly Gly
    130                 135                 140

Ala Gly Gly Ala Gly Ala Asp Ser Ala Pro Asp Ser Asn Asp Gln Glu
145                 150                 155                 160

Asp Ala Gly Lys Lys Asp Gly Gly Leu Leu Gly Gly Ile Gly Ser Ala
                165                 170                 175

Leu Gly Met

<210> 73
<211> 178
<212> PRT
<213> Ustilago maydis

<400> 73

Met Met Thr Thr Leu Val Gln Thr Thr Leu Leu Ser Leu Ala Leu Val
1               5                   10                  15

Leu Leu Gly Ser Thr Val Pro Val His Ala Asp Ala Ala Gly Ala Val
            20                  25                  30

Pro Leu Pro Asn Phe Lys Val Asp Pro Gln Pro Leu Ala Ser Thr Phe
        35                  40                  45

Tyr Trp Phe Ser Ser Val Glu Val Gly Val Cys Tyr Asn Pro Gln Ala
    50                  55                  60

Arg Val Gly Ser Ile Lys Gly Ala Leu His Cys Thr His Gln Glu Asn
65                  70                  75                  80

Tyr Asp Arg Asp Asn Asn Ser Tyr Thr Leu Pro Gln Thr Cys Val Ala
                85                  90                  95

Leu Lys Pro Leu Gly Lys Ala Phe Ser Ser Asn Val Arg Asp Ser Cys
            100                 105                 110

Thr Asn Ala Lys Gly Ile Phe Asn Val Ile Val Pro Ala Ser Ser Asn
        115                 120                 125

Ala Leu Gly Ser Gln Ala Tyr Asp Ala Val Gln Ala Lys Gly Gly Thr
    130                 135                 140

Gly Gly Thr Gly Thr Asp Asp Asp Thr Ser Ala Pro Asp Ser Asn Asp
145                 150                 155                 160

Gln Glu Lys Lys Gly Gly Gly Leu Leu Gly Gly Ile Gly Ser Met Phe
                165                 170                 175

Gly Met

<210>  74
<211>  175
<212>  PRT
<213>  Ustilago esculenta

<400>  74

Met Cys Lys Ile Ser Leu Thr Gly Ala Thr Phe Phe Ala Ile Gly Ser
1               5                   10                  15

Leu Leu Ala Ile Asn Leu Lys Cys Val Ser Ala Asp Ala Ala Gly Leu
            20                  25                  30

Gly Ile Pro Asp Tyr Asn Gly Gln Lys Tyr Pro Val Ala Ser Thr Phe
        35                  40                  45

Tyr Trp Phe Ser Ser Val Glu Val Ala Ile Cys Tyr Asn Pro Gln Gly
    50                  55                  60

Arg Val Ala Ser Ile Lys Gly Gln Ile Asp Cys Thr His Gln Gly Lys
65                  70                  75                  80

Tyr Asp Ile Asp Asn Asn Thr Trp Thr Leu Pro Gln Thr Cys Val Ala
                85                  90                  95

Leu Gln Pro Leu Gly Gly Pro Leu Ser Thr Ala Val His Asp Ser Cys
            100                 105                 110

Lys Leu Ala Lys Gly Ile Tyr Asn Val Ile Thr Pro Ala Ser Ser Asn
        115                 120                 125

Glu Asp Gly Ser Gln Ala Tyr Lys Ala Ile Gln Asp Lys Ser Ser Gly
    130                 135                 140

Gly Thr Ser Asp Ala Pro Asp Ser Asn Asp Gln Leu Ala Lys Gly Asp
145                 150                 155                 160

Gly Gly Gly Gly Phe Leu Gly Gly Leu Gly Gly Leu Leu His Lys
                165                 170                 175

<210>  75
<211>  170
<212>  PRT
<213>  Ustilago trichophora

<400>  75

Met Lys Lys Thr Phe Gly Val Thr Thr Leu Leu Ser Leu Ala Ser Phe
1               5                   10                  15

Leu Val Leu Cys Phe Asp His Val Ser Ala Asp Ile Gly Gly Ala Val
            20                  25                  30

Gly Ala Pro Asp Phe Lys Val Ser Ser His Pro Val Ala Ser Thr Phe
        35                  40                  45

Tyr Trp Phe Ser Ser Val Glu Val Gly Val Cys Phe Gly Pro Glu Ala
    50                  55                  60

Arg Val Gly Ser Ile Lys Gly Ala Ile His Cys Thr His Gln Gly Asn
65                  70                  75                  80

Tyr Asp Ile Asp Asn Asn Thr Trp Thr Leu Pro Gln Thr Cys Val Ala
                85                  90                  95

Leu Lys Pro Leu Gly Gly Pro Leu Ser Thr Ala Val Arg Asp Ser Cys
            100                 105                 110

Ala Asn Ala Lys Gly Val Phe Asn Thr Ile Thr Pro Ala Ala Ala Asn
        115                 120                 125

Thr Glu Gly Gly Gln Ala Tyr Asn Ala Ile Lys Ser Pro Gly Gly Ala
    130                 135                 140

Gly Gly Asp Gly Gly Asp Gly Asp Gly Lys Lys Glu Glu Gly Gly Leu
145                 150                 155                 160

Leu Ser Gly Leu Gly Ser Met Phe Gly Leu
                165                 170

<210>  76
<211>  175

<212>  PRT
<213>  Ustilago hordei (host: oats)

<400>  76

Met Lys Leu Thr Leu Asn Thr Ala Phe Leu Leu Thr Leu Ala Ser Leu
1               5                   10                  15

Leu Val Ile Ser Ile Asp Ser Val Asp Ala Asp Val Pro Leu Pro Asp
            20                  25                  30

Tyr Thr Ala His Asp Tyr Pro Leu Ala Ala Thr Phe Tyr Trp Phe Ser
        35                  40                  45

Ser Val Glu Val Gly Val Cys Tyr Ser Pro Lys Ala Arg Val Gly Ser
    50                  55                  60

Ile Lys Gly Ala Leu His Cys Thr His Gln Glu Asn Tyr Asp Ile Glu
65                  70                  75                  80

Asn Asn Thr Trp Thr Leu Pro Gln Thr Cys Val Ala Leu Lys Pro Leu
                85                  90                  95

Gly Glu Pro Leu Ser Asn Ala Val Arg Asp Ser Cys Lys Asn Ala Lys
            100                 105                 110

Gly Ser Phe Asn Leu Ile Lys Pro Ala Ser Ser Asn Ala Ala Gly Ser
        115                 120                 125

Gln Ala Tyr Asn Ala Ile Ser Asn Lys Gly Ala Gly Gly Ala Ala Ser
    130                 135                 140

Asp Pro Gly Ser Gln Thr Gly Ser Asp Asp Asp Glu Asp Asp Lys Asn
145                 150                 155                 160

Lys Asp Gly Gly Met Leu Gly Gly Leu Gly Lys Met Phe Gly Leu
                165                 170                 175

<210>  77
<211>  174
<212>  PRT
<213>  Ustilago tritici

<400>  77

Met Arg Phe Thr Leu Ala Glu Ala Ser Leu Val Trp Leu Gly Ser Met
1               5                   10                  15

Leu Leu Ser Gln Leu His Leu Val Ser Ala Asp Ala Gly Ser Glu Ala
            20                  25                  30

Val Ala Leu Pro Asn Tyr Lys Gly Ser Asp Ala Val Leu Ala Ala Thr
        35                  40                  45

Phe Tyr Trp Tyr Ser Ser Val Glu Leu Gly Val Cys Tyr Asp Pro Lys
    50                  55                  60

Ala Arg Val Ala Ser Val Lys Gly Ala Ile His Cys Asn Ala Gln Gly
65                  70                  75                  80

Ser Tyr Asp Lys Asp Asn Asn Ser Trp Thr Leu Pro Gln Thr Cys Val
                85                  90                  95

Ala Val Gln Pro Leu Gly Gly Ser Leu Ser Asn Ala Val Arg Asp Ser
            100                 105                 110

Cys Lys Asn Ala Gly Gly Thr Phe Asn Thr Ile Gln Pro Ala Ala Ser
        115                 120                 125

Asn Ala Asn Gly Ala Gln Ala Val Ser Thr Ile Gln Gln Asn Ser Ala
    130                 135                 140

Gly Ser Ala Gly Gly Gly Ser Ala Leu Asp Ser Asp Asp Gln Asp Gly
145                 150                 155                 160

Gly Glu Lys Lys Ser Gly Gly Phe Leu Ser Gly Leu Phe Gly
                165                 170

## Description

The present invention relates to a compound or a combination of compounds which can be used as anti-fungal agents directed against plant pathogenic basidiomycete fungi, and especially for inhibiting the virulence of corn smut fungus *Ustilago maydis* and related smut fungi, and of rust fungus *Uromyces fabae* and related rust fungi, as well as compositions comprising at least one of those compounds and/or agriculturally acceptable salts thereof as agriculturally active ingredients.

### Background of the invention

Fungicides are compounds of natural or synthetic origin, which act to protect and cure plants against damage caused by agriculturally relevant fungi. Generally, no single fungicide is useful in all situations. Consequently, research is ongoing to produce fungicides that may have better performance, are easier to use, and cost-effective.
The basidiomycete fungus *Ustilago maydis* is a biotrophic fungal pathogen that causes smut disease in its host plant maize, a disease of considerable relevance in agriculture.
The smut fungus *U. maydis* is an important pathogen of corn (Maize, Zea *mays*). Primary disease symptoms are plant tumors in which fungal hyphae differentiate into spores. *U. maydis* is a biotrophic pathogen requiring living plant tissue for proliferation. In its dikaryotic form *U. maydis* develops appressoria that penetrate the plant cuticle and cell wall. Invading hyphae become encased by the plant plasma membrane resulting in an extended interaction zone. Plant responses elicited at this stage by fungal MAMPs (microbe associated molecular patterns) are actively suppressed by a cocktail of mostly novel secreted effectors. A large number of these effectors are expressed during establishment of the biotrophic stage and in this group are all six *U. maydis* virulence promoting effectors functionally studied to date. These effectors all interact with specific plant proteins and modulate their function either in the interface between fungus and surrounding plant plasma membrane or after translocation into invaded host cells. The molecular mechanisms by which translocation is achieved are largely unknown.

*Uromyces fabae* (*U. vicia-fabae*) attacks several important crop species such as broad bean (*Vicia faba*), pea, and lentil, as well as more than 50 other Vicia species, and about 20 Lathyrus species. However, it is most commonly referred to as rust of faba bean where yield losses of up to 50% have been reported. The pathogen has served as a model organism for almost half a century.

Infestation of crops by pathogenic basidiomycete fungi has continued to have a major impact by reducing yield and quality, emphasizing the need to identify new targets and develop new agents to improve methods of crop protection.

It is the objective of the present invention to provide compounds and/or agriculturally acceptable salts thereof which can be used as anti-fungal agents, especially for inhibiting the virulence of plant pathogenic basidiomycete fungi, and especially of corn smut fungus *Ustilago maydis* and related smut fungi, and of rust fungus *Uromyces fabae* and related rust fungi, as well as compositions comprising at least one of those compounds and/or agriculturally acceptable salts thereof as agriculturally active ingredients.

The objective of the present invention is solved by the teaching of the independent claims. Further advantageous features, aspects and details of the invention are evident from the dependent claims, the description, the figures, and the examples of the present application.

### Brief description of the invention

The present invention relates to a compound for use or a combination of compounds for use in inhibiting virulence of one or more plant pathogenic basidiomycete fungi, wherein the compound(s) is/are selected from the general formula (I) - (III) which are defined as follows: 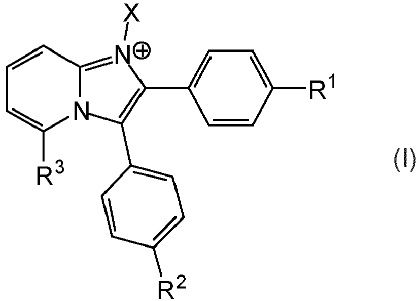 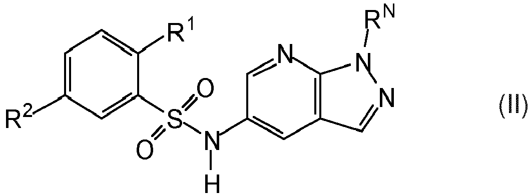 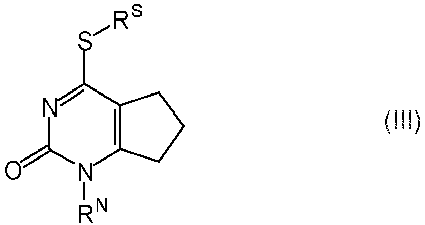 wherein
R¹ and R² represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -CH₂-OCH₃, -CH₂-OC₂H₅, -SH, -SCH₃, -SC₂H₅, -SC₃H₇, -S-cyclo-C₃H₅, -SCH(CH₃)₂, -SC(CH₃)₃, -NO₂, -F, -CI, -Br, -P(O)(OH)₂, -P(O)(OCH₃)₂, -P(O)(OC₂H₅)₂, -P(O)(OCH(CH₃)₂)₂, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, , -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₆, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CON(cyclo-C₃H₅)₂, -NH-CO-N[CH(CH₃)₂]₂, -NH-CS-N(C₂H₅)₂, -NH-CON[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₃, -C₂H₅, -C₃H₇, -CH(CH₃)₂, -CH₂F, -CHF₂, -CF₃, -CH₂Cl, -CH₂Br, -CH₂-CH₂F, -CH₂-CHF₂, -CH₂-CF₃, -CH₂-CH₂Cl, or -CH₂-CH₂Br, and wherein R¹ or R² is different from hydrogen;
R³ represents -H, -CH₃, -C₂H₅, -C₃H₇, cyclo-C₃H₅, -CH(CH₃)₂ or -C(CH₃)₃;
R^{N} represents -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -C₆H₁₃, -Ph, -L-Ph, -L- cyclo-C₅H₉, -L-R³, -L-R*, cyclo-C₃H₅, cyclo-C₄H₇, cyclo-C₅H₉, cyclo-C₆H₁₁, cyclo-C₇H₁₃, 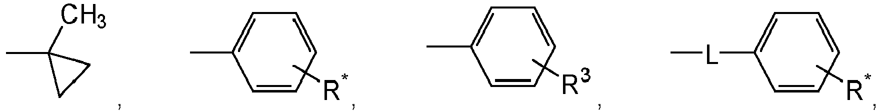 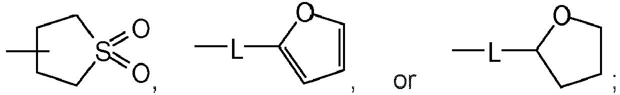 R^{S} represents -CH₃, -C₂H₅, -C₃H₇, cyclo-C₃H₅, -CH(CH₃)₂, -CH₂-CN, -CH₂-Ar, -CH(NH₂)-Ar, Ar represents -Ph, 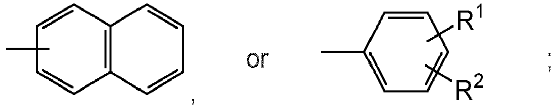 R* represents -OCH₃, -OC₂H₅, -OC₃H₇, -O-cyclo-C₃H₅, -OCH(CH₃)₂, -OC(CH₃)₃, -OC₄H₉, -COCH₃, -COC₂H₅, -COC₃H₇, -CO-cyclo-C₃H₅, -COCH(CH₃)₂, or -COC(CH₃)₃;
L represents -CH₂-, -C₂H₄-, -C₃H₆-, or -CH₂-CO-O-;
X represents -O⁻, -OCH₃, -OC₂H₅, or -OC₃H₇;
and salts especially agriculturally acceptable salts thereof.

The present invention also relates to a compound for use or the combination of compounds for use in inhibiting virulence of one or more plant pathogenic basidiomycete fungi, wherein the compound(s) is/are selected from the general formula (I) - (III) as follows: 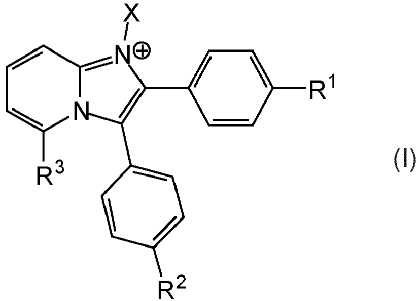 wherein
R¹ and R² represent independently of each other -H, -OCH₃, -OC₂H₅, -NO₂, -F, -Cl, -Br, -N₃, -CN, -OCN, -NCO, -SCN, -NCS, -CHO, -COCH₃, -COC₂H₅, -COOH, -COCN, -COOCH₃, -COOC₂H₅, -OOC-CH₃, -OOC-C₂H₅, -CONH₂, -CONHCH₃, -CONHC₂H₅, -CON(CH₃)₂, -CON(C₂H₅)₂, -NHCOCH₃, -NHCOC₂H₅, -NH₂, -NHCH₃, -NHC₂H₅, -N(CH₃)₂, -N(C₂H₅)₂, -SOCH₃, -SOC₂H₅, -SO₂CH₃, -SO₂C₂H₅, -SO₃H, -SO₃CH₃, -SO₃C₂H₅, -SO₂NH₂, -SO₂NHCH₃, -SO₂NHC₂H₅, -SO₂N(CH₃)₂, -SO₂N(C₂H₅)₂, -O-S(=O)CH₃, -O-S(=O)C₂H₅, -NH-SO₂-CH₃, -NH-SO₂-C₂H₅, -O-SO₂-CH₃, -O-SO₂-C₂H₅, -OCF₃, -CH₂-OCF₃, -OC₂F₅, -O-COOCH₃, -O-COOC₂H₅, -NH-CO-NH₂, -NH-CS-NH₂, -NH-C(=NH)-NH₂, -O-CO-NH₂, -O-CO-OCH₃, -O-CO-OC₂H₅, -CH₃, -C₂H₅, -C₃H₇, -CH(CH₃)₂, -CH₂F, -CHF₂, -CF₃, -CH₂Cl, -CH₂Br, and wherein R¹ or R² is different from hydrogen;
R³ represents -H or -CH₃;
X represents -O⁻ or -OCH₃;
and salts especially agriculturally acceptable salts thereof; 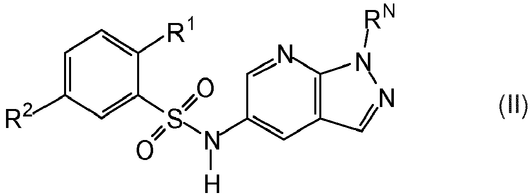
wherein
R¹ and R² represent independently of each other -H, -OCH₃, -OC₂H₅, -NO₂, -F, -Cl, -Br, -N₃, -CN, -OCN, -NCO, -SCN, -NCS, -CHO, -COCH₃, -COC₂H₅, -COOH, -COCN, -COOCH₃, -COOC₂H₅, -OOC-CH₃, -OOC-C₂H₅, -CONH₂, -CONHCH₃, -CONHC₂H₅, -CON(CH₃)₂, -CON(C₂H₅)₂, -NHCOCH₃, -NHCOC₂H₅, -NH₂, -NHCH₃, -NHC₂H₅, -N(CH₃)₂, -N(C₂H₅)₂, -SOCH₃, -SOC₂H₅, -SO₂CH₃, -SO₂C₂H₅, -SO₃H, -SO₃CH₃, -SO₃C₂H₅, -SO₂NH₂, -SO₂NHCH₃, -SO₂NHC₂H₅, -SO₂N(CH₃)₂, -SO₂N(C₂H₅)₂, -O-S(=O)CH₃, -O-S(=O)C₂H₅, -NH-SO₂-CH₃, -NH-SO₂-C₂H₅, -O-SO₂-CH₃, -O-SO₂-C₂H₅, -OCF₃, -CH₂-OCF₃, -OC₂F₅, -O-COOCH₃, -O-COOC₂H₅, -NH-CO-NH₂, -NH-CS-NH₂, -NH-C(=NH)-NH₂, -O-CO-NH₂, -O-CO-OCH₃, -O-CO-OC₂H₅, -CH₃, -C₂H₅, -C₃H₇, -CH(CH₃)₂, -CH₂F, -CHF₂, -CF₃, -CH₂Cl, -CH₂Br, and wherein R¹ or R² is different from hydrogen;
R^{N} represents -CH₃, -C₂H₅, -C₃H₇, -CH(CH₃)₂, -C₄H₉, -CH₂-CH(CH₃)₂, -C(CH₃)₃, -C₅H₁₁, -Ph, -L-Ph, -L-R³, cyclo-C₃H₅, cyclo-C₄H₇, cyclo-C₅H₉, cyclo-C₆H₁₁, cyclo-C₇H₁₃;
L represents -CH₂-, -C₂H₄-, or -CH₂-CO-O-;
R³ represents -H, -CH₃, -C₂H₅, -C₃H₇, cyclo-C₃H₅, -CH(CH₃)₂ or -C(CH₃)₃;
and salts especially agriculturally acceptable salts thereof; 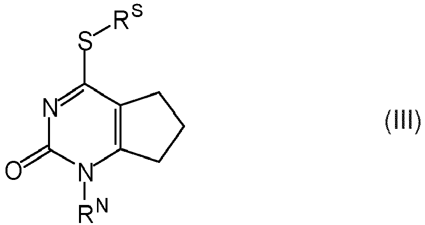 wherein
R^{N} represents -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -C₆H₁₃, -Ph, -L-Ph, -L- cyclo-C₅H₉, -L-R³, -L-R*, cyclo-C₃H₅, cyclo-C₄H₇, cyclo-C₅H₉, cyclo-C₆H₁₁, cyclo-C₇H₁₃, 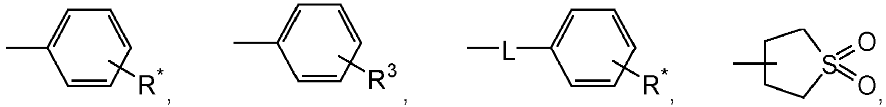 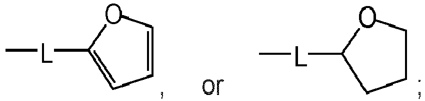
R^{S} represents -CH₃, -C₂H₅, -C₃H₇, cyclo-C₃H₅, -CH(CH₃)₂, -CH₂-CN, -CH₂-Ar, -CH(NH₂)-Ar,
Ar represents -Ph, 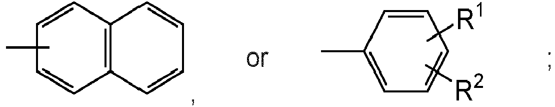
R* represents -OCH₃, -OC₂H₅, -OC₃H₇, -O-cyclo-C₃H₅, -OCH(CH₃)₂, -COCH₃, -COC₂H₅, -COC₃H₇, -CO-cyclo-C₃H₅, or -COCH(CH₃)₂;
R¹ and R² represent independently of each other -H, -OCH₃, -OC₂H₅, -NO₂, -F, -Cl, -Br, -N₃, -CN, -OCN, -NCO, -SCN, -NCS, -CHO, -COCH₃, -COC₂H₅, -COOH, -COCN, -COOCH₃, -COOC₂H₅, -OOC-CH₃, -OOC-C₂H₅, -CONH₂, -CONHCH₃, -CONHC₂H₅, -CON(CH₃)₂, -CON(C₂H₅)₂, -NHCOCH₃, -NHCOC₂H₅, -NH₂, -NHCH₃, -NHC₂H₅, -N(CH₃)₂, -N(C₂H₅)₂, -SOCH₃, -SOC₂H₅, -SO₂CH₃, -SO₂C₂H₅, -SO₃H, -SO₃CH₃, -SO₃C₂H₅, -SO₂NH₂, -SO₂NHCH₃, -SO₂NHC₂H₅, -SO₂N(CH₃)₂, -SO₂N(C₂H₅)₂, -O-S(=O)CH₃, -O-S(=O)C₂H₅, -NH-SO₂-CH₃, -NH-SO₂-C₂H₅, -O-SO₂-CH₃, -O-SO₂-C₂H₅, -OCF₃, -CH₂-OCF₃, -OC₂F₅, -O-COOCH₃, -O-COOC₂H₅, -NH-CO-NH₂, -NH-CS-NH₂, -NH-C(=NH)-NH₂, -O-CO-NH₂, -O-CO-OCH₃, -O-CO-OC₂H₅, -CH₃, -C₂H₅, -C₃H₇, -CH(CH₃)₂, -CH₂F, -CHF₂, -CF₃, -CH₂Cl, -CH₂Br, and wherein R¹ or R² is different from hydrogen;
R³ represents -H, -CH₃, or -C₂H₅;
L represents -CH₂-, -C₂H₄-, or -C₃H₆-;
and salts especially agriculturally acceptable salts thereof.

The present invention further relates to a compound for use or the combination of compounds for use in inhibiting virulence of one or more plant pathogenic, wherein the compound(s) is/are selected from: 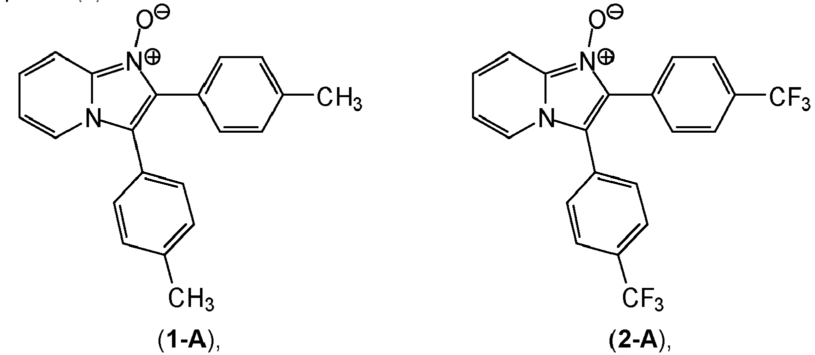 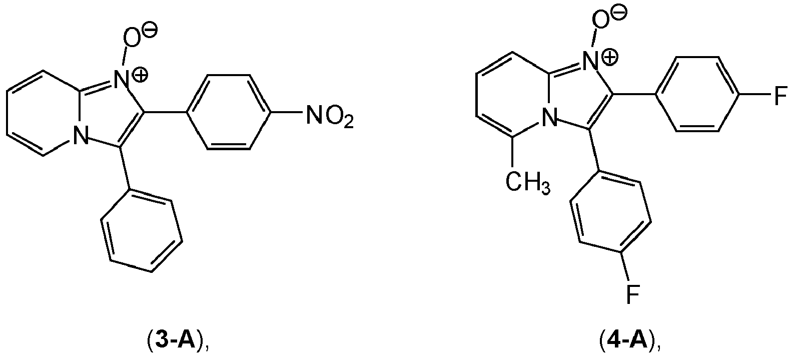 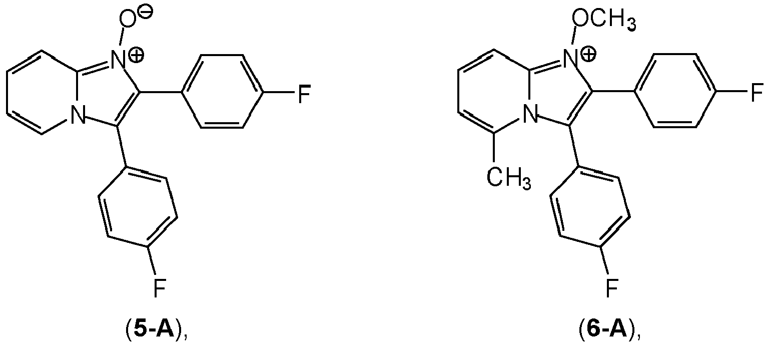 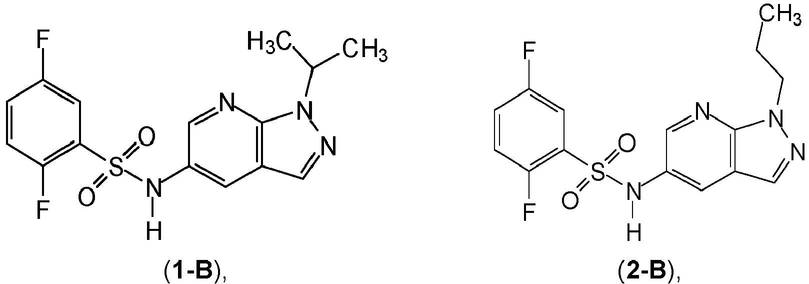 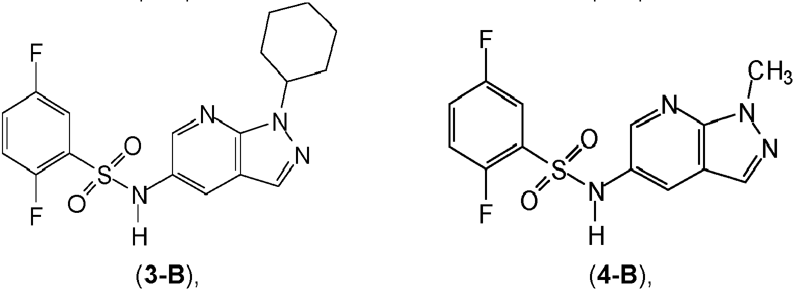 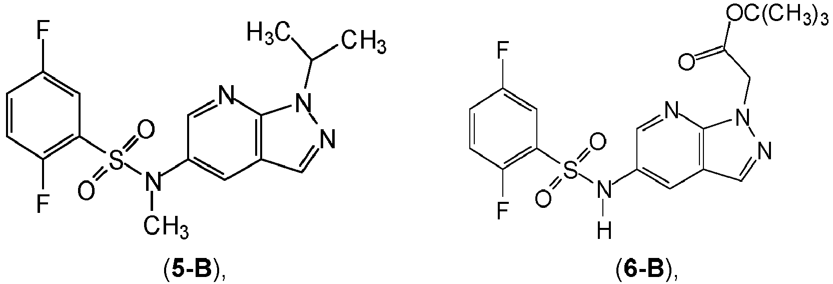 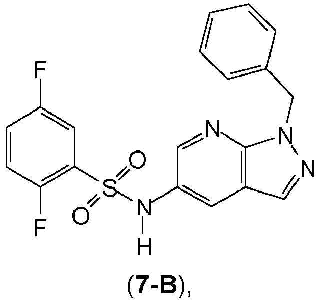 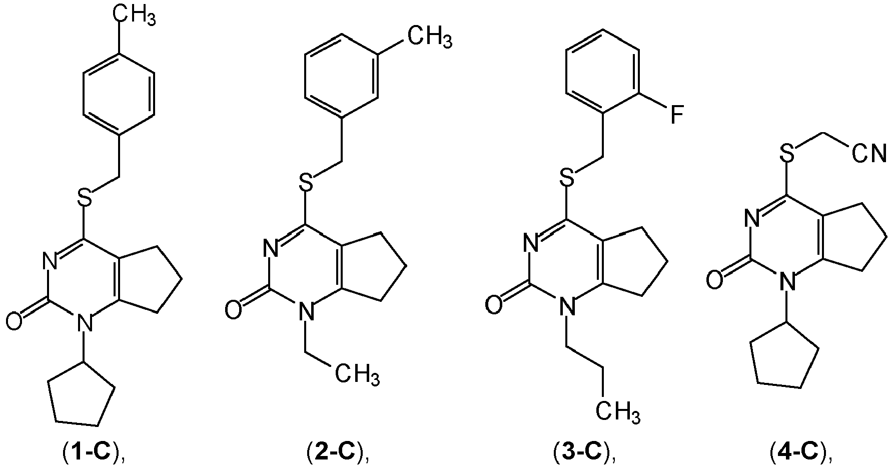 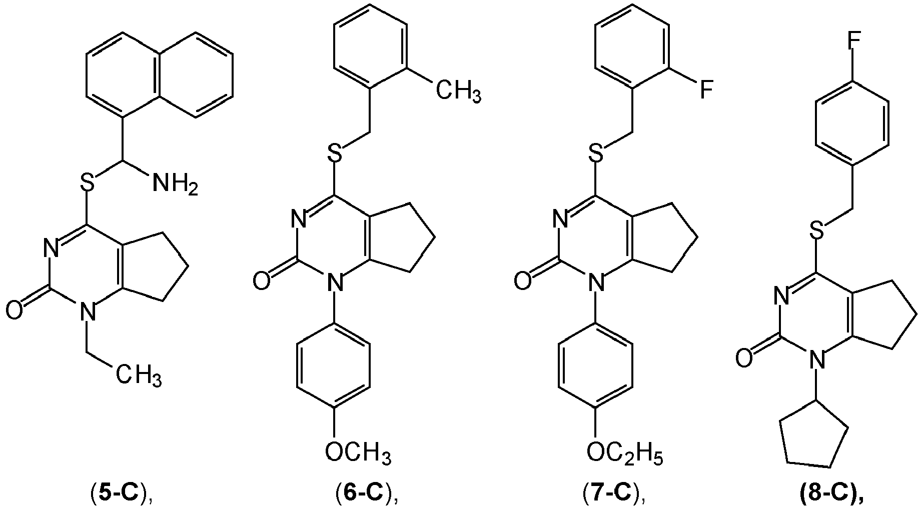 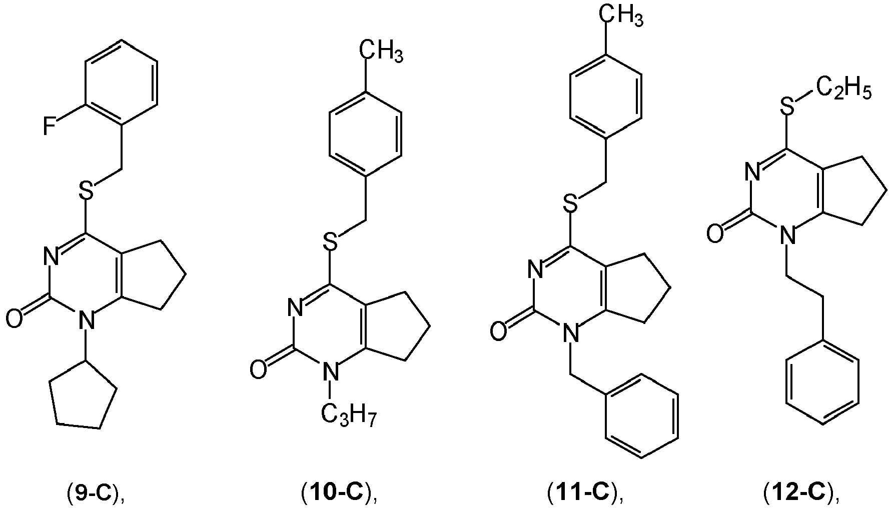 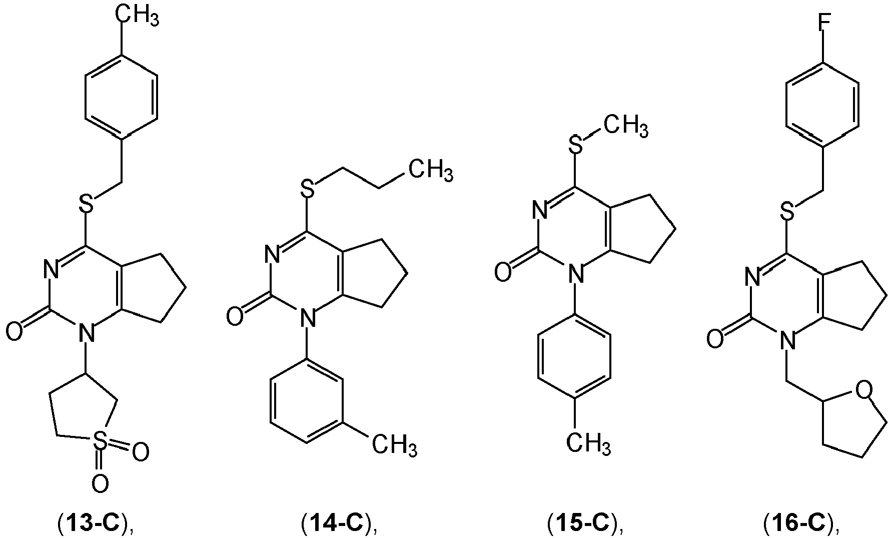 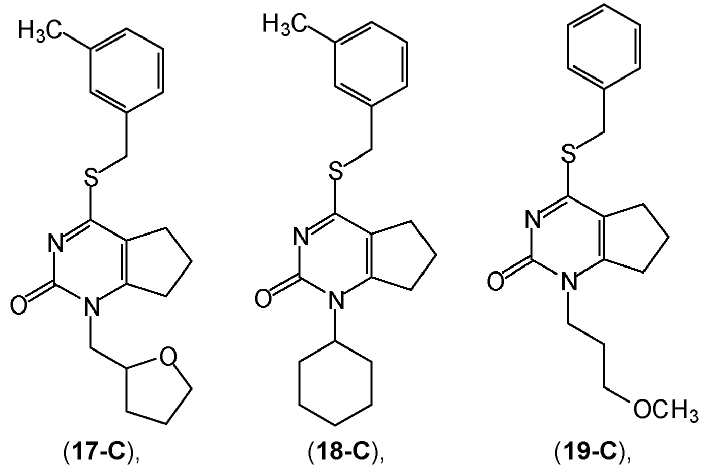 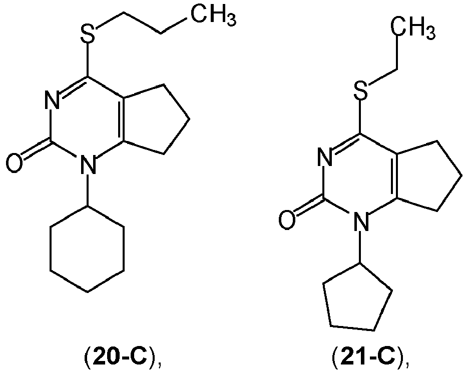 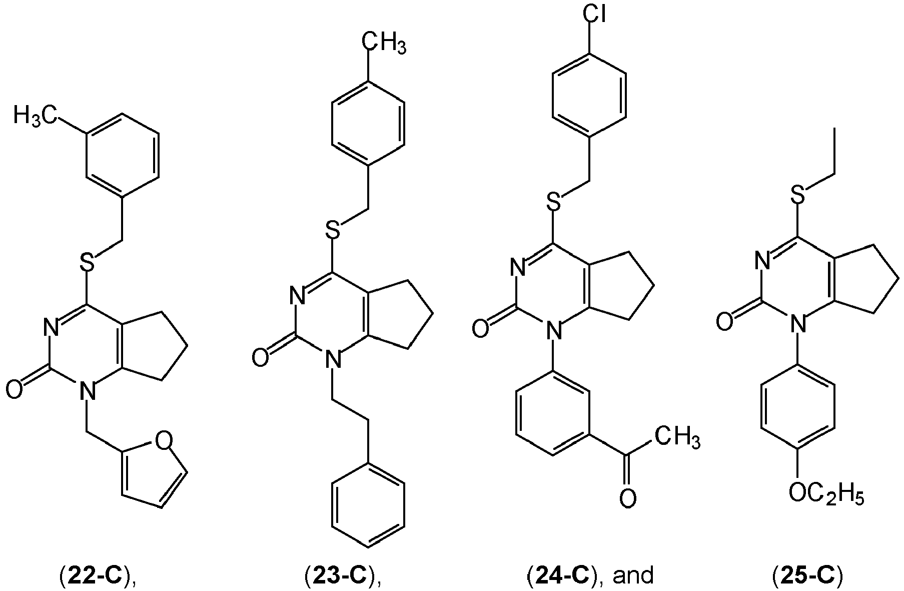 and salts especially agriculturally acceptable salts thereof.

A preferred embodiment of the invention is directed to a compound for use or the combination of compounds for use as described above, wherein the one or more plant pathogenic basidiomycete fungi are selected from the group consisting of smut fungi and rust fungi, wherein the smut fungi are *Ustilago hordei, Ustilago avenae, Ustilago nuda, Ustilago nigra, Ustilago tritici, Ustilago maydis, Ustilago scitaminea, Ustilago esculenta, Ustilago striiformis, Ustilago trichophora, Sporisorium reilianum, Sporisorium reilianum f.sp. zeae, Sporisorium reilianum f.sp. reilianum, Sporisorium scitamineum, Angiosorus solani, Urocystis agropyri, Urocystis occulta, Tilletia indica, Tilletia caries, Tilletia barclayana* or *Tilletia controversa,* or wherein the rust fungi is *Uromyces fabae, Uromyces appendiculatus, Uromyces beticola, Puccinia graminis f. sp. tritici, Puccinia striiformis* f. sp. *tritici, Puccinia triticina, Puccinia graminis f. sp. secalis, Puccinia arachidis, Puccinia coronata f. sp. avenae, Puccinia sorghi, Puccinia melanocephala, Puccinia kuehnii, Puccinia recondita, Puccinia pitteriana, Puccinia graminis, Puccinia polysora, Phakopsora pachyrhizi, Phakopsora meibomiae, Hemileia vastatrix, Melampsora-larici-populina, Melampsora lini, Cronartium ribicola, Cronartium quercuum f. sp. fusiforme, Gymnosporangium sabinae, Gymnosporangium yamadae, Gymnosporangium juniperi-virginianae.*

Another preferred embodiment of the invention is directed to a compound for use or the combination of compounds for use as described above, wherein the one or more plant pathogenic basidiomycete fungi are selected from the group consisting of smut fungi and rust fungi, wherein the smut fungi are *Ustilago maydis, Ustilago esculenta, Ustilago trichophora, Ustilago tritici, Sporisorium reilianum f.sp. zeae, Sporisorium reilianum f.sp. reilianum, Sporisorium scitamineum, Ustilago hordei,* or wherein the rust fungi is *Uromyces fabae.*

Another embodiment of the invention provides a fungicidal composition comprising a compound for use or a combination of compounds for use in treating, preventing, inhibiting, or eliminating the virulence of one or more plant pathogenic basidiomycete fungi in a plant or a plant material, wherein the compound(s) is/are selected from the general formula (I) - (III) as defined above,
wherein the fungicidal composition further comprises at least one suitable agriculturally acceptable carrier, excipient, solvent and/or adjuvant.

A preferred embodiment of the invention provides a fungicidal composition comprising a compound for use or a combination of compounds for use in treating, preventing, inhibiting, or eliminating the virulence of one or more plant pathogenic basidiomycete fungi in a plant or a plant material as described above, wherein the one or more plant pathogenic basidiomycete fungi are selected from the group consisting of smut fungi and rust fungi, wherein the smut fungi are *Ustilago hordei, Ustilago avenae, Ustilago nuda, Ustilago nigra, Ustilago tritici, Ustilago maydis, Ustilago scitaminea, Ustilago esculenta, Ustilago striiformis, Ustilago trichophora, Sporisorium reilianum, Sporisorium reilianum f.sp. zeae, Sporisorium reilianum f.sp. reilianum, Sporisorium scitamineum, Angiosorus solani, Urocystis agropyri, Urocystis occulta, Tilletia indica, Tilletia caries, Tilletia barclayana* or *Tilletia controversa,* or wherein the rust fungi is *Uromyces fabae, Uromyces appendiculatus, Uromyces beticola, Puccinia graminis f. sp. tritici, Puccinia striiformis* f. sp. *tritici, Puccinia triticina, Puccinia graminis f. sp. secalis, Puccinia arachidis, Puccinia coronata f. sp. avenae, Puccinia sorghi, Puccinia melanocephala, Puccinia kuehnii, Puccinia recondita, Puccinia pitteriana, Puccinia graminis, Puccinia polysora, Phakopsora pachyrhizi, Phakopsora meibomiae, Hemileia vastatrix, Melampsora-larici-populina, Melampsora lini, Cronartium ribicola, Cronartium quercuum f. sp. fusiforme, Gymnosporangium sabinae, Gymnosporangium yamadae, Gymnosporangium juniperi-virginianae.*

A further preferred embodiment of the invention provides a fungicidal composition comprising a compound for use or a combination of compounds for use in treating, preventing, inhibiting, or eliminating the virulence of one or more plant pathogenic basidiomycete fungi in a plant or a plant material as described above, wherein the one or more plant pathogenic basidiomycete fungi are selected from the group consisting of smut fungi and rust fungi, wherein the smut fungi are *Ustilago maydis, Ustilago esculenta, Ustilago trichophora, Ustilago tritici, Sporisorium reilianum f.sp. zeae, Sporisorium reilianum f.sp. reilianum, Sporisorium scitamineum, Ustilago hordei,* or wherein the rust fungi is *Uromyces fabae.*

In another aspect, the present invention provides a method of treating, preventing, inhibiting, or eliminating virulence of one or more plant pathogenic basidiomycete fungi in a plant or a plant material comprising the step of applying onto a plant, to a plant material or in the vicinity of said plant or plant material a compound or a combination of compounds or a fungicidal composition comprising the same, wherein the compound(s) is/are selected from the general formula (I) - (III) as defined above.

In a particular aspect, the present invention provides the method of treating, preventing, inhibiting, or eliminating the virulence of one or more plant pathogenic basidiomycete fungi in a plant or a plant material as defined above, wherein the one or more plant pathogenic basidiomycete fungi are selected from the group consisting of smut fungi and rust fungi, wherein the smut fungi are *Ustilago hordei, Ustilago avenae, Ustilago nuda, Ustilago nigra, Ustilago tritici, Ustilago maydis, Ustilago scitaminea, Ustilago esculenta, Ustilago striiformis, Ustilago trichophora, Sporisorium reilianum, Sporisorium reilianum f.sp. zeae, Sporisorium reilianum f.sp. reilianum, Sporisorium scitamineum, Angiosorus solani, Urocystis agropyri, Urocystis occulta, Tilletia indica, Tilletia caries, Tilletia barclayana* or *Tilletia controversa,* or wherein the rust fungi is *Uromyces fabae, Uromyces appendiculatus, Uromyces beticola, Puccinia graminis f. sp. tritici, Puccinia striiformis* f. sp. *tritici, Puccinia triticina, Puccinia graminis f. sp. secalis, Puccinia arachidis, Puccinia coronata f. sp. avenae, Puccinia sorghi, Puccinia melanocephala, Puccinia kuehnii, Puccinia recondita, Puccinia pitteriana, Puccinia graminis, Puccinia polysora, Phakopsora pachyrhizi, Phakopsora meibomiae, Hemileia vastatrix, Melampsora-larici-populina, Melampsora lini, Cronartium ribicola, Cronartium quercuum f. sp. fusiforme, Gymnosporangium sabinae, Gymnosporangium yamadae, Gymnosporangium juniperi-virginianae.*

In a further particular aspect, the present invention provides the method of treating, preventing, inhibiting, or eliminating the virulence of one or more plant pathogenic basidiomycete fungi in a plant or a plant material as defined above, wherein the one or more plant pathogenic basidiomycete fungi are selected from the group consisting of smut fungi and rust fungi, wherein the smut fungi are *Ustilago maydis, Ustilago esculenta, Ustilago trichophora, Ustilago tritici, Sporisorium reilianum f.sp. zeae, Sporisorium reilianum f.sp. reilianum, Sporisorium scitamineum, Ustilago hordei,* or wherein the rust fungi is *Uromyces fabae.*

### Description of the invention

The basidiomycete fungus *Ustilago maydis* is a biotrophic fungal pathogen that causes smut disease in its host plant maize, a disease of considerable relevance in agriculture. There are about 1500 different smut species which parasitize different grasses including important crop plants like maize, sorghum, sugar cane, wheat and barley. To allow colonization *U. maydis* secretes a cocktail of novel effector proteins which suppress plant defense responses and manipulate the physiology of the host to support fungal growth and nutrition acquisition. Such effector proteins function either in the apoplast, i.e. a compartment between fungal hyphae and surrounding plant plasma membrane or are taken up by plant cells and function inside defined plant cell compartments. The mechanism how such effectors are translocated to the host is unknown.

By analyzing effectors that are induced during the stage when biotrophy is established, i.e. 2 days after infection, Ludwig et al. have identified three effector mutants where single gene deletions, thereafter named *stp1, stp2,* and *stp3,* abolish virulence completely (Ludwig et al., A protein complex with an essential virulence function in a fungal plant pathogen. Manuscript under review). Co-immunoprecipitation and mass spectrometry (Co-IP-MS) analysis showed that these effectors form a complex with two additional effector proteins, Stp4 and Pep1, and two fungal transmembrane proteins, Stp5 and Stp6. This complex was termed Stp, Stop after penetration, complex. Mutants in the additional four genes also abolished virulence completely. The genes were termed *stp1, stp2, stp3, stp4, stp5, stp6* and *pep1.* Pep1 was previously published as an apoplastic inhibitor of maize peroxidase (Hemetsberger et al., The Ustilago maydis effector Pep1 suppresses plant immunity by inhibition of host peroxidase activity. PLoS Pathog. 2012, 8, e1002684). Individual mutants were still able to penetrate the epidermis, but become arrested in the epidermal cell layer and did not manage to suppress plant defense responses. Immunoelectron microscopy analysis showed that the complex members reside in structures extending from the fungus into host cells. Moreover, it was shown that the complex interacts with pore-forming proteins residing in the plant plasma membrane, which encases biotrophic hyphae. Reconstitution of the complex by constitutively expressing all seven genes in *U. maydis* revealed surface-exposed structures. The inability of complex mutants to suppress defense after infection of a susceptible host as well as to elicit non-host resistance after barley infection, processes likely depending on translocated effectors, strongly suggests that Stp complex mediates effector translocation to the host, which is of crucial importance for successful infection.

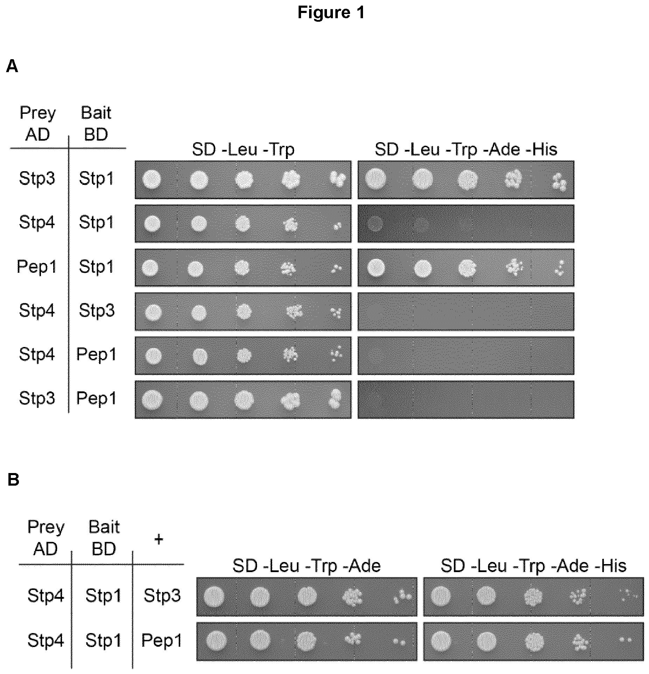

Based on the crucial importance of the Stp complex for successful plant colonization of *U. maydis,* the complex was considered as an interesting new fungicide target. A screening platform to analyse interaction of several complex members was developed in a *Saccharomyces cerevisiae* 2-hybrid system, strain named NL73, where gene activation relies on protein interaction. Results showed interaction between Stp1, Stp3 and Stp4 to form a stable subcomplex **(****Figure 1****)** and this could be also verified by Co-IP-MS analysis **(Table 3**).

The compounds of the COMAS library (http://comas.mpi-dortmund.mpg.de/) were tested for their activity on yeast viability using an engineered NL73 strain, in which viability depends on formation of a stable Stp1, Stp3 and Stp4 complex and is assessed with the Alamar Blue viability assay. In these experiments, 10 compounds from 10 different chemical classes showed activity in the nanomolar or low micromolar range (listed in **Table 4**).

These identified 10 hit compounds were then tested for their effect on virulence of *U*. *maydis* in *Zea mays.* The results revealed significant reductions in virulence by compounds **1-C, 1-B** and **1-A (Table 4).**

Activity of these compounds and of other structurally related compounds was thereafter tested using three types of assays: Alamar Blue viability assay in the NL73 strain, virulence inhibition in *U. maydis* and virulence inhibition in *Uromyces fabae.* Indeed, previous data suggest that rust fungi might possess a Stp complex related structure (Kemen et al., The Plant Journal 2013, 75, 767-780).

Several imidazopyridines, like **1-A,** showed nanomolar potency in the Alamar Blue assay with NL73 **(Table 5).** Moreover, three imidazopyridines compounds (**1-A, 2-A, 6-A**) were able to inhibit virulence of *U. maydis* with a concentration dependent inhibitory effect **(****Figure 2A** - **2C****).** Moreover, five imidazopyridines compounds (**1-A, 2-A, 3-A, 5-A,** and **6-A**) were able to inhibit pustule formation of *U. fabae* at 1mM (**Figure 3**), and two of them also at 0.5 mM final concentration in the inoculum **(****Figure 4****).**

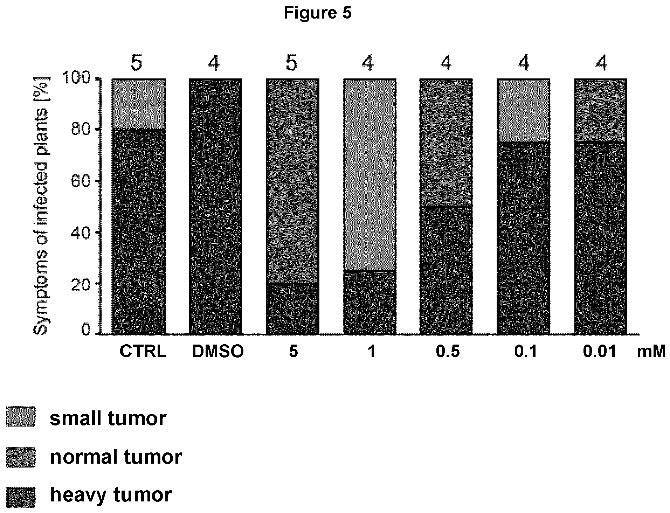

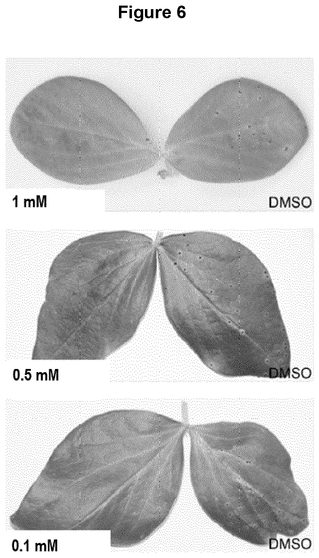

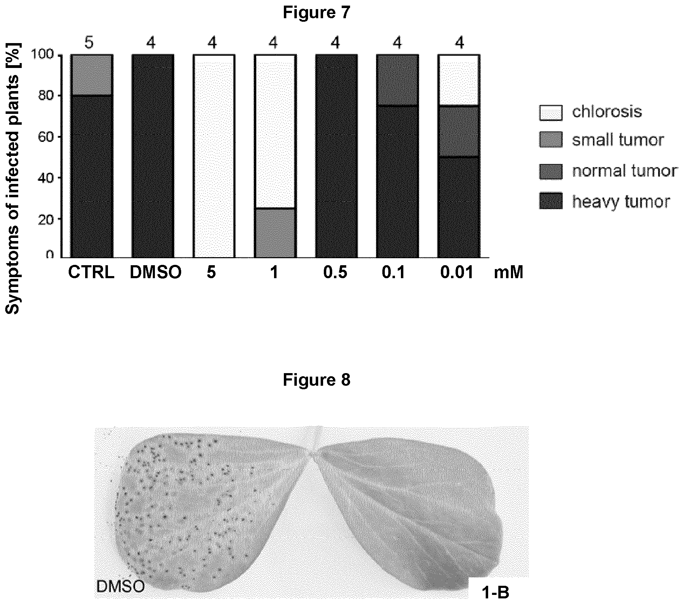

Among the tetrahydro-cyclopentapyrimidine, compound **1-C** showed strong inhibitory activity on *U. maydis* virulence **(****Figure 5****),** and on *U. fabae* virulence **(****Figure 6****).** Among the pyrazolopyridin-benzenesulfonamide, **1-B** showed an IC₅₀ of 2.5 µM in the Alamar Blue viability assay of NL73. Compound **1-B** inhibited *U. maydis* virulence very strongly at 5 mM and 1 mM **(****Figure 7**), and also *U. fabae* virulence at 1mM **(****Figure 8**).

These data show that the inventors were able to discover novel anti-fungicidal compounds able to efficiently and specifically targeting the Stp complex in smut fungi and a related structure in rust fungi, resulting in inhibition of their virulence.

Moreover, the inventors showed that the Stp complex members Stp1 - Stp6 and Pep1 are expressed in different smut fungi **(Example 7**), suggesting that the Stp complex is also present in others basidiomycete fungi, so that the inventive compounds are able to inhibit virulence also of these other basidiomycete fungi relying on the Stp complex for plant colonization.

Currently, the use of fungicides to combat diseases caused by smut and rust fungi is a common practice and the most used chemicals belong to three classes. The compounds "succinate dehydrogenase inhibitors" (SDHI) inhibit succinate dehydrogenase, "quinone outside inhibitors" (QoI) inhibit mitochondrial respiration, and demethylation inhibitors (DMIs) inhibit sterol biosynthesis. These are all rather general targets and compounds from these 3 classes are usually used in combination (Carmona et al., Role of Fungicide Applications on the Integrated Management of Wheat Stripe Rust. Front. Plant Sci., 2020, 11:733; Foster , Identification of Fungicide Targets in Pathogenic Fungi, In: Anke T., Schüffler A. (eds) Physiology and Genetics. The Mycota 2018, vol 15. Springer, Cham.) to avoid development of fungicide resistant strains.

In contrast, the Stp complex has an essential role for disease progression, represents a highly specific target that is expressed only during colonization of plants by plant pathogenic basidiomycete fungi. Therefore, use of the compounds described here either alone or in combination with the existing compound classes could become a treatment not easily overcome by development of resistant fungal strains.

Thus, the present invention provides a compound for use or a combination of compounds for use in inhibiting virulence of one or more plant pathogenic basidiomycete fungi, wherein the compound(s) is/are selected from the general formula (I) - (III) which are defined as follows: 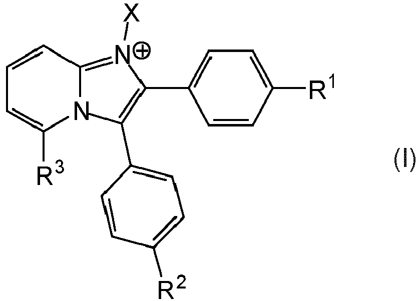 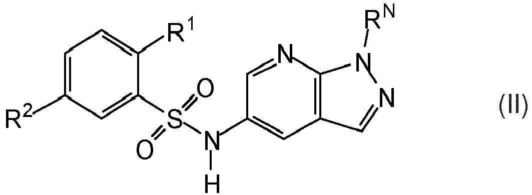 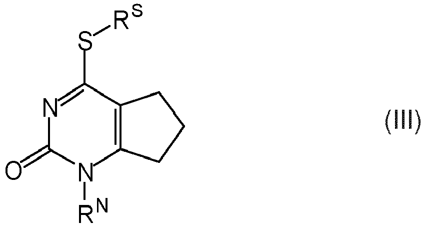 wherein
R¹ and R² represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -CH₂-OCH₃, -CH₂-OC₂H₅, -SH, -SCH₃, -SC₂H₅, -SC₃H₇, -S-cyclo-C₃H₅, -SCH(CH₃)₂, -SC(CH₃)₃, -NO₂, -F, -CI, -Br, -P(O)(OH)₂, -P(O)(OCH₃)₂, -P(O)(OC₂H₅)₂, -P(O)(OCH(CH₃)₂)₂, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₆, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₃, -C₂H₅, -C₃H₇, -CH(CH₃)₂, -CH₂F, -CHF₂, -CF₃, -CH₂Cl, -CH₂Br, -CH₂-CH₂F, -CH₂-CHF₂, -CH₂-CF₃, -CH₂-CH₂Cl, or -CH₂-CH₂Br, and
wherein R¹ or R² is different from hydrogen;
R³ represents -H, -CH₃, -C₂H₅, -C₃H₇, cyclo-C₃H₅, -CH(CH₃)₂ or -C(CH₃)₃;
R^{N} represents -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -C₆H₁₃, -Ph, -L-Ph, -L-cyclo-C₅H₉, -L-R³, -L-R*, cyclo-C₃H₅, cyclo-C₄H₇, cyclo-C₅H₉, cyclo-C₆H₁₁, cyclo-C₇H₁₃, 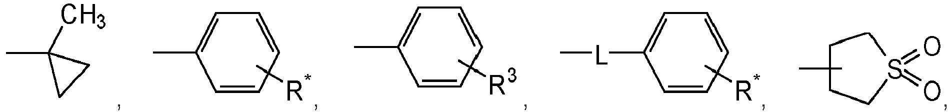 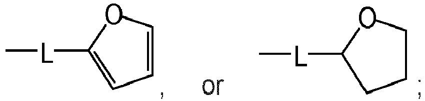
R^{S} represents -CH₃, -C₂H₅, -C₃H₇, cyclo-C₃H₅, -CH(CH₃)₂, -CH₂-CN, -CH₂-Ar, -CH(NH₂)-Ar,
Ar represents -Ph, 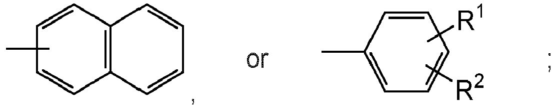
R* represents -OCH₃, -OC₂H₅, -OC₃H₇, -O-cyclo-C₃H₅, -OCH(CH₃)₂, -OC(CH₃)₃, -OC₄H₉, -COCH₃, -COC₂H₅, -COC₃H₇, -CO-cyclo-C₃H₅, -COCH(CH₃)₂, or -COC(CH₃)₃;
L represents -CH₂-, -C₂H₄-, -C₃H₆-, or -CH₂-CO-O-;
X represents -O⁻, -OCH₃, -OC₂H₅, or -OC₃H₇;
and salts especially agriculturally acceptable salts thereof.

The present invention also relates to a compound for use or the combination of compounds for use in inhibiting virulence of one or more plant pathogenic basidiomycete fungi, wherein the compound(s) is/are selected from the general formula (I) - (III) which are defined as follows: 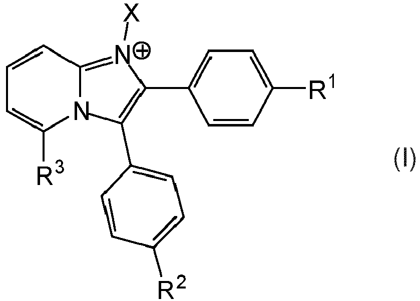 wherein
R¹ and R² represent independently of each other -H, -OCH₃, -OC₂H₅, -NO₂, -F, -Cl, -Br, -N₃, -CN, -OCN, -NCO, -SCN, -NCS, -CHO, -COCH₃, -COC₂H₅, -COOH, -COCN, -COOCH₃, -COOC₂H₅, -OOC-CH₃, -OOC-C₂H₅, -CONH₂, -CONHCH₃, -CONHC₂H₅, -CON(CH₃)₂, -CON(C₂H₅)₂, -NHCOCH₃, -NHCOC₂H₅, -NH₂, -NHCH₃, -NHC₂H₅, -N(CH₃)₂, -N(C₂H₅)₂, -SOCH₃, -SOC₂H₅, -SO₂CH₃, -SO₂C₂H₅, -SO₃H, -SO₃CH₃, -SO₃C₂H₅, -SO₂NH₂, -SO₂NHCH₃, -SO₂NHC₂H₅, -SO₂N(CH₃)₂, -SO₂N(C₂H₅)₂, -O-S(=O)CH₃, -O-S(=O)C₂H₅, -NH-SO₂-CH₃, -NH-SO₂-C₂H₅, -O-SO₂-CH₃, -O-SO₂-C₂H₅, -OCF₃, -CH₂-OCF₃, -OC₂F₅, -O-COOCH₃, -O-COOC₂H₅, -NH-CO-NH₂, -NH-CS-NH₂, -NH-C(=NH)-NH₂, -O-CO-NH₂, -O-CO-OCH₃, -O-CO-OC₂H₅, -CH₃, -C₂H₅, -C₃H₇, -CH(CH₃)₂, -CH₂F, -CHF₂, -CF₃, -CH₂Cl, -CH₂Br, and wherein R¹ or R² is different from hydrogen;
R³ represents -H or -CH₃;
X represents -O⁻ or -OCH₃;
and salts especially agriculturally acceptable salts thereof; 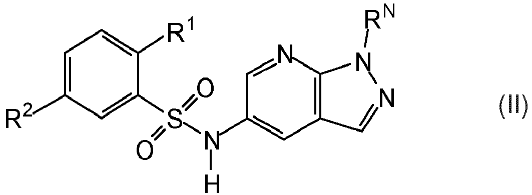
wherein
R¹ and R² represent independently of each other -H, -OCH₃, -OC₂H₅, -NO₂, -F, -Cl, -Br, -N₃, -CN, -OCN, -NCO, -SCN, -NCS, -CHO, -COCH₃, -COC₂H₅, -COOH, -COCN, -COOCH₃, -COOC₂H₅, -OOC-CH₃, -OOC-C₂H₅, -CONH₂, -CONHCH₃, -CONHC₂H₅, -CON(CH₃)₂, -CON(C₂H₅)₂, -NHCOCH₃, -NHCOC₂H₅, -NH₂, -NHCH₃, -NHC₂H₅, -N(CH₃)₂, -N(C₂H₅)₂, -SOCH₃, -SOC₂H₅, -SO₂CH₃, -SO₂C₂H₅, -SO₃H, -SO₃CH₃, -SO₃C₂H₅, -SO₂NH₂, -SO₂NHCH₃, -SO₂NHC₂H₅, -SO₂N(CH₃)₂, -SO₂N(C₂H₅)₂, -O-S(=O)CH₃, -O-S(=O)C₂H₅, -NH-SO₂-CH₃, -NH-SO₂-C₂H₅, -O-SO₂-CH₃, -O-SO₂-C₂H₅, -OCF₃, -CH₂-OCF₃, -OC₂F₅, -O-COOCH₃, -O-COOC₂H₅, -NH-CO-NH₂, -NH-CS-NH₂, -NH-C(=NH)-NH₂, -O-CO-NH₂, -O-CO-OCH₃, -O-CO-OC₂H₅, -CH₃, -C₂H₅, -C₃H₇, -CH(CH₃)₂, -CH₂F, -CHF₂, -CF₃, -CH₂Cl, -CH₂Br, and wherein R¹ or R² is different from hydrogen;
R^{N} represents -CH₃, -C₂H₅, -C₃H₇, -CH(CH₃)₂, -C₄H₉, -CH₂-CH(CH₃)₂, -C(CH₃)₃, -C₅H₁₁, -Ph, -L-Ph, -L-R³, cyclo-C₃H₅, cyclo-C₄H₇, cyclo-C₅H₉, cyclo-C₆H₁₁, cyclo-C₇H₁₃;
L represents -CH₂-, -C₂H₄-, or -CH₂-CO-O-;
R³ represents -H, -CH₃, -C₂H₅, -C₃H₇, cyclo-C₃H₅, -CH(CH₃)₂ or -C(CH₃)₃;
and salts especially agriculturally acceptable salts thereof; 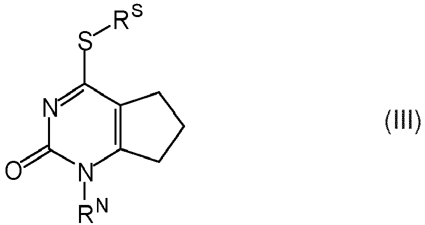
wherein
R^{N} represents -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -C₆H₁₃, -Ph, -L-Ph, -L- cyclo-C₅H₉, -L-R³, -L-R* cyclo-C₃H₅, cyclo-C₄H₇, cyclo-C₅H₉, cyclo-C₆H₁₁, cyclo-C₇H₁₃, 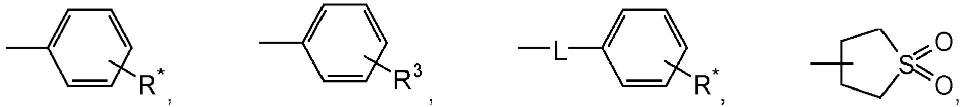 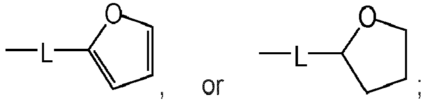
R^{S} represents -CH₃, -C₂H₅, -C₃H₇, cyclo-C₃H₅, -CH(CH₃)₂, -CH₂-CN, -CH₂-Ar, -CH(NH₂)-Ar,
Ar represents -Ph, 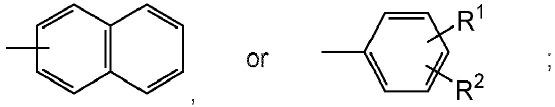
R* represents -OCH₃, -OC₂H₅, -OC₃H₇, -O-cyclo-C₃H₅, -OCH(CH₃)₂, -COCH₃, -COC₂H₅, -COC₃H₇, -CO-cyclo-C₃H₅, or -COCH(CH₃)₂;
R¹ and R² represent independently of each other -H, -OCH₃, -OC₂H₅, -NO₂, -F, -CI, -Br, -N₃, -CN, -OCN, -NCO, -SCN, -NCS, -CHO, -COCH₃, -COC₂H₅, -COOH, -COCN, -COOCH₃, -COOC₂H₅, -OOC-CH₃, -OOC-C₂H₅, -CONH₂, -CONHCH₃, -CONHC₂H₅, -CON(CH₃)₂, -CON(C₂H₅)₂, -NHCOCH₃, -NHCOC₂H₅, -NH₂, -NHCH₃, -NHC₂H₅, -N(CH₃)₂, -N(C₂H₅)₂, -SOCH₃, -SOC₂H₅, -SO₂CH₃, -SO₂C₂H₅, -SO₃H, -SO₃CH₃, -SO₃C₂H₅, -SO₂NH₂, -SO₂NHCH₃, -SO₂NHC₂H₅, -SO₂N(CH₃)₂, -SO₂N(C₂H₅)₂, -O-S(=O)CH₃, -O-S(=O)C₂H₅, -NH-SO₂-CH₃, -NH-SO₂-C₂H₅, -O-SO₂-CH₃, -O-SO₂-C₂H₅, -OCF₃, -CH₂-OCF₃, -OC₂F₅, -O-COOCH₃, -O-COOC₂H₅, -NH-CO-NH₂, -NH-CS-NH₂, -NH-C(=NH)-NH₂, -O-CO-NH₂, -O-CO-OCH₃, -O-CO-OC₂H₅, -CH₃, -C₂H₅, -C₃H₇, -CH(CH₃)₂, -CH₂F, -CHF₂, -CF₃, -CH₂Cl, -CH₂Br, and wherein R¹ or R² is different from hydrogen;
R³ represents -H, -CH₃, or -C₂H₅;
L represents -CH₂-, -C₂H₄-, or -C₃H₆-;
and salts especially agriculturally acceptable salts thereof.

The present invention further relates to a compound for use or the combination of compounds for use in inhibiting virulence of one or more plant pathogenic basidiomycete fungi, wherein the compound(s) is/are selected from: 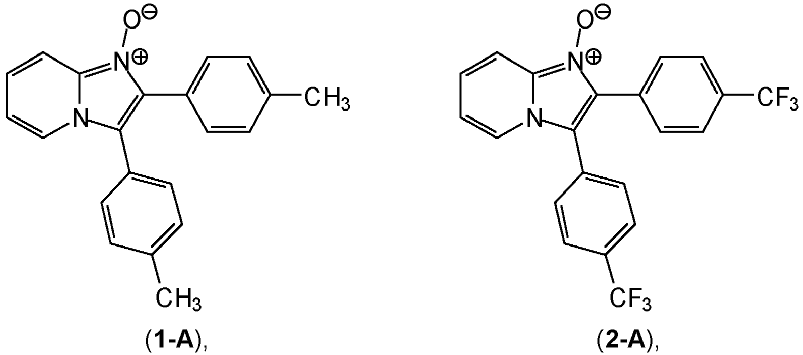 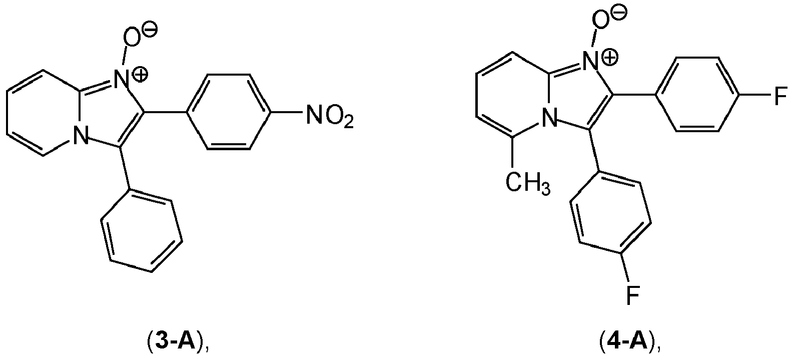 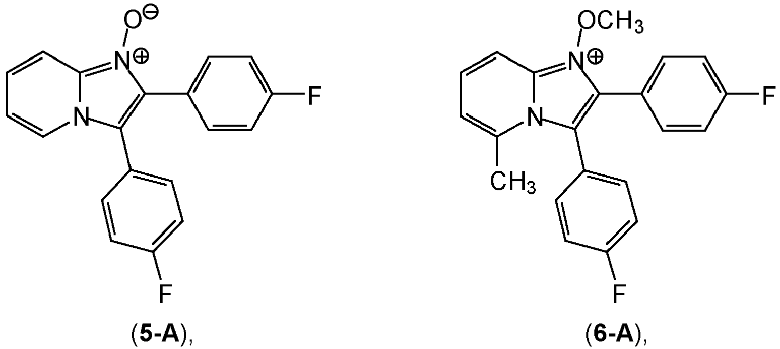 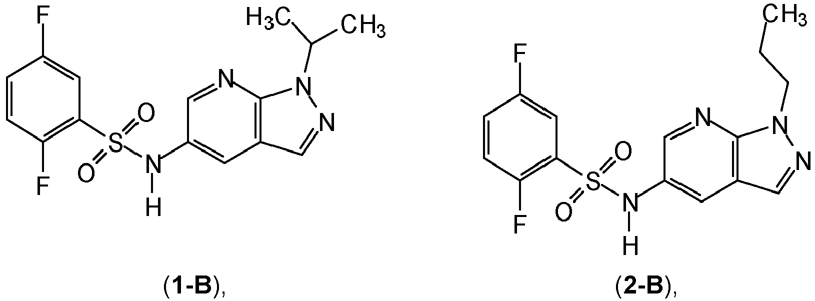 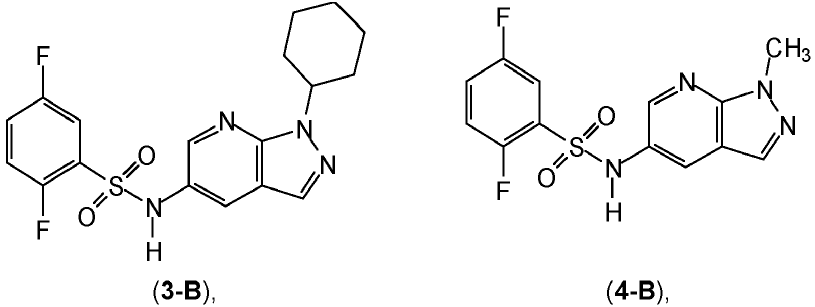 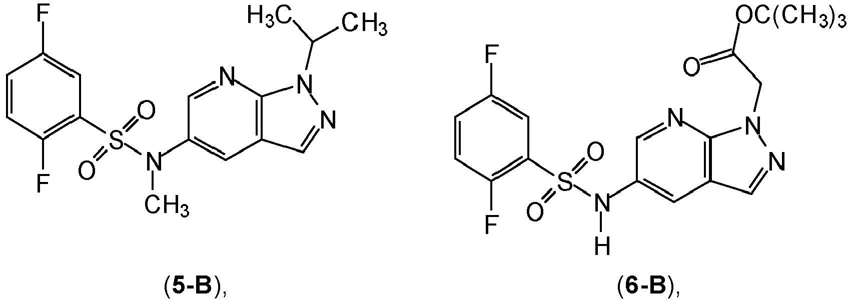 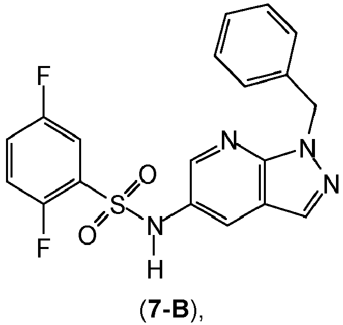 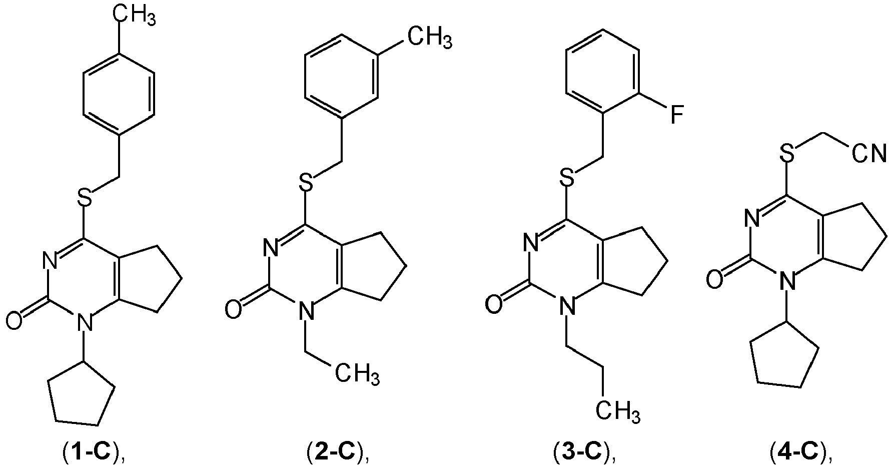 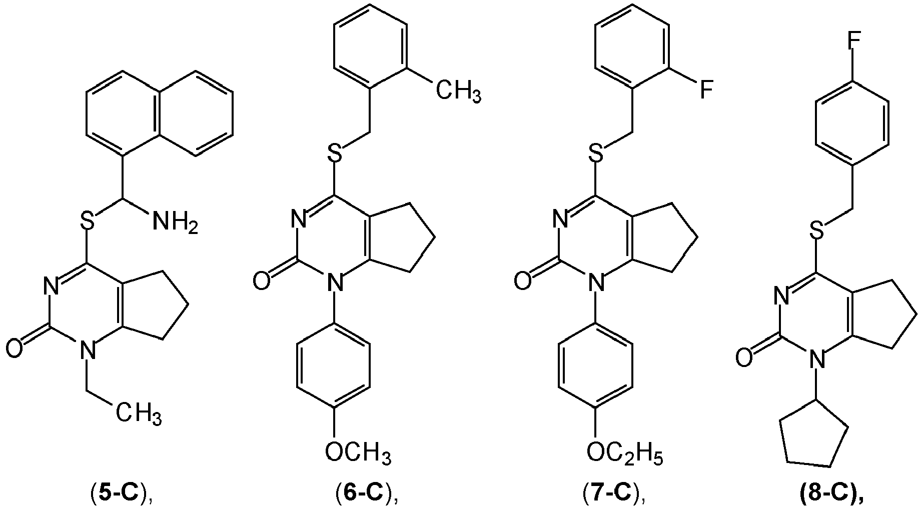 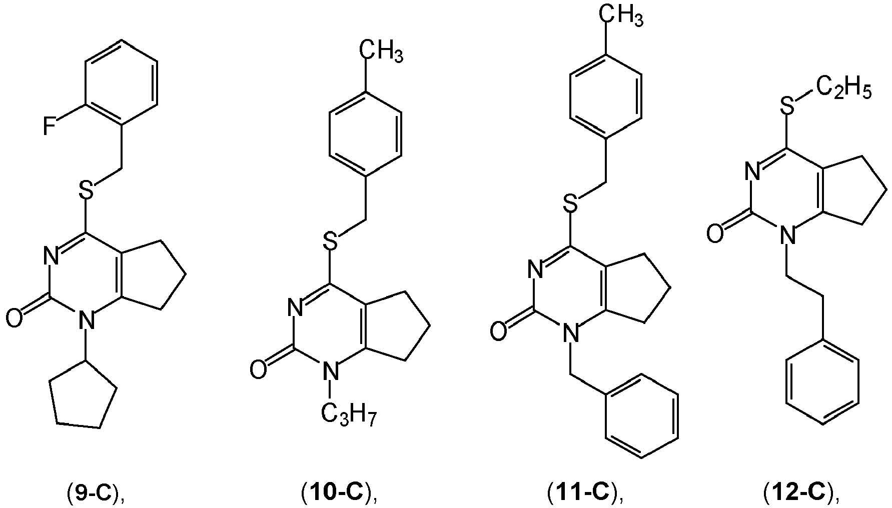 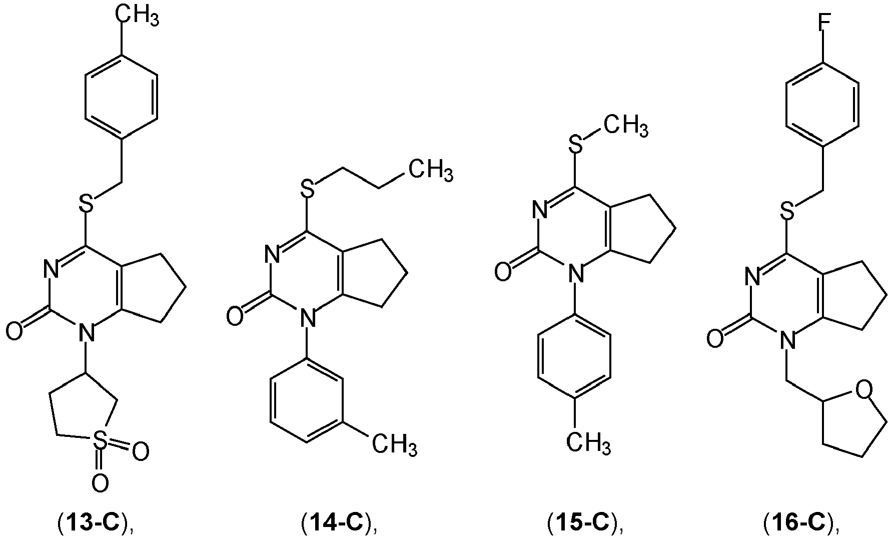 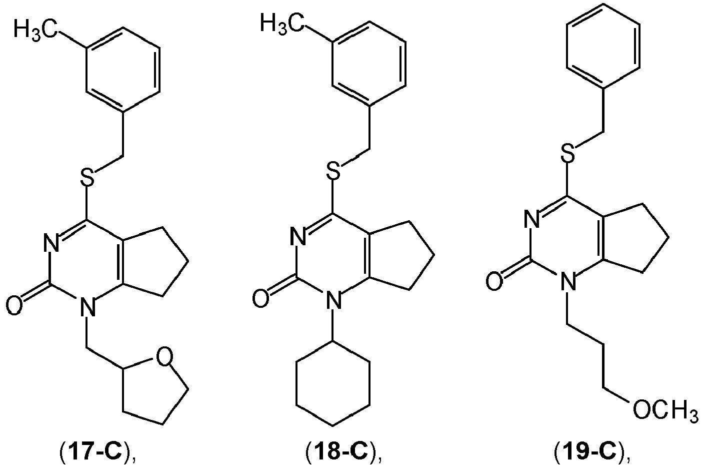 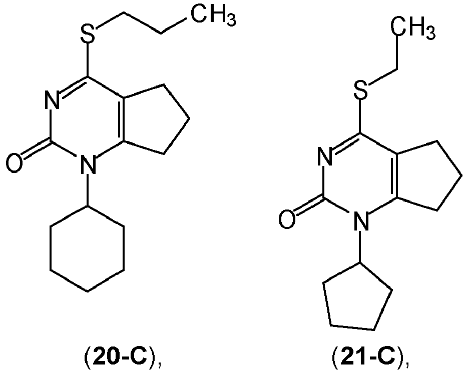 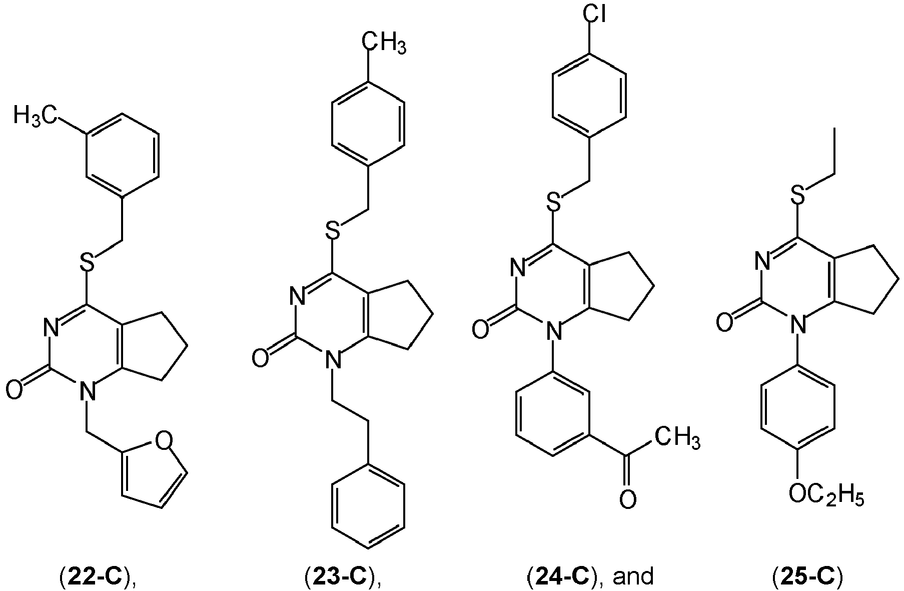 and salts especially agriculturally acceptable salts thereof.

More in particular, the present invention provides a compound for use or a combination of compounds for use in inhibiting virulence of one or more plant pathogenic basidiomycete fungi, wherein the compound(s) is/are selected from the general formula (I) - (III) which are defined as described above, and wherein the one or more plant pathogenic basidiomycete fungi are selected from the group consisting of smut fungi and rust fungi.

### Definitions

### Target plant pathogenic fungi

The terms **"plant pathogenic basidiomycete fungi"** and **"phytopathogenic basidiomycete fungi"** are used interchangeably to refer to fungi of the phyla Basidiomycota causing plant diseases. Basidiomycota (informally, basidiomycetes) is a large group, comprising about 32,000 known species. Morphologically, ecologically, and taxonomically this is a very diverse group, but its members share the feature that their sexual spores are exospores, formed on a basidium (and are therefore called basidiospores). Basidiomycota includes the plant pathogens that cause smut and rust diseases, ectomycorrhizal species which are of key significance to forest ecosystems, saprotrophic species that can decay the lignin ('white-rot fungi') as well the cellulose of plant litter, and the most noticeable and frequently encountered mushroom fungi.

The **"smut fungi"** represent the largest known group of plant pathogens following rusts, with more than 1,500 known species. Many smut fungi infect members of the Poaceae, which includes economically important cereal crops such as maize, wheat, barley, sorghum, and sugarcane. Smut fungi are characterized by their ability to produce massive amounts of black or dark brown teliospores. These cells are typically produced in the host's floral organs, directly affecting reproduction in infected plants. An exception with regard to infection style is *Ustilago maydis*, which causes common smut in maize. The smut fungi were traditionally divided into the phragmobasidiate Ustilaginaceae and the holobasidiate Tilletiaceae families. The last comprehensive compilation of taxonomy for smut fungi was produced in the book publication "Smut Fungi of the World" by K. G. Vánky (2012, The American Phytopathological Society, St. Paul, MN). In it, Vánky listed 1,650 recognized species of smut fungi, which he believed to only represent one third of all basidiomycetous smut fungi. These species within basidiomycota were found across two subphyla, four classes, eight orders, 26 families, and 93 genera. The classification system in use today applies classical methods of taxonomic classification relying on morphological characteristics of teliospores and basidiospores but has been augmented by molecular techniques, including DNA and, especially, comparative genome analyses.

The **"rust fungi"** form the order of the Pucciniales representing one of the largest fungal orders (Lorrain et al. 2019, Advances in understanding obligate biotrophy in rust fungi, New Phytologist 222: 1190-1206) with > 8000 species already described. These intriguing parasites have the ability to infect a wide variety of host plants, from ferns to monocots and gymnosperms to angiosperms, which suggests an ancestral adaptation to the biotrophic lifestyle. Worldwide, rust fungi are considered among the most serious threats to both agricultural crops (e.g. wheat (*Triticum aestivum*), soybean (*Glycine max*) or coffee), and tree species used for wood production and bioenergy (e.g. poplar, eucalypt or pines). To fend off rust diseases, the plant breeding industry primarily prioritized developing disease-resistant plants. Over the years, rust fungi have adapted to completely overcome plant resistances. The recent literature provides examples of loss estimates directly attributed to rust fungi. Overall, annual global losses in wheat production associated with rust fungi are estimated at US $4-5 billion.

**Table 1** provides examples of smut fungi und rust fungi, and plant diseases associated therewith, that can be treated or prevented using the compounds or a combination of compounds and related methods described herein.

**Table 1: Smut fungi and rungi examples**

| **Order** | **Example species** | **Host** | **Disease** |
|---|---|---|---|
| Smut fungi | | | |
| *Ustilaginales* | *Ustilago avenae* | oat | loose smut |
| *Ustilaginales* | *Ustilago hordei* | barley | covered smut |
| *Ustilaginales* | *Ustilago nuda* | barley | loose smut |
| *Ustilaginales* | *Ustilago nigra* | barley | false loose smut |
| *Ustilaginales* | *Ustilago tritici* | barley, rye, wheat | loose smut |
| *Ustilaginales* | *Ustilago maydis* | maize, teosinte | smut |
| *Ustilaginales* | *Ustilago scitaminea* | sugarcane | smut |
| *Ustilaginales* | *Ustilago esculenta* | asian wild rice, *Zizania latifolia* | smut |
| *Ustilaginales* | *Ustilago striiformis* | grasses | stripe smut |
| *Ustilaginales* | *Ustilago trichophora* | barnyard grasses | smut |
| *Microbotryales* | *Sporisorium reilianum* | maize, sorghum | head smut |
| *Microbotryales* | *Sporisorium reilianum f.sp.* | maize | head smut |
| *Microbotryales* | *Sporisorium reilianum f.sp. reilianum* | sorghum | head smut |
| *Ustilaginales* | *Sporisorium scitamineum* | sugarcane | smut |
| *Urocystales* | *Angiosorus solani* | potato | smut |
| *Urocystales* | *Urocystis agropyri* | wheat | flag smut |
| *Urocystales* | *Urocystis occulta* | rye | flag smut |
| *Tilletiales* | *Tilletia indica* | wheat, durum wheat, triticale | karnal bunt |
| *Tilletiales* | *Tilletia caries* | wheat, rye, other grasses | stinking smut of wheat |
| *Tilletiales* | *Tilletia controversa* | winter wheat | TCK smut |
| *Tilletiales* | *Tilletia barclayana* | rice, pearl millet | |

| Rust Fungi | | | |
|---|---|---|---|
| *Uromyces* | *Uromyces appendiculatus* | bean | common bean rust |
| *Uromyces* | *Uromyces beticola* | beet | rust of beet |
| *Uromyces* | *Uromyces fabae* | broad bean, pea, lentil | broad bean rust |
| *Pucciniales* | *Puccinia graminis f. sp. tritici* | wheat | wheat stem rust |
| *Pucciniales* | *Puccinia striiformis f. sp. tritici* | wheat | wheat yellow rust |
| *Pucciniales* | *Puccinia triticina* | wheat | wheat leaf rust |
| *Pucciniales* | *Puccinia graminis f. sp. secalis* | rye | rye stem rust |
| *Pucciniales* | *Puccinia arachidis* | peanut | peanut rust |
| *Pucciniales* | *Puccinia coronata f. sp. avenae* | oat | oat crown rust |
| *Pucciniales* | *Puccinia sorghi* | maize | common maize rust |
| *Pucciniales* | *Puccinia melanocephala* | sugarcane | sugarcane brown rust |
| *Pucciniales* | *Puccinia kuehnii* | sugarcane | orange rust |
| *Pucciniales* | *Puccinia recondita* | wheat and rye | leaf rust |
| | *Puccinia pitteriana* | potato and tomato | common potato and tomato rust |
| *Pucciniales* | *Puccinia graminis* | oat | oat stem rust |
| | *Puccinia po*/*ysora* | maize | rust, southern corn |
| *Pucciniales* | *Phakopsora pachyrhizi* | soybean | asian soybean rust |
| *Pucciniales* | *Phakopsora meibomiae* | soybean | (American) soybean rust |
| *Pucciniales* | *Hemileia vastatrix* | coffee | coffee leaf rust |
| *Pucciniales* | *Melampsora-larici-populina* | *Populus, Salicaceae* | poplar rust |
| *Pucciniales* | *Melampsora lini* | flax | flax rust |
| *Pucciniales* | *Cronartium ribicola* | five-needle pines | white pine blister |
| *Pucciniales* | *Cronartium quercuum f. sp. fusiforme* | pines | fusiform rust |
| *Pucciniales* | *Gymnosporangium sabinae* | pear, juniper | pear rust |
| *Pucciniales* | *Gymnosporangium yamadae* | japanese apple | rust |
| *Pucciniales* | *Gymnosporangium juniperi-virginianae* | cedar, apple | rust |

| | | | |
|---|---|---|---|
| TCK smut = *tilletia controversa kuhn* | | | |

Therefore, the present invention is directed to the compound for use or the combination of compounds for use, wherein the compound(s) is/are selected from the general formula (I) - (III) as disclosed in this invention, for use in inhibiting virulence of one or more plant pathogenic basidiomycete fungi selected from the group consisting of smut fungi and rust fungi.

More in particular, the present invention is directed to the compound for use or the combination of compounds for use, wherein the compound(s) is/are selected from the general formula (I) - (III) as disclosed in this invention, for use in inhibiting virulence of one or more plant pathogenic basidiomycete fungi selected from the group consisting of smut fungi and rust fungi, wherein the smut fungi are *Ustilago hordei, Ustilago avenae, Ustilago nuda, Ustilago nigra, Ustilago tritici, Ustilago maydis, Ustilago scitaminea, Ustilago esculenta, Ustilago striiformis, Ustilago trichophora, Sporisorium reilianum, Sporisorium reilianum f.sp. zeae, Sporisorium reilianum f.sp. reilianum, Sporisorium scitamineum, Angiosorus solani, Urocystis agropyri, Urocystis occulta, Tilletia indica, Tilletia caries, Tilletia barclayana* or *Tilletia controversa,* or wherein the rust fungi is *Uromyces fabae, Uromyces appendiculatus, Uromyces beticola, Puccinia graminis f. sp. tritici, Puccinia striiformis* f. sp. *tritici, Puccinia triticina, Puccinia graminis f. sp. secalis, Puccinia arachidis, Puccinia coronata f. sp. avenae, Puccinia sorghi, Puccinia melanocephala, Puccinia kuehnii, Puccinia recondita, Puccinia pitteriana, Puccinia graminis, Puccinia polysora, Phakopsora pachyrhizi, Phakopsora meibomiae, Hemileia vastatrix, Melampsora-larici-populina, Melampsora lini, Cronartium ribicola, Cronartium quercuum f. sp. fusiforme, Gymnosporangium sabinae, Gymnosporangium yamadae, Gymnosporangium juniperi-virginianae.*

More preferably, the one or more plant pathogenic basidiomycete fungi as disclosed in this invention are selected from the group consisting of smut fungi and rust fungi, wherein the smut fungi are *Ustilago maydis, Ustilago esculenta, Ustilago trichophora, Ustilago tritici, Sporisorium reilianum f.sp. zeae, Sporisorium reilianum f.sp. reilianum, Sporisorium scitamineum, Ustilago hordei,* or wherein the rust fungi is *Uromyces fabae.*

Therefore, the present invention is directed to a compound for use or a combination of compounds for use in inhibiting virulence of one or more plant pathogenic basidiomycete fungi, wherein the compound(s) is/are selected from the general formula (I) - (III) which are defined as follows: 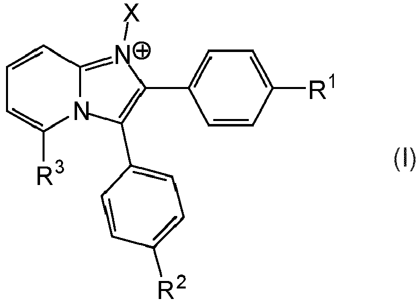 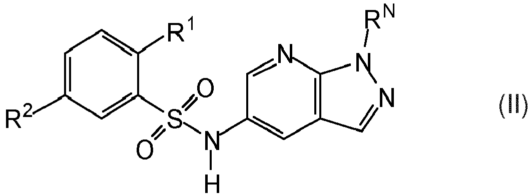 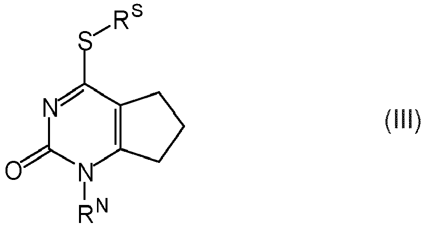 wherein
R¹ and R² represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -CH₂-OCH₃, -CH₂-OC₂H₅, -SH, -SCH₃, -SC₂H₅, -SC₃H₇, -S-cyclo-C₃H₅, -SCH(CH₃)₂, -SC(CH₃)₃, -NO₂, -F, -Cl, -Br, -P(O)(OH)₂, -P(O)(OCH₃)₂, -P(O)(OC₂H₅)₂, -P(O)(OCH(CH₃)₂)₂, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₆, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂Hₛ)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₆, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₃, -C₂H₅, -C₃H₇, -CH(CH₃)₂, -CH₂F, -CHF₂, -CF₃, -CH₂Cl, -CH₂Br, -CH₂-CH₂F, -CH₂-CHF₂, -CH₂-CF₃, -CH₂-CH₂Cl, or -CH₂-CH₂Br, and
wherein R¹ or R² is different from hydrogen;
R³ represents -H, -CH₃, -C₂H₅, -C₃H₇, cyclo-C₃H₅, -CH(CH₃)₂ or -C(CH₃)₃;
R^{N} represents -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -C₆H₁₃, -Ph, -L-Ph, -L- cyclo-C₅H₉, -L-R³, -L-R* cyclo-C₃H₅, cyclo-C₄H₇, cyclo-C₅H₉, cyclo-C₆H₁₁, cyclo-C₇H₁₃, 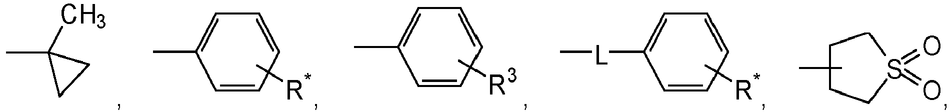 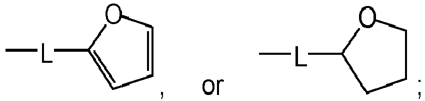
R^{S} represents -CH₃, -C₂H₅, -C₃H₇, cyclo-C₃H₅, -CH(CH₃)₂, -CH₂-CN, -CH₂-Ar, -CH(NH₂)-Ar,
Ar represents -Ph, 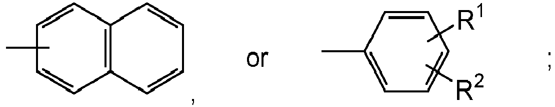
R* represents -OCH₃, -OC₂H₅, -OC₃H₇, -O-cyclo-C₃H₅, -OCH(CH₃)₂, -OC(CH₃)₃, -OC₄H₉, -COCH₃, -COC₂H₅, -COC₃H₇, -CO-cyclo-C₃H₅, -COCH(CH₃)₂, or -COC(CH₃)₃;
L represents -CH₂-, -C₂H₄-, -C₃H₆-, or -CH₂-CO-O-;
X represents -O⁻, -OCH₃, -OC₂H₅, or -OC₃H₇;
and salts especially agriculturally acceptable salts thereof,
wherein the one or more plant pathogenic basidiomycete fungi are selected from the group consisting of smut fungi and rust fungi, wherein the smut fungi are *Ustilago hordei, Ustilago avenae, Ustilago nuda, Ustilago nigra, Ustilago tritici, Ustilago maydis, Ustilago scitaminea, Ustilago esculenta, Ustilago striiformis, Ustilago trichophora, Sporisorium reilianum, Sporisorium reilianum f.sp. zeae, Sporisorium reilianum f.sp. reilianum, Sporisorium scitamineum, Angiosorus solani, Urocystis agropyri, Urocystis occulta, Tilletia indica, Tilletia caries, Tilletia barclayana* or *Tilletia controversa,* or wherein the rust fungi is *Uromyces fabae, Uromyces appendiculatus, Uromyces beticola, Puccinia graminis f. sp. tritici, Puccinia striiformis* f. sp. *tritici, Puccinia triticina, Puccinia graminis f. sp. secalis, Puccinia arachidis, Puccinia coronata f. sp. avenae, Puccinia sorghi, Puccinia melanocephala, Puccinia kuehnii, Puccinia recondita, Puccinia pitteriana, Puccinia graminis, Puccinia polysora, Phakopsora pachyrhizi, Phakopsora meibomiae, Hemileia vastatrix, Melampsora-larici-populina, Melampsora lini, Cronartium ribicola, Cronartium quercuum f. sp. fusiforme, Gymnosporangium sabinae, Gymnosporangium yamadae, Gymnosporangium juniperi-virginianae.*

The present invention is also directed to a compound for use or the combination of compounds for use in inhibiting virulence of one or more plant pathogenic basidiomycete fungi, wherein the compound(s) is/are selected from the general formula (I) - (III) which are defined as follows: 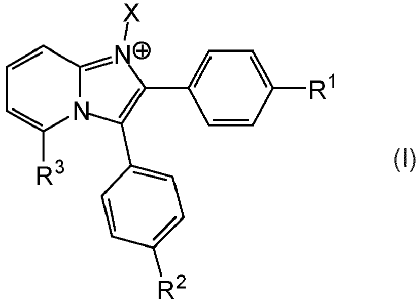 wherein
R¹ and R² represent independently of each other -H, -OCH₃, -OC₂H₅, -NO₂, -F, -Cl, -Br, -N₃, -CN, -OCN, -NCO, -SCN, -NCS, -CHO, -COCH₃, -COC₂H₅, -COOH, -COCN, -COOCH₃, -COOC₂H₅, -OOC-CH₃, -OOC-C₂H₅, -CONH₂, -CONHCH₃, -CONHC₂H₅, -CON(CH₃)₂, -CON(C₂H₅)₂, -NHCOCH₃, -NHCOC₂H₅, -NH₂, -NHCH₃, -NHC₂H₅, -N(CH₃)₂, -N(C₂H₅)₂, -SOCH₃, -SOC₂H₅, -SO₂CH₃, -SO₂C₂H₅, -SO₃H, -SO₃CH₃, -SO₃C₂H₅, -SO₂NH₂, -SO₂NHCH₃, -SO₂NHC₂H₅, -SO₂N(CH₃)₂, -SO₂N(C₂Hₛ)₂, -O-S(=O)CH₃ᵢ -O-S(=O)C₂H₅, -NH-SO₂-CH₃, -NH-SO₂-C₂H₅, -O-SO₂-CH₃, -O-SO₂-C₂H₅, -OCF₃, -CH₂-OCF₃, -OC₂F₅, -O-COOCH₃, -O-COOC₂H₅, -NH-CO-NH₂, -NH-CS-NH₂, -NH-C(=NH)-NH₂, -O-CO-NH₂, -O-CO-OCH₃, -O-CO-OC₂H₅, -CH₃, -C₂H₅, -C₃H₇, -CH(CH₃)₂, -CH₂F, -CHF₂, -CF₃, -CH₂Cl, -CH₂Br, and wherein R¹ or R² is different from hydrogen;
R³ represents -H or -CH₃;
X represents -O⁻ or -OCH₃;
and salts especially agriculturally acceptable salts thereof; 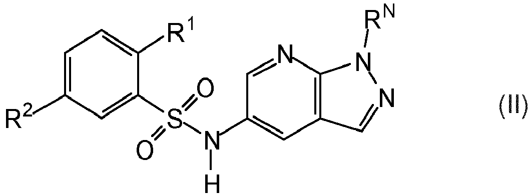
wherein
R¹ and R² represent independently of each other -H, -OCH₃, -OC₂H₅, -NO₂, -F, -Cl, -Br, -N₃, -CN, -OCN, -NCO, -SCN, -NCS, -CHO, -COCH₃, -COC₂H₅, -COOH, -COCN, -COOCH₃, -COOC₂H₅, -OOC-CH₃, -OOC-C₂H₅, -CONH₂, -CONHCH₃, -CONHC₂H₅, -CON(CH₃)₂, -CON(C₂H₅)₂, -NHCOCH₃, -NHCOC₂H₅, -NH₂, -NHCH₃, -NHC₂H₅, -N(CH₃)₂, -N(C₂H₅)₂, -SOCH₃, -SOC₂H₅, -SO₂CH₃, -SO₂C₂H₅, -SO₃H, -SO₃CH₃, -SO₃C₂H₅, -SO₂NH₂, -SO₂NHCH₃, -SO₂NHC₂H₅, -SO₂N(CH₃)₂, -SO₂N(C₂H₅)₂, -O-S(=O)CH₃, -O-S(=O)C₂H₅, -NH-SO₂-CH₃, -NH-SO₂-C₂H₅, -O-SO₂-CH₃, -O-SO₂-C₂H₅, -OCF₃, -CH₂-OCF₃, -OC₂F₅, -O-COOCH₃, -O-COOC₂H₆, -NH-CO-NH₂, -NH-CS-NH₂, -NH-C(=NH)-NH₂, -O-CO-NH₂, -O-CO-OCH₃, -O-CO-OC₂H₅, -CH₃, -C₂H₅, -C₃H₇, -CH(CH₃)₂, -CH₂F, -CHF₂, -CF₃, -CH₂Cl, -CH₂Br, and wherein R¹ or R² is different from hydrogen;
R^{N} represents -CH₃, -C₂H₅, -C₃H₇, -CH(CH₃)₂, -C₄H₉, -CH₂-CH(CH₃)₂, -C(CH₃)₃, -C₅H₁₁, -Ph, -L-Ph, -L-R³, cyclo-C₃H₅, cyclo-C₄H₇, cyclo-C₅H₉, cyclo-C₆H₁₁, cyclo-C₇H₁₃;
L represents -CH₂-, -C₂H₄-, or -CH₂-CO-O-;
R³ represents -H, -CH₃, -C₂H₅, -C₃H₇, cyclo-C₃H₅, -CH(CH₃)₂ or -C(CH₃)₃;
and salts especially agriculturally acceptable salts thereof; 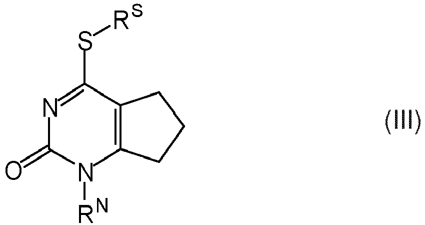
wherein
R^{N} represents -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -C₆H₁₃, -Ph, -L-Ph, -L- cyclo-C₅H₉, -L-R³, -L-R* cyclo-C₃H₅, cyclo-C₄H₇, cyclo-C₅H₉, cyclo-C₆H₁₁, cyclo-C₇H₁₃, 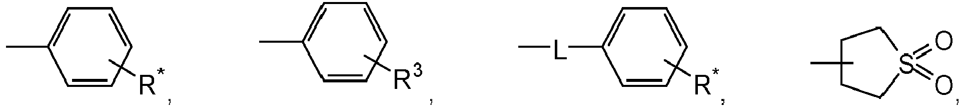 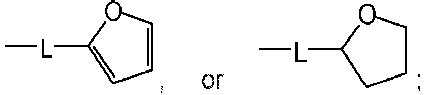
R^{S} represents -CH₃, -C₂H₅, -C₃H₇, cyclo-C₃H₅, -CH(CH₃)₂, -CH₂-CN, -CH₂-Ar, -CH(NH₂)-Ar,
Ar represents -Ph, 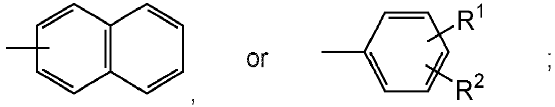
R* represents -OCH₃, -OC₂H₅, -OC₃H₇, -O-cyclo-C₃H₅, -OCH(CH₃)₂, -COCH₃, -COC₂H₅, -COC₃H₇, -CO-cyclo-C₃H₅, or -COCH(CH₃)₂;
R¹ and R² represent independently of each other -H, -OCH₃, -OC₂H₅, -NO₂, -F, -Cl, -Br, -N₃, -CN, -OCN, -NCO, -SCN, -NCS, -CHO, -COCH₃, -COC₂H₅, -COOH, -COCN, -COOCH₃, -COOC₂H₅, -OOC-CH₃, -OOC-C₂H₅, -CONH₂, -CONHCH₃, -CONHC₂H₅, -CON(CH₃)₂, -CON(C₂H₅)₂, -NHCOCH₃, -NHCOC₂H₅, -NH₂, -NHCH₃, -NHC₂H₅, -N(CH₃)₂, -N(C₂H₅)₂, -SOCH₃, -SOC₂H₅, -SO₂CH₃, -SO₂C₂H₅, -SO₃H, -SO₃CH₃, -SO₃C₂H₅, -SO₂NH₂, -SO₂NHCH₃, -SO₂NHC₂H₅, -SO₂N(CH₃)₂, -SO₂N(C₂H₅)₂, -O-S(=O)CH₃, -O-S(=O)C₂H₅, -NH-SO₂-CH₃, -NH-SO₂-C₂H₅, -O-SO₂-CH₃, -O-SO₂-C₂H₅, -OCF₃, -CH₂-OCF₃, -OC₂F₅, -O-COOCH₃, -O-COOC₂H₅, -NH-CO-NH₂, -NH-CS-NH₂, -NH-C(=NH)-NH₂, -O-CO-NH₂, -O-CO-OCH₃, -O-CO-OC₂H₅, -CH₃, -C₂H₅, -C₃H₇, -CH(CH₃)₂, -CH₂F, -CHF₂, -CF₃, -CH₂Cl, -CH₂Br, and wherein R¹ or R² is different from hydrogen;
R³ represents -H, -CH₃, or -C₂H₅;
L represents -CH₂-, -C₂H₄-, or -C₃H₆-;
and salts especially agriculturally acceptable salts thereof,
wherein the one or more plant pathogenic basidiomycete fungi are selected from the group consisting of smut fungi and rust fungi, wherein the smut fungi are *Ustilago hordei, Ustilago avenae, Ustilago nuda, Ustilago nigra, Ustilago tritici, Ustilago maydis, Ustilago scitaminea, Ustilago esculenta, Ustilago striiformis, Ustilago trichophora, Sporisorium reilianum, Sporisorium reilianum f.sp. zeae, Sporisorium reilianum f.sp. reilianum, Sporisorium scitamineum, Angiosorus solani, Urocystis agropyri, Urocystis occulta, Tilletia indica, Tilletia caries, Tilletia barclayana* or *Tilletia controversa,* or wherein the rust fungi is *Uromyces fabae, Uromyces appendiculatus, Uromyces beticola, Puccinia graminis f. sp. tritici, Puccinia striiformis* f. sp. *tritici, Puccinia triticina, Puccinia graminis f. sp. secalis, Puccinia arachidis, Puccinia coronata f. sp. avenae, Puccinia sorghi, Puccinia melanocephala, Puccinia kuehnii, Puccinia recondita, Puccinia pitteriana, Puccinia graminis, Puccinia polysora, Phakopsora pachyrhizi, Phakopsora meibomiae, Hemileia vastatrix, Melampsora-larici-populina, Melampsora lini, Cronartium ribicola, Cronartium quercuum f. sp. fusiforme, Gymnosporangium sabinae, Gymnosporangium yamadae, Gymnosporangium juniperi-virginianae.*

The present invention is further directed to a compound for use or the combination of compounds for use in inhibiting virulence of one or more plant pathogenic basidiomycete fungi, wherein the compound(s) is/are selected from: **1-A, 2-A, 3-A, 4-A, 5-A, 6-A, 1-B, 2-B, 3-B, 4-B, 5-B, 6-B, 7-B, 1-C, 2-C, 3-C, 4-C, 5-C, 6-C, 7-C, 8-C, 9-C, 10-C, 11-C, 12-C, 13-C, 14-C, 15-C, 16-C, 17-C, 18-C, 19-C, 20-C, 21-C, 22-C, 23-C, 24-C,** and **25-C,** and salts especially agriculturally acceptable salts thereof,
wherein the one or more plant pathogenic basidiomycete fungi are selected from the group consisting of smut fungi and rust fungi, wherein the smut fungi are *Ustilago hordei, Ustilago avenae, Ustilago nuda, Ustilago nigra, Ustilago tritici, Ustilago maydis, Ustilago scitaminea, Ustilago esculenta, Ustilago striiformis, Ustilago trichophora, Sporisorium reilianum, Sporisorium reilianum f.sp. zeae, Sporisorium reilianum f.sp. reilianum, Sporisorium scitamineum, Angiosorus solani, Urocystis agropyri, Urocystis occulta, Tilletia indica, Tilletia caries, Tilletia barclayana* or *Tilletia controversa,* or wherein the rust fungi is *Uromyces fabae, Uromyces appendiculatus, Uromyces beticola, Puccinia graminis f. sp. tritici, Puccinia striiformis* f. sp. *tritici, Puccinia triticina, Puccinia graminis f. sp. secalis, Puccinia arachidis, Puccinia coronata f. sp. avenae, Puccinia sorghi, Puccinia melanocephala, Puccinia kuehnii, Puccinia recondita, Puccinia pitteriana, Puccinia graminis, Puccinia polysora, Phakopsora pachyrhizi, Phakopsora meibomiae, Hemileia vastatrix, Melampsora-larici-populina, Melampsora lini, Cronartium ribicola, Cronartium quercuum f. sp. fusiforme, Gymnosporangium sabinae, Gymnosporangium yamadae, Gymnosporangium juniperi-virginianae.*

Moreover, the present invention is directed to a compound for use or a combination of compounds for use in inhibiting virulence of one or more plant pathogenic basidiomycete fungi, wherein the compound(s) is/are selected from the general formula (I) - (III) which are defined as follows: 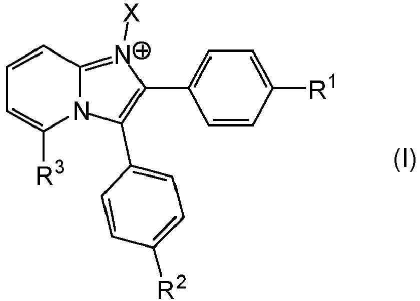 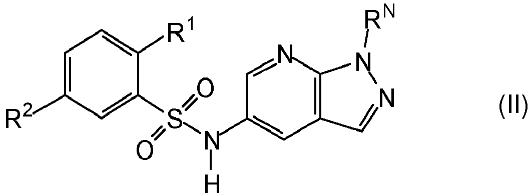 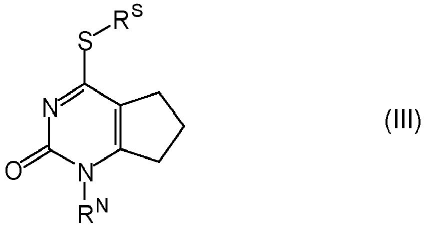
wherein R¹, R², R³, R^{N}, R^{S}, R*, Ar, L and X have the meanings as disclosed herein and salts especially agriculturally acceptable salts thereof,
wherein the one or more plant pathogenic basidiomycete fungi are selected from the group consisting of smut fungi and rust fungi.

The present invention is further directed to a compound for use or the combination of compounds for use in inhibiting virulence of one or more plant pathogenic basidiomycete fungi, wherein the compound(s) is/are selected from **1-A, 2-A, 3-A, 4-A, 5-A, 6-A, 1-B, 2-B, 3-B, 4-B, 5-B, 6-B, 7-B, 1-C, 2-C, 3-C, 4-C, 5-C, 6-C, 7-C, 8-C, 9-C, 10-C, 11-C, 12-C, 13-C, 14-C, 15-C, 16-C, 17-C, 18-C, 19-C, 20-C, 21-C, 22-C, 23-C, 24-C,** and **25-C,** and salts especially agriculturally acceptable salts thereof, wherein the one or more plant pathogenic basidiomycete fungi are selected from the group consisting of smut fungi and rust fungi.

Further, the present invention is directed to a compound for use or a combination of compounds for use in inhibiting virulence of one or more plant pathogenic basidiomycete fungi, wherein the compound(s) is/are selected from the general formula (I) - (III) which are defined as follows: 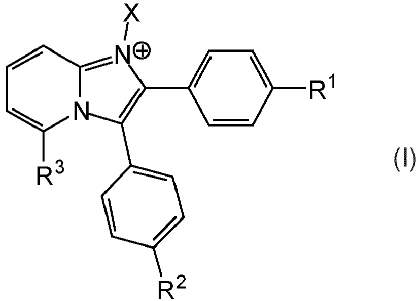 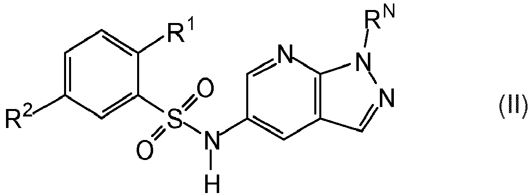 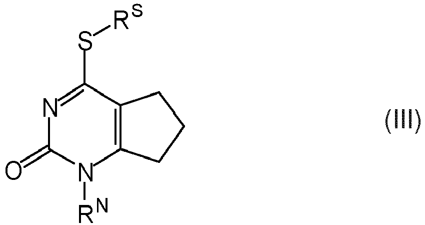
wherein R¹, R², R³, R^{N}, R^{S}, R*, Ar, L and X have the meanings as disclosed herein and salts especially agriculturally acceptable salts thereof,
wherein the one or more plant pathogenic basidiomycete fungi are selected from the group consisting of smut fungi and rust fungi, wherein the smut fungi are *Ustilago maydis, Ustilago esculenta, Ustilago trichophora, Ustilago tritici, Sporisorium reilianum f.sp. zeae, Sporisorium reilianum f.sp. reilianum, Sporisorium scitamineum, Ustilago hordei,* or wherein the rust fungi is *Uromyces fabae.*

The present invention is further directed to a compound for use or the combination of compounds for use in inhibiting virulence of one or more plant pathogenic basidiomycete fungi, wherein the compound(s) is/are selected from **1-A, 2-A, 3-A, 4-A, 5-A, 6-A, 1-B, 2-B, 3-B, 4-B, 5-B, 6-B, 7-B, 1-C, 2-C, 3-C, 4-C, 5-C, 6-C, 7-C, 8-C, 9-C, 10-C, 11-C, 12-C, 13-C, 14-C, 15-C, 16-C, 17-C, 18-C, 19-C, 20-C, 21-C, 22-C, 23-C, 24-C,** and **25-C,** and salts especially agriculturally acceptable salts thereof,
wherein the one or more plant pathogenic basidiomycete fungi are selected from the group consisting of smut fungi and rust fungi, wherein the smut fungi are *Ustilago maydis, Ustilago esculenta, Ustilago trichophora, Ustilago tritici, Sporisorium reilianum f.sp. zeae, Sporisorium reilianum f.sp. reilianum, Sporisorium scitamineum, Ustilago hordei,* or wherein the rust fungi is *Uromyces fabae.*

As used herein, the terms **"fungicide", "fungicidal compound", "fungicidal composition", "antifungal agent", "antifungal compound",** and **"antifungal composition"** are used interchangeably to refer to a substance that kills or inhibits the growth, proliferation, division, reproduction, virulence or spread of fungi, such as plant pathogenic fungi.

The term **"fungi pathogenicity"** refers to the capacity of a fungus to cause damage in a host.
**"Virulence"** is the degree of pathogenicity, i.e., the amount of measureable disease or damage that fungi can cause. Virulence may be determined for example by mortality due to a strain or mix of strains. More in particular, virulence of plant pathogenic fungi may be determined for example by quantification of the damages or symptoms to the host plant.

### Fungicidal composition

A further embodiment of the invention provides a fungicidal composition comprising a compound for use or a combination of compounds for use in treating, preventing, inhibiting, or eliminating the virulence of one or more plant pathogenic basidiomycete fungi in a plant or a plant material, wherein the compound(s) is/are selected from the general formula (I) - (III) as follows: 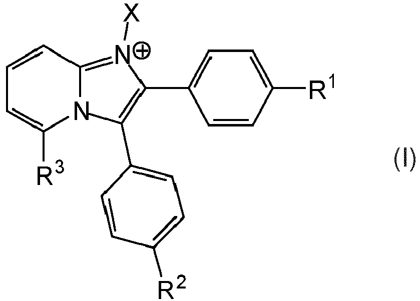 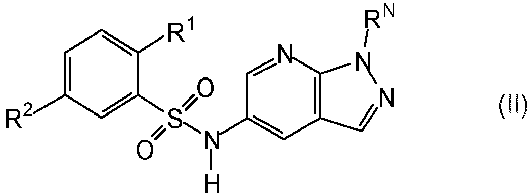 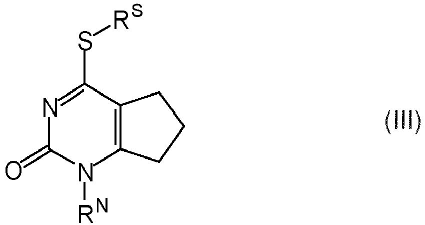
wherein R¹, R², R³, R^{N}, R^{S}, R*, Ar, L and X have the meanings as disclosed herein and salts especially agriculturally acceptable salts thereof; and
wherein the fungicidal composition further comprises at least one suitable agriculturally acceptable carrier, excipient, solvent and/or adjuvant.

A further more particular embodiment of the invention provides a fungicidal composition comprising a compound for use or a combination of compounds for use in treating, preventing, inhibiting, or eliminating the virulence of one or more plant pathogenic basidiomycete fungi in a plant or a plant material, wherein the compound(s) is/are selected from **1-A, 2-A, 3-A, 4-A, 5-A, 6-A, 1-B, 2-B, 3-B, 4-B, 5-B, 6-B, 7-B, 1-C, 2-C, 3-C, 4-C, 5-C, 6-C, 7-C, 8-C, 9-C, 10-C, 11-C, 12-C, 13-C, 14-C, 15-C, 16-C, 17-C, 18-C, 19-C, 20-C, 21-C, 22-C, 23-C, 24-C,** and **25-C,** and salts especially agriculturally acceptable salts thereof;
wherein the fungicidal composition further comprises at least one suitable agriculturally acceptable carrier, excipient, solvent and/or adjuvant.

A further embodiment of the invention provides a fungicidal composition comprising a compound for use or a combination of compounds for use in treating, preventing, inhibiting, or eliminating the virulence of one or more plant pathogenic basidiomycete fungi in a plant or a plant material, wherein the compound(s) is/are selected from the general formula (I) - (III) as follows: 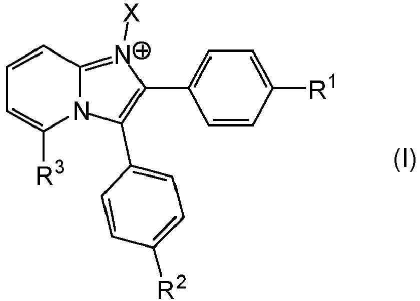 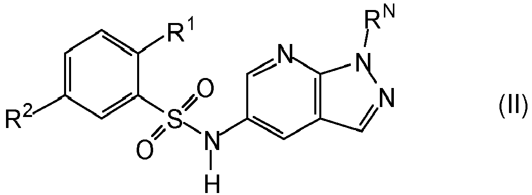 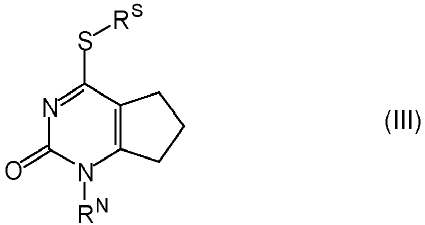
wherein R¹, R², R³, R^{N}, R^{S}, R*, Ar, L and X have the meanings as disclosed herein and salts especially agriculturally acceptable salts thereof; and
wherein the fungicidal composition further comprises at least one suitable agriculturally acceptable carrier, excipient, solvent and/or adjuvant, and
wherein the one or more plant pathogenic basidiomycete fungi are selected from the group consisting of smut fungi and rust fungi.

A further embodiment of the invention provides a fungicidal composition comprising a compound for use or a combination of compounds for use in treating, preventing, inhibiting, or eliminating the virulence of one or more plant pathogenic basidiomycete fungi in a plant or a plant material, wherein the compound(s) is/are selected from the general formula (I) - (III) as follows: 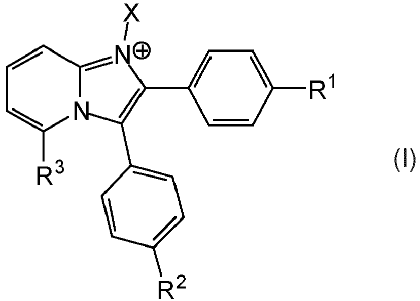 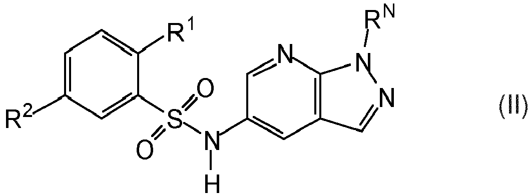 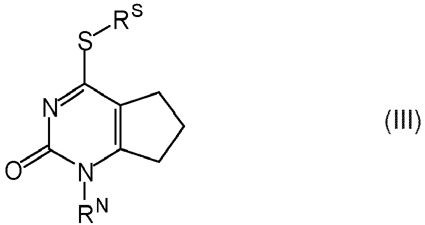
wherein R¹, R², R³, R^{N}, R^{S}, R*, Ar, L and X have the meanings as disclosed herein and salts especially agriculturally acceptable salts thereof; and
wherein the fungicidal composition further comprises at least one suitable agriculturally acceptable carrier, excipient, solvent and/or adjuvant, and
wherein the one or more plant pathogenic basidiomycete fungi are selected from the group consisting of smut fungi and rust fungi, wherein the smut fungi are *Ustilago hordei, Ustilago avenae, Ustilago nuda, Ustilago nigra, Ustilago tritici, Ustilago maydis, Ustilago scitaminea, Ustilago esculenta, Ustilago striiformis, Ustilago trichophora, Sporisorium reilianum, Sporisorium reilianum f.sp. zeae, Sporisorium reilianum f.sp. reilianum, Sporisorium scitamineum, Angiosorus solani, Urocystis agropyri, Urocystis occulta, Tilletia indica, Tilletia caries, Tilletia barclayana* or *Tilletia controversa,* or wherein the rust fungi is *Uromyces fabae, Uromyces appendiculatus, Uromyces beticola, Puccinia graminis f. sp. tritici, Puccinia striiformis* f. sp. *tritici, Puccinia triticina, Puccinia graminis f. sp. secalis, Puccinia arachidis, Puccinia coronata f. sp. avenae, Puccinia sorghi, Puccinia melanocephala, Puccinia kuehnii, Puccinia recondita, Puccinia pitteriana, Puccinia graminis, Puccinia polysora,Phakopsora pachyrhizi, Phakopsora meibomiae, Hemileia vastatrix, Melampsora-larici-populina, Melampsora lini, Cronartium ribicola, Cronartium quercuum f. sp. fusiforme, Gymnosporangium sabinae, Gymnosporangium yamadae, Gymnosporangium juniperi-virginianae.*

A further still more particular embodiment of the invention provides a fungicidal composition comprising a compound for use or a combination of compounds for use in treating, preventing, inhibiting, or eliminating the virulence of one or more plant pathogenic basidiomycete fungi in a plant or a plant material, wherein the compound(s) is/are selected from **1-A, 2-A, 3-A, 4-A, 5-A, 6-A, 1-B, 2-B, 3-B, 4-B, 5-B, 6-B, 7-B, 1-C, 2-C, 3-C, 4-C, 5-C, 6-C, 7-C, 8-C, 9-C, 10-C, 11-C, 12-C, 13-C, 14-C, 15-C, 16-C, 17-C, 18-C, 19-C, 20-C, 21-C, 22-C, 23-C, 24-C,** and **25-C,** and salts especially agriculturally acceptable salts thereof;
wherein the fungicidal composition further comprises at least one suitable agriculturally acceptable carrier, excipient, solvent and/or adjuvant, and
wherein the one or more plant pathogenic basidiomycete fungi are selected from the group consisting of smut fungi and rust fungi, wherein the smut fungi are *Ustilago hordei, Ustilago avenae, Ustilago nuda, Ustilago nigra, Ustilago tritici, Ustilago maydis, Ustilago scitaminea, Ustilago esculenta, Ustilago striiformis, Ustilago trichophora, Sporisorium reilianum, Sporisorium reilianum f.sp. zeae, Sporisorium reilianum f.sp. reilianum, Sporisorium scitamineum, Angiosorus solani, Urocystis agropyri, Urocystis occulta, Tilletia indica, Tilletia caries, Tilletia barclayana* or *Tilletia controversa,* or wherein the rust fungi is *Uromyces fabae, Uromyces appendiculatus, Uromyces beticola, Puccinia graminis f. sp. tritici, Puccinia striiformis* f. sp. *tritici, Puccinia triticina, Puccinia graminis f. sp. secalis, Puccinia arachidis, Puccinia coronata f. sp. avenae, Puccinia sorghi, Puccinia melanocephala, Puccinia kuehnii, Puccinia recondita, Puccinia pitteriana, Puccinia graminis, Puccinia polysora,Phakopsora pachyrhizi, Phakopsora meibomiae, Hemileia vastatrix, Melampsora-larici-populina, Melampsora lini, Cronartium ribicola, Cronartium quercuum f. sp. fusiforme, Gymnosporangium sabinae, Gymnosporangium yamadae, Gymnosporangium juniperi-virginianae.*

A further embodiment of the invention provides a fungicidal composition comprising a compound for use or a combination of compounds for use in treating, preventing, inhibiting, or eliminating the virulence of one or more plant pathogenic basidiomycete fungi in a plant or a plant material, wherein the compound(s) is/are selected from the general formula (I) - (III) as follows: 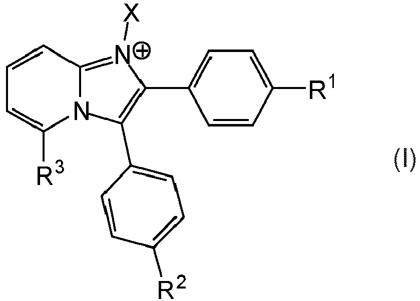 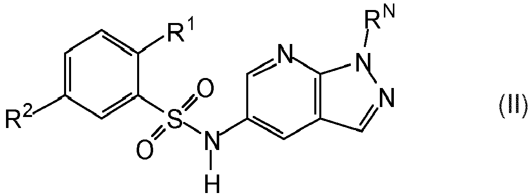 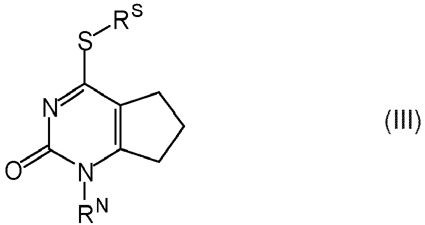
wherein R¹, R², R³, R^{N}, R^{S}, R*, Ar, L and X have the meanings as disclosed herein and salts especially agriculturally acceptable salts thereof; and
wherein the fungicidal composition further comprises at least one suitable agriculturally acceptable carrier, excipient, solvent and/or adjuvant, and
wherein the one or more plant pathogenic basidiomycete fungi are selected from the group consisting of smut fungi and rust fungi, wherein the smut fungi are *Ustilago maydis, Ustilago esculenta, Ustilago trichophora, Ustilago tritici, Sporisorium reilianum f.sp. zeae, Sporisorium reilianum f.sp. reilianum, Sporisorium scitamineum, Ustilago hordei,* or wherein the rust fungi is *Uromyces fabae.*

Another more particular embodiment of the invention provides a fungicidal composition comprising a compound for use or a combination of compounds for use in treating, preventing, inhibiting, or eliminating the virulence of one or more plant pathogenic basidiomycete fungi in a plant or a plant material, wherein the compound(s) is/are selected from **1-A, 2-A, 3-A, 4-A, 5-A, 6-A, 1-B, 2-B, 3-B, 4-B, 5-B, 6-B, 7-B, 1-C, 2-C, 3-C, 4-C, 5-C, 6-C, 7-C, 8-C, 9-C, 10-C, 11-C, 12-C, 13-C, 14-C, 15-C, 16-C, 17-C, 18-C, 19-C, 20-C, 21-C, 22-C, 23-C, 24-C,** and **25-C,** and salts especially agriculturally acceptable salts thereof;
wherein the fungicidal composition further comprises at least one suitable agriculturally acceptable carrier, excipient, solvent and/or adjuvant, and
wherein the one or more plant pathogenic basidiomycete fungi are selected from the group consisting of smut fungi and rust fungi, wherein the smut fungi are *Ustilago maydis, Ustilago esculenta, Ustilago trichophora, Ustilago tritici, Sporisorium reilianum f.sp. zeae, Sporisorium reilianum f.sp. reilianum, Sporisorium scitamineum, Ustilago hordei, or wherein the rust fungi is Uromyces fabae.*

In the context of the present invention, **"treating or inhibiting the virulence of a plant pathogenic fungus"** means a reduction in infection or infestation by the **plant pathogenic fungus,** compared with the untreated plant, preferably a reduction by 25 - 50 %, compared with the untreated plant (100 %), more preferably a reduction by 40-79 %, compared with the untreated plant (100 %); even more preferably, the infection by the **plant pathogenic fungus** is almost entirely suppressed or eliminated (by 70-100 %).
In the context of the present invention, **"eliminating the virulence of a plant pathogenic fungus"** means a reduction in infection or infestation by the **plant pathogenic fungus,** compared with the untreated plant, by 100 %, compared with the untreated plant (100 %), i.e. the infection by the **plant pathogenic fungus** is entirely suppressed.
In the context of the present invention, **"preventing the virulence of a plant pathogenic fungus"** means to decrease the likelihood of infection by said fungus relative to the likelihood of infection in an untreated plant. For example, the method can decrease the likelihood of infection by 1%, 2%, 5%, 10%, 20%, 30%, 40%, 50%, 60%, 70%, 80%, 90%, 95%, 99%, 100%, or more than 100% relative to an untreated plant. In some instances, the method can decrease the likelihood of infection by about 2x-fold, 5x-fold, 10x-fold, 25x-fold, 50x-fold, 75x-fold, 100x-fold, or more than 100x-fold relative to an untreated plant.

The infection or infestation by the plant pathogenic fungus can be determined by using a particular method depending on the fungus and plant being tested. Infection and associated symptoms can be identified by any means of identifying infection or related symptoms. In one aspect, the methods may involve macroscopic or microscopic screening for infection and/or symptoms, quantitative PCR, or the use of microarrays for detection of infection related genes (e.g. Systemic Acquired Resistance genes, defensin genes, and the like). Macroscopic and microscopic methods for determining infestation in a plant are known in the art and include the identification of damage on plant tissue caused by the infection or by the presence of lesions, necrosis, pustules, tumors, spores, hyphae, growth of fungal mycelium, wilts, blights, spots on fruits, rots, galls and stunts, or the like. Such symptoms can be compared to non-infested plants, photos, or illustrations of infected plants or combinations thereof to determine the presence of an infection or the identity of the pathogen or both. Photos and illustrations of the symptoms of pathogen infection are widely available in the art. In some instances, the symptoms are visible to the naked eye or by a specified magnification (e.g., 2x, 3x, 4x, 5x, 10x, or 50x). In some instances, the infection or associated symptom can be identified using commercially available test kits to identify pests in plants. Such test kits are available, for example, from local agricultural extensions or cooperatives. In some instances, identifying a crop plant in need of treatment is by prediction of weather and environmental conditions conducive for disease development.

The infection or infestation by the plant pathogenic fungus is determined for example by quantification of tumor formation when analysing *U. maydis* infection in corn (Kamper et al. 2006), or of formation of spore pustules when analysing *U. fabae* infection in *Vicia faba* plants (Sillero and Rubiales 2002, Histological Characterization of Resistance to Uromyces viciae-fabae in Faba Bean, Phytopathology 92 (3), 294-299).

An **"fungicidally effective amount"** or " **fungicidally effective phytologically acceptable amount"** means an amount of the inventive compound or composition which is sufficient to **treating, preventing, inhibiting, or eliminating the virulence of one plant pathogenic basidiomycete fungus** in a plant or a plant material, and which, at the same time, does not cause any significant symptoms of phytotoxicity in the treated plant or plant material.
In general, the effective amount may vary within a relatively wide range. It depends on several factors, for example on the fungus to be controlled, the stage of growth thereof, the plant, or the part of the plant or other plant material to be contacted with the compound, the climatic conditions and the ingredients of the inventive compositions.

Suitable organic **solvents** include all polar and non-polar organic solvents usually employed for formulation purposes. Preferable the solvents are selected from ketones, e.g. methyl-isobutyl-ketone and cyclohexanone, amides, e.g. dimethyl formamide and alkanecarboxylic acid amides, e.g. N,N-dimethyl decaneamide and N,N-dimethyl octanamide, furthermore cyclic solvents, e.g. N-methyl-pyrrolidone, N-octyl-pyrrolidone, N-dodecyl-pyrrolidone, N-octyl-caprolactame, N-dodecyl-caprolactame and butyrolactone, furthermore strong polar solvents, e.g. dimethylsulfoxide, and aromatic hydrocarbons, e.g. xylol, Solvesso^{™}, mineral oils, e.g. white spirit, petroleum, alkyl benzenes and spindle oil, also esters, e.g. propyleneglycol-monomethylether acetate, adipic acid dibutylester, acetic acid hexylester, acetic acid heptylester, citric acid tri-n-butylester and phthalic acid di-n-butylester, and also alkohols, e.g. benzyl alcohol and 1-methoxy-2-propanol.

According to the invention, **carrier** is to be understood as meaning a natural or synthetic, organic or inorganic substance, which is mixed or combined with the active compounds for better applicability, in particular for application to plants or plant parts or seeds. The **carrier,** which may be solid or liquid, is generally inert and should be suitable for use in agriculture.
According to the invention, a **carrier** is a natural or synthetic, organic or inorganic substance with which the active ingredients are mixed or combined for better applicability, in particular for application to plants or plant parts or seed. The **carrier,** which may be solid or liquid, is generally inert and should be suitable for use in agriculture.
Useful **solid or liquid carriers** include for example ammonium salts and natural rock dusts, such as kaolins, clays, talc, chalk, quartz, attapulgite, montmorillonite or diatomaceous earth, and synthetic rock dusts, such as finely divided silica, alumina and natural or synthetic silicates, resins, waxes, solid fertilizers, water, alcohols, especially butanol, organic solvents, mineral and vegetable oils, and derivatives thereof. Mixtures of such carriers can likewise be used. Suitable **solid carrier** include inorganic particles, e.g. carbonates, silikates, sulphates and oxides with an average particle size of between 0.005 and 20 mm, preferably of between 0.02 to 10 mm, for example ammonium sulphate, ammonium phosphate, urea, calcium carbonate, calcium sulphate, magnesium sulphate, magnesium oxide, aluminium oxide, silicium dioxide, so-called fine-particle silica, silica gels, natural or synthetic silicates, and alumosilicates and plant products like cereal flour, wood powder/sawdust and cellulose powder. Useful **solid carriers for granules** include for example crushed and fractionated natural rocks such as calcite, marble, pumice, sepiolite, dolomite, and synthetic granules of inorganic and organic meals, and also granules of organic material such as sawdust, coconut shells, maize cobs and tobacco stalks. Useful **liquefied gaseous carriers** are those liquids which are gaseous at standard temperature and under standard pressure, for example aerosol propellants such as halohydrocarbons, and also butane, propane, nitrogen and carbon dioxide.

The **fungicidal compositions** according to the invention may comprise additional further components, such as, for example, **surfactants.** Suitable surfactants are emulsifiers, dispersants or wetting agents having ionic or nonionic properties, or mixtures of these surfactants. Examples of these are salts of polyacrylic acid, salts of lignosulphonic acid, salts of phenolsulphonic acid or naphthalenesulphonic acid, polycondensates of ethylene oxide with fatty alcohols or with fatty acids or with fatty amines, substituted phenols (preferably alkylphenols or arylphenols), salts of sulphosuccinic esters, taurine derivatives (preferably alkyl taurates), phosphoric esters of polyethoxylated alcohols or phenols, fatty esters of polyols, and derivatives of the compounds containing sulphates, sulphonates and phosphates. The presence of a **surfactant** is required if one of the active compounds and/or one of the inert carriers is insoluble in water and when the application takes place in water. The proportion of surfactants is between 5 and 40 per cent by weight of the composition according to the invention.
Suitable **surfactants** (**adjuvants**, emulsifiers, dispersants, protective colloids, wetting agent and adhesive) include all common ionic and non-ionic substances, for example ethoxylated nonylphenols, polyalkylene glycolether of linear or branched alcohols, reaction products of alkyl phenols with ethylene oxide and/or propylene oxide, reaction products of fatty acid amines with ethylene oxide and/or propylene oxide, furthermore fattic acid esters, alkyl sulfonates, alkyl sulphates, alkyl ethersulphates, alkyl etherphosphates, arylsulphate, ethoxylated arylalkylphenols, e.g. tristyrylphenol-ethoxylates, furthermore ethoxylated and propoxylated arylalkylphenols like sulphated or phosphated arylalkylphenol-ethoxylates and -ethoxy- and -propoxylates. Further examples are natural and synthetic, water soluble polymers, e.g. lignosulphonates, gelatine, gum arabic, phospholipides, starch, hydrophobic modified starch and cellulose derivatives, in particular cellulose ester and cellulose ether, further polyvinyl alcohol, polyvinyl acetate, polyvinyl pyrrolidone, polyacrylic acid, polymethacrylic acid and co-polymerisates of (meth)acrylic acid and (meth)acrylic acid esters, and further co-polymerisates of methacrylic acid and methacrylic acid esters which are neutralized with alkalimetal hydroxide and also condensation products of optionally substituted naphthalene sulfonic acid salts with formaldehyde.

If appropriate, other additional components may also be present, for example protective colloids, binders, adhesives, thickeners, thixotropic substances, penetrants, stabilizers, sequestering agents, complex formers. In general, the active compounds can be combined with any solid or liquid additive customarily used for formulation purposes.

The inventive **fungicidal compositions** can be used as such or, depending on their particular physical and/or chemical properties, in the form of their formulations or the use forms prepared therefrom, such as liquids, gels, solutions, suspensions, sprays, powders, pellets, briquettes, bricks aerosols, capsule suspensions, cold-fogging concentrates, warm-fogging concentrates, encapsulated granules, fine granules, flowable concentrates for the treatment of seed, ready-to-use solutions, dustable powders, emulsifiable concentrates, oil-in-water emulsions, water-in-oil emulsions, macrogranules, microgranules, oil-dispersible powders, oil-miscible flowable concentrates, oil miscible liquids, gas (under pressure), gas generating product, foams, pastes, pesticide coated seed, suspension concentrates, suspoemulsion concentrates, soluble concentrates, suspensions, wettable powders, soluble powders, dusts and granules, water-soluble and water-dispersible granules or tablets, water-soluble and water-dispersible powders for the treatment of seed, wettable powders, natural products and synthetic substances impregnated with active ingredient, and also microencapsulations in polymeric substances and in coating materials for seed, and also ULV cold-fogging and warm-fogging formulations.

In general, the **fungicidal compositions** according to the invention comprise between 0.05 and 99 per cent by weight, 0.01 and 98 per cent by weight, preferable between 0.1 and 95 per cent by weight, particularly preferred between 0.5 and 90 per cent by weight of a **compound** of general formula (I) - (III) as described herein or of a combination of compounds according to the invention.

The fungicidal compositions according to the invention can be prepared using the standard method known in the art.

The fungicidal compositions according to the invention do not only comprise ready-to-use compositions which can be applied with suitable apparatus to the plant or the seed, but also commercial concentrates which have to be diluted with water prior to use.

According to the invention all **plants, plant materials or plant parts** can be treated. By **plants** is meant all plants and plant populations such as desirable and undesirable wild plants, cultivars and plant varieties, whether or not protectable by plant variety or plant breeder's rights. Cultivars and plant varieties can be plants obtained by conventional propagation and breeding methods which can be assisted or supplemented by one or more biotechnological methods such as by use of double haploids, protoplast fusion, random and directed mutagenesis, molecular or genetic markers or by bioengineering and genetic engineering methods. By **plant materials or parts** is meant all above ground and below ground parts and organs of plants such as shoot, leaves, needles, stems, branches, petioles, internodes, bark, pubescence, tillers,blossoms, fruiting bodies, fruits, stipules, seed (including embryo, endosperm, cotyledons, and seed coat), as well as roots, corms, tubers, rhizomes, fronds, blades, flowers and floral organs/structures (e.g., bracts, sepals, petals, stamens, carpels, anthers and ovules), plant tissue (e.g., vascular tissue, ground tissue, and the like), cells (e.g., guard cells, egg cells, and the like), and the like. Crops and vegetative and generative propagating material, for example cuttings, corms, rhizomes, runners and seeds also belong to plant parts.

Examples of plants which can be treated in accordance with the invention are listed in **Table 1.**

### Methods using the fungicidal compounds or compositions.

An embodiment of the present invention relates to a method of treating, preventing, inhibiting, or eliminating virulence of one or more plant pathogenic basidiomycete fungi in a plant or a plant material comprising the step of applying onto a plant, to a plant material or in the vicinity of said plant or plant material a compound or a combination of compounds or a fungicidal composition comprising the same, wherein the compound(s) is/are selected from the general formula (I) - (III) as follows: 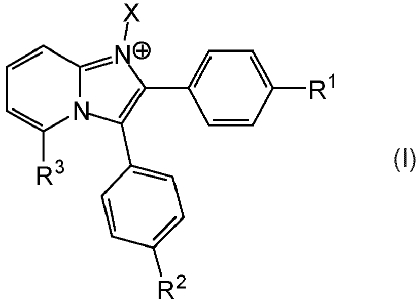 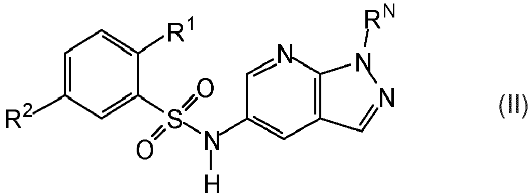 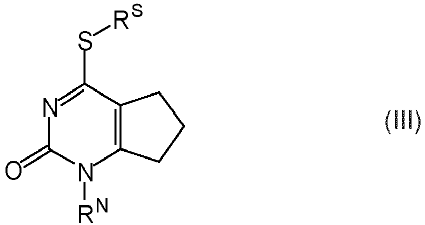 wherein R¹, R², R³, R^{N}, R^{S}, R*, Ar, L and X have the meanings as disclosed herein and salts especially agriculturally acceptable salts thereof.

A particular embodiment of the present invention relates to a method of treating, preventing, inhibiting, or eliminating virulence of one or more plant pathogenic basidiomycete fungi in a plant or a plant material comprising the step of applying onto a plant, to a plant material or in the vicinity of said plant or plant material a compound or a combination of compounds or a fungicidal composition comprising the same, wherein the compound(s) is/are selected from **1-A, 2-A, 3-A, 4-A, 5-A, 6-A, 1-B, 2-B, 3-B, 4-B, 5-B, 6-B, 7-B, 1-C, 2-C, 3-C, 4-C, 5-C, 6-C, 7-C, 8-C, 9-C, 10-C, 11-C, 12-C, 13-C, 14-C, 15-C, 16-C, 17-C, 18-C, 19-C, 20-C, 21-C, 22-C, 23-C, 24-C,** and **25-C,** and salts especially agriculturally acceptable salts thereof.

A further embodiment of the present invention relates to a method of treating, preventing, inhibiting, or eliminating virulence of one or more plant pathogenic basidiomycete fungi in a plant or a plant material comprising the step of applying onto a plant, to a plant material or in the vicinity of said plant or plant material a compound or a combination of compounds or a fungicidal composition comprising the same, wherein the compound(s) is/are selected from the general formula (I) - (III) as follows: 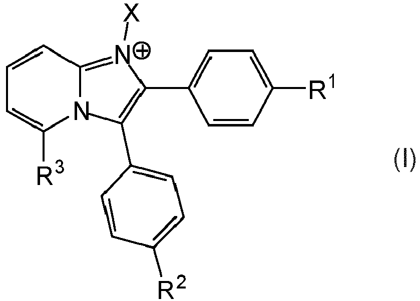 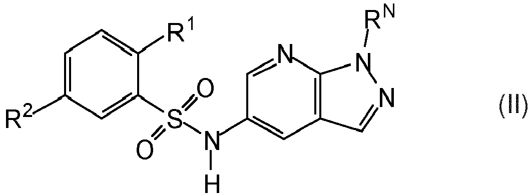 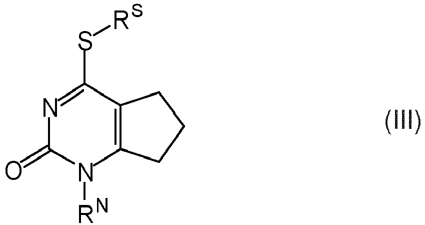
wherein R¹, R², R³, R^{N}, R^{S}, R*, Ar, L and X have the meanings as disclosed herein and salts especially agriculturally acceptable salts thereof, and
wherein the one or more plant pathogenic basidiomycete fungi are selected from the group consisting of smut fungi and rust fungi, wherein the smut fungi are *Ustilago hordei, Ustilago avenae, Ustilago nuda, Ustilago nigra, Ustilago tritici, Ustilago maydis, Ustilago scitaminea, Ustilago esculenta, Ustilago striiformis, Ustilago trichophora, Sporisorium reilianum, Sporisorium reilianum f.sp. zeae, Sporisorium reilianum f.sp. reilianum, Sporisorium scitamineum, Angiosorus solani, Urocystis agropyri, Urocystis occulta, Tilletia indica, Tilletia caries, Tilletia barclayana* or *Tilletia controversa,* or wherein the rust fungi is *Uromyces fabae, Uromyces appendiculatus, Uromyces beticola, Puccinia graminis f. sp. tritici, Puccinia striiformis* f. sp. *tritici, Puccinia triticina, Puccinia graminis f. sp. secalis, Puccinia arachidis, Puccinia coronata f. sp. avenae, Puccinia sorghi, Puccinia melanocephala, Puccinia kuehnii, Puccinia recondita, Puccinia pitteriana, Puccinia graminis, Puccinia polysora,Phakopsora pachyrhizi, Phakopsora meibomiae, Hemileia vastatrix, Melampsora-larici-populina, Melampsora lini, Cronartium ribicola, Cronartium quercuum f. sp. fusiforme, Gymnosporangium sabinae, Gymnosporangium yamadae, Gymnosporangium juniperi-virginianae.*

A particular embodiment of the present invention relates to a method of treating, preventing, inhibiting, or eliminating virulence of one or more plant pathogenic basidiomycete fungi in a plant or a plant material comprising the step of applying onto a plant, to a plant material or in the vicinity of said plant or plant material a compound or a combination of compounds or a fungicidal composition comprising the same, wherein the compound(s) is/are selected from **1-A, 2-A, 3-A, 4-A, 5-A, 6-A, 1-B, 2-B, 3-B, 4-B, 5-B, 6-B, 7-B, 1-C, 2-C, 3-C, 4-C, 5-C, 6-C, 7-C, 8-C, 9-C, 10-C, 11-C, 12-C, 13-C, 14-C, 15-C, 16-C, 17-C, 18-C, 19-C, 20-C, 21-C, 22-C, 23-C, 24-C,** and **25-C,** and salts especially agriculturally acceptable salts thereof, and
wherein the one or more plant pathogenic basidiomycete fungi are selected from the group consisting of smut fungi and rust fungi, wherein the smut fungi are *Ustilago hordei, Ustilago avenae, Ustilago nuda, Ustilago nigra, Ustilago tritici, Ustilago maydis, Ustilago scitaminea, Ustilago esculenta, Ustilago striiformis, Ustilago trichophora, Sporisorium reilianum, Sporisorium reilianum f.sp. zeae, Sporisorium reilianum f.sp. reilianum, Sporisorium scitamineum, Angiosorus solani, Urocystis agropyri, Urocystis occulta, Tilletia indica, Tilletia caries, Tilletia barclayana* or *Tilletia controversa,* or wherein the rust fungi is *Uromyces fabae, Uromyces appendiculatus, Uromyces beticola, Puccinia graminis f. sp. tritici, Puccinia striiformis* f. sp. *tritici, Puccinia triticina, Puccinia graminis f. sp. secalis, Puccinia arachidis, Puccinia coronata f. sp. avenae, Puccinia sorghi, Puccinia melanocephala, Puccinia kuehnii, Puccinia recondita, Puccinia pitteriana, Puccinia graminis, Puccinia polysora,Phakopsora pachyrhizi, Phakopsora meibomiae, Hemileia vastatrix, Melampsora-larici-populina, Melampsora lini, Cronartium ribicola, Cronartium quercuum f. sp. fusiforme, Gymnosporangium sabinae, Gymnosporangium yamadae, Gymnosporangium juniperi-virginianae.*

An embodiment of the present invention relates to a method of treating, preventing, inhibiting, or eliminating virulence of one or more plant pathogenic basidiomycete fungi in a plant or a plant material comprising the step of applying onto a plant, to a plant material or in the vicinity of said plant or plant material a compound or a combination of compounds or a fungicidal composition comprising the same, wherein the compound(s) is/are selected from the general formula (I) - (III) as follows: 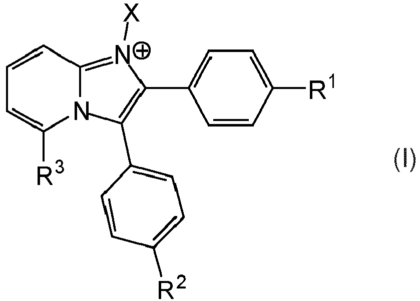 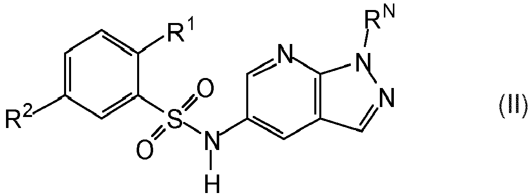 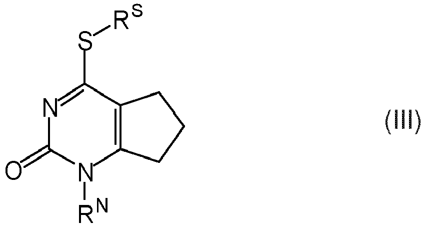
wherein R¹, R², R³, R^{N}, R^{S}, R*, Ar, L and X have the meanings as disclosed herein and salts especially agriculturally acceptable salts thereof, and
wherein the one or more plant pathogenic basidiomycete fungi are selected from the group consisting of smut fungi and rust fungi, wherein the smut fungi are *Ustilago maydis, Ustilago esculenta, Ustilago trichophora, Ustilago tritici, Sporisorium reilianum f.sp. zeae, Sporisorium reilianum f.sp. reilianum, Sporisorium scitamineum, Ustilago hordei,* or wherein the rust fungi is *Uromyces fabae.*

A particular embodiment of the present invention relates to a method of treating, preventing, inhibiting, or eliminating virulence of one or more plant pathogenic basidiomycete fungi in a plant or a plant material comprising the step of applying onto a plant, to a plant material or in the vicinity of said plant or plant material a compound or a combination of compounds or a fungicidal composition comprising the same, wherein the compound(s) is/are selected from **1-A, 2-A, 3-A, 4-A, 5-A, 6-A, 1-B, 2-B, 3-B, 4-B, 5-B, 6-B, 7-B, 1-C, 2-C, 3-C, 4-C, 5-C, 6-C, 7-C, 8-C, 9-C, 10-C, 11-C, 12-C, 13-C, 14-C, 15-C, 16-C, 17-C, 18-C, 19-C, 20-C, 21-C, 22-C, 23-C, 24-C,** and **25-C,** and salts especially agriculturally acceptable salts thereof, and
wherein the one or more plant pathogenic basidiomycete fungi are selected from the group consisting of smut fungi and rust fungi, wherein the smut fungi are *Ustilago maydis, Ustilago esculenta, Ustilago trichophora, Ustilago tritici, Sporisorium reilianum f.sp. zeae, Sporisorium reilianum f.sp. reilianum, Sporisorium scitamineum, Ustilago hordei,* or wherein the rust fungi is *Uromyces fabae.*

An embodiment of the present invention relates to a method of treating, preventing, inhibiting, or eliminating virulence of one or more plant pathogenic basidiomycete fungi in a plant or a plant material comprising the step of applying onto a plant, to a plant material or in the vicinity of said plant or plant material a compound or a combination of compounds or a fungicidal composition comprising the same, wherein the compound(s) is/are selected from the general formula (I) - (III) as follows: 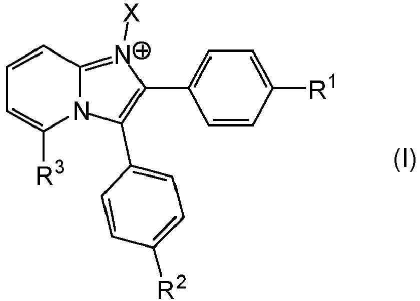 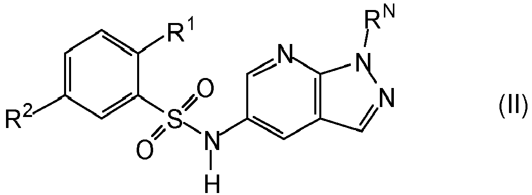 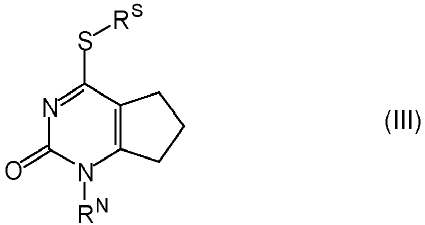
wherein R¹, R², R³, R^{N}, R^{S}, R*, Ar, L and X have the meanings as disclosed herein and salts especially agriculturally acceptable salts thereof, and
wherein the one or more plant pathogenic basidiomycete fungi are selected from the group consisting of smut fungi and rust fungi.

A particular embodiment of the present invention relates to a method of treating, preventing, inhibiting, or eliminating virulence of one or more plant pathogenic basidiomycete fungi in a plant or a plant material comprising the step of applying onto a plant, to a plant material or in the vicinity of said plant or plant material a compound or a combination of compounds or a fungicidal composition comprising the same, wherein the compound(s) is/are selected from **1-A, 2-A, 3-A, 4-A, 5-A, 6-A, 1-B, 2-B, 3-B, 4-B, 5-B, 6-B, 7-B, 1-C, 2-C, 3-C, 4-C, 5-C, 6-C, 7-C, 8-C, 9-C, 10-C, 11-C, 12-C, 13-C, 14-C, 15-C, 16-C, 17-C, 18-C, 19-C, 20-C, 21-C, 22-C, 23-C, 24-C,** and **25-C,** and salts especially agriculturally acceptable salts thereof, and
wherein the one or more plant pathogenic basidiomycete fungi are selected from the group consisting of smut fungi and rust fungi.

**The step of applying in the vicinity of said plant or plant material** a compound or a combination of compounds or a fungicidal composition comprises applying to the surroundings, habitat, soil, or culture medium of said plant or plant material (for example, roots, foliage, flower or a locus of the fungus), a **"fungicidally effective amount"** of one or more of the compounds of general formula (I) - (III) described above, or of **a combination** of said compounds or a fungicidal composition comprising said compounds.. For example, the compound may be applied to the roots or foliage of plants for the control of various fungi, without damaging the commercial value of the plants.

The **"fungicidally effective amount"** to be applied is dependent on several factors, for example on the fungus to be controlled, the stage of growth thereof, the plant, or the part of the plant or other plant material to be contacted with the compound, the climatic conditions and the ingredients of the inventive compositions. Thus, all the compounds, and fungicidal compositions comprising the same, may not be equally effective at similar concentrations against the same fungal species. The compounds are effective in use with plants in a **"fungicidally effective amount",** which is also a **"fungicidally effective phytologically acceptable amount".**

The term **"fungicidally effective amount"** and **"fungicidally effective phytologically acceptable amount"** refers to an amount of a compound or a combination of compounds that **treats, prevents, inhibits, or eliminate virulence of** one plant pathogenic basidiomycete fungus for which control is desired, but is not significantly toxic to the plant. The exact **fungicidally effective amount** of a compound varies with the fungal disease to be controlled, the type of formulation employed, the method of application, the particular plant species, climate conditions, and the like. A suitable application rate can be for example 0.0001 , 0.001, 0.005, 0.01, 0.1 , 1, 2, 10, 100, 1,000, 2,000, 5,000 kg/ha (kg/hectare), or any range between about 0.0001 and 5,000. For example, about 0.0001 to about 0.01 , about 0.01 to about 10, about 10 to about 1,000, about 1 ,000 to about 5,000 kg/ha.

Any range or desired value given herein may be extended or altered without losing the effects sought, as is apparent to the skilled person for an understanding of the teachings herein.

**The step of applying onto a plant, to a plant material or in the vicinity of said plant or plant material a compound or a combination** of **compounds or a fungicidal composition comprising the same** is carried out directly or by action on their surroundings, habitat, soil, or culture medium using customary treatment methods, for example by dipping, spraying, atomizing, irrigating, evaporating, dusting, fogging, broadcasting, foaming, painting, spreading-on, watering (drenching), drip irrigating and, in the case of propagation material, in particular in the case of seeds, furthermore as a powder for dry seed treatment, a solution for seed treatment, a water-soluble powder for slurry treatment, by incrusting, by coating with one or more layers, etc. It is furthermore possible to apply the active compounds by the ultra-low volume method, or to inject the active compound preparation or the active compound itself into the soil.

**A compound or a combination** of **compounds or a fungicidal composition comprising the same** as defined in the present invention may be applied in the form of any of the generally used formulation types, for example, as solutions, dusts, wettable powders, flowable concentrate, or emulsifiable concentrates.

Preferably, the compounds of the present disclosure are applied in the form of a formulation, comprising one or more of the compounds of general formula (I) - (III) described above with a suitable **"agriculturally acceptable carrier".** Concentrated formulations may be dispersed in water, or other liquids, for application, or formulations may be dust-like or granular, which may then be applied without further treatment. The formulations can be prepared according to procedures that are conventional in the agricultural chemical art.
The present disclosure contemplates all carriers by which one or more of the compounds may be formulated for delivery and used as a fungicide. Typically, formulations are applied as aqueous suspensions or emulsions. Such suspensions or emulsions may be produced from water-soluble, water-suspendible, or emulsifiable formulations which are solids, usually known as wettable powders; or liquids, usually known as emulsifiable concentrates, aqueous suspensions, or suspension concentrates. As will be readily appreciated, any material to which these compounds may be added may be used, provided it yields the desired utility without significant interference with the activity of these compounds as antifungal agents.

Wettable powders, which may be compacted to form **water-dispersible granules,** comprise an intimate mixture of one or more of the compounds of general formula (I) - (III) described above, an inert carrier and surfactants. The concentration of the compound in the wettable powder may be from about 10 percent to about 90 percent by weight based on the total weight of the wettable powder, more preferably about 25 weight percent to about 75 weight percent. In the preparation of wettable powder formulations, the compounds may be compounded with any finely divided solid, such as prophyllite, talc, chalk, gypsum, Fuller's earth, bentonite, attapulgite, starch, casein, gluten, montmorillonite clays, diatomaceous earths, purified silicates or the like. In such operations, the finely divided carrier and surfactants are typically blended with the compound(s) and milled.

Emulsifiable concentrates of the compounds of general formula (I) - (III) described above may comprise a convenient concentration, such as from about 1 weight percent to about 50 weight percent of the compound, in a suitable liquid, based on the total weight of the concentrate. The compounds may be dissolved in an inert carrier, which is either a water-miscible solvent or a mixture of water-immiscible organic solvents, and emulsifiers. The concentrates may be diluted with water and oil to form spray mixtures in the form of oil-in-water emulsions. Useful organic solvents include aromatics, especially the high-boiling naphthalenic and olefmic portions of petroleum such as heavy aromatic naphtha. Other organic solvents may also be used, for example, terpenic solvents, including rosin derivatives, aliphatic ketones, such as cyclohexanone, and complex alcohols, such as 2-ethoxyethanol.

The compounds of general formula (I) - (III) described above also be applied as **granular formulations,** which are particularly useful for applications to the soil. Granular formulations generally contain from about 0.5 to about 10 weight percent, based on the total weight of the granular formulation of the compound(s), dispersed in an inert carrier which consists entirely or in large part of coarsely divided inert material such as attapulgite, bentonite, diatomite, clay or a similar inexpensive substance. Such formulations are usually prepared by dissolving the compounds in a suitable solvent and applying it to a granular carrier, which has been preformed to the appropriate particle size, in the range of from about 0.5 to about 3 mm. A suitable solvent is a solvent in which the compound is substantially or completely soluble. Such formulations may also be prepared by making a dough or paste of the carrier and the compound and solvent, and crushing and drying to obtain the desired granular particle.

The compounds of general formula (I) - (III) described above may be used alone, in composition or in combination, such as for example in a fungicidal composition, or in a combination wherein the compounds are administered in a simultaneous, separate or sequential application. If the single fungicidal compounds are applied in a sequential manner, i.e. at different times, they are applied one after the other within a reasonably short period, such as a few hours or days.

In a combination or in a fungicidal composition, the compounds of general formula (I) - (III) described above may be used in a wide **variety of weight ratios.** For example, in a **two-component combination or composition,** the following weight ratios can be used: 100:1 to 1:100, 50:1 to 1:50, 20:1 to 1:20, 10:1 to 1:10, 5:1 to 1:5, 3:1 to 1:3, 2:1 to 1:2, or 1:1.

### Applications Rates and Timing

When using the inventive compounds or fungicidal combinations, the application rates can be varied within a relatively wide range, depending on the kind of application. The application rate of the inventive active ingredients is
a) in the **case of treatment of plant parts,** for example leaves: from 0.1 to 10 000 g/ha, preferably from 10 to 1000 g/ha, more preferably from 10 to 800 g/ha, even more preferably from 50 to 300 g/ha (in the case of application by watering or dripping, it is even possible to reduce the application rate, especially when inert substrates such as rockwool or perlite are used);
b) in the case of **seed treatment:** from 2 to 200 g per 100 kg of seed, preferably from 3 to 150 g per 100 kg of seed, more preferably from 2.5 to 25 g per 100 kg of seed, even more preferably from 2.5 to 12.5 g per 100 kg of seed;
c) in the case of soil treatment: from 0.1 to 10 000 g/ha, preferably from 1 to 5000 g/ha.

These application rates are merely by way of example and are not limiting for the purposes of the invention. The inventive compositions can thus be used to protect plants from attack by the pathogens mentioned for a certain **period of time after treatment.**

### Description of the Figures:

- **Figure 1**: shows yeast 2-hybrid analysis of complex members. A) Pairwise interaction screens of proteins listed on the left reveal strong interaction between Stp3 and Stp1 as well as between Pep1 and Stp1. Stp1 and Stp4 interact only weakly. B) The weak interaction between Stp4 and Stp1 is enhanced when either Stp3 or Pep1 are additionally expressed.

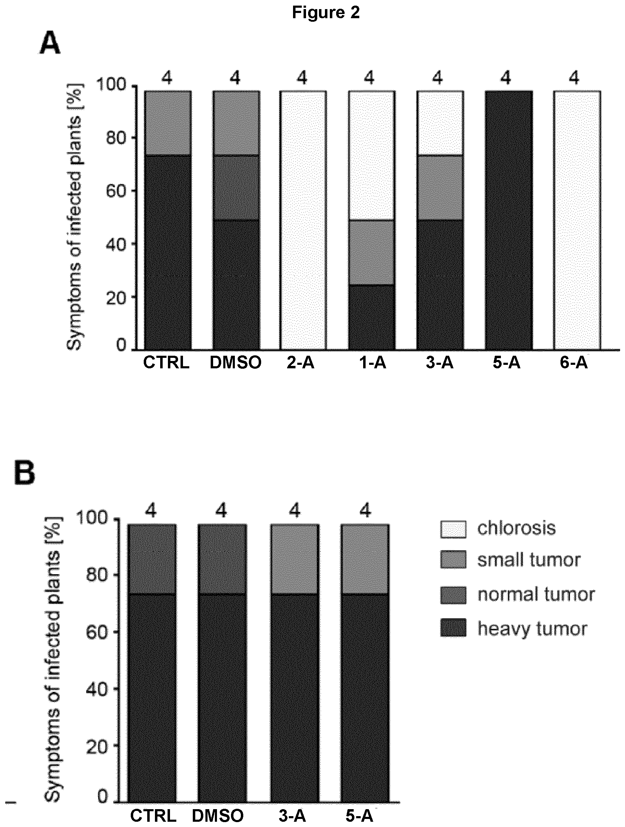

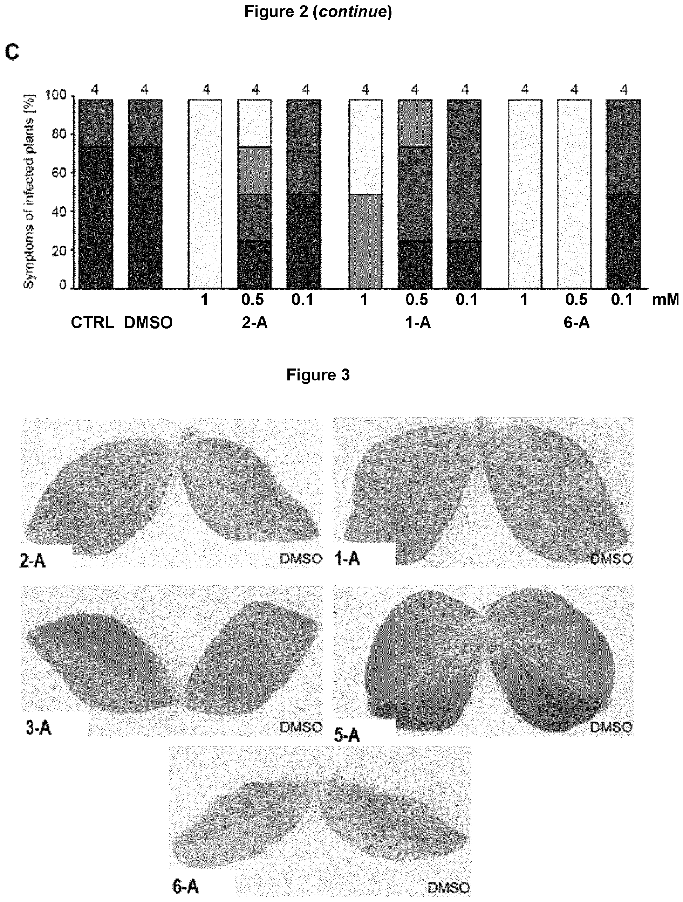

- **Figure 2**: shows that imidazopyridines can inhibit virulence of *U. maydis.* Four plants per assay (number on column) were infected with FB1xFB2 and compounds were added directly to the inoculum. A) Indicated compounds were added at 1 mM final concentration. B) Retesting of compounds inactive in A) at 1 mM final concentration. C) Restesting of compounds active in A) in 1 mM, 0.5 mM and 0.1 mM final concentration. The color scheme indicates symptom severity based on the strongest symptoms scored. Symptom severity of each infected plant was assessed according to Table 2 and plants were categorized according to the most severe symptoms they displayed. For example, 50% in the dark red category indicates that two of the four tested plants had heavy tumors.
- **Figure 3**: shows that imidazopyridines can inhibit virulence of *U. fabae.* Infections were done by applying indicated compounds at 1mM or DMSO to left and right parts of the same leaf together with spores of *U. fabae*

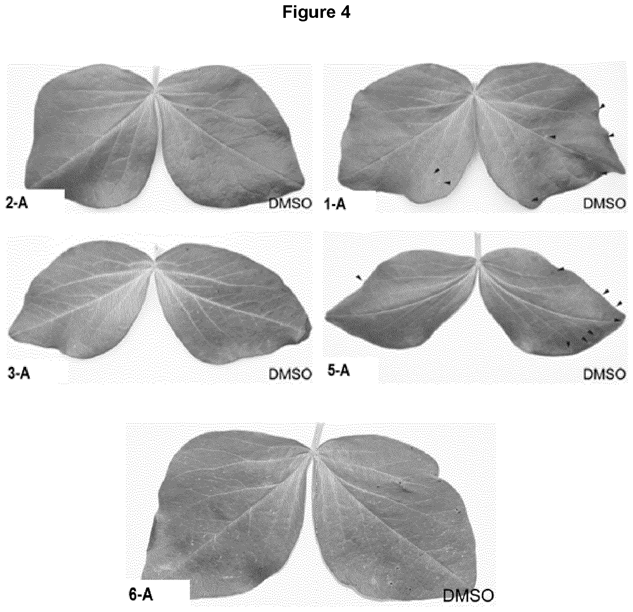

- **Figure 4**: shows that imidazopyridines can inhibit virulence of *U. fabae.* Infections were done by applying indicated compounds at 0.5 mM or DMSO to left and right parts of the same leaf together with spores of *U. fabae.* Because spore pustules are small all pustules present on leaflets are marked by black arrow tips.
- **Figure 5**: shows that tetrahydro-cyclopentapyrimidines can inhibit virulence of *U. maydis.* Per assay, 4-5 plants (number on column) were infected with FB1xFB2 and **1-C** was added directly to the inoculum at the indicated final concentrations. The color scheme indicates symptom severity based on the strongest symptoms scored.
- **Figure 6**: shows that tetrahydro-cyclopentapyrimidines can inhibit virulence of *U. fabae*. Infections were done by applying **1-C** in a dilution series or DMSO to left and right parts of the same leaf.
- **Figure 7**: shows that pyrazolopyridin-benzenesulfonamides can inhibit virulence of *U. maydis.* Per assay, 4 5 plants (number on column) were infected with FB1xFB2 and **1-B** was added directly to the inoculum at the indicated final concentrations. The color scheme indicates symptom severity based on the strongest symptoms scored.
- **Figure 8**: shows that pyrazolopyridin-benzenesulfonamides can inhibit virulence of *U. fabae.* Infections were done by applying 1 mM **1-B** or DMSO to left and right parts of the same leaf together with spores of *U. fabae.*

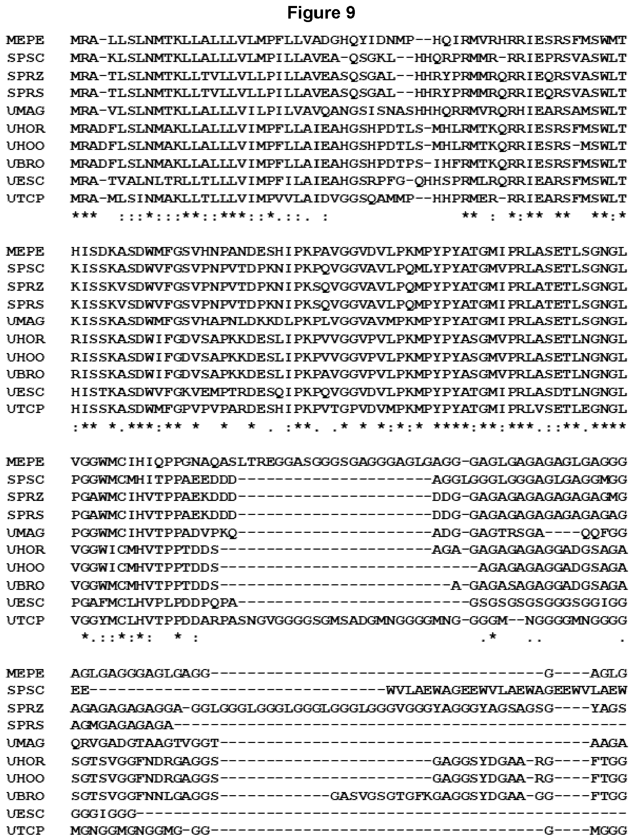

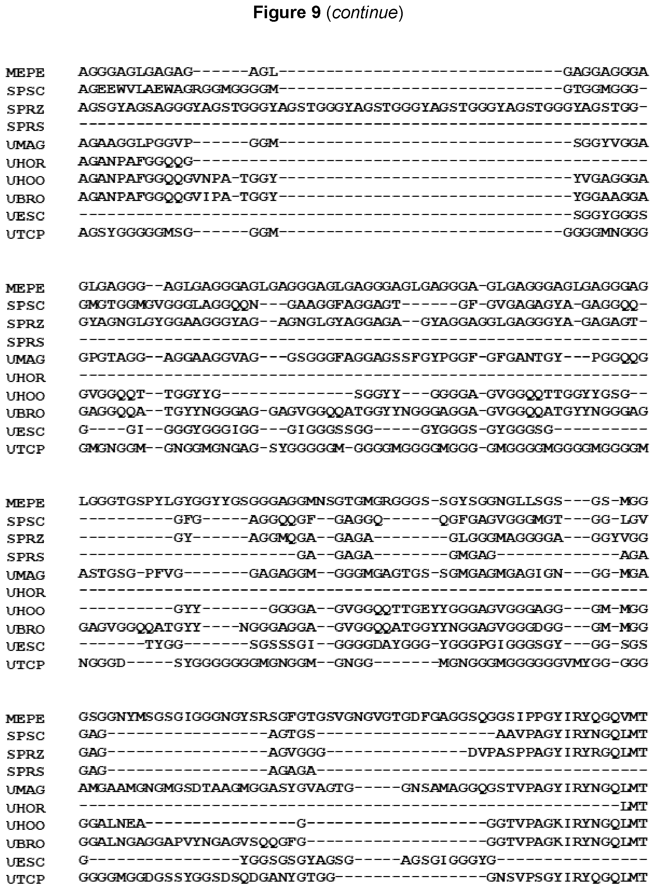

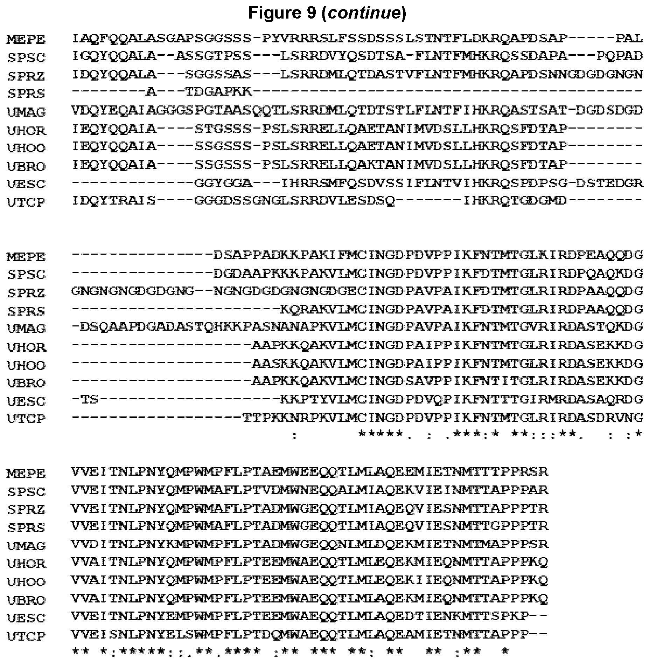

- **Figure 9**: shows alignment of the protein sequences of Stp1 in the indicated fungi. MEPE: *Melanopsichium pennsylvanicum;* UESC: *Ustilago esculenta*; SPSC: *Sporisorium scitamineum;* SPRZ: *Sporisorium reilianum f. sp. zeae*; SPRS: *Sporisorium reilianum f. sp. sorghi*; UTCP: *Ustilago trichophora*; UTRI: *Ustilago tritici*; UMAG: *U. maydis*; UBRO: *Ustilago bromivira*; UHOR: *Ustilago hordei* (host: barley); UHOO: *Ustilago hordei* (host: oat).

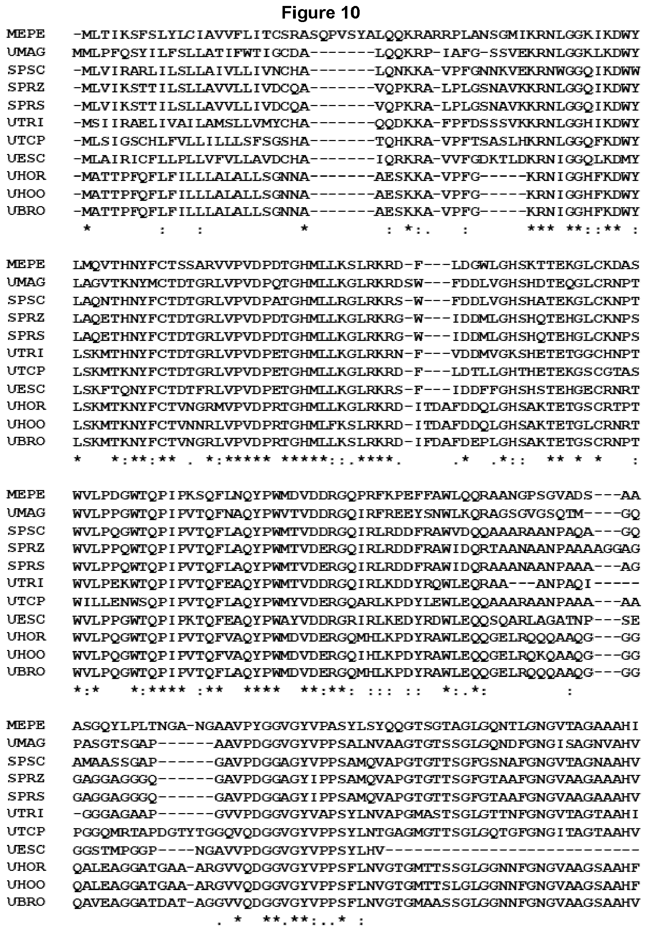

- **Figure 10**: shows alignment of the protein sequences of Stp2 in the indicated fungi. Abbreviations as in Figure 9.

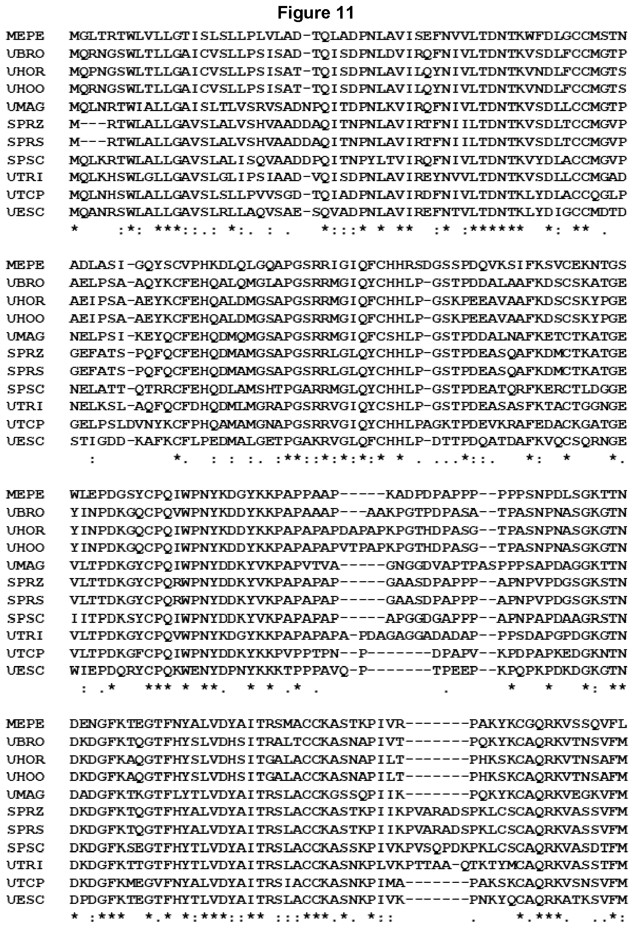

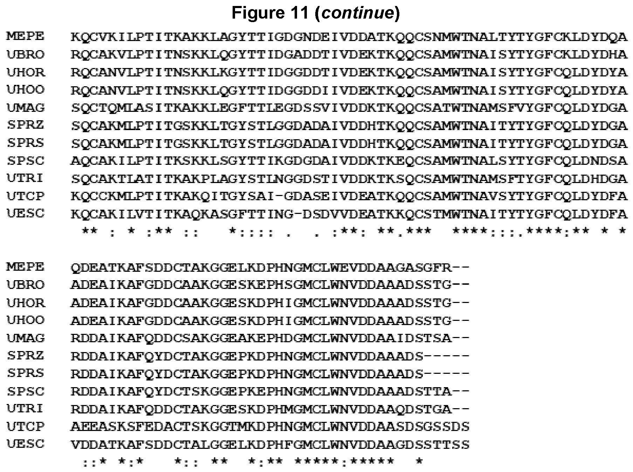

- **Figure 11**: shows alignment of the protein sequences of Stp3 in the indicated fungi. Abbreviations as in Figure 9.

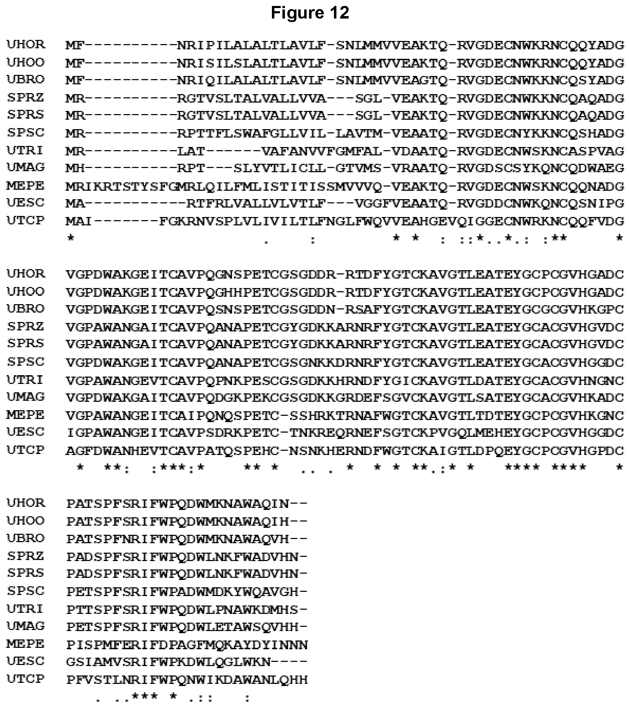

- **Figure 12**: shows alignment of the protein sequences of Stp4 in the indicated fungi. Abbreviations as in Figure 9.

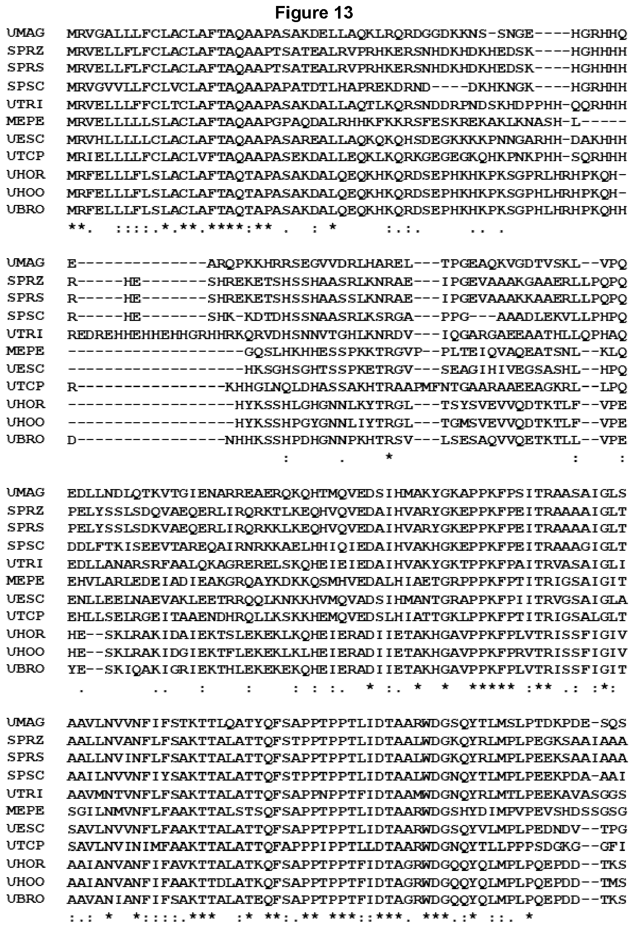

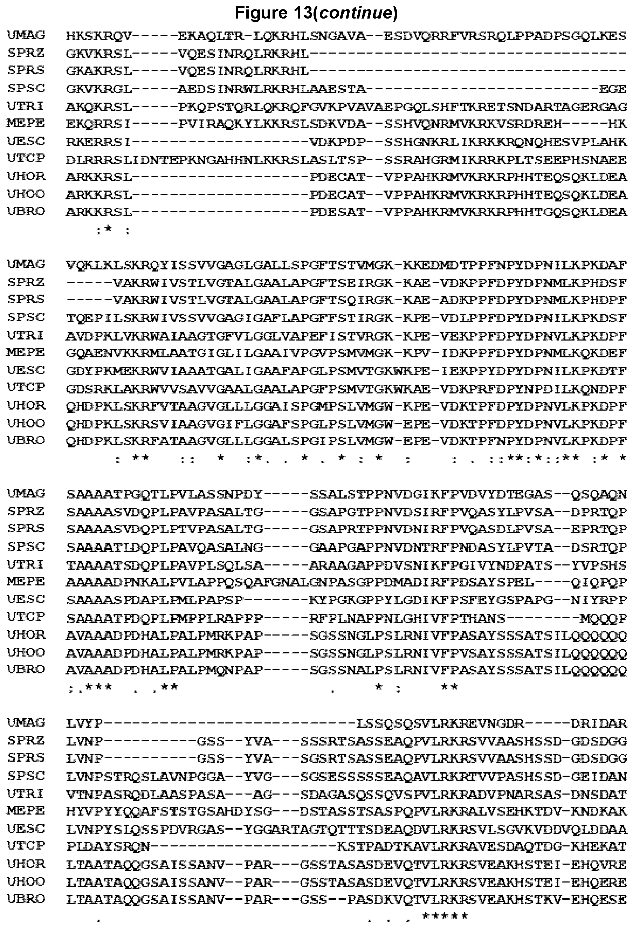

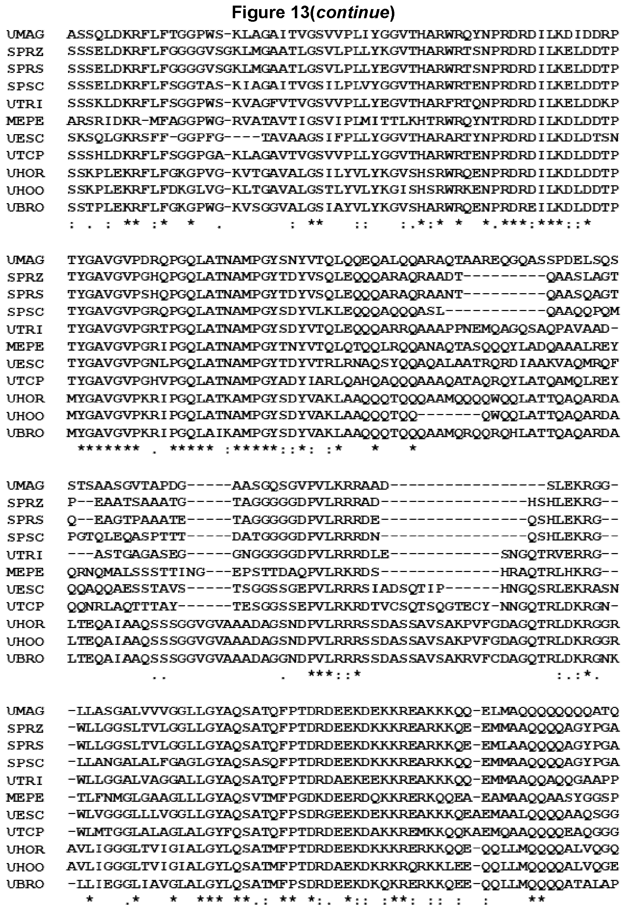

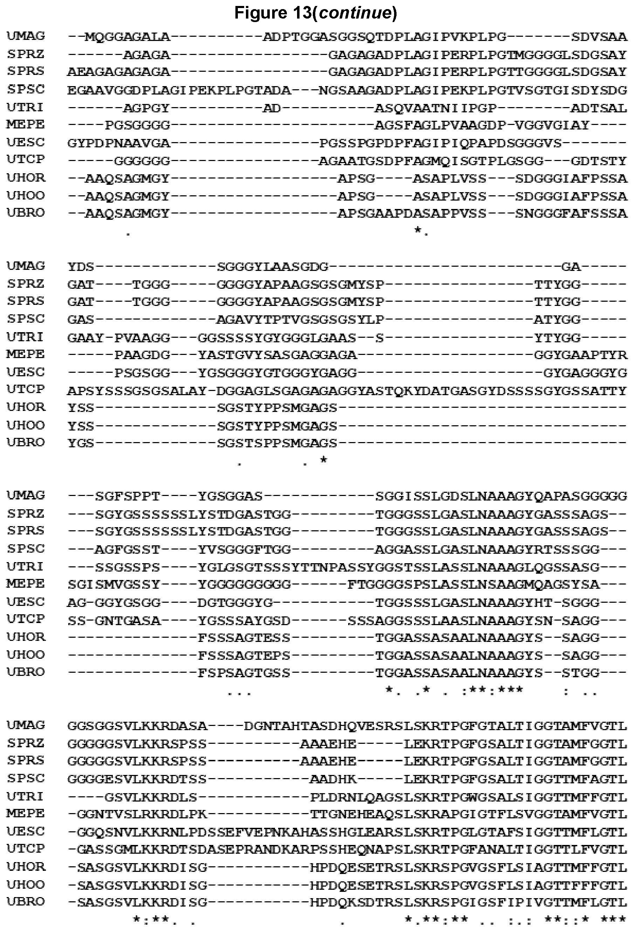

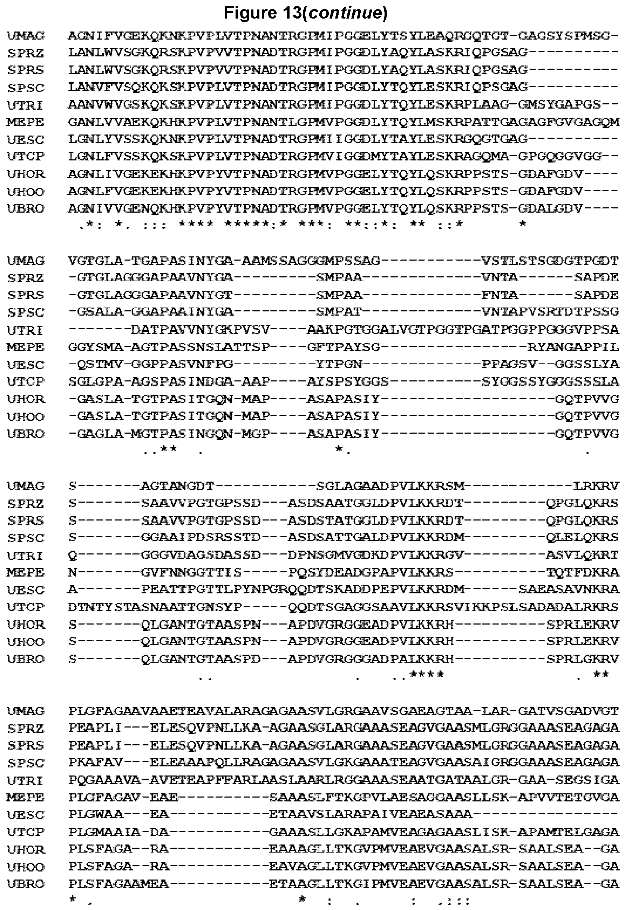

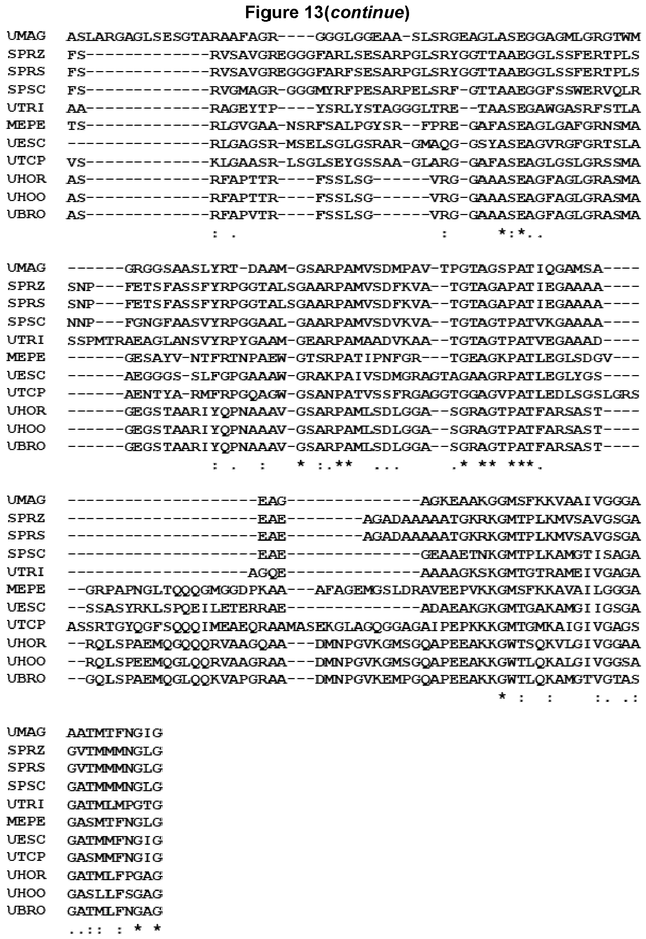

- **Figure 13**: shows alignment of the protein sequences of Stp5 in the indicated fungi. Abbreviations as in Figure 9.

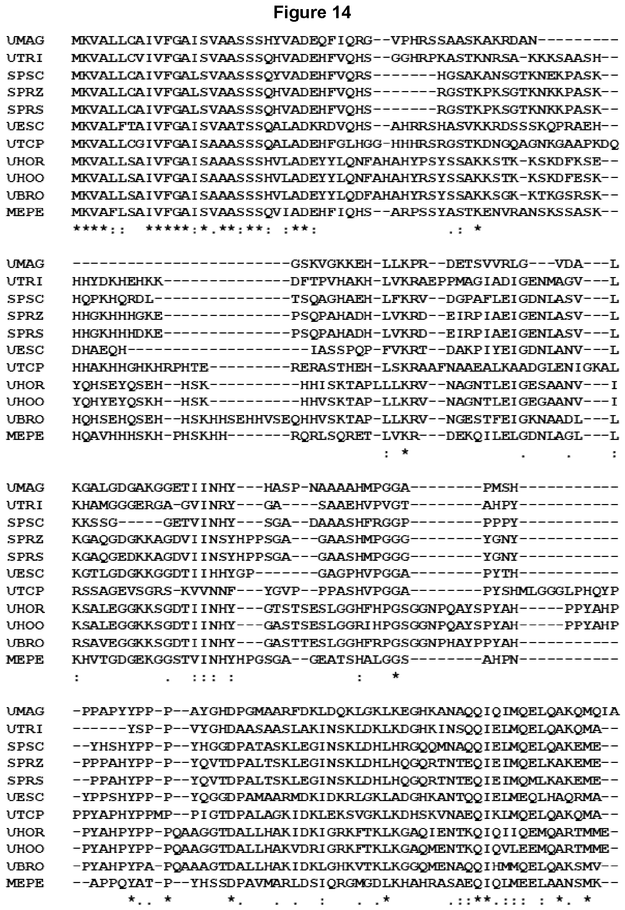

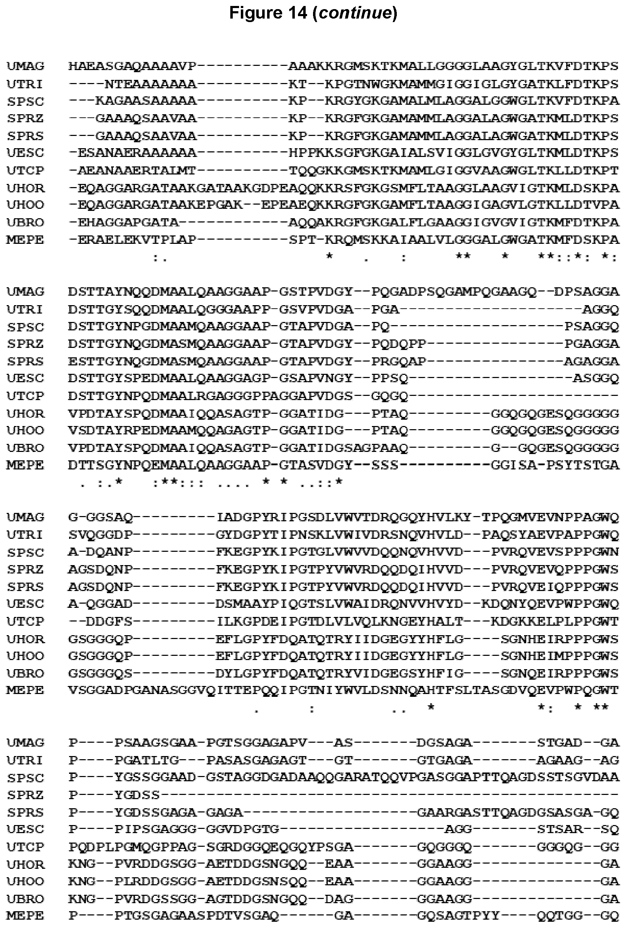

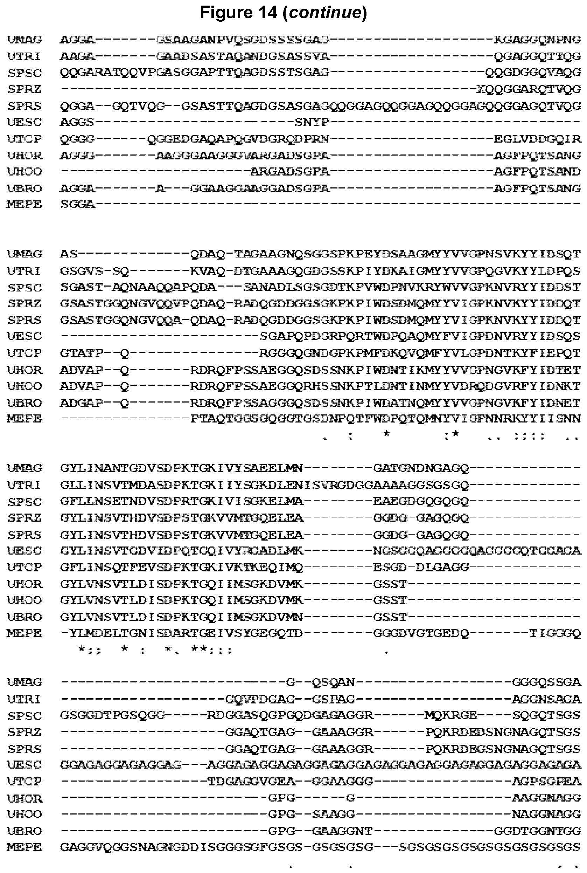

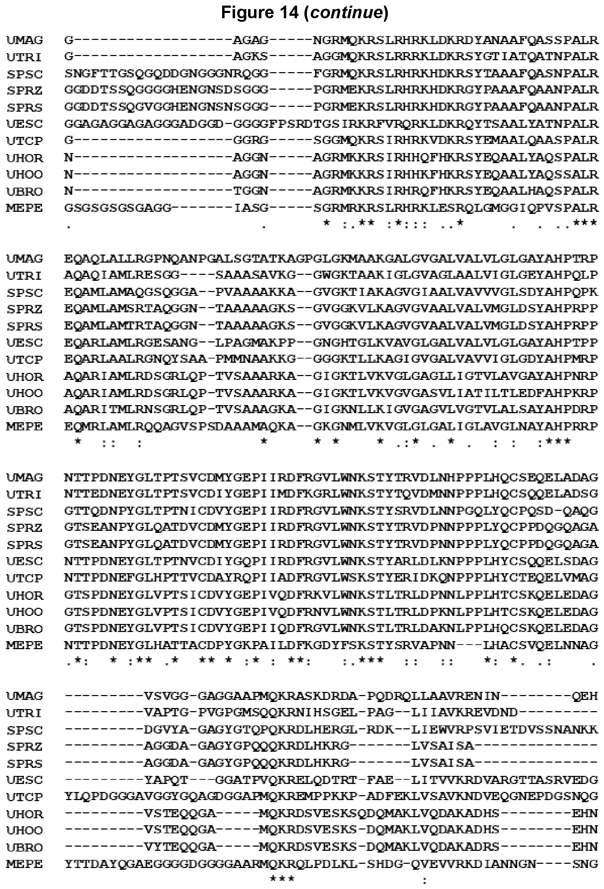

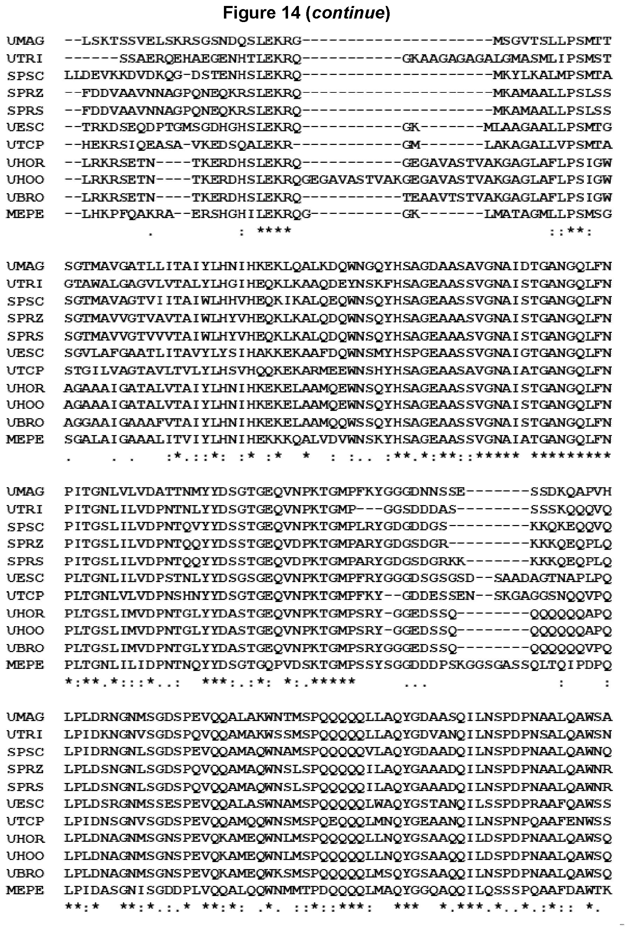

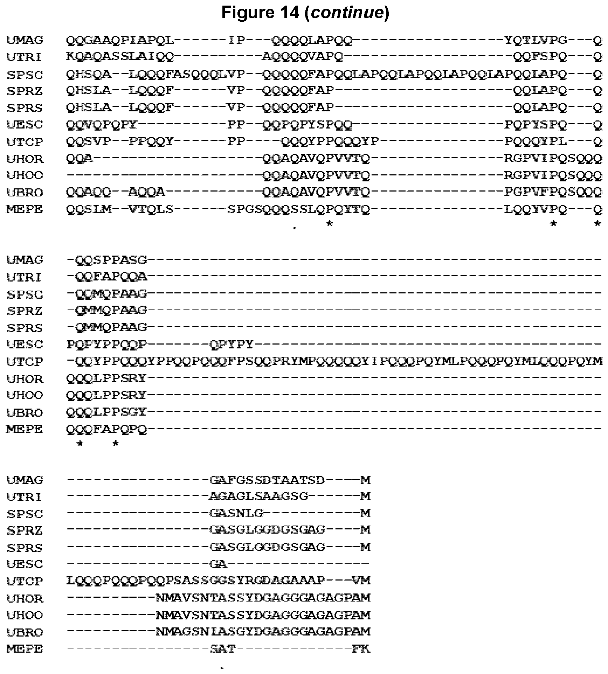

- **Figure 14**: shows alignment of the protein sequences of Stp6 in the indicated fungi. Abbreviations as in Figure 9.

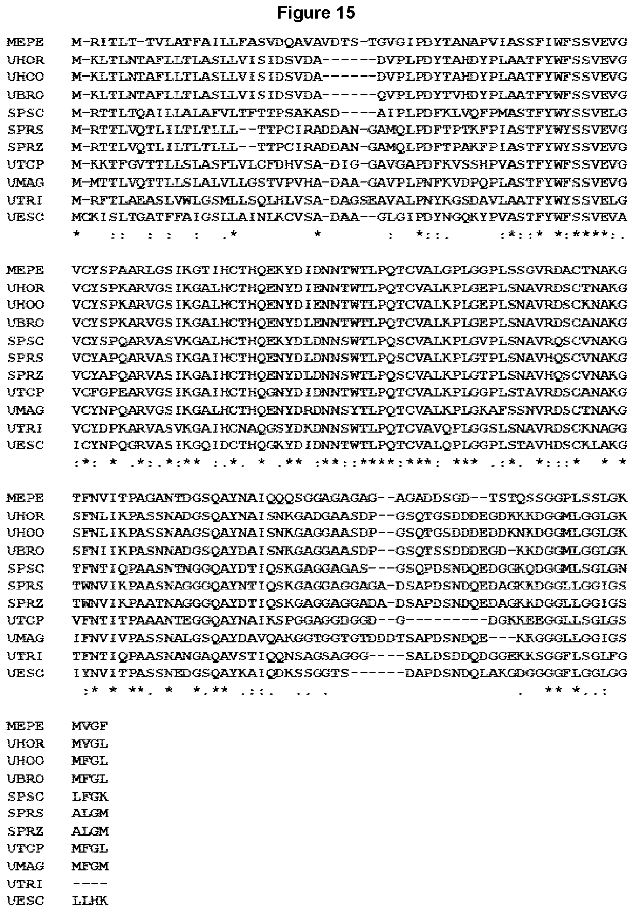

- **Figure 15**: shows alignment of the protein sequences of Pep1 in the indicated fungi. Abbreviations as in Figure 9.

The following examples are included to demonstrate preferred embodiments of the invention. It should be appreciated by those of skill in the art that the techniques disclosed in the following examples represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### EXAMPLES

### METHODS:

### Synthesis of the compounds

The imidazopyrimines compounds (Formula I) were synthetized according to the method published by Manna et al. 2015. Regioselective annulation of nitrosopyridine with alkynes: straightforward synthesis of N-oxide-imidazopyridines. Chem. Commun. 51, 6119.
The tetrahydro-cyclopentapyrimidine compounds (Formula III) were purchased from ChemDiv using the "*search for catalog numbers*" option https://chemistryondemand.com:8443/eShop/. Catalog numbers are listed in Table 6 and 7.

The hit compound **1-B** of pyrazolopyridin-benzenesulfonamides (Formula II) was purchased from Enamine, catalog nr. #Z365137036. The further pyrazolopyridin-benzenesulfonamide analogs were synthetized *in house* with the procedure described below.

### Chemical synthesis of pyrazolopyridin-benzenesulfonamide analogs.

Reactions were performed in heat gun dried flasks under an argon atmosphere. All reagents purchased from commercial sources were used directly without further purification. Thin-layer chromatography was performed on pre-coated silica gel F-254 plates from Merck. The spots were visualized by UV light and/or by staining of the TLC plate with potassium permanganate stain followed, if necessary, by heating with a heat gun. For column chromatography, silica gel 60 from Merck with a particle size of 0.040-0.063 mm was used. ¹H- and ¹³C-NMR spectra were recorded on Bruker AV 400 Avance III HD NanoBay (400 MHz), Bruker AV 500 Avance III HD (500 MHz), Bruker AV 600 Avance III HD (600 MHz) and Bruker AV 700 Avance III HD (700 MHz) spectrometers at room temperature. HPLC-MS analyses were performed with an Agilent 1100 Series connected to a Thermo LCQ Advantage mass spectrometer using a C18 HPLC column 3 µm from Macherey Nagel.

### General procedure A: pyrazole alkylation

To a solution of 3 (1.0 equiv.) in DMF (0.2-0.3 M) was added the respective alkyl halide (1.1 equiv.) and Cs₂CO₃ (1.5 equiv.) and it was stirred at room temperature for 18-20 h. The reaction mixture was poured into EtOAc, washed with sat. aq. NaCl solution (2 ×), dried over MgSO₄ and the solvent was removed under reduced pressure. Column chromatography (PE/EtOAc) afforded the *N*1-alkylated pyrazole and in some cases also the *N*2-alkylated pyrazole as minor product.

### General procedure B: reduction of the nitro group

To a degassed solution of the aromatic nitro compound 3 or 4 (1.0 equiv.) in EtOH (0.2-0.3 M) was added Pd/C (10 wt%) followed by dropwise addition of triethylsilane (5.0 equiv.) and it was stirred at room temperature for 1 h. The reaction mixture was filtered over Celite, washed with EtOAc and the solvent was removed under reduced pressure. The corresponding aniline was used in the next step without further purification.

### General procedure C: sulfonamide formation

To a solution of the aniline 5 (1.0 equiv.) in THF (0.1 M) at 0 °C was added NEt₃ (1.2 equiv.) and the corresponding sulfonyl chloride (1.1 equiv.) and it was allowed to reach room temperature over 16-18 h. The solvent was removed under reduced pressure. Column chromatography (PE/EtOAc) afforded the sulfonamide.

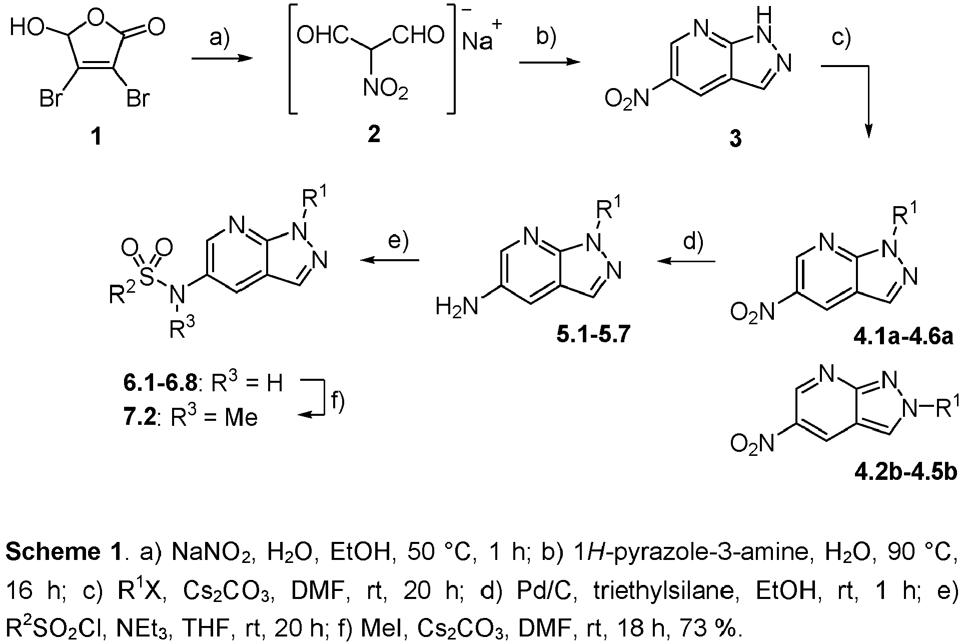

### Sodium nitromalonaldehyde (2) (Org. Synth. 1952, 32, 95).

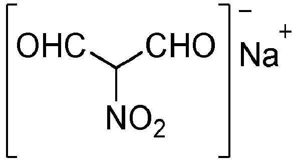

To a solution of NaNO₂ (25.0 g, 362 mmol) in H₂O (25 mL) was added dropwise a solution of Mucobromic acid (25.0 g, 97.0 mmol) in EtOH (25 mL) at 60 °C over 1 h, and the mixture was stirred at the same temperature for 15 min. The reaction was cooled to 0 °C and the solid was filtered, washed with cold EtOH and dried in the air to give an orange/beige colored wet solid (15.3 g). It was used without further purification in the next step.

### 5-Nitro-1H-pyrazolor3,4-b]pyridine (3) (Eur. J. Med Chem. 2017, 131, 1-13).

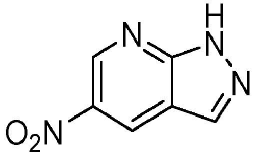

To a solution of sodium nitromalonaldehyde (15.3 g) in H₂O (100 mL) was added 1*H-*Pyrazol-3-amine (3.00 g, 36.1 mmol, 1.0 equiv.) and it was stirred at 90 °C for 19 h. After cooling to room temperature the reaction mixture was adjusted to pH 5 using AcOH. EtOAc was added, the reaction mixture was filtered, extracted with EtOAc (2 × 75 mL), dried over MgSO₄ and the solvent was removed under reduced pressure. Column chromatography (DCM/MeOH 50:1) afforded a light yellow solid (1.47 g, 8.96 mmol, 9 % over 2 steps).
¹H-NMR (DMSO-d₆, 700 MHz): δ = 8.44 (s, 1 H, Ar-H), 9.19 (d, *J* = 2.5 Hz, 1 H, Ar-H), 9.33 (d, *J* = 2.5 Hz, 1 H, Ar-H), 14.37 (s, 1 H, NH).
¹³C-NMR (DMSO-d₆, 175 MHz): δ = 113.5, 127.8, 136.6, 139.1, 144.6, 152.6.
MS (ESI) *m*/*z* (%) = 165.0 (100) [M+H]⁺.

### 1-Methyl-5-nitro-1H-pyrazolo[3,4-b]pyridine (4.1a)

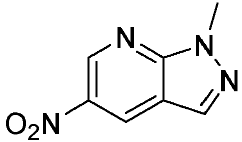

Following general procedure A: From 3 (150 mg, 0.914 mmol) and lodomethane (63 µL, 1.01 mmol). Purification by column chromatography (PE/EtOAc 3:1) afforded a colorless solid (111 mg, 0.623 mmol, 68 %).
¹H-NMR (CDCl₃, 500 MHz): δ = 4.22 (s, 3 H, CH₃), 8.24 (s, 1 H, Ar-H), 8.95 (d, *J =* 2.4 Hz, 1 H, Ar-H), 9.41 (d, *J* = 2.4 Hz, 1 H, Ar-H).
¹³C-NMR (CDCl₃, 125 MHz): δ = 34.67, 114.2, 127.1, 134.7, 139.5, 144.9, 151.3. MS (ESI) *m*/*z* (%) = 179.0 (100) [M+H]⁺.

### 1-Isopropyl-5-nitro-1H-pyrazolo[3,4-b]pyridine (4.2a)

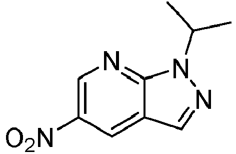

Following general procedure A: From 3 (150 mg, 0.914 mmol) and 2-lodopropane (101 µL, 1.01 mmol). Purification by column chromatography (PE/EtOAc 3:1 → 1:1) afforded a slightly yellow oil (83.0 mg, 0.403 mmol, 44 %).
¹H-NMR (CDCl₃, 700 MHz): δ = 1.62 (d, *J* = 6.7 Hz, 6 H, 2 × CH₃), 5.35 (hept, *J* = 6.7 Hz, 1 H, CH), 8.24 (s, 1 H, Ar-H), 8.93 (d, *J* = 2.4 Hz, 1 H, Ar-H), 9.39 (d, *J* = 2.4 Hz, 1 H, Ar-H).
¹³C-NMR (CDCl3, 175 MHz): δ = 22.10, 49.79, 114.4, 127.0, 134.5, 139.4, 144.5, 150.3.
MS (ESI) *m*/*z* (%) = 207.0 (100) [M+H]⁺.

### 1-Allyl-5-nitro-1H-pyrazolo[3,4-b]pyridine (4.3a)

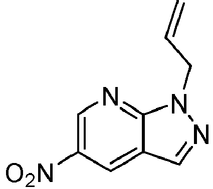

Following general procedure A: From 3 (150 mg, 0.914 mmol) and allyl bromide (85 µL, 1.01 mmol). Purification by column chromatography (PE/EtOAc 3:1 → 1:1) afforded a slightly yellow oil (95.0 mg, 0.465 mmol, 51 %).
¹H-NMR (CDCl₃, 400 MHz): δ = 5.18-5.31 (m, 4 H, 2 × CH, CH₂), 6.09 (ddt, *J* = 17.0, 10.3, 5.9 Hz, 1 H, CH), 8.27 (s, 1 H, Ar-H), 8.96 (d, *J* = 2.4 Hz, 1 H, Ar-H), 9.41 (d, *J* = 2.4 Hz, 1 H, Ar-H).
¹³C-NMR (CDCl3, 100 MHz): δ = 50.16, 114.4, 119.0, 127.1, 131.9, 135.1, 139.7, 145.0, 151.0.
MS (ESI) *m*/*z* (%) = 205.0 (100) [M+H]⁺.

### 1-(Cyclohex-2-en-1-yl)-5-nitro-1H-pyrazolo[3,4-b]pyridine (4.4a)

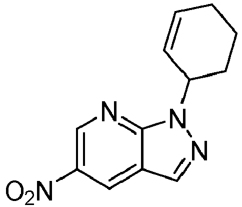

Following general procedure A: From 3 (150 mg, 0.914 mmol) and 3-bromocyclohexene (116 µL, 1.01 mmol). Purification by column chromatography (PE/EtOAc 3:1 → 1:1) afforded a slightly yellow oil (96.0 mg, 0.393 mmol, 43 %).
¹H-NMR (CDCl₃, 500 MHz): δ = 1.76-1.88 (m, 1 H, CH), 1.93-2.04 (m, 1 H, CH), 2.07-2.23 (m, 3 H, 3 × CH), 2.23-2.34 (m, 1 H, CH), 5.72-5.81 (m, 2 H, 2 × CH), 6.08-6.19 (m, 1 H, CH), 8.26 (s, 1 H, Ar-H), 8.95 (d, *J* = 2.4 Hz, 1 H, Ar-H), 9.40 (d, *J* = 2.4 Hz, 1 H, Ar-H).
¹³C-NMR (CDCl₃, 125 MHz): δ = 20.55, 24.65, 29.67, 53.50, 114.4, 125.7, 127.1, 132.4, 134.8, 139.5, 144.6, 150.7.
MS (ESI) *m*/*z* (%) = 245.0 (100) [M+H]⁺.

### 1-Benzyl-5-nitro-1H-pyrazolo[3,4-b]pyridine (4.5a)

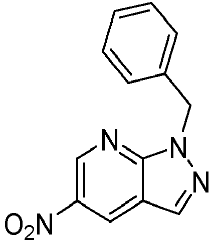

Following general procedure A: From 3 (150 mg, 0.914 mmol) and benzyl bromide (119 µL, 1.01 mmol). Purification by column chromatography (PE/EtOAc 3:1 → 1:1) afforded a slightly yellow oil (112 mg, 0.441 mmol, 48 %).
¹H-NMR (CDCl₃, 500 MHz): δ = 5.75 (s, 2 H, CH₂), 7.27-7.41 (m, 5 H, 5 × Ar-H), 8.25 (s, 1 H, Ar-H), 8.95 (d, *J* = 2.4 Hz, 1 H, Ar-H), 9.44 (d, *J* = 2.4 Hz, 1 H, Ar-H). ¹³C-NMR (CDCl3, 125 MHz): δ = 51.59, 114.4, 127.2, 128.3, 128.4, 129.0, 135.2, 135.9, 139.7, 145.1, 151.1.
MS (ESI) *m*/*z* (%) = 255.1 (100) [M+H]⁺.

### tert-Butyl 2-(5-nitro-1H-pyrazolo[3,4-b]pyridin-1-yl)acetate (4.6a)

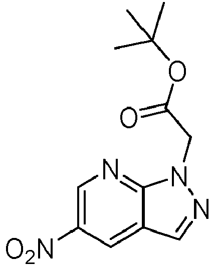

Following general procedure A: From 3 (150 mg, 0.914 mmol) and tert-butyl bromoacetate (149 µL, 1.01 mmol). Purification by column chromatography (PE/EtOAc 3:1 → 1:1) afforded a colorless solid (91.0 mg, 0.327 mmol, 36 %).
¹H-NMR (CDCl₃, 500 MHz): δ = 1.46 (s, 9 H, 3 × CH₃), 5.26, (s, 2 H, CH₂), 8.30 (s, 1 H, Ar-H), 8.97 (d, *J* = 2.4 Hz, 1 H, Ar-H), 9.42 (d, *J* = 2.4 Hz, 1 H, Ar-H).
¹³C-NMR (CDCl3, 125 MHz): δ = 28.13, 49.51, 83.52, 114.5, 127.2, 135.7, 139.9, 145.2, 151.9, 166.3.
MS (ESI) *m*/*z* (%) = 223.0 (100) [M-*t*Bu+2H]⁺.

### 2,5-Difluoro-N-(1H-pyrazolo[3,4-b]pyridin-5-yl)benzenesulfonamide (6.1)

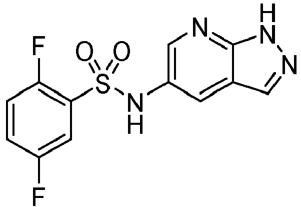

Following general procedure B: From 3 (40.0 mg, 0.244 mmol).
Following general procedure C: From aniline 5.1 (0.244 mmol) and 2,5-Difluorobenzenesulfonyl chloride (36 µL, 0.268 mmol, 1.1 equiv.). Purification by column chromatography (PE/EtOAc 1:1) afforded a beige colored solid (19.0 mg, 61.2 µmol, 25 %).
¹H-NMR (DMSO-d₆, 500 MHz): δ = 7.48-7.64 (m, 3 H, 3 × Ar-H), 7.93 (d, *J* = 2.4 Hz, 1 H, Ar-H), 8.11 (s, 1 H, Ar-H), 8.25 (d, *J* = 2.4 Hz, 1 H, Ar-H), 10.8 (s, 1 H, NH), 13.7 (S_{br}, 1 H, NH).
¹³C-NMR (DMSO-d₆, 125 MHz): δ = 114.1, 116.73 (d, *J* = 27.1 Hz), 119.47 (dd, *J* = 24.4, 8.8 Hz), 122.7 (dd, *J* = 24.1, 9.2 Hz), 123.6, 126.5, 127.9 (dd, *J* = 17.1, 7.4 Hz), 133.5, 145.0, 149.8, 154.4 (d, *J* = 251 Hz), 157.3 (d, *J* = 247 Hz).
MS (ESI) *m*/*z* (%) = 311.1 (100) [M+H]⁺.

### 2,5-Difluoro-N-(1-methyl-1H-pyrazolo[3,4-b]pyridin-5-yl)benzenesulfonamide (6.2)

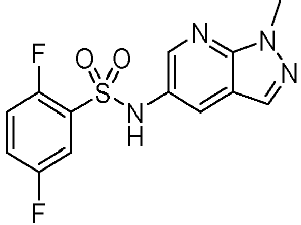

Following general procedure B: From 4.1a (40.0 mg, 0.225 mmol).

Following general procedure C: From aniline 5.2 (0.225 mmol) and 2,5-Difluorobenzenesulfonyl chloride (33 µL, 0.247 mmol, 1.1 equiv.). Purification by column chromatography (PE/EtOAc 2:1 → 1:1) afforded a colorless oil (49.0 mg, 0.151 mmol, 67%).
¹H-NMR (CDCl₃, 500 MHz): δ = 4.10 (s, 3 H, CH₃), 7.19-7.25 (m, 2 H, 2 × Ar-H), 7.40-7.45 (m, 1 H, Ar-H), 7.50 (S_{br}, 1 H, NH), 7.96 (d, *J* = 2.4 Hz, 1 H, Ar-H), 7.97 (s, 1 H, Ar-H), 8.28 (d, *J* = 2.4 Hz, 1 H, Ar-H).
¹³C-NMR (CDCl₃, 125 MHz): δ = 34.27, 115.4, 117.86 (d, *J* = 27.1 Hz), 118.55 (dd, *J* = 24.1, 7.8 Hz), 122.44 (dd, *J* = 24.0, 8.7 Hz), 125.5, 126.1, 127.80 (dd, *J* = 16.2, 6.9 Hz), 132.1, 145.7, 154.74 (dd, *J* = 250, 2.8 Hz), 157.97 (dd, *J* = 249, 2.6 Hz).
MS (ESI) *m*/*z* (%) = 325.2 (100) [M+H]⁺.

### N-(1-isopropyl-1H-pyrazolo[3,4-b]pyridin-5-yl)methanesulfonamide (6.3)

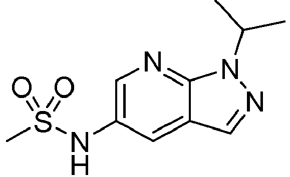

Following general procedure B: From 4.2a (30.0 mg, 0.170 mmol).
Following general procedure C: From aniline 5.3 (0.170 mmol) and Methanesulfonyl chloride (15 µL, 0.187 mmol, 1.1 equiv.). Purification by column chromatography (PE/EtOAc 2:1 → 1:1) afforded a yellow oil (37.0 mg, 0.145 mmol, 85 %).
¹H-NMR (CDCl₃, 700 MHz): δ = 1.59 (d, *J* = 6.8 Hz, 6 H, 2 × CH₃), 3.03 (s, 3 H, CH₃), 5.27 (hept, *J* = 6.7 Hz, 1 H, CH), 7.03 (S_{br}, 1 H, NH), 8.02 (s, 1 H, Ar-H), 8.08 (d, *J* = 2.4 Hz, 1 H, Ar-H), 8.45 (d, *J* = 2.4 Hz, 1 H, Ar-H).
¹³C-NMR (CDCl₃, 175 MHz): δ = 22.18, 39.52, 49.17, 115.7, 125.6, 126.6, 131.9, 145.4, 148.0.
MS (ESI) *m*/*z* (%) = 255.1 (100) [M+H]⁺.

### 2,5-Difluoro-N-(1-isopropyl-1H-pyrazolo[3,4-b]pyridin-5-yl)benzenesulfonamide (6.4)

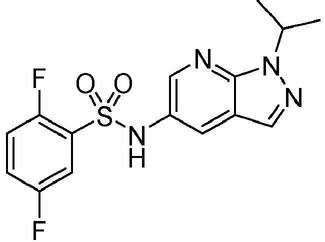

Following general procedure B: From 4.2a (30.0 mg, 0.170 mmol).
Following general procedure C: From aniline 5.3 (0.170 mmol) and 2,5-Difluorobenzenesulfonyl chloride (25 µL, 0.187 mmol, 1.1 equiv.). Purification by column chromatography (PE/EtOAc 3:1 → 2-1) afforded a colorless solid (43.0 mg, 0.122 mmol, 72 %).
¹H-NMR (CDCl₃, 700 MHz): δ = 1.55 (d, *J* = 6.8 Hz, 6 H, 2 × CH₃), 5.21 (hept, *J* = 6.7 Hz, 1 H, CH), 7.12 (S_{br}, 1 H, NH), 7.20-7.26 (m, 2 H, 2 × Ar-H), 7.42-7.47 (m, 1 H, Ar-H), 7.93 (d, *J* = 2.4 Hz, 1 H, Ar-H), 7.98 (s, 1 H, Ar-H), 8.25 (d, *J* = 2.4 Hz, 1 H, Ar-H).
¹³C-NMR (CDCl₃, 175 MHz): δ = 22.17, 49.09, 115.1, 117.9 (d, *J* = 27.0 Hz), 118.6 (dd, *J* = 24.1, 7.8 Hz), 122.4 (dd, *J* = 24.0, 8.7 Hz), 125.4, 126.1, 128.0 (dd, *J* = 16.1, 7.0 Hz), 131.9, 145.3, 148.0, 154.8 (dd, *J* = 250.3, 3.0 Hz), 158.0 (dd, *J* = 249.0, 2.5 Hz).
MS (ESI) *m*/*z* (%) = 353.2 (100) [M+H]⁺.

### 2,5-Difluoro-N-(1-propyl-1H-pyrazolo[3,4-b]pyridin-5-yl)benzenesulfonamide (6.5)

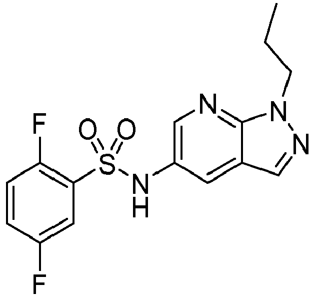

Following general procedure B: From 4.3a (40.0 mg, 0.196 mmol).
Following general procedure C: From aniline 5.4 (0.196 mmol) and 2,5-Difluorobenzenesulfonyl chloride (29 µL, 0.215 mmol, 1.1 equiv.). Purification by column chromatography (PE/EtOAc 4:1) afforded a colorless solid (32.0 mg, 90.8 µmοl, 46 %).
¹H-NMR (CDCl₃, 400 MHz): δ = 0.90 (t, *J* = 7.4 Hz, 3 H, CH₃), 1.89-1.99 (m, 2 H, CH₂), 4.39- 4.44 (m, 2 H, CH₂), 7.20-7.27 (m, 2 H, , 2 × Ar-H), 7.41-7.47 (m, 1 H, Ar-H), 7.95 (d, *J* = 2.4 Hz, 1 H, Ar-H), 7.97 (s, 1 H, Ar-H), 8.26 (d, *J* = 2.4 Hz, 1 H, Ar-H).
¹³C-NMR (CDCl₃, 100 MHz): δ = 11.36, 23.19, 49.15, 115.4, 117.9 (d, *J* = 26.9 Hz), 118.6 (dd, *J* = 24.1, 7.9 Hz), 122.4 (dd, *J* = 24.1, 8.8 Hz), 125.4, 126.2, 128.0 (dd, *J* = 16.1, 7.1 Hz), 132.1, 145.5, 148.8, 154.8 (dd, *J* = 251, 2.8 Hz), 158.0 (dd, *J* = 249, 2.4 Hz).
MS (ESI) *m*/*z* (%) = 353.2 (100) [M+H]⁺.

### N-(1-cyclohexyl-1H-pyrazolo[3,4-b]pyridin-5-yl)-2,5-difluorobenzenesulfonamide (6.6)

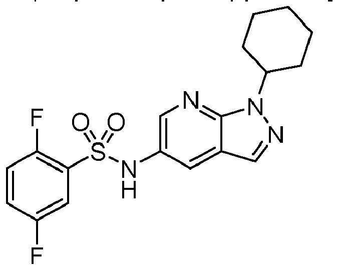

Following general procedure B: From 4.4a (40.0 mg, 0.164 mmol).
Following general procedure C: From aniline 5.5 (0.164 mmol) and 2,5-Difluorobenzenesulfonyl chloride (24 µL, 0.181 mmol, 1.1 equiv.). Purification by column chromatography (PE/EtOAc 4:1 → 3:1) afforded a yellow oil (40.0 mg, 0.102 mmol, 62 %).
¹H-NMR (CDCl₃, 500 MHz): δ = 1.26-1.37 (m, 1 H, CH), 1.40-1.54 (m, 2 H, 2 × CH), 1.70- 1.78 (m, 1 H, CH), 1.85-1.94 (m, 2 H, 2 × CH), 1.95-2.01 (m, 4 H, 4 × CH), 4.73-4.86 (m, 1 H, CH), 7.18 -7.27 (m, 2 H, 2 × Ar-H), 7.40-7.47 (m, 1 H, Ar-H), 7.94 (d, *J* = 2.4 Hz, 1 H, Ar-H), 7.97 (s, 1 H, Ar-H), 8.25 (d, *J* = 2.4 Hz, 1 H, Ar-H).
¹³C-NMR (CDCl₃, 125 MHz): δ = 25.43, 25.75, 32.57, 56.44, 115.4, 117.9 (d, *J* = 26.9 Hz), 118.54(dd, *J* = 24.0, 8.0 Hz), 122.4 (dd, *J* = 24.1, 8.6 Hz), 125.3, 126.2, 127.9 (dd, *J* = 16.2, 6.9 Hz), 131.8, 142.3, 148.0, 154.8 (dd, *J* = 250, 2.7 Hz), 154.8 (dd, *J* = 250, 2.7 Hz).
MS (ESI) *m*/*z* (%) = 393.2 (100) [M+H]⁺.

### N-(-benzyl-1H-pyrazolo[3,4-b]pyridin-5-yl)-2,5-difluorobenzenesulfonamide (6.7)

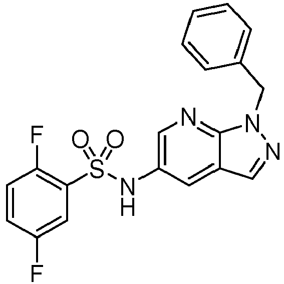

Following general procedure B: From 4.5a (40.0 mg, 0.157 mmol).

Following general procedure C: From aniline 5.6 (0.157 mmol) and 2,5-Difluorobenzenesulfonyl chloride (23 µL, 0.173 mmol, 1.1 equiv.). Purification by column chromatography (PE/EtOAc 3:1) afforded a colorless oil (26.0 mg, 64.9 µmol, 41 %).
¹H-NMR (CDCl₃, 400 MHz): δ = 5.64 (s, 2 H, CH₂), 7.18-7.34 (m, 7 H, 7 × Ar-H), 7.41-7.51 (m, 1 H, Ar-H), 7.94 (d, *J* = 2.3 Hz, 1 H, Ar-H), 7.99 (s, 1 H, Ar-H), 8.29 (d, *J* = 2.3 Hz, 1 H, Ar-H).
¹³C-NMR (CDCl₃, 100 MHz): δ = 51.12, 115.5, 117.9 (d, *J* = 27.0 Hz), 118.6 (dd, *J* = 24.0, 7.9 Hz), 122.5 (dd, *J* = 24.2, 8.8 Hz), 125.7, 126.0, 128.1, 128.1, 128.8, 132.7, 136.6, 145.8, 148.8, 154.8 (d, *J* = 254 Hz), 158.0 (d, *J* = 246 Hz).
MS (ESI) *m*/*z* (%) = 401.2 (100) [M+H]⁺.

### tert-Butyl 2-(5-((2,5-difluorophenyl)sulfonamido)-1H-pyrazolo[3,4-b]pyridin-1-yl)acetate (5.9)

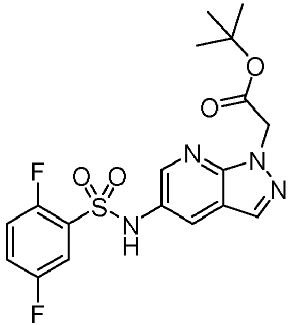

Following general procedure B: From 4.6a (80.0 mg, 0.288 mmol).
Following general procedure C: From aniline 5.5 (0.288 mmol) and 2,5-Difluorobenzenesulfonyl chloride (43 µL, 0.316 mmol, 1.1 equiv.). Purification by column chromatography (PE/EtOAc 2:1) afforded a colorless solid (72.0 mg, 0.170 mmol, 59 %).
¹H-NMR (DMSO-d₆, 500 MHz): δ = 1.33 (s, 9 H, 3 × CH₃), 5.17 (s, 2 H, CH₂), 7.47-7.63 (m, 3 H, 3 × Ar-H), 7.98 (d, *J* = 2.4 Hz, 1 H, Ar-H), 8.17 (s, 1 H, Ar-H), 8.28 (d, *J* = 2.3 Hz, 1 H, Ar-H), 10.9 (s, 1 H, NH).
¹³C-NMR (DMSO-d₆, 125 MHz): δ = 27.57, 48,93, 81.85, 114.9, 116.8 (d, *J* = 27.2 Hz), 119.5 (dd, *J* = 24.0, 8.4 Hz), 122.8 (dd, *J* = 24.0, 8.7 Hz), 124.2, 127.2, 127.8 (dd, *J* = 16.5, 6.5 Hz), 133.2, 145.1, 148.4, 154.4 (d, *J* = 249 Hz), 157.3 (d, *J* = 244 Hz), 166.9.
MS (ESI) *m*/*z* (%) = 424.8 (100) [M+H]⁺, 369.1 (45) [M-*t*Bu+H]⁺,

### 2,5-Difluoro-N-(1-isopropyl-1H-pyrazolo[3,4-b]pyhdin-5-yl)-N-methylbenzenesulfonamide (7.2)

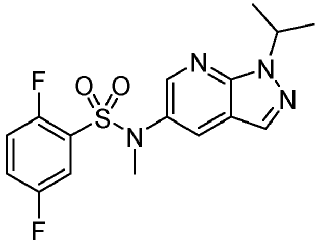

To a solution of 6.4 (25.0 mg, 71.0 µmmol) in DMF (0.5 mL) was added lodomethane (5 µL, 78.1 µmmol, 1.1 equiv.) and CS₂CO₃ (34.7 mg, 0.106 mmol, 1.5 equiv.) and it was stirred at room temperature for 18 h. The reaction mixture was poured into EtOAc (15 mL), washed with sat. aq. NaCl solution (2 × 15 mL), dried over MgSO₄ and the solvent was removed under reduced pressure. Column chromatography (PE/EtOAc 3:1) afforded a colorless oil (19.0 mg, 51.9 µmmol, 73 %).
¹H-NMR (CDCl₃, 500 MHz): δ = 1.58 (d, *J* = 6.8 Hz, 6 H, 2 × CH₃), 3.45 (d, *J* = 2.1 Hz, 3 H, CH₃), 5.26 (hept, *J* = 6.7 Hz, 1 H, CH), 7.18-7.31 (m, 2 H, 2 × Ar-H), 7.35-7.42 (m, 1 H, Ar-H), 7.88 (d, *J* = 2.3 Hz, 1 H, Ar-H), 7.99 (s, 1 H, Ar-H), 8.31 (d, *J* = 2.3 Hz, 1 H, Ar-H).
¹³C-NMR (CDCl₃, 125 MHz): δ = 22.21, 39.95 (d, *J* = 4.0 Hz), 49.03, 115.5., 118.3 (d, *J* = 26.8 Hz), 118.83 (dd, *J* = 24.9, 7.8 Hz), 122.1 (dd, *J* = 24.0, 8.5 Hz), 127.43 (dd, *J* = 17.4, 6.6 Hz), 129.1, 130.9, 132.1, 147.9, 148.1, 155.0 (dd, *J* = 252, 2.8 Hz), 157.9 (dd, *J* = 248, 2.6 Hz).
MS (ESI) *m*/*z* (%) = 367.2 (100) [M+H]⁺.

### Preparation and storing of chemical compounds.

The candidate chemical compounds of the COMAS library (http://comas.mpi-dortmund.mpg.de/index.php/workflow) were obtained from the chemical providers ChemDiv and Enamine in form of dry film which was dissolved in DMSO to yield a 10 mM stock solution and stored at -20°C for long term storage and at room temperature for short term storage. Additional compounds were obtained from academic collaboration partners, including Andrey Antonchick from Technical University of Dortmund in the form of 10 mM DMSO solution and stored at -20°C for long term storage and at room temperature for short term storage.
Before use in the screening campaign, the compounds were not diluted but directly added from 10 mM DMSO stocks to assay plates using acoustic nanoliter dispensing technology.

### Establishment of a S. cerevisiae 2-hybrid system to assay Stp complex formation.

To develop a screening platform based on Stp complex formation, a *Saccharomyces cerevisiae* 2-hybrid system was used. The *stp1*(gene ID: 23563214, UMAG_02475), *stp3* (gene ID: 23561938, UMAG_00715), *stp4* (gene ID: 23567950, UMAG_12197) and *pep1* (gene ID: 23562847, UMAG_01987) genes were cloned into both the bait vector pGBKT7 and the prey vector pGADT7, generating in-frame fusions with the gene encoding the yeast GAL4 binding (BD) and activation domain (AD), respectively. After co-transformation, reporter gene transcription is activated only if the two fusion proteins interact in the yeast nucleus. Moreover, the *S. cerevisiae* strain AH109 harbours the reporter genes ADE2 and HIS3, which encode for two enzymes that are crucial for adenine (Ade) and histidine (His) biosynthesis and thereby enable nutritional selection as a read-out for the interaction between proteins. In the following experiments, growth on high-stringency synthetic defined (SD) medium (SD -Leu -Trp -Ade -His) was used to identify if the complex members interact between them causing expression of ADE2 and HIS3. Additionally, strains were also grown on low-stringency medium (SD -Leu -Trp) to confirm their viability. Expression of all fusion proteins was confirmed by analyzing total cell extracts via western blot using anti-HA (AD fusion proteins) and anti-myc (BD fusion proteins) antibodies (not shown). All experiments were done with three independent transformants to exclude false positives due to spontaneous mutations. When tested for autoactivation, BD-Stp4 showed an activation of the reporter genes and was therefore used exclusively when fused to the AD. Neither BD-Stp1, BD-Stp3 nor BD-Pep1 showed autoactivation (not shown).

### Preparation of yeast NL73 strain and NL70 control.

The *S. cerevisiae* strain AH109 from Clontech (Saint-Germain-en-Laye, France) is auxotrophic for tryptophan, leucine, adenine and histidine. In addition, *lacz* is under control of the MEL1 UAS to be used as readout for quantitative β-galactosidase assays. AH109Aabc9 is a derivative of AH109. In this strain, nine ABC transporter- related genes are deleted by standard methods using PCR product-mediated homologous recombination, making it highly permeable and sensitive for small molecules (Wong, et al., 2017. A yeast two-hybrid system for the screening and characterization of small-molecule inhibitors of protein-protein interactions identifies a novel putative Mdm2-binding site in p53. *BMC Biology* 15, 108).

To prove that ABC9Δ behaves like its progenitor strain AH109 with regard to expression and growth after expression of complex member combinations, several representative interaction assays for complex members were conducted. In all cases, the results were comparable to those previously observed in AH109 (not shown). The resulting test strain NL73 (AD-Stp4/BD-Stp1/myc-Stp3) expresses the Stp1/Stp4/Stp3 subcomplex. NL73 carries the following three plasmids:
pGBKT7-stp1: pGBKT7 vector expressing proteins fused to the GAL4 DNA binding domain (DNA-BD). pGBKT7 also contains the T7 promoter, a c-Myc epitope tag, and a MCS. The vector carries a Kanamycin resistance gene (Kanr) for selection in *E. coli* and the *TRP1* nutritional marker for selection in yeast.
pGAD-stp4: pGADT7 vector expressing proteins fused to a GAL4 activation domain (AD). The GAL4 AD fusion contains an N-terminal nuclear localization signal that targets the protein to the yeast nucleus, and a HA Tag, located between the GAL4 AD and the protein of interest. The vector carries an ampicillin resistance gene (Ampr) for selection in *E. coli* and a *LEU2* nutritional marker for selection in yeast.
pYEA-stp3-myc: pYEA vector expressing proteins under the control of the phosphoglycerate kinase promoter. The vector carries an ampicillin resistance gene (Ampr) for selection in *E. coli* and an *ADE2* nutritional marker for selection in yeast. As control strain, NL70 containing empty vectors was generated (AH109Δabc9 pGAD::LEU2 pGBK::TRP1 pYEA::ADE2).

### Incubation of NL73 and NL70 with candidate compounds and viability assay

Overnight cultures of the NL73 test strain and NL70 control strain (unable to grow on high stringency medium) were harvested by centrifugation, washed 2 times and resuspended in high stringency medium (0.67% (w/v) yeast nitrogen base (BD #291920), 0.16% (w/v) dropout supplements without adenine, histidine, leucine and tryptophan (Clonetech #630428), 2% glucose) to an OD₆₀₀ of 0.5.

25 µl of compound stock solution were pre-dispensed into black 384-well assay plates (Corning 4513) using Echo520 Nanoliter dispenser (Labcyte). After that, 25 µl of NL73 cell suspension were dispensed into the assay plates using Multidrop Combi (Thermofisher Scientific) giving a final screening concentration of 10 µM. One column of the assay plate was filled with 25 µl of NL70 control strain cell suspension as negative control. Plates were sealed with Breathe-Easy sealing membrane (Sigma, Z380059), lidded and incubated in a humid chamber over night at 30°C and 140 rpm in an HT Multitron Pro (Infors). After approximately 19 h, plates were centrifuged for 1 min at 500 rpm (Eppendorf centrifuge 5804R). For the viability assay, the alamar Blue HS Cell Viability reagent (Invitrogen, #A50101) was pre-diluted using an equal amount of dVE-H₂O and 5 µl of diluted Alamar Blue were added to the screening plates using Multidrop Combi. The plates were sealed again and incubated for 4 h at room temperature, unlidded. Fluorescence readout was carried out on a Spectramax Paradigm detection platform (Molecular Devices) at Ex/Em 535nm/595nm for 0.14 s. Screening data was normalized to DMSO-treated NL73 cells (that received DMSO in the same concentration as in samples where compounds dissolved in DMSO were added) being 100% and DMSO-treated NL70 cells representing 0% viability and analyzed with Quattro Software Suite (Quattro Research GmbH).

To determine whether reduced viability of NL73 in the presence of the compound did result from toxicity of the compound rather than from destruction of the complex, an Alamar Blue based toxicity counter-assay was developed. To this end an overnight culture of the control strain NL70 was harvested by centrifugation, washed 2 times and resuspended in low stringency medium (high stringency medium + 0.02 g/L histidine (Sigma) to an OD₆₀₀ of 0.5. 25 µl of compound stock solution were pre-dispensed into black 384-well assay plates using Echo520 Nanoliter dispenser. After that, 25 µl of NL70 cell suspension were dispensed into the assay plates using Multidrop Combi giving a final screening concentration of 10 µM. One column of the assay plate was filled with 25 µl of NL70 cell suspension in high stringency medium as negative control. Plates were sealed with Breathe-Easy sealing membrane, lidded and incubated in a humid chamber over night at 30°C and 140 rpm in an Infors HT Multitron Pro. After approximately 19 h, plates were centrifuged for 1 min at 500 rpm. Alamar Blue HS Cell Viability reagent was pre-diluted using an equal amount of dVE-H₂O and 5 µl of Alamar Blue were added using Multidrop Combi. The plates were sealed again and incubated for 4 h at room temperature, unlidded. Fluorescence readout was carried out on a Spectramax Paradigm detection platform at Ex/Em 535nm/595nm for 0.14 s. Data were normalized using DMSO-treated NL70 cells in low stringency vs. high stringency medium. Screening data was analysed with Quattro Software Suite (Quattro Research GmbH).

Hits which affected growth of NL73 but showed no toxicity at 10 µM concentration in NL70 were subjected to a confirmatory assay using the Beta-Glo assay system (Promega #E4740). This system allows luminescence-based quantification of β-galactosidase expression in cells which serves as a readout for promoter activation by interacting complex members Stp1, Stp3 and Stp4. Overnight cultures of NL73 and NL70 strains were harvested as described above. Dilution series of compound stock solution were pre-dispensed into black 384-well assay plates (Corning 4513) using Echo520 Nanoliter dispenser starting from 10 µM with 3-fold dilution over 8 steps. In the viability setup, 15 µl of NL73 cell suspension were dispensed into the assay plates using Multidrop Combi. One column of the assay plate was filled with 15 µl of NL70 cell suspension. In the toxicity setup, 15 µl of NL70 cell suspension in low stringency medium were dispensed into the assay plates with one column being filled with 15 µl of NL70 cell suspension in high stringency medium. Plates were sealed with Breathe-Easy sealing membrane, lidded and incubated in a humid chamber over night at 30°C and 140 rpm in an Infors HT Multitron Pro. After approximately 19 h, plates were centrifuged for 1 min at 500 rpm. 4 µl of Beta-Glo assay solution were added using Multidrop Combi. The plates were sealed again and incubated for 2.5 h at room temperature, unlidded. Luminescence readout was determined on a Spectramax Paradigm detection platform for 0.2 s. Dose response data was analysed with Quattro Software Suite.

### Activity of the hit compounds in a maize U. maydis infection model

To test whether the identified ten hit compounds had an effect on virulence of *U. maydis, Zea mays* seedlings of the variety Golden Bantam (Bingenheimer Saatgut#G455) were infected with *U. maydis* strains FB1 and FB2 following an established protocol. Compounds to be tested were added to the inoculum. Briefly, 6 day old maize seedlings were syringe-infected with about 0.5 ml of fungal suspension in H₂O which was amended with the hit compound. The infection and symptoms assessment were done as described in Kämper et al., (Insights from the genome of the biotrophic fungal plant pathogen Ustilago maydis. Nature 2006, 444, 97-101). During the syringe-infection into the stem, the fungal-compound mix is dispersed between the rolled-up leaves and fills the leaf whorl. The compounds were applied only at the stage of mixing with the inoculum, which is subsequently diluted with existing apoplastic fluid, during the infection. In addition, compounds have to reach the Stp complex in biotrophic hyphae from the initial inoculum. This explains that significantly higher concentrations are needed compared to the tests in yeast strain NL73. Since compounds are dissolved in DMSO, the tolerance of *U. maydis* towards DMSO was tested by adding DMSO to the inoculum at concentrations between 10 and 50% final concentration. Results showed that up to 20% are tolerated in the inoculum without adverse effects on virulence (not shown).

Symptom scoring was done 12 days post infection (dpi) in case of *U. maydis* seedling infections. Symptom scoring occurred according to the categories described in **Table 2.**

**Table 2: Categories of virulence symptoms of U. maydis maize seedling infection (according to Kämper et al., 2006).**

| **Plant symptom** | **Description** |
|---|---|
| No symptoms | No symptoms observable |
| Chlorosis / necrosis | Plant shows discoloration |
| Small tumors | Small tumors (< 1 mm) on leaves or very few tumors (> 1 mm) |
| Normal tumors | Tumors on leaves and/or stem |
| Heavy tumors | Tumors on base of stem and/or change of growth axis |
| Dead | Plant died due to the infection |

### Uromyces fabae - Vicia faba system

A previous study of Kemen et al. showed by immunoelectron microscopy that *Uromyces fabae* possesses structures highly similar to the Stp complex structures of *U. maydis.* Of particular relevance is that the RTP1 effector of *U. fabae* was revealed to reside in structures extending from fungal feeding structures, i.e. haustoria, into neighboring plant cells (Kemen et *al.*, 2013). These data suggest that rust fungi might possess a Stp complex related structure.

The *Uromyces fabae* - *Vicia faba* system was established by initially obtaining spores as well as broad bean seeds from the Institut für Phytomedizin at Hohenheim University, Germany. Briefly, *Vicia faba* plants (Bingenheimer Saatgut Frühe Weißkeimige, G142) were propagated in soil up to about the 4 leaf stage (2-3 plants per pot). Plants were transferred to a chamber lined with wet paper. 100 mg *U. fabae* spores were mixed with 160 mg skimmed milk powder in 200 ml sterile H₂O. Tween 20 was added to a final concentration of 0.01 % and the mixture was stirred in the dark for 45 min. Leaves were sprayed with the suspension on both the upper and lower side and afterwards the entire compartment was fogged with H₂O before closure and kept in the dark for 20 h. Plants were then gradually allowed to dry and were placed in a growth chamber under a light regime of 16 h light (24°C) and 8 h (20°C) dark until symptoms developed (10-12 days). Plants were tossed and spores were collected on underlying aluminum foil, and aliquots were frozen at -80°C.

For the infection with compounds added, 10 mg of *U. fabae* spores were mixed with 16 mg skimmed milk powder in 20 ml sterile H₂O. Tween 20 was added to a final concentration of 0.01 % and the mixture was stirred in the dark for 45 min. Compounds were diluted to the desired concentration into in the spore suspension. The controls received the same amount of DMSO. Per bean plant, one pinnate leaf with two leaflets was selected and one of the leaflets was infected with spores + compound and the other with spores + DMSO, respectively. To this end leaves were lightly sprayed with H₂O and the 100µl spore solution containing the compound or DMSO respectively was applied on the upper part of the using a small painting brush. Afterwards the plants were placed in a chamber lined with wet paper, fogged with H₂O before closure and kept in the dark for 20 h. Plants were then gradually allowed to dry and were placed in a growth chamber under a light regime of 16 h light (24°C) and 8 h (20°C) dark until symptoms developed. The symptoms were quantified by using a previously described method (Sillero and Rubiales, 2002) with minor modifications. Comparisons of pustule formation were done as follow: leaf treated with spores *plus* DMSO (control) *versus* leaf treated with spores *plus* compound in DMSO (test). A single bifurcated leaf was used for this assay, and one half received the control and the other half receive spores with compound. Then it was determined whether pustule formation occurs or is inhibited. Leaves were scored for the development of brownish-red pustules. Only when brownish-red pustules containing powdery spores (urediniospores) appeared on the control leaf an infection was counted as valid. All compounds were tested in three biological replicates. Pictures were taken at 12-14 days post infection.

### Example 1. Establishment of Stp complex in yeast

Based on the crucial importance of the Stp complex for successful plant colonization of *U. maydis,* the inventors have considered it as a potential new fungicide target. A screening platform based on Stp complex formation was developed using a *Saccharomyces cerevisiae* 2-hybrid system as described in the methods. The *stp1 stp3, stp4,* and *pep1* genes were cloned in order to have each of them expressed with AD or BD fusion proteins.

Testing strains containing all possible combinations of four complex members revealed that all strains could grow on low-stringency plates (SD -Leu -Trp). The yeast strains expressing AD-Stp3/BD-Stp1 and AD-Pep1/BD-Stp1 were able to grow on high-stringency plates (SD -Leu -Trp -Ade -His), illustrating direct interaction between the respective protein pairs (**Figure 1A**). For the ADStp4/BD-Stp1 expressing strains, growth could be observed on high-stringency plates, but appeared much weaker, suggesting a weaker, more transient interaction between Stp4 and Stp1 (**Figure 1A**). Combinations of AD-Stp3/BD-Pep1, AD-Stp4/BD-Stp3 and AD-Stp4/BD-Pep1 failed to provide for growth on high-stringency plates, indicating that there is no direct interaction between the respective protein pairs (**Figure 1A**). Next we tested whether expression of an additional complex member could increase interaction between the weakly interacting AD-Stp4/BD-Stp1 and the non-interacting effectors Stp3, Stp4 and Pep1. To this end, both myc-Pep1 and myc-Stp3 were cloned separately into the vector pYEA and co-transformed with AD-Stp4/BD-Stp1. pYEA contains an ADE2 nutritional marker allowing the selection on medium lacking adenine. The yeast strains expressing AD-Stp4/BD-Stp1/myc-Stp3 or AD-Stp4/BD-Stp1/myc-Pep1 showed full growth on both low (SD -Leu -Trp -Ade) and high (SD -Ade -His -Leu -Trp) stringency plates (**Figure 1B**). This demonstrates that the weak interaction between Stp1 and Stp4 is strengthened when either Stp3 or Pep1 are present.

The interaction of the respective three complex components revealed by yeast 2-Hybrid analysis was verified in a co-immunoprecipitation in combination with mass spectrometry (co-IP/MS) experiment in which the total lysate of strain AH109 AD-Stp4/BD-Stp1/myc-Stp3 was incubated with HA-coated magnetic beads to immunoprecipitate the HA-tagged AD-Stp4. Samples were analyzed by LC-MS/MS. Shortly, a total lysate of NL73 was incubated with HA-coated magnetic beads to immunoprecipitate the HA-tagged AD-Stp4 protein and interacting proteins. Precipitated proteins were analyzed by MS and numbers in **Table 3** are spectral counts representing the unique peptides of the respective proteins. The presence of spectral counts of Stp3 and Stp1 indicates that these proteins interacted with Stp4. The results showed an interaction between Stp1, Stp3 and Stp4 (**Table 3**), confirming the existence of a stable subcomplex.

**Table 3: Interaction between Stp1, Stp3 and Stp4 in S. cerevisiae revealed by co-IP/MS.**

| Peptides | Protein Stp4-HA |
|---|---|
| Stp1 | 26 |
| Stp3 | 14 |
| Stp4 | 58 |

### Example 2: Yeast 2Hybrid-analysis for HTS screening of small molecules

The interaction of Stp1, Stp3 and Stp4 in the yeast 2Hybrid-system was monitored for HTS screening of small molecules inhibiting growth and thus, complex formation. Small compounds identified by virtue of inhibiting the three-component complex in *S. cerevisiae,* would most probably inhibit also biotrophic development of *U. maydis.*

To this end, the HTS test strain NL73 (AD-Stp4/BD-Stp1/myc-Stp3) expressing the Stp1/Stp4/Stp3 subcomplex, and the control strain NL70 were prepared as described in the methods. The growth of NL73 on high stringency medium depends on the interaction of Stp1, Stp4, and Stp3. Any compound interfering with assembly of the complex should eliminate or reduce growth/viability on high stringency medium. NL73 test strain and NL70 control strain were incubated with the compounds as described in the methods, and then tested for viability with the Alamar Blue based viability assay. To determine whether reduced viability of NL73 in the presence of the compound did result from toxicity of the compound rather than from destruction of the complex, an Alamar Blue based toxicity counter-assay was used. Hits which affected growth of NL73 but showed no toxicity at 10 µM concentration in NL70 were also subjected to a confirmatory assay using the Beta-Glo assay system (see methods). Briefly, this system allows luminescence-based quantification of β-galactosidase expression in cells which serves as a readout for promoter activation by interacting complex members Stp1, Stp3 and Stp4.

The established assay system was used to screen the compounds of the COMAS library (*http:*//*comas.mpi-dortmund.mpg.de*/*index.php*) consisting mostly of screening compounds from ChemDiv and Enamine and also includes compounds with known mode of action as well as proprietary compounds from the Department of Chemical Biology (MPI Dortmund) as well as national and international collaboration partners. A primary screening of around 200,000 molecules was carried out using the Alamar Blue viability screening assay. Typically, signal-over-background factors of 10-15 and Z' of approximatively 0.6 were achieved. Hits were defined as having a residual viability of <50% with regard to the DMSO treated control cells. 1700 hits were picked from the compound storage and validated in the screening assay, the toxicity and the confirmatory beta-glo assays with a triplicate measurement at 10 µM. Hits that showed a mean residual viability of <40% in both the Alamar Blue and the Beta-Glo assays while displaying not more than 25% toxicity in the respective counter-assays were chosen for further analysis in dose response mode. This reduced the initial hits to 237 compounds, which were further analyzed in all assays in triplicate. Ten compounds from ten different chemical classes showed activity in the nanomolar or low micromolar range. Some compounds were part of a small hit cluster, while others were singletons. Eight of ten compounds were commercially available and were thus re-ordered from the supplier as powder.

### Example 3: Activity of the hit compounds identified in the S. cerevisiae in a maize infection model with U. maydis

To test whether the identified ten hit compounds had an effect on virulence of *U. maydis, Zea mays* seedlings of the variety Golden Bantam were infected with *U. maydis* strains FB1 and FB2 following an established protocol, as described in the methods.

The ten hits from the HTS screen were assessed by applying the compounds at a final concentration of 5 mM in the inoculum of FB1xFB2 (**Table 4**) using 4-7 plants per assay. The results revealed significant reductions in virulence by compounds **1-A, 1-B, 1-C,** and **1-D.** Retesting of those four compounds for an inhibition of virulence, confirmed the results for **1-C** (a tetrahydro-cyclopentapyrimidine), **1-B** (a pyrazolopyridin-benzenesulfonamide) and **1-A** (a imidazopyridine). These results showed that three of the ten hit compounds initially detected in the yeast assay also inhibited virulence of *U. maydis.*

Considering the three identified hit compounds, activity of structurally related compounds was also tested both with the Alamar Blue viability assay as well as with respect to virulence inhibition in *U. maydis* and *U. fabae.*

**Table 4: Virulence of U. maydis with 5 mM hit compound added to inoculum**

| Compound | Severity of disease symptoms | |
|---|---|---|
| | First infection | Second infection |
| DMSO control | XXXXX* | XXXXX |
| **1-A** | X | X |
| **1-B** | XX | X |
| **1-C** | XXX | XXX |
| **1-D** | XX | XXXXX |
| **1-E** | XXXXX | Not retested |
| **1-F** | XXXX | Not retested |
| **1-G** | XXXXX | Not retested |
| **1-H** | XXXXX | Not retested |
| **1-I** | XXXX | Not retested |
| **1-L** | XXXXX | |

| | | |
|---|---|---|
| *Full virulence is symbolized by (XXXXX) attenuated virulence is indicated (XXX - slightly reduced virulence; XX - reduced virulence; x - strongly reduced virulence) | | |

### Example 4: Testing of imidazopyridines

Imidazopyridines (Manna et al. 2015. Regioselective annulation of nitrosopyridine with alkynes: straightforward synthesis of N-oxide-imidazopyridines. Chem. Commun. 51, 6119) showed nanomolar potency in the Alamar Blue assay with NL73 strain (**Table 5** below).

**Table 5:**

| COMPOUND | A-blue IC₅₀ | B-glo IC₅₀ |
|---|---|---|
| **1-A** | 0.14 µM | 0.76 µM |
| **2-A** | 0.02 µM | 0.23 µM |
| **3-A** | 0.25 µM | 0.86 µM |

Imidazopyridines compounds (**Table 5**) were then tested for virulence inhibition of *U. maydis* **(****Figure 2A)** at 1 mM final concentration in the inoculum of *Zea Mays.* **5-A** was unable to inhibit virulence while **3-A** only mildly (**Figure 2A****, B**). The three compounds **1-A, 2-A,** and **6-A,** able to reduce virulence were next tested at 1 mM, 0.5 mM and 0.1 mM final concentration in the inoculum. In all three cases the inhibitory effect was concentration dependent (**Figure 2C**).

Selected imidazopyridines compounds were also tested for virulence inhibition of *U. fabae* at 1 mM final concentration in the inoculum of *Vicia faba* (**Figure 3**). The five tested compounds **1-A, 2-A, 3-A, 5-A,** and **6-A,** were all able to inhibit pustule formation (**Figure 3**). This result could be confirmed in three biological replicates (not shown). Next, we tested all compounds at 0.5 mM final concentration in the inoculum. Whereas plants treated with **1-A, 3-A** and **5-A** now also developed symptoms, **2-A** and **6-A** were still able to fully inhibit pustule formation (**Figure 4**).

### Example 5: Testing of tetrahydro-cyclopentapyrimidines

The second hit class was tetrahydro-cyclopentapyrimidines, like **1-C**, which was found to be active in the nanomolar range with NL73. Structure searching revealed further analogs, which had been inactive in the primary screening. Upon IC₅₀ determination, some analogs showed inhibitory activity on NL73 viability (**Table 6** below).

**Table 6:**

| COMPOUND | ChemDiv IDs | IC₅₀ |
|---|---|---|
| **1-C** | D468-0807 | 0.67 µM |
| **2-C** | D468-0522 | 6.2 µM |
| **3-C** | D468-0555 | 6.4 µM |
| **4-C** | D468-0823 | 2 µM |
| **5-C** | D468-0520 | 2 µM |
| **6-C** | D468-1059 | 4.9 µM |
| **7-C** | D468-1112 | 9.4 µM |

Based on these results, a collection of further analogs was selected and acquired from the supplier. A further round of testing revealed that analogs **8-C, 9-C,** and **10-C** showed appreciable activity in the Alamar Blue assay (**Table 7**, below).

**Table 7:**

| COMPOUND | ChemDiv IDs | IC₅₀ |
|---|---|---|
| **1-C** | D468-0807 | 0.67 µM |
| **8-C** | D468-0808 | 0.81 µM |
| **9-C** | D468-0809 | 0.6 µM |
| **10-C** | D468-0553 | 5 µM |

Effect on *U. maydis* virulence was investigated with compound **1-C.** In a dilution series, **1-C** was tested between 5 mM and 0.01 mM final concentration. Virulence inhibition with **1-C** resulted concentration dependent (**Figure 5**).
Also *U. fabae* virulence was investigated with compound **1-C.** In a dilution series, **1-C** was tested at 1 mM, 0.5mM and 0.1 mM final concentration. Results showed that **1-C** inhibited pustule formation at all three tested concentrations (**Figure 6**).

### Example 6: Testing of pyrazolopyridin-benzenesulfonamides

The third validated hit compound that revealed activity in NL73 was the pyrazolopyridin-benzenesulfonamide **1-B.** As no close analogs were available in the COMAS library, close analogs of **1-B** were synthesized *in-house* as described in the Method section. A round of testing revealed that some analogs of **1-B** showed appreciable activity in the Alamar Blue assay as shown in**Table 8** below.

**Table 8**

| COMPOUND | IC₅₀ |
|---|---|
| **1-B** | 2.5 µM |
| **2-B** | 4.74 µM |
| **3-B** | 2.20 µM |
| **4-B** | 10.18 µM |
| **Ref1-B** | inactive |
| **Ref2-B** | inactive |

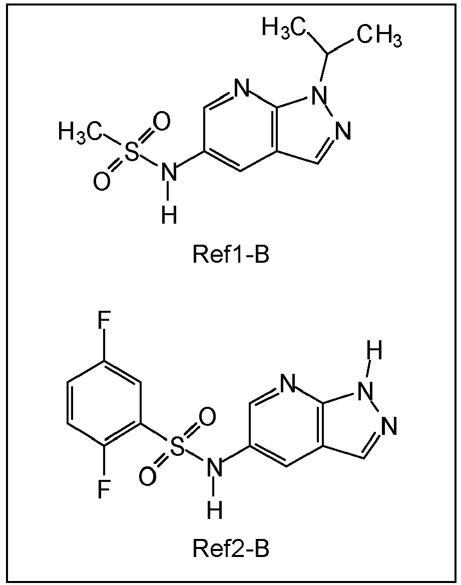

Effect of **1-B** on *U. maydis* virulence was investigated in a dilution series between 5 mM and 0.01 mM final concentration. Virulence inhibition was very strong at 5 mM and 1 mM but negligible at 0.5 mM (**Figure 7**).
Effect of **1-B** on *U. fabae* virulence was investigated with 1mM final compound concentration. At this concentration pustule formation was completely inhibited **(****Figure 8****).**

### Example 7: Alignment of Stp orthologs from eleven sequenced smut fungi.

The sequence of the orthologs of the seven members of the Stp complex were alligned to investigate the presence of the complex in different smut fungi.

For genomes lacking annotation amino acid sequences were extracted with the program CLC benchtop by running tBlastn predictions of the genome sequence data using the respective *U. maydis* (UMAG) or the *U. hordei* (UHOR) protein as a reference. The Stp members have, respectively, the following protein IDs in *Ustilago maydis* 521, and NCBI Reference Sequence IDs: Stp1: UMAG_02475, XP_011388756.1, Stp2: UMAG_10067, XP_011388794.1; Stp3: UMAG_00715, XP_011386505.1; Stp4: UMAG_12197, XP_011389576.1; Stp5: UMAG_04342, XP_011391052.1; Stp6: UMAG_01695, XP_011387671.1; Pep1: UMAG_01987, XP_011387901.1, and in UHOR: Stp1: UHOR_12209, CCF47935.1; Stp2: UHOR_03606, CCF52300.1; Stp3: UHOR_01096, CCF51205.1; Stp4: UHOR_04531, CCF52452.1; Stp5: UHOR_06791, CCF50905.1; Stp6: UHOR_02518, CCF53394.1; Pep1: UHOR_02965, CCF53137.1.
The alignments were generated with MAFFT (v7.475). "*" indicates perfect alignment. ":" indicates a site belonging to group exhibiting strong similarity. "." indicates a site belonging to a group exhibiting weak similarity. MEPE: *Melanopsichium pennsylvanicum*; UESC: *Ustilago esculenta*; SPSC: *Sporisorium scitamineum*; SPRZ: *Sporisorium reilianum f. sp.* zeae; SPRS: *Sporisorium reilianum f. sp. sorghi*; UTCP: *Ustilago trichophora*; UTRI: *Ustilago tritici*; UMAG: *U. maydis*; UBRO: *Ustilago bromivira*; UHOR: *Ustilago hordei* (host: barley); UHOO: *Ustilago hordei* (host: oats).

DNA sequences of Stp orthologues from the 11 sequenced smuts were derived from the following data sources: MEPE: ENA (European Nucleotide Archive) study no. PRJEB4565, sequence IDs HG529494 to HG52992869; UESC: Genbank accession JTLW00000000, version JTLW01000000; SPSC: ENA sequence IDs LK056649-LK05669566; SPRZ: EMBL sequence IDs FQ311430 - FQ311474; SPRS: ENA sequence IDs LT795054-LT795076; UTCP: Genbank accession LVYE00000000, version LVYE01000000; UTRI: Genbank accession LVYE00000000, version LVYE010000006; UBRO: ENA study no. PRJEB7751; UMAG: https://mycocosm.jgi.doe.gov/Ustma2_2/Ustma2_2.home.html; UHOR: ENA sequence IDs CAGI01000001 - CAGI01000713; UHOO: GenBank accession NSDP00000000 version NSDP01000000.

The amino acid sequences of the seven Stp members used in the Figures 9 - 15 are reported in the Sequence Listing, and listed in **Table 9** below:

**Table 9**

| **Sequence nr** | **Protein** | **Species** |
|---|---|---|
| Sequence 1 | Stp1 | *Melanopsichium pennsylvanicum* |
| Sequence 2 | Stp1 | *Sporisorium scitamineum* |
| Sequence 3 | Stp1 | *Ustilago hordei* (host: barley) |
| Sequence 4 | Stp1 | *Ustilago bromivira* |
| Sequence 5 | Stp1 | *Sporisorium reilianum* f. sp. zeae |
| Sequence 6 | Stp1 | *Sporisorium reilianum* f. sp. *sorghi* |
| Sequence 7 | Stp1 | *Ustilago maydis* |
| Sequence 8 | Stp1 | *Ustilago esculenta* |
| Sequence 9 | Stp1 | *Ustilago trichophora* |
| Sequence 10 | Stp1 | *Ustilago hordei* (host: oats) |
| Sequence 11 | Stp1 | *Ustilago tritici* |
| Sequence 12 | Stp2 | *Melanopsichium pennsylvanicum* |
| Sequence 13 | Stp2 | *Sporisorium scitamineum* |
| Sequence 14 | Stp2 | *Ustilago hordei* (host: barley) |
| Sequence 15 | Stp2 | *Ustilago bromivira* |
| Sequence 16 | Stp2 | *Sporisorium reilianum* f. sp. zeae |
| Sequence 17 | Stp2 | *Sporisorium reilianum* f. sp. *sorghi* |
| Sequence 18 | Stp2 | *Ustilago maydis* |
| Sequence 19 | Stp2 | *Ustilago esculenta* |
| Sequence 20 | Stp2 | *Ustilago trichophora* |
| Sequence 21 | Stp2 | *Ustilago hordei* (host: oats) |
| Sequence 22 | Stp2 | *Ustilago tritici* |
| Sequence 23 | Stp3 | *Melanopsichium pennsylvanicum* |
| Sequence 24 | Stp3 | *Sporisorium scitamineum* |
| Sequence 25 | Stp3 | *Ustilago hordei* (host: barley) |
| Sequence 26 | Stp3 | *Ustilago bromivira* |
| Sequence 27 | Stp3 | *Sporisorium reilianum* f. sp. zeae |
| Sequence 28 | Stp3 | *Sporisorium reilianum* f. sp. *sorghi* |
| Sequence 29 | Stp3 | *Ustilago maydis* |
| Sequence 30 | Stp3 | *Ustilago esculenta* |
| Sequence 31 | Stp3 | *Ustilago trichophora* |
| Sequence 32 | Stp3 | *Ustilago hordei* (host: oats) |
| Sequence 33 | Stp3 | *Ustilago tritici* |
| Sequence 34 | Stp4 | *Melanopsichium pennsylvanicum* |
| Sequence 35 | Stp4 | *Sporisorium scitamineum* |
| Sequence 36 | Stp4 | *Ustilago hordei* (host: barley) |
| Sequence 37 | Stp4 | *Ustilago bromivira* |
| Sequence 38 | Stp4 | *Sporisorium reilianum* f. sp. zeae |
| Sequence 39 | Stp4 | *Sporisorium reilianum* f. sp. *sorghi* |
| Sequence 40 | Stp4 | *Ustilago maydis* |
| Sequence 41 | Stp4 | *Ustilago esculenta* |
| Sequence 42 | Stp4 | *Ustilago trichophora* |
| Sequence 43 | Stp4 | *Ustilago hordei* (host: oats) |
| Sequence 44 | Stp4 | *Ustilago tritici* |
| Sequence 45 | Stp5 | *Melanopsichium pennsylvanicum* |
| Sequence 46 | Stp5 | *Sporisorium scitamineum* |
| Sequence 47 | Stp5 | *Ustilago hordei* (host: barley) |
| Sequence 48 | Stp5 | *Ustilago bromivira* |
| Sequence 49 | Stp5 | *Sporisorium reilianum* f. sp. zeae |
| Sequence 50 | Stp5 | *Sporisorium reilianum* f. sp. *sorghi* |
| Sequence 51 | Stp5 | *Ustilago maydis* |
| Sequence 52 | Stp5 | *Ustilago esculenta* |
| Sequence 53 | Stp5 | *Ustilago trichophora* |
| Sequence 54 | Stp5 | *Ustilago hordei* (host: oats) |
| Sequence 55 | Stp5 | *Ustilago tritici* |
| Sequence 56 | Stp6 | *Melanopsichium pennsylvanicum* |
| Sequence 57 | Stp6 | *Sporisorium scitamineum* |
| Sequence 58 | Stp6 | *Ustilago hordei* (host: barley) |
| Sequence 59 | Stp6 | *Ustilago bromivira* |
| Sequence 60 | Stp6 | *Sporisorium reilianum* f. sp. zeae |
| Sequence 61 | Stp6 | *Sporisorium reilianum* f. sp. *sorghi* |
| Sequence 62 | Stp6 | *Ustilago maydis* |
| Sequence 63 | Stp6 | *Ustilago esculenta* |
| Sequence 64 | Stp6 | *Ustilago trichophora* |
| Sequence 65 | Stp6 | *Ustilago hordei* (host: oats) |
| Sequence 66 | Stp6 | *Ustilago tritici* |
| Sequence 67 | Pep1 | *Melanopsichium pennsylvanicum* |
| Sequence 68 | Pep1 | *Sporisorium scitamineum* |
| Sequence 69 | Pep1 | *Ustilago hordei* (host: barley) |
| Sequence 70 | Pep1 | *Ustilago bromivira* |
| Sequence 71 | Pep1 | *Sporisorium reilianum* f. sp. zeae |
| Sequence 72 | Pep1 | *Sporisorium reilianum* f. sp. *sorghi* |
| Sequence 73 | Pep1 | *Ustilago maydis* |
| Sequence 74 | Pep1 | *Ustilago esculenta* |
| Sequence 75 | Pep1 | *Ustilago trichophora* |
| Sequence 76 | Pep1 | *Ustilago hordei* (host: oats) |
| Sequence 77 | Pep1 | *Ustilago tritici* |

## Claims

1. A compound for use or a combination of compounds for use in inhibiting virulence of one or more plant pathogenic basidiomycete fungi, wherein the compound(s) is/are selected from the general formula (I) - (III) which are defined as follows: 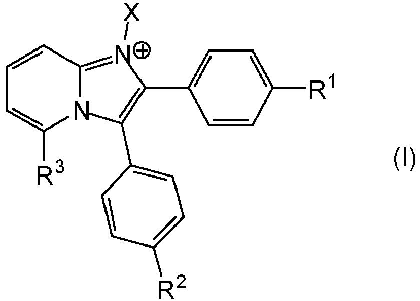 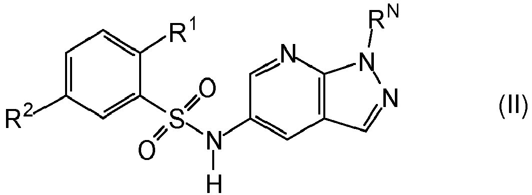 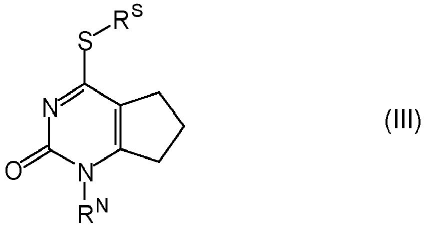 wherein
R¹ and R² represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -CH₂-OCH₃, -CH₂-OC₂H₅, -SH, -SCH₃, -SC₂H₅, -SC₃H₇, -S-cyclo-C₃H₅, -SCH(CH₃)₂, -SC(CH₃)₃, -NO₂, -F, -Cl, -Br, -P(O)(OH)₂, -P(O)(OCH₃)₂, -P(O)(OC₂H₅)₂, -P(O)(OCH(CH₃)₂)₂, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]_{2,} -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂,-O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, , -S(=O)(=NH)CH₃, -S(=O)(=NH)C₂H₅, -S(=O)(=NH)C₃H₇ -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CON(cyclo-C₃H₅)₂, -NH-CO-N[CH(CH₃)₂]₂, -NH-CS-N(C₂H₅)₂, -NH-CON[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₃, -C₂H₅, -C₃H₇, -CH(CH₃)₂, -CH₂F, -CHF₂, -CF₃, -CH₂Cl, -CH₂Br, -CH₂-CH₂F, -CH₂-CHF₂, -CH₂-CF₃, -CH₂-CH₂Cl, or -CH₂-CH₂Br, and
wherein R¹ or R² is different from hydrogen;
R³ represents -H, -CH₃, -C₂H₅, -C₃H₇, cyclo-C₃H₅, -CH(CH₃)₂ or -C(CH₃)₃;
R^{N} represents -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -C₆H₁₃, -Ph, -L-Ph, -L- cyclo-C₅H₉, -L-R³, -L-R* cyclo-C₃H₅, cyclo-C₄H₇, cyclo-C₅H₉, cyclo-C₆H₁₁, cyclo-C₇H₁₃, 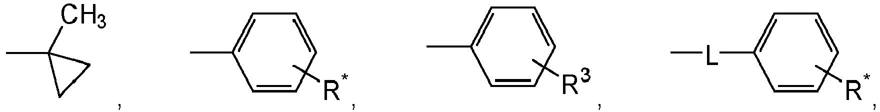 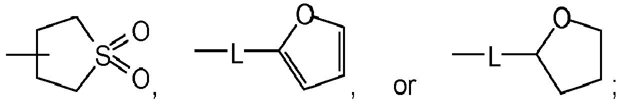
R^{S} represents -CH₃, -C₂H₅, -C₃H₇, cyclo-C₃H₅, -CH(CH₃)₂, -CH₂-CN, -CH₂-Ar, -CH(NH₂)-Ar,
Ar represents -Ph, 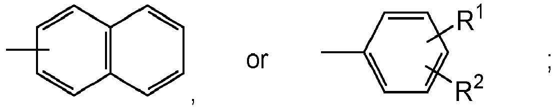
R* represents -OCH₃, -OC₂H₅, -OC₃H₇, -O-cyclo-C₃H₅, -OCH(CH₃)₂, -OC(CH₃)₃, -OC₄H₉, -COCH₃, -COC₂H₅, -COC₃H₇, -CO-cyclo-C₃H₅, -COCH(CH₃)₂, or -COC(CH₃)₃;
L represents -CH₂-, -C₂H₄-, -C₃H₆-, or -CH₂-CO-O-;
X represents -O⁻, -OCH₃, -OC₂H₅, or -OC₃H₇;
and salts especially agriculturally acceptable salts thereof.

2. The compound for use or the combination of compounds for use according to claim 1, wherein the compound(s) is/are selected from the general formula (I) - (III) as follows: 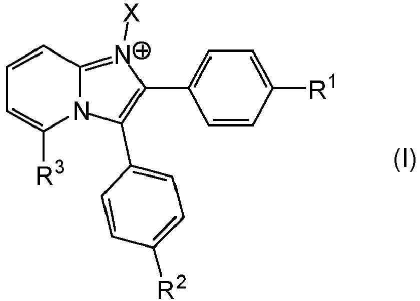 wherein
R¹ and R² represent independently of each other -H, -OCH₃, -OC₂H₅, -NO₂, -F, -Cl, -Br, -N₃, -CN, -OCN, -NCO, -SCN, -NCS, -CHO, -COCH₃, -COC₂H₅, -COOH, -COCN, -COOCH₃, -COOC₂H₅, -OOC-CH₃, -OOC-C₂H₅, -CONH₂, -CONHCH₃, -CONHC₂H₅, -CON(CH₃)₂, -CON(C₂H₅)₂, -NHCOCH₃, -NHCOC₂H₅, -NH₂, -NHCH₃, -NHC₂H₅, -N(CH₃)₂, -N(C₂H₅)₂, -SOCH₃, -SOC₂H₅, -SO₂CH₃, -SO₂C₂H₅, -SO₃H, -SO₃CH₃, -SO₃C₂H₅, -SO₂NH₂, -SO₂NHCH₃, -SO₂NHC₂H₅, -SO₂N(CH₃)₂, -SO₂N(C₂H₅)₂, -O-S(=O)CH₃, -O-S(=O)C₂H₅, -NH-SO₂-CH₃, -NH-SO₂-C₂H₅, -O-SO₂-CH₃, -O-SO₂-C₂H₅, -OCF₃, -CH₂OCF₃, -OC₂F₅, -O-COOCH₃, -O-COOC₂H₅, -NH-CO-NH₂, -NH-CS-NH₂, -NH-C(=NH)-NH₂, -O-CO-NH₂, -O-CO-OCH₃, -O-CO-OC₂H₅, -CH₃, -C₂H₅, -C₃H₇, -CH(CH₃)₂, -CH₂F, -CHF₂, -CF₃, -CH₂Cl, -CH₂Br, and wherein R¹ or R² is different from hydrogen;
R³ represents -H or -CH₃;
X represents -O⁻ or -OCH₃;
and salts especially agriculturally acceptable salts thereof; 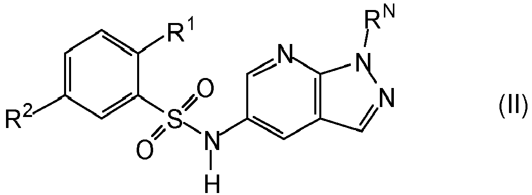
wherein
R¹ and R² represent independently of each other -H, -OCH₃, -OC₂H₅, -NO₂, -F, -Cl, -Br, -N₃, -CN, -OCN, -NCO, -SCN, -NCS, -CHO, -COCH₃, -COC₂H₅, -COOH, -COCN, -COOCH₃, -COOC₂H₅, -OOC-CH₃, -OOC-C₂H₅, -CONH₂, -CONHCH₃, -CONHC₂H₅, -CON(CH₃)₂, -CON(C₂H₅)₂, -NHCOCH₃, -NHCOC₂H₅, -NH₂, -NHCH₃, -NHC₂H₅, -N(CH₃)₂, -N(C₂H₅)₂, -SOCH₃, -SOC₂H₅, -SO₂CH₃, -SO₂C₂H₅, -SO₃H, -SO₃CH₃, -SO₃C₂H₅, -SO₂NH₂, -SO₂NHCH₃, -SO₂NHC₂H₅, -SO₂N(CH₃)₂, -SO₂N(C₂H₅)₂, -O-S(=O)CH₃, -O-S(=O)C₂H₅, -NH-SO₂-CH₃, -NH-SO₂-C₂H₅, -O-SO₂-CH₃, -O-SO₂-C₂H₅, -OCF₃, -CH₂-OCF₃, -OC₂F₅, -O-COOCH₃, -O-COOC₂H₅, -NH-CO-NH₂, -NH-CS-NH₂, -NH-C(=NH)-NH₂, -O-CO-NH₂, -O-CO-OCH₃, -O-CO-OC₂H₅, -CH₃, -C₂H₅, -C₃H₇, -CH(CH₃)₂, -CH₂F, -CHF₂, -CF₃, -CH₂Cl, -CH₂Br, and wherein R¹ or R² is different from hydrogen;
R^{N} represents -CH₃, -C₂H₅, -C₃H₇, -CH(CH₃)₂, -C₄H₉, -CH₂-CH(CH₃)₂, -C(CH₃)₃, -C₅H₁₁, -Ph, -L-Ph, -L-R³, cyclo-C₃H₅, cyclo-C₄H₇, cyclo-C₅H₉, cyclo-C₆H₁₁, cyclo-C₇H₁₃;
L represents -CH₂-, -C₂H₄-, or -CH₂-CO-O-;
R³ represents -H, -CH₃, -C₂H₅, -C₃H₇, cyclo-C₃H₅, -CH(CH₃)₂ or -C(CH₃)₃;
and salts especially agriculturally acceptable salts thereof; 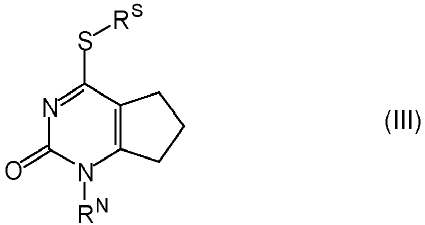 wherein
R^{N} represents -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -C₆H₁₃, -Ph, -L-Ph, -L- cyclo-C₅H₉, -L-R³, -L-R* cyclo-C₃H₅, cyclo-C₄H₇, cyclo-C₅H₉, cyclo-C₆H₁₁, cyclo-C₇H₁₃, 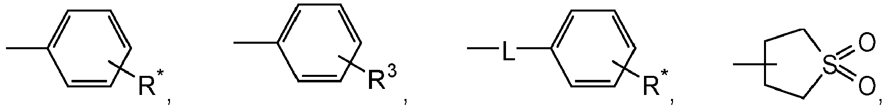 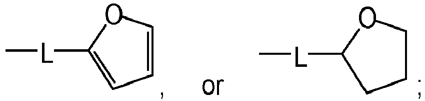
R^{S} represents -CH₃, -C₂H₅, -C₃H₇, cyclo-C₃H₅, -CH(CH₃)₂, -CH₂-CN, -CH₂-Ar, -CH(NH₂)-Ar,
Ar represents -Ph, 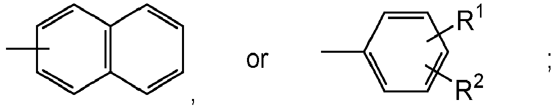
R* represents -OCH₃, -OC₂H₅, -OC₃H₇, -O-cyclo-C₃H₅, -OCH(CH₃)₂, -COCH₃, -COC₂H₅, -COC₃H₇, -CO-cyclo-C₃H₅, or -COCH(CH₃)₂;
R¹ and R² represent independently of each other -H, -OCH₃, -OC₂H₅, -NO₂, -F, -Cl, -Br, -N₃, -CN, -OCN, -NCO, -SCN, -NCS, -CHO, -COCH₃, -COC₂H₅, -COOH, -COCN, -COOCH₃, -COOC₂H₅, -OOC-CH₃, -OOC-C₂H₅, -CONH₂, -CONHCH₃, -CONHC₂H₅, -CON(CH₃)₂, -CON(C₂H₅)₂, -NHCOCH₃, -NHCOC₂H₅, -NH₂, -NHCH₃, -NHC₂H₅, -N(CH₃)₂, -N(C₂H₅)₂, -SOCH₃, -SOC₂H₅, -SO₂CH₃, -SO₂C₂H₅, -SO₃H, -SO₃CH₃, -SO₃C₂H₅, -SO₂NH₂, -SO₂NHCH₃, -SO₂NHC₂H₅, -SO₂N(CH₃)₂, -SO₂N(C₂H₅)₂, -O-S(=O)CH₃, -O-S(=O)C₂H₅, -NH-SO₂-CH₃, -NH-SO₂-C₂H₅, -O-SO₂-CH₃, -O-SO₂-C₂H₅, -OCF₃, -CH₂-OCF₃, -OC₂F₅, -O-COOCH₃, -O-COOC₂H₅, -NH-CO-NH₂, -NH-CS-NH₂, -NH-C(=NH)-NH₂, -O-CO-NH₂, -O-CO-OCH₃, -O-CO-OC₂H₅, -CH₃, -C₂H₅, -C₃H₇, -CH(CH₃)₂, -CH₂F, -CHF₂, -CF₃, -CH₂Cl, -CH₂Br, and wherein R¹ or R² is different from hydrogen;
R³ represents -H, -CH₃, or -C₂H₅;
L represents -CH₂-, -C₂H₄-, or -C₃H₆-;
and salts especially agriculturally acceptable salts thereof.

3. The compound for use or the combination of compounds for use according to claim 1, wherein the compound(s) is/are selected from: 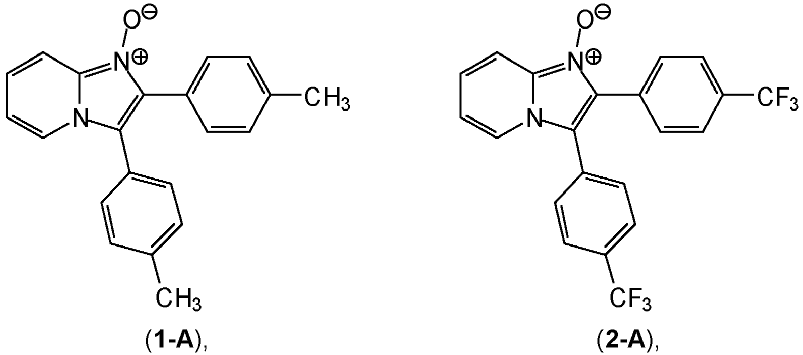 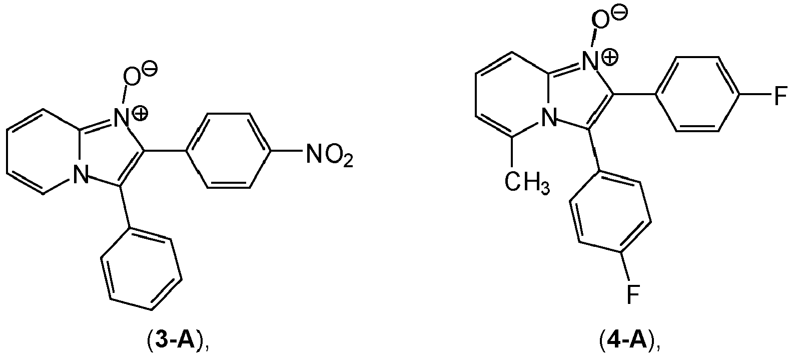 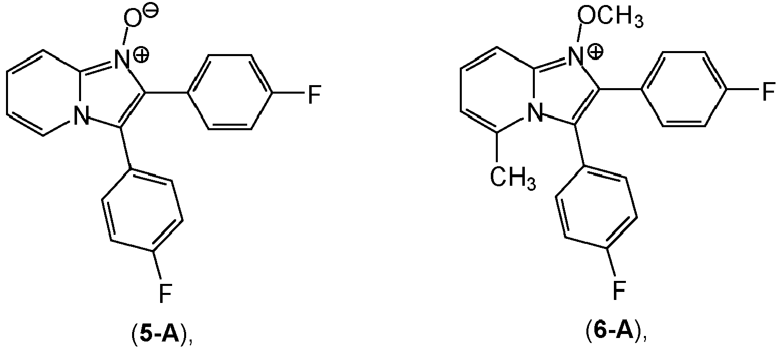 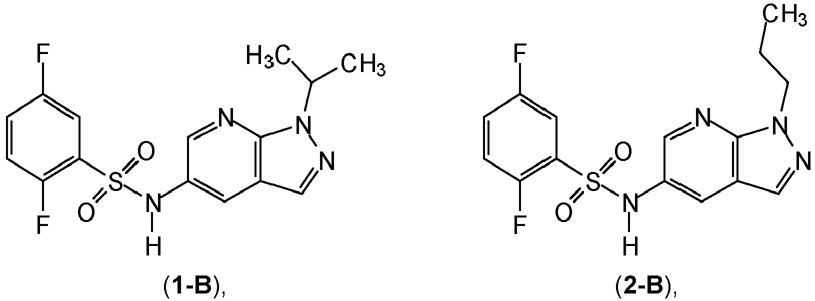 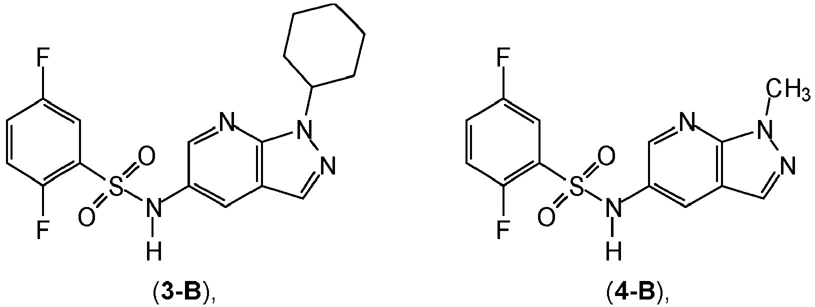 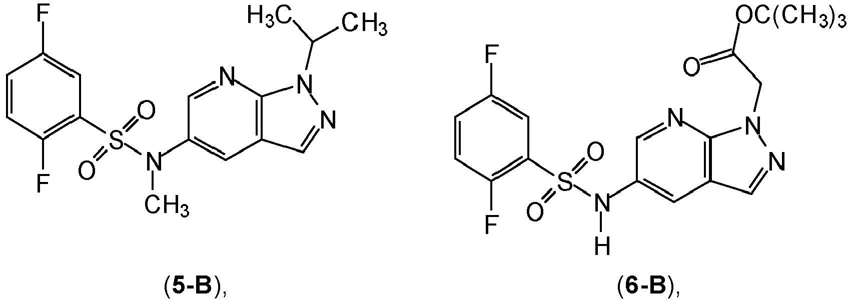 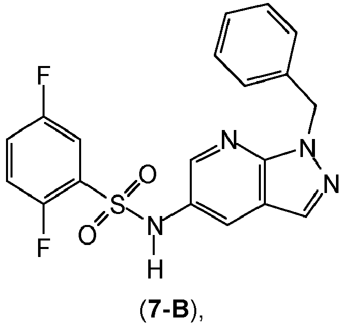 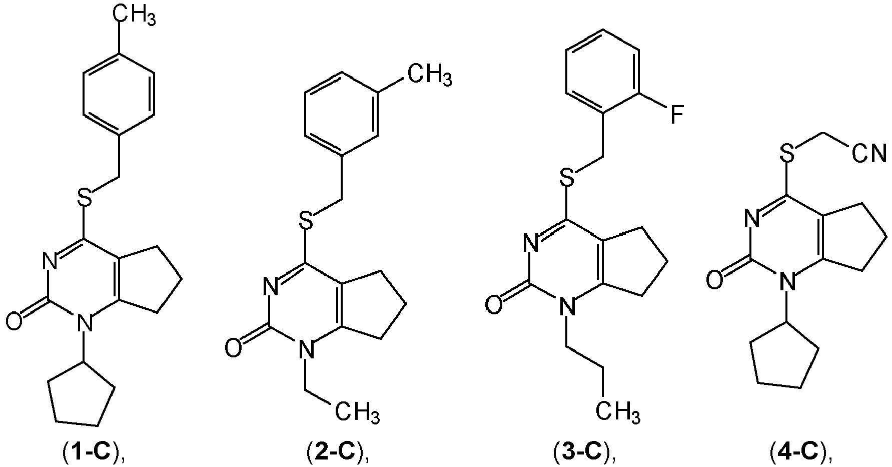 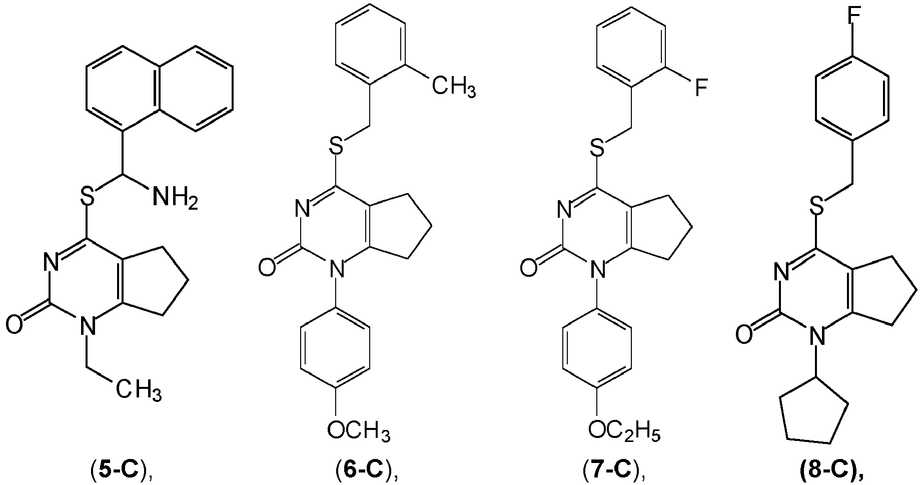 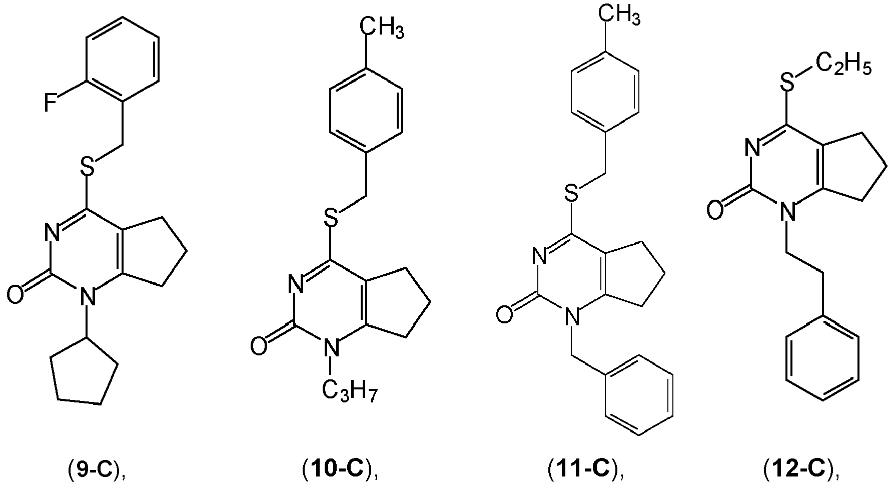 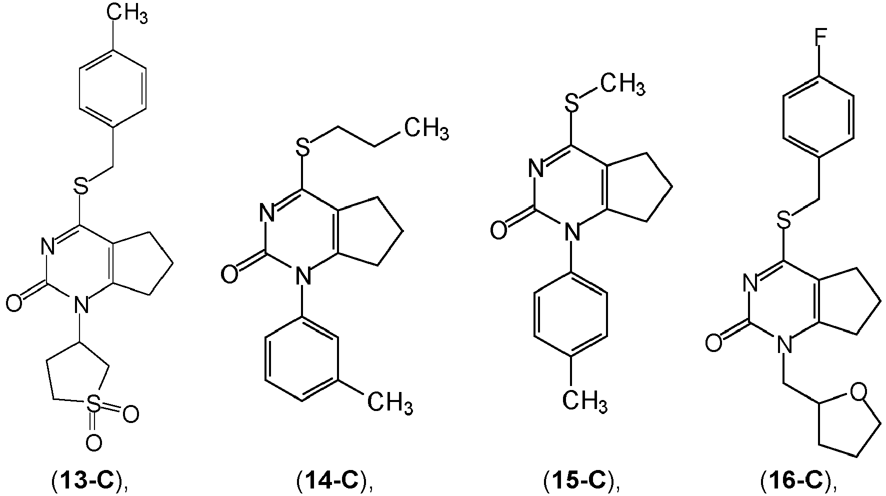 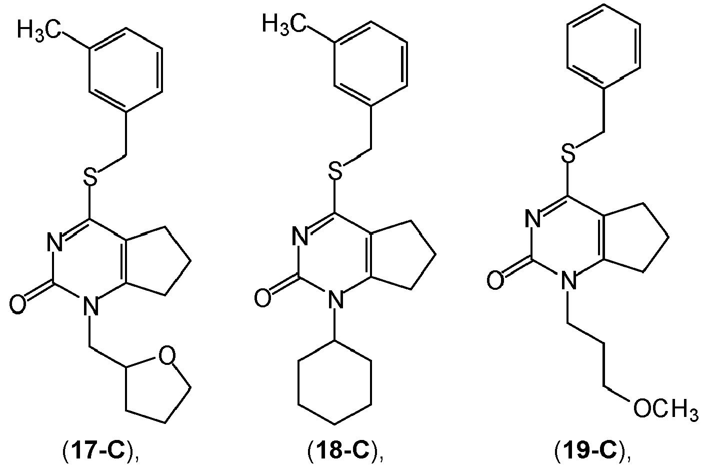 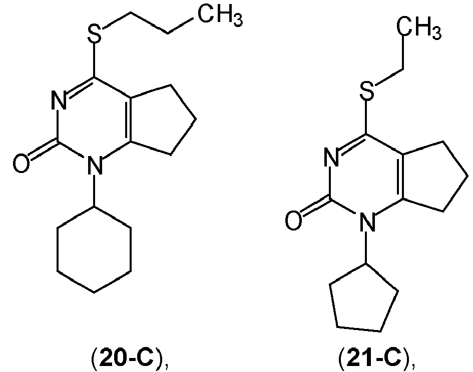 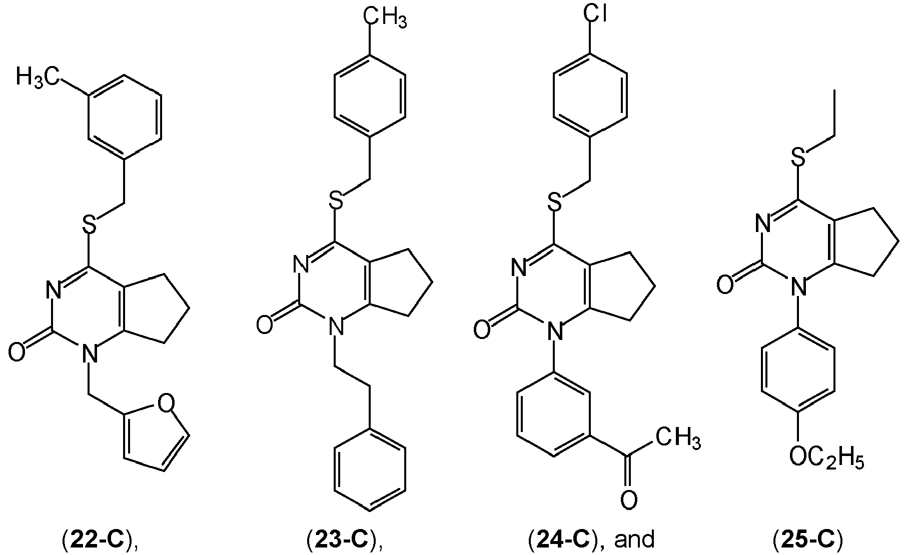 and salts especially agriculturally acceptable salts thereof.

4. The compound for use or the combination of compounds for use according to any of the claims 1 - 3, wherein the one or more plant pathogenic basidiomycete fungi are selected from the group consisting of smut fungi and rust fungi, wherein the smut fungi are *Ustilago hordei, Ustilago avenae, Ustilago nuda, Ustilago nigra, Ustilago tritici, Ustilago maydis, Ustilago scitaminea, Ustilago esculenta, Ustilago striiformis, Ustilago trichophora, Sporisorium reilianum, Sporisorium reilianum f.sp.* zeae, *Sporisorium reilianum f.sp. reilianum, Sporisorium scitamineum, Angiosorus solani, Urocystis agropyri, Urocystis occulta, Tilletia indica, Tilletia caries, Tilletia barclayana* or *Tilletia controversa,* or wherein the rust fungi is *Uromyces fabae, Uromyces appendiculatus, Uromyces beticola, Puccinia graminis f. sp. tritici, Puccinia striiformis* f. sp. *tritici, Puccinia triticina, Puccinia graminis f. sp. secalis, Puccinia arachidis, Puccinia coronata f. sp. avenae, Puccinia sorghi, Puccinia melanocephala, Puccinia kuehnii, Puccinia recondita, Puccinia pitteriana, Puccinia graminis, Puccinia polysora,Phakopsora pachyrhizi, Phakopsora meibomiae, Hemileia vastatrix, Melampsora-larici-populina, Melampsora lini, Cronartium ribicola, Cronartium quercuum f. sp. fusiforme, Gymnosporangium sabinae, Gymnosporangium yamadae, Gymnosporangium juniperi-virginianae.*

5. The compound for use or the combination of compounds for use according to any of the claims 1 - 3, wherein the one or more plant pathogenic basidiomycete fungi are selected from the group consisting of smut fungi and rust fungi, wherein the smut fungi are *Ustilago maydis, Ustilago esculenta, Ustilago trichophora, Ustilago tritici, Sporisorium reilianum f.sp.* zeae, *Sporisorium reilianum f.sp. reilianum, Sporisorium scitamineum, Ustilago hordei,* or wherein the rust fungi is *Uromyces fabae.*

6. A fungicidal composition comprising a compound for use or a combination of compounds for use in treating, preventing, inhibiting, or eliminating the virulence of one or more plant pathogenic basidiomycete fungi in a plant or a plant material, wherein the compound(s) is/are selected from the general formula (I) - (III) as defined in claim 1;
wherein the fungicidal composition further comprises at least one agriculturally acceptable carrier, excipient, solvent and/or adjuvant.

7. The fungicidal composition comprising a compound for use or a combination of compounds for use in treating, preventing, inhibiting, or eliminating the virulence of one or more plant pathogenic basidiomycete fungi in a plant or a plant material according to claim 6, wherein the compound(s) is/are selected from the general formula (I) - (III) as defined in claim 2.

8. The fungicidal composition comprising a compound for use or a combination of compounds for use in treating, preventing, inhibiting, or eliminating the virulence of one or more plant pathogenic basidiomycete fungi in a plant or a plant material according to claim 6, wherein the compound(s) is/are selected from the list as defined in claim 3.

9. The fungicidal composition comprising a compound for use or a combination of compounds for use in treating, preventing, inhibiting, or eliminating the virulence of one or more plant pathogenic basidiomycete fungi in a plant or a plant material according to any of the claims 6 - 8, wherein the one or more plant pathogenic basidiomycete fungi are selected from the group consisting of smut fungi and rust fungi, wherein the smut fungi are *Ustilago hordei, Ustilago avenae, Ustilago nuda, Ustilago nigra, Ustilago tritici, Ustilago maydis, Ustilago scitaminea, Ustilago esculenta, Ustilago striiformis, Ustilago trichophora, Sporisorium reilianum, Sporisorium reilianum f.sp.* zeae, *Sporisorium reilianum f.sp. reilianum, Sporisorium scitamineum, Angiosorus solani, Urocystis agropyri, Urocystis occulta, Tilletia indica, Tilletia caries, Tilletia barclayana* or *Tilletia controversa,* or wherein the rust fungi is *Uromyces fabae, Uromyces appendiculatus, Uromyces beticola, Puccinia graminis f. sp. tritici, Puccinia striiformis* f. sp. *tritici, Puccinia triticina, Puccinia graminis f. sp. secalis, Puccinia arachidis, Puccinia coronata f. sp. avenae, Puccinia sorghi, Puccinia melanocephala, Puccinia kuehnii, Puccinia recondita, Puccinia pitteriana, Puccinia graminis, Puccinia polysora,Phakopsora pachyrhizi, Phakopsora meibomiae, Hemileia vastatrix, Melampsora-larici-populina, Melampsora lini, Cronartium ribicola, Cronartium quercuum f. sp. fusiforme, Gymnosporangium sabinae, Gymnosporangium yamadae, Gymnosporangium juniperi-virginianae.*

10. The fungicidal composition comprising a compound for use or a combination of compounds for use in treating, preventing, inhibiting, or eliminating the virulence of one or more plant pathogenic basidiomycete fungi in a plant or a plant material according to any of the claims 6 - 8, wherein the one or more plant pathogenic basidiomycete fungi are selected from the group consisting of smut fungi and rust fungi, wherein the smut fungi are *Ustilago maydis, Ustilago esculenta, Ustilago trichophora, Ustilago tritici, Sporisorium reilianum f.sp.* zeae, *Sporisorium reilianum f.sp. reilianum, Sporisorium scitamineum, Ustilago hordei,* or wherein the rust fungi is *Uromyces fabae.*

11. A method of treating, preventing, inhibiting, or eliminating virulence of one or more plant pathogenic basidiomycete fungi in a plant or a plant material comprising the step of applying onto a plant, to a plant material or in the vicinity of said plant or plant material a compound or a combination of compounds or a fungicidal composition comprising the same, wherein the compound(s) is/are selected from the general formula (I) - (III) as defined in claim 1.

12. The method of treating, preventing, inhibiting, or eliminating virulence of one or more plant pathogenic basidiomycete fungi in a plant or a plant material according to claim 11, wherein the compound(s) is/are selected from the general formula (I) - (III) as defined in claim 2.

13. The method of treating, preventing, inhibiting, or eliminating the virulence of one or more plant pathogenic basidiomycete fungi in a plant or a plant material according to claim 11, wherein the compound(s) is/are selected from the list as defined in claim 3.

14. The method of treating, preventing, inhibiting, or eliminating the virulence of one or more plant pathogenic basidiomycete fungi in a plant or a plant material according to any of the claims 11 - 13, wherein the one or more plant pathogenic basidiomycete fungi are selected from the group consisting of smut fungi and rust fungi, wherein the smut fungi are *Ustilago hordei, Ustilago avenae, Ustilago nuda, Ustilago nigra, Ustilago tritici, Ustilago maydis, Ustilago scitaminea, Ustilago esculenta, Ustilago striiformis, Ustilago trichophora, Sporisorium reilianum, Sporisorium reilianum f.sp.* zeae, *Sporisorium reilianum f.sp. reilianum, Sporisorium scitamineum, Angiosorus solani, Urocystis agropyri, Urocystis occulta, Tilletia indica, Tilletia caries, Tilletia barclayana* or *Tilletia controversa,* or wherein the rust fungi is *Uromyces fabae, Uromyces appendiculatus, Uromyces beticola, Puccinia graminis f. sp. tritici, Puccinia striiformis* f. sp. *tritici, Puccinia triticina, Puccinia graminis f. sp. secalis, Puccinia arachidis, Puccinia coronata f. sp. avenae, Puccinia sorghi, Puccinia melanocephala, Puccinia kuehnii, Puccinia recondita, Puccinia pitteriana, Puccinia graminis, Puccinia polysora,Phakopsora pachyrhizi, Phakopsora meibomiae, Hemileia vastatrix, Melampsora-larici-populina, Melampsora lini, Cronartium ribicola, Cronartium quercuum f. sp. fusiforme, Gymnosporangium sabinae, Gymnosporangium yamadae, Gymnosporangium juniperi-virginianae.*

15. The method of treating, preventing, inhibiting, or eliminating the virulence of one or more plant pathogenic basidiomycete fungi in a plant or a plant material according to any of the claims 11 - 13, wherein the one or more plant pathogenic basidiomycete fungi are selected from the group consisting of smut fungi and rust fungi, wherein the smut fungi are *Ustilago maydis, Ustilago esculenta, Ustilago trichophora, Ustilago tritici, Sporisorium reilianum f.sp.* zeae, *Sporisorium reilianum f.sp. reilianum, Sporisorium scitamineum, Ustilago hordei,* or wherein the rust fungi is *Uromyces fabae.*
